(19) Europäisches Patentamt
European Patent Office
Office européen des brevets

(11) EP 3 793 613 B1

(12) EUROPEAN PATENT SPECIFICATION

(45) Date of publication and mention of the grant of the patent:
**01.01.2025 Bulletin 2025/01**

(21) Application number: **19728294.0**

(22) Date of filing: **17.05.2019**

(51) International Patent Classification (IPC):
***A61K 47/68*** *(2017.01)* ***A61P 35/00*** *(2006.01)*
*C07K 16/32* *(2006.01)*

(52) Cooperative Patent Classification (CPC):
**A61K 47/6889; A61K 47/6803; A61K 47/6849; A61K 47/6851; A61K 47/6855; A61P 35/00;**
C07K 16/32; C07K 2317/52

(86) International application number:
**PCT/US2019/032947**

(87) International publication number:
**WO 2019/222676 (21.11.2019 Gazette 2019/47)**

# (54) IMMUNOCONJUGATES

IMMUNOKONJUGATE

IMMUNOCONJUGUÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.05.2018 US 201862673015 P**
**29.08.2018 US 201862724259 P**

(43) Date of publication of application:
**24.03.2021 Bulletin 2021/12**

(73) Proprietors:
- **Bolt Biotherapeutics, Inc.**
  **Redwood City, CA 94063 (US)**
- **The Board of Trustees of the Leland Stanford Junior University**
  **Stanford, CA 94305-2038 (US)**

(72) Inventors:
- **ALONSO, Michael N.**
  **Santa Clara, California 95051 (US)**
- **JACKSON, David Y.**
  **Belmont, California 94002 (US)**
- **SAFINA, Brian**
  **Belmont, California 94002 (US)**
- **ACKERMAN, Shelley Erin**
  **Mountain View, California 94040 (US)**
- **ENGLEMAN, Edgar George**
  **Atherton, CA 94027 (US)**

(74) Representative: **Maiwald GmbH**
**Elisenhof**
**Elisenstraße 3**
**80335 München (DE)**

(56) References cited:
**WO-A1-2018/009916** **WO-A2-2007/100634**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 793 613 B1

## Description

A computer-readable nucleotide/amino acid sequence listing submitted concurrently herewith is identified as follows: One 102,498 Byte ASCII (Text) file named "743565_ST25.TXT," created on May 17, 2019.

BACKGROUND OF THE INVENTION

It is now well appreciated that tumor growth necessitates the acquisition of mutations that facilitate immune evasion. Even so, tumorigenesis results in the accumulation of mutated antigens, or neoantigens, that are readily recognized by the host immune system following *ex vivo* stimulation. Why and how the immune system fails to recognize neoantigens are beginning to be elucidated. Groundbreaking studies by Carmi et al. (Nature, 521: 99-104 (2015)) have indicated that immune ignorance can be overcome by delivering neoantigens to activated dendritic cells via antibody-tumor immune complexes. In these studies, simultaneous delivery of tumor binding antibodies and dendritic cell adjuvants via intratumoral injections resulted in robust anti-tumor immunity. New compositions and methods for the delivery of antibodies and dendritic cell adjuvants are needed in order to reach inaccessible tumors and to expand treatment options for cancer patients and other subjects. The invention addresses this and other needs.

WO 2018/009916 A1 discloses immunoconjugates comprising an antibody construct which includes an antigen binding domain and an Fc domain, an adjuvant moiety, and a linker, wherein each adjuvant moiety is covalently bonded to the antibody via the linker. WO 2007/100634 A2 discloses immune response modifier (IRM) conjugates that includes an IRM moiety and a second active moiety covalently linked to the IRM moiety in which the covalent link does not depend on UV irradiation.

BRIEF SUMMARY OF THE INVENTION

In a first aspect, the invention provides an immunoconjugate comprising (a) an antibody construct comprising (i) an antigen binding domain and (ii) an Fc domain and (b) 1-8 adjuvant cores, wherein each adjuvant core is covalently bonded to the antibody construct via a linker, wherein each adjuvant core comprises a 2-amino nitrogen moiety with a pendant nitrogen atom and a point of attachment of the linker to the adjuvant core, and wherein the distance between the pendant nitrogen atom and the point of attachment of the linker is greater than about 5 Å,

the immunoconjugate is of formula:

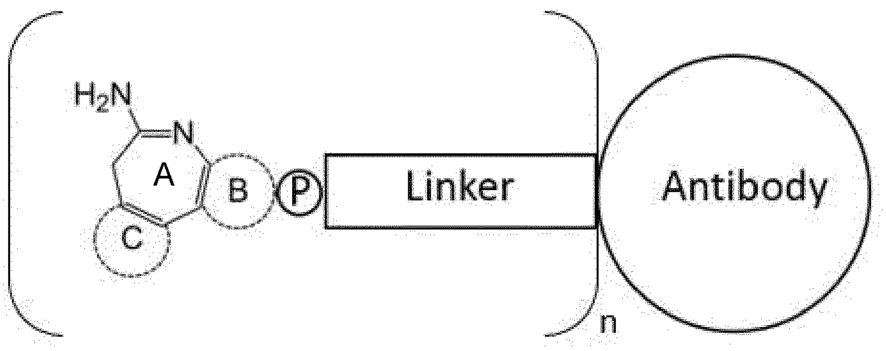

Immunoconjugate D2,

wherein the adjuvant core is represented by fused rings A, B and C, wherein A and B are present and C is optionally present, and B and C denote 5-, 6-, or 7-membered rings, optionally comprising double bonds, and optionally comprising heteroatoms in addition to the 2-amino nitrogen moiety, and the adjuvant core is optionally substituted such that the substitution pattern around the adjuvant core is not particularly limited, as long as the 2-amino nitrogen moiety remains unsubstituted, P represents the point of attachment of the linker to the B ring, and n is an integer from 1 to 8, or

the immunoconjugate is of formula:

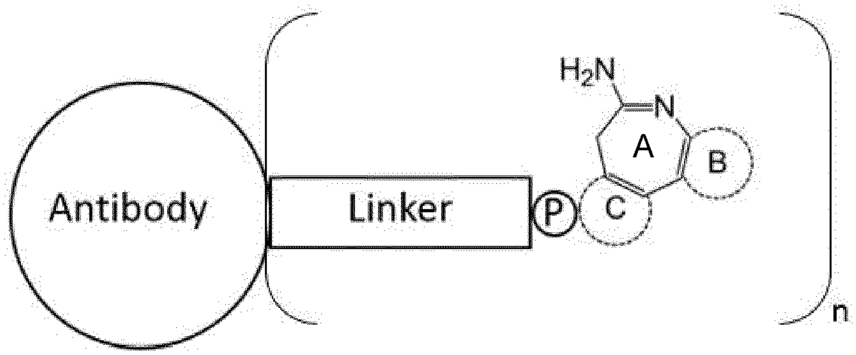

Immunoconjugate D4,

wherein the adjuvant core is represented by fused rings A, B and C, wherein A and C are present and B is optionally present, and B and C denote 5-, 6-, or 7-membered rings, optionally comprising double bonds, and optionally comprising heteroatoms in addition to the 2-amino nitrogen moiety, and the adjuvant core is optionally substituted such that the substitution pattern around the adjuvant core is not particularly limited, as long as the 2-amino nitrogen moiety remains unsubstituted, P represents the point of attachment of the linker to the C ring, and n is an integer from 1 to 8..

In a further aspect, the invention provides a composition comprising a plurality of immunoconjugates of the invention.

In another aspect, the invention provides an immunconjugate of the invention or a composition of the invention for use in treating cancer.

BRIEF DESCRIPTION OF THE DRAWINGS

FIG. 1 is a depiction of an exemplary adjuvant moiety in relation to a binding domain of a TLR, where A, B, and C represent the adjuvant core, "HBA" is a hydrogen bond-accepting amino acid within the binding domain of the TLR, "HP" is a hydrogen pocket within the binding domain of the TLR, "$R_H$" is a hydrophobic substituent on the adjuvant core, "P" is a point of attachment of a linker to the adjuvant core, " " denotes the distance from the 2-amino nitrogen moiety to the hydrophobic substituent, and " " denotes the distance from the 2-amino nitrogen moiety to the point of attachment of the linker.

FIG. 2A is a graph showing the effect of Immunoconjugate A on myeloid activation in myeloid APC-tumor co-cultures, using the HCC1954 human ductal carcinoma tumor cell line. Median fluorescence intensity of co-stimulatory molecule CD40 (cells gated on viable CD45+CD11c+HLA-DR+) was measured by flow cytometry and is shown for trastuzumab (dotted line, circle), trastuzumab + Compound 7 (dashed line, triangle), and Immunoconjugate A (solid line, square).

FIG. 2B is a graph showing the effect of Immunoconjugate A on myeloid activation in myeloid APC-tumor co-cultures, using the HCC1954 human ductal carcinoma tumor cell line. Median fluorescence intensity of co-stimulatory molecule CD86 (cells gated on viable CD45+CD11c+HLA-DR+) was measured by flow cytometry and is shown for trastuzumab (dotted line, circle), trastuzumab + Compound 7 (dashed line, triangle), and Immunoconjugate A (solid line, square).

FIG. 2C is a graph showing the effect of Immunoconjugate A on myeloid activation in myeloid APC-tumor co-cultures, using the HCC1954 human ductal carcinoma tumor cell. TNF$\alpha$ secretion was measured by cytokine bead array (cells gated on viable CD45+CD11c+HLA-DR+) for trastuzumab (dotted line, circle), trastuzumab + Compound 7 (dashed line, triangle), and Immunoconjugate A (solid line, square).

FIG. 2D is a graph showing the effect of Immunoconjugate A on myeloid activation in myeloid APC-tumor co-cultures, using the JIMT-1 human ductal carcinoma tumor cell line. Median fluorescence intensity of co-stimulatory molecule CD40 (cells gated on viable CD45+CD11c+HLA-DR+) was measured by flow cytometry and is shown for trastuzumab (dotted line, circle), trastuzumab + Compound 7 (dashed line, triangle), and Immunoconjugate A (solid line, square).

FIG. 2E is a graph showing the effect of Immunoconjugate A on myeloid activation in myeloid APC-tumor co-cultures, using the JIMT-1 human ductal carcinoma tumor cell line. Median fluorescence intensity of co-stimulatory molecule CD86 (cells gated on viable CD45+CD11c+HLA-DR+) was measured by flow cytometry and is shown for trastuzumab (dotted line, circle), trastuzumab + Compound 7 (dashed line, triangle), and Immunoconjugate A (solid line, square).

FIG. 2F is a graph showing the effect of Immunoconjugate A on myeloid activation in myeloid APC-tumor co-cultures,

using the JIMT-1 human ductal carcinoma tumor cell. TNFα secretion was measured by cytokine bead array (cells gated on viable CD45+CD11c+HLA-DR+) for trastuzumab (dotted line, circle), trastuzumab + Compound 7 (dashed line, triangle), and Immunoconjugate A (solid line, square).

FIG. 2G is a graph showing the effect of Immunoconjugate A on myeloid activation in myeloid APC-tumor co-cultures, using the COLO 205 human colon adenocarcinoma cell line. Median fluorescence intensity of co-stimulatory molecule CD40 (cells gated on viable CD45+CD11c+HLA-DR+) was measured by flow cytometry and is shown for trastuzumab (dotted line, circle), trastuzumab + Compound 7 (dashed line, triangle), and Immunoconjugate A (solid line, square).

FIG. 2H is a graph showing the effect of Immunoconjugate A on myeloid activation in myeloid APC-tumor co-cultures, using the COLO 205 human colon adenocarcinoma cell line. Median fluorescence intensity of co-stimulatory molecule CD86 (cells gated on viable CD45+CD11c+HLA-DR+) was measured by flow cytometry and is shown for trastuzumab (dotted line, circle), trastuzumab + Compound 7 (dashed line, triangle), and Immunoconjugate A (solid line, square).

FIG. 2I is a graph showing the effect of Immunoconjugate A on myeloid activation in myeloid APC-tumor co-cultures, using the COLO 205 human colon adenocarcinoma cell line. TNFα secretion was measured by cytokine bead array (cells gated on viable CD45+CD1 1c+HLA-DR+) for trastuzumab (dotted line, circle), trastuzumab + Compound 7 (dashed line, triangle), and Immunoconjugate A (solid line, square).

FIG. 3A is a graph showing that Immunoconjugate B elicits myeloid differentiation as indicated by CD14 downregulation.

FIG. 3B is a graph showing that Immunoconjugate B elicits myeloid activation as indicated by CD40 upregulation.

FIG. 3C is a graph showing that Immunoconjugate B elicits myeloid activation as indicated by CD86 upregulation.

FIG. 3D is a graph showing TNFα secretion from myeloid cells following an 18 hour incubation with Immunoconjugate B.

FIG. 4A is a graph showing that Immunoconjugate C elicits myeloid differentiation as indicated by CD14 downregulation.

FIG. 4B is a graph showing that Immunoconjugate C elicits myeloid activation as indicated by CD40 upregulation.

FIG. 4C is a graph showing that Immunoconjugate C elicits myeloid activation as indicated by CD86 upregulation.

FIG. 4D is a graph showing TNFα secretion from myeloid cells following an 18 hour incubation with Immunoconjugate C.

FIG. 5A is a graph showing that Immunoconjugate D elicits myeloid differentiation as indicated by CD14 downregulation.

FIG. 5B is a graph showing that Immunoconjugate D elicits myeloid activation as indicated by CD40 upregulation.

FIG. 5C is a graph showing that Immunoconjugate D elicits myeloid activation as indicated by CD86 upregulation.

FIG. 5D is a graph showing TNFα secretion from myeloid cells following an 18 hour incubation with Immunoconjugate D.

FIG. 6A is a graph showing that Immunoconjugate E elicits myeloid differentiation as indicated by CD14 downregulation.

FIG. 6B is a graph showing that Immunoconjugate E elicits myeloid activation as indicated by CD40 upregulation.

FIG. 6C is a graph showing that Immunoconjugate E elicits myeloid activation as indicated by CD86 upregulation.

FIG. 6D is a graph showing TNFα secretion from myeloid cells following an 18 hour incubation with Immunoconjugate E.

FIG. 6E is a graph showing TNFα secretion from myeloid cells following an 18 hour incubation with Immunoconjugate E.

FIG. 7A is a graph showing that Immunoconjugate F elicits myeloid differentiation as indicated by CD14 downregulation.

FIG. 7B is a graph showing that Immunoconjugate F elicits myeloid activation as indicated by CD40 upregulation.

FIG. 7C is a graph showing that Immunoconjugate F elicits myeloid activation as indicated by CD86 upregulation.

FIG. 7D is a graph showing TNFα secretion from myeloid cells following an 18 hour incubation with Immunoconjugate F.

FIG. 8A is a graph showing that Immunoconjugate G elicits myeloid differentiation as indicated by CD14 downregulation.

FIG. 8B is a graph showing that Immunoconjugate G elicits myeloid activation as indicated by CD40 upregulation.

FIG. 8C is a graph showing that Immunoconjugate G elicits myeloid activation as indicated by CD86 upregulation.

FIG. 8D is a graph showing TNFα secretion from myeloid cells following an 18 hour incubation with Immunoconjugate G.

FIG. 9 is a graph showing TNFα secretion from myeloid cells following an 18 hour incubation with Immunoconjugate H.

FIG. 10A is a graph showing that Immunoconjugate I elicits myeloid differentiation as indicated by CD14 downregulation.

FIG. 10B is a graph showing that Immunoconjugate I elicits myeloid activation as indicated by CD40 upregulation.
FIG. 10C is a graph showing that Immunoconjugate I elicits myeloid activation as indicated by CD86 upregulation.
FIG. 10D is a graph showing TNFα secretion from myeloid cells following an 18 hour incubation with Immunoconjugate I.
FIG. 11A is a graph showing that Immunoconjugate J elicits myeloid activation as indicated by CD40 upregulation.
FIG. 11B is a graph showing that Immunoconjugate J elicits myeloid activation as indicated by CD86 upregulation.
FIG. 11C is a graph showing that Immunoconjugate J elicits myeloid activation as indicated by CD123 upregulation.
FIG. 12 is a set of two graphs illustrating the importance of the pendant nitrogen of the 2-amino nitrogen moiety for maintaining activity of an adjuvant, as evidenced by HEK293 reporter cells expressing human TLR7 and human TLR8.
FIG. 13 is a set of two graphs illustrating the importance of the pendant nitrogen of the 2-amino nitrogen moiety for maintaining activity of an immunoconjugate, as measured by upregulation of costimulatory molecules CD40 and CD86.
FIG. 14 is a set of three graphs illustrating the importance of the pendant nitrogen of the 2-amino nitrogen moiety for inducing dendritic cell differentiation, as measured by CD14, CD16, and CD123 expression.

## DETAILED DESCRIPTION OF THE INVENTION

## GENERAL

Antibody-adjuvant immunoconjugates which are covalently attached, i.e., wherein the antibody is covalently bonded to the linker which is covalently bonded to the adjuvant, are quantitatively and qualitatively more effective at eliciting immune activation than non-covalently attached antibody-adjuvant immunoconjugates. Further, antibody-adjuvant immunoconjugates linked according to the invention are much more effective than other known immunoconjugates. Systemic administration of the adjuvant-antibody conjugates allows for the simultaneous targeting of the primary tumor and associated metastases without the need for intra-tumoral injections and surgical resection.

The effectiveness of the immunoconjugates described herein can be considered in terms of the adjuvant moiety's ability to bind to its receptor. Immunoconjugates of the invention have increased adjuvant activity due to one or more of the following characteristics: (i) the adjuvant comprises a 2-amino nitrogen moiety that remains unsubstituted, (ii) the point of attachment of the linker to the adjuvant core is in a location relative to the 2-amino nitrogen moiety that allows for necessary alignment in the binding domain of its receptor, and (iii) the adjuvant moiety may further comprises a hydrophobic substituent with at least 1 carbon atom (e.g., at least 2 carbon atoms, at least 3 carbon atoms, at least 4 carbon atoms, or at least 6 carbon atoms).

## DEFINITIONS

As used herein, the term "immunoconjugate" refers to an antibody construct, or antibody, that is covalently bonded to a non-naturally occurring chemical moiety as described herein. The terms "immunoconjugate" and "antibody-adjuvant immunoconjugate" are used interchangeably herein.

As used herein, the phrase "antibody construct" refers to polypeptide comprising an antigen binding domain and an Fc domain. An antibody construct can comprise or be an antibody.

As used herein, the phrase "antigen binding domain" refers to a protein, or a portion of a protein, that specifically binds a specified antigen (e.g., a paratope), for example, that portion of an antigen-binding protein that contains the amino acid residues that interact with an antigen and confer on the antigen-binding protein its specificity and affinity for the antigen.

As used herein, the phrase "Fc domain" refers to the fragment crystallizable region, or the tail region of an antibody. The Fc domain interacts with Fc receptors on cell surfaces.

As used herein, the phrase "targeting binding domain" refers to a protein, or a portion of a protein, that specifically binds a second antigen that is distinct from the antigen bound by the antigen binding domain of the immunoconjugates. The targeting binding domain can be conjugated to the antibody construct at a C-terminal end of the Fc domain.

As used herein, the term "antibody" refers to a polypeptide comprising an antigen binding region (including the complementarity determining region (CDRs)) from an immunoglobulin gene or fragments thereof that specifically binds and recognizes an antigen. The recognized immunoglobulin genes include the kappa, lambda, alpha, gamma, delta, epsilon, and mu constant region genes, as well as numerous immunoglobulin variable region genes.

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain ($V_L$) and variable heavy chain ($V_H$) refer to these light and heavy chains,

respectively. Light chains are classified as either kappa or lambda. Heavy chains are classified as gamma, mu, alpha, delta, or epsilon, which in turn define the immunoglobulin classes IgG, IgM, IgA, IgD, and IgE, respectively.

IgG antibodies are large molecules of about 150 kDa composed of four peptide chains. IgG antibodies contain two identical class $\gamma$ heavy chains of about 50 kDa and two identical light chains of about 25 kDa, forming a tetrameric quaternary structure. The two heavy chains are linked to each other and to a light chain each by disulfide bonds. The resulting tetramer has two identical halves, which together form the Y-like shape. Each end of the fork contains an identical antigen binding site. There are four IgG subclasses (IgG1, 2, 3, and 4) in humans, named in order of their abundance in serum (IgG1 being the most abundant). Typically, the antigen-binding region of an antibody will be most critical in specificity and affinity of binding.

Dimeric IgA antibodies are about 320 kDa. IgA has two subclasses (IgA1 and IgA2) and can be produced as a monomeric as well as a dimeric form. The IgA dimeric form (secretory or sIgA) is the most abundant.

Antibodies can exist, for examples, as intact immunoglobulins or as a number of well-characterized fragments produced by digestion with various peptidases. Thus, for example, pepsin digests an antibody below the disulfide linkages in the hinge region to produce $F(ab)'_2$, a dimer of Fab which itself is a light chain joined to $V_H$-$C_H1$ by a disulfide bond. The $F(ab)'_2$ may be reduced under mild conditions to break the disulfide linkage in the hinge region, thereby converting the $F(ab)'_2$ dimer into a Fab' monomer. The Fab' monomer is essentially Fab with part of the hinge region (see, e.g., Fundamental Immunology (Paul, editor, 7th edition, 2012)). While various antibody fragments are defined in terms of the digestion of an intact antibody, such fragments may be synthesized *de novo* either chemically or by using recombinant DNA methodology. Thus, the term antibody, as used herein, also includes antibody fragments produced by the modification of whole antibodies, synthesized *de novo* using recombinant DNA methodologies (e.g., single chain Fv), or identified using phage display libraries (see, e.g., McCafferty et al., Nature, 348: 552-554 (1990)).

The term "antibody" is used in the broadest sense and specifically encompasses monoclonal antibodies (including full length monoclonal antibodies), polyclonal antibodies, multispecific antibodies (e.g., bispecific antibodies), and antibody fragments so long as they exhibit the desired biological activity. "Antibody fragment" and all grammatical variants thereof as used herein are defined as a portion of an intact antibody comprising the antigen binding site or variable region of the intact antibody, wherein the portion is free of the constant heavy chain domains (i.e., CH2, CH3, and CH4, depending on antibody isotype) of the Fc region of the intact antibody. Examples of antibody fragments include Fab, Fab', Fab'-SH, $F(ab')_2$, and Fv fragments; diabodies; any antibody fragment that is a polypeptide having a primary structure consisting of one uninterrupted sequence of contiguous amino acid residues (referred to herein as a "single-chain antibody fragment" or "single chain polypeptide"), including without limitation (1) single-chain Fv (scFv) molecules; (2) single chain polypeptides containing only one light chain variable domain, or a fragment thereof that contains the three CDRs of the light chain variable domain, without an associated heavy chain moiety; (3) single chain polypeptides containing only one heavy chain variable region, or a fragment thereof containing the three CDRs of the heavy chain variable region, without an associated light chain moiety; (4) nanobodies comprising single Ig domains from non-human species or other specific single-domain binding modules; and (5) multispecific or multivalent structures formed from antibody fragments. In an antibody fragment comprising one or more heavy chains, the heavy chain(s) can contain any constant domain sequence (e.g., CH1 in the IgG isotype) found in a non-Fc region of an intact antibody, and/or can contain any hinge region sequence found in an intact antibody, and/or can contain a leucine zipper sequence fused to or situated in the hinge region sequence or the constant domain sequence of the heavy chain(s).

As used herein, the term "biosimilar" in reference to a biological product means that the biological product is highly similar to the reference product notwithstanding minor differences in clinically inactive components, and there are no clinically meaningful differences between the biological product and the reference product in terms of the safety, purity, and potency of the product.

As used herein, the term "epitope" means any antigenic determinant on an antigen to which binds the antigen-binding site, also referred to as the paratope, of an antibody. Epitopic determinants usually consist of chemically active surface groupings of molecules such as amino acids or sugar side chains and usually have specific three-dimensional structural characteristics, as well as specific charge characteristics.

The terms "polypeptide," "peptide," and "protein" are used interchangeably herein to refer to a polymer of amino acid residues. The terms also apply to amino acid polymers in which one or more amino acid residues are artificial chemical mimetics of a corresponding naturally occurring amino acids, as well as to naturally occurring amino acid polymers and non-naturally occurring amino acid polymer.

As used herein, the term "adjuvant" refers to a substance capable of eliciting an immune response in a subject exposed to the adjuvant.

As used herein, the phrase "adjuvant moiety" refers to an adjuvant that is covalently bonded to an antibody as described herein. The adjuvant moiety can elicit the immune response while bonded to the antibody or after cleavage (e.g., enzymatic cleavage) from the antibody following administration of an immunoconjugate to the subject.

As used herein, the phrase "pattern recognition receptor" and term "PRR" refer to any member of a class of conserved mammalian proteins which recognize pathogen-associated molecular patterns (PAMPs) or damage-associated molecular patterns (DAMPs), and act as key signaling elements in innate immunity. Pattern recognition receptors are divided into membrane-bound PRRs, cytoplasmic PRRs, and secreted PRRs. Examples of membrane-bound PRRs include Toll-like receptors (TLRs) and C-type lectin receptors (CLRs). Examples of cytoplasmic PRRs include NOD-like receptors (NLRs) and Rig-I-like receptors (RLRs).

As used herein, the phrase "Toll-like receptor" and term "TLR" refer to any member of a family of highly-conserved mammalian proteins which recognize pathogen-associated molecular patterns and act as key signaling elements in innate immunity. TLR polypeptides share a characteristic structure that includes an extracellular domain that has leucine-rich repeats, a transmembrane domain, and an intracellular domain that is involved in TLR signaling.

The phrase "Toll-like receptor 1" and term "TLR1" refer to nucleic acids or polypeptides sharing at least 70%; 80%, 90%, 95%, 96%, 97%, 98%, 99%, or more sequence identity to a publicly-available TLR1 sequence, e.g., GenBank accession number AAY85643 for human TLR1 polypeptide, or GenBank accession number AAG37302 for murine TLR1 polypeptide.

The phrase "Toll-like receptor 2" and term "TLR2" refer to nucleic acids or polypeptides sharing at least 70%; 80%, 90%, 95%, 96%, 97%, 98%, 99%, or more sequence identity to a publicly-available TLR2 sequence, e.g., GenBank accession number AAY85648 for human TLR2 polypeptide, or GenBank accession number AAD49335 for murine TLR2 polypeptide.

The phrase "Toll-like receptor 3" and term "TLR3" refer to nucleic acids or polypeptides sharing at least 70%; 80%, 90%, 95%, 96%, 97%, 98%, 99%, or more sequence identity to a publicly-available TLR3 sequence, e.g., GenBank accession number AAC34134 for human TLR3 polypeptide, or GenBank accession number AAK26117 for murine TLR3 polypeptide.

The phrase "Toll-like receptor 4" and term "TLR4" refer to nucleic acids or polypeptides sharing at least 70%; 80%, 90%, 95%, 96%, 97%, 98%, 99%, or more sequence identity to a publicly-available TLR4 sequence, e.g., GenBank accession number AAY82270 for human TLR4 polypeptide, or GenBank accession number AAD29272 for murine TLR4 polypeptide.

The phrase "Toll-like receptor 5" and term "TLR5" refer to nucleic acids or polypeptides sharing at least 70%; 80%, 90%, 95%, 96%, 97%, 98%, 99%, or more sequence identity to a publicly-available TLR5 sequence, e.g., GenBank accession number ACM69034 for human TLR5 polypeptide, or GenBank accession number AAF65625 for murine TLR5 polypeptide.

The phrase "Toll-like receptor 6" and term "TLR6" refer to nucleic acids or polypeptides sharing at least 70%; 80%, 90%, 95%, 96%, 97%, 98%, 99%, or more sequence identity to a publicly-available TLR6 sequence, e.g., GenBank accession number ABY67133 for human TLR6 polypeptide, or GenBank accession number AAG38563 for murine TLR6 polypeptide.

The phrase "Toll-like receptor 7" and term "TLR7" refer to nucleic acids or polypeptides sharing at least 70%; 80%, 90%, 95%, 96%, 97%, 98%, 99%, or more sequence identity to a publicly-available TLR7 sequence, e.g., GenBank accession number AAZ99026 for human TLR7 polypeptide, or GenBank accession number AAK62676 for murine TLR7 polypeptide.

The phrase "Toll-like receptor 8" and term "TLR8" refer to nucleic acids or polypeptides sharing at least 70%; 80%, 90%, 95%, 96%, 97%, 98%, 99%, or more sequence identity to a publicly-available TLR8 sequence, e.g., GenBank accession number AAZ95441 for human TLR8 polypeptide, or GenBank accession number AAK62677 for murine TLR8 polypeptide.

The phrase "Toll-like receptor 7/8" and term "TLR7/8" refer to nucleic acids or polypeptides that are both TLR7 agonists and TLR8 agonists.

The phrase "Toll-like receptor 9" and term "TLR9" refer to nucleic acids or polypeptides sharing at least 70%; 80%, 90%, 95%, 96%, 97%, 98%, 99%, or more sequence identity to a publicly-available TLR9 sequence, e.g., GenBank accession number AAF78037 for human TLR9 polypeptide, or GenBank accession number AAK28488 for murine TLR9 polypeptide.

The phrase "Toll-like receptor 10" and term "TLR10" refer to nucleic acids or polypeptides sharing at least 70%; 80%, 90%, 95%, 96%, 97%, 98%, 99%, or more sequence identity to a publicly-available TLR10 sequence, e.g., GenBank accession number AAK26744 for human TLR10 polypeptide.

The phrase "Toll-like receptor 11" and term "TLR11" refer to nucleic acids or polypeptides sharing at least 70%; 80%, 90%, 95%, 96%, 97%, 98%, 99%, or more sequence identity to a publicly-available TLR11 sequence, e.g., GenBank accession number AAS83531 for murine TLR11 polypeptide.

A "TLR agonist" is a substance that binds, directly or indirectly, to a TLR (e.g., TLR7 and/or TLR8) to induce TLR signaling. Any detectable difference in TLR signaling can indicate that an agonist stimulates or activates a TLR. Signaling differences can be manifested, for example, as changes in the expression of target genes, in the phosphorylation of signal transduction components, in the intracellular localization of downstream elements such as NK-xB, in the association of certain components (such as IRAK) with other proteins or intracellular structures, or in the biochemical activity of components such as kinases (such as MAPK).

As used herein, the term "amino acid" refers to any monomeric unit that can be incorporated into a peptide, polypeptide, or protein. Amino acids include naturally-occurring $\alpha$-amino acids and their stereoisomers, as well as unnatural (non-naturally occurring) amino acids and their stereoisomers. "Stereoisomers" of a given amino acid refer to isomers having the same molecular formula and intramolecular bonds but different three-dimensional arrangements of bonds and atoms (e.g., an L-amino acid and the corresponding D-amino acid).

Naturally-occurring amino acids are those encoded by the genetic code, as well as those amino acids that are later modified, e.g., hydroxyproline, $\gamma$-carboxyglutamate, and O-phosphoserine. Naturally-occurring $\alpha$-amino acids include, without limitation, alanine (Ala), cysteine (Cys), aspartic acid (Asp), glutamic acid (Glu), phenylalanine (Phe), glycine (Gly), histidine (His), isoleucine (Ile), arginine (Arg), lysine (Lys), leucine (Leu), methionine (Met), asparagine (Asn), proline (Pro), glutamine (Gln), serine (Ser), threonine (Thr), valine (Val), tryptophan (Trp), tyrosine (Tyr), and combinations thereof. Stereoisomers of a naturally-occurring $\alpha$-amino acids include, without limitation, D-alanine (D-Ala), D-cysteine (D-Cys), D-aspartic acid (D-Asp), D-glutamic acid (D-Glu), D-phenylalanine (D-Phe), D-histidine (D-His), D-isoleucine (D-Ile), D-arginine (D-Arg), D-lysine (D-Lys), D-leucine (D-Leu), D-methionine (D-Met), D-asparagine (D-Asn), D-proline (D-Pro), D-glutamine (D-Gln), D-serine (D-Ser), D-threonine (D-Thr), D-valine (D-Val), D-tryptophan (D-Trp), D-tyrosine (D-Tyr), and combinations thereof.

Unnatural (non-naturally occurring) amino acids include, without limitation, amino acid analogs, amino acid mimetics, synthetic amino acids, N-substituted glycines, and *N*-methyl amino acids in either the L- or D-configuration that function in a manner similar to the naturally-occurring amino acids. For example, "amino acid analogs" can be unnatural amino acids that have the same basic chemical structure as naturally-occurring amino acids (i.e., a carbon that is bonded to a hydrogen, a carboxyl group, an amino group) but have modified side-chain groups or modified peptide backbones, e.g., homoserine, norleucine, methionine sulfoxide, methionine methyl sulfonium. "Amino acid mimetics" refer to chemical compounds that have a structure that is different from the general chemical structure of an amino acid, but that functions in a manner similar to a naturally-occurring amino acid. Amino acids may be referred to herein by either the commonly known three letter symbols or by the one-letter symbols recommended by the IUPAC-IUB Biochemical Nomenclature Commission.

As used herein, the term "immune checkpoint inhibitors" refers to any modulator that inhibits the activity of the immune checkpoint molecule. Immune checkpoint inhibitors can include, but are not limited to, immune checkpoint molecule binding proteins, small molecule inhibitors, antibodies, antibody-derivatives (including Fc fusions, Fab fragments and scFvs), antibody-drug conjugates, antisense oligonucleotides, siRNA, aptamers, peptides and peptide mimetics.

Useful bonds for connecting linking moieties to proteins and other materials include, but are not limited to, amides, amines, esters, carbamates, ureas, thioethers, thiocarbamates, thiocarbonates, and thioureas. A "divalent" linking moiety contains two points of attachment for linking two functional groups; polyvalent linking moieties can have additional points of attachment for linking further functional groups. For example, divalent linking moieties include divalent polymer moieties such as divalent poly(ethylene glycol), divalent polypropylene glycol), and divalent poly(vinyl alcohol).

As used herein, when the term "optionally present" is used to refer to a chemical structure (e.g., "X" or "Y"), if that chemical structure is not present, the bond originally made to the chemical structure is made directly to the adjacent atom.

As used herein, the term "linker" refers to a functional group that covalently bonds two or more moieties in a compound or material. For example, the linker can serve to covalently bond an adjuvant core to an antibody construct in an immunoconjugate.

As used herein, the term "spacer" refers to a functional group that covalently bonds two or more moieties in a compound or material. For example, the spacer can covalently bond an adjuvant moiety to an antibody construct in an immunoconjugate.

As used herein, the term "alkyl" refers to a straight or branched, saturated, aliphatic radical having the number of carbon atoms indicated. Alkyl can include any number of carbons, such as $C_{1-2}$, $C_{1-3}$, $C_{1-4}$, $C_{1-5}$, $C_{1-6}$, $C_{1-7}$, $C_{1-8}$, $C_{1-9}$, $C_{1-10}$, $C_{2-3}$, $C_{2-4}$, $C_{2-5}$, $C_{2-6}$, $C_{3-4}$, $C_{3-5}$, $C_{3-6}$, $C_{4-5}$, $C_{4-6}$ and $C_{5-6}$. For example, $C_{1-6}$ alkyl includes, but is not limited to, methyl, ethyl, propyl, isopropyl, butyl, isobutyl, sec-butyl, tert-butyl, pentyl, isopentyl, hexyl, etc. Alkyl can also refer to alkyl groups having up to 30 carbons atoms, such as, but not limited to heptyl, octyl, nonyl, decyl, etc. Alkyl groups can be substituted or unsubstituted. "Substituted alkyl" groups can be substituted with one or more groups selected from halo, hydroxy, amino, oxo (=O), alkylamino, amido, acyl, nitro, cyano, and alkoxy. The term "alkylene" refers to a divalent alkyl radical.

As used herein, the term "heteroalkyl" refers to an alkyl group as described herein, wherein one or more carbon atoms are optionally and independently replaced with a heteroatom selected from N, O, and S. The term "heteroalkylene" refers to a divalent heteroalkyl radical.

As used herein, the term "cycloalkyl" refers to a saturated or partially unsaturated, monocyclic, fused bicyclic, or bridged polycyclic ring assembly containing from 3 to 12 ring atoms, or the number of atoms indicated. Cycloalkyls can include any number of carbons, such as $C_{3-6}$, $C_{4-6}$, $C_{5-6}$, $C_{3-8}$, $C_{4-8}$, $C_{5-8}$, $C_{6-8}$, $C_{3-9}$, $C_{3-10}$, $C_{3-11}$, and $C_{3-12}$. Saturated monocyclic carbocyclic rings include, for example, cyclopropyl, cyclobutyl, cyclopentyl, cyclohexyl, and cyclooctyl.

Saturated bicyclic and polycyclic carbocyclic rings include, for example, norbornane, [2.2.2] bicyclooctane, decahydronaphthalene and adamantane. Cycloalkyl groups can also be partially unsaturated, having one or more double or triple bonds in the ring. Representative carbocyclic groups that are partially unsaturated include, but are not limited to, cyclobutene, cyclopentene, cyclohexene, cyclohexadiene (1,3- and 1,4-isomers), cycloheptene, cycloheptadiene, cyclooctene, cyclooctadiene (1,3-, 1,4- and 1,5-isomers), norbornene, and norbornadiene.

Unsaturated carbocyclic groups also include aryl groups. The term "aryl" refers to an aromatic ring system having any suitable number of ring atoms and any suitable number of rings. Aryl groups can include any suitable number of ring atoms, such as, 6, 7, 8, 9, 10, 11, 12, 13, 14, 15, or 16 ring atoms, as well as from 6 to 10, 6 to 12, or 6 to 14 ring members. Aryl groups can be monocyclic, fused to form bicyclic or tricyclic groups, or linked by a bond to form a biaryl group. Representative aryl groups include phenyl, naphthyl and biphenyl. Other aryl groups include benzyl, having a methylene linking group. Some aryl groups have from 6 to 12 ring members, such as phenyl, naphthyl or biphenyl. Other aryl groups have from 6 to 10 ring members, such as phenyl or naphthyl. Aryl groups can be substituted or unsubstituted. "Substituted aryl" groups can be substituted with one or more groups selected from halo, hydroxy, amino, oxo (=O), alkylamino, amido, acyl, nitro, cyano, alkyl, and alkoxy.

A "divalent" cycloalkyl refers to a carbocyclic group having two points of attachment for covalently linking two moieties in a molecule or material. Cycloalkyl groups can be substituted or unsubstituted. "Substituted cycloalkyl" groups can be substituted with one or more groups selected from halo, hydroxy, amino, oxo (=O), alkylamino, amido, acyl, nitro, cyano, alkyl, and alkoxy.

As used herein, the term "heterocycle" refers to heterocycloalkyl groups and heteroaryl groups. "Heteroaryl," by itself or as part of another substituent, refers to a monocyclic or fused bicyclic or tricyclic aromatic ring assembly containing 5 to 16 ring atoms, where from 1 to 5 of the ring atoms are a heteroatom such as N, O or S. Additional heteroatoms can also be useful, including, but not limited to, B, Al, Si and P. The heteroatoms can be oxidized to form moieties such as, but not limited to, -S(O)- and $-S(O)_2-$. Heteroaryl groups can include any number of ring atoms, such as 3 to 6, 4 to 6, 5 to 6, 3 to 8, 4 to 8, 5 to 8, 6 to 8, 3 to 9, 3 to 10, 3 to 11, or 3 to 12 ring members. Any suitable number of heteroatoms can be included in the heteroaryl groups, such as 1, 2, 3, 4, or 5, or 1 to 2, 1 to 3, 1 to 4, 1 to 5, 2 to 3, 2 to 4, 2 to 5, 3 to 4, or 3 to 5. The heteroaryl group can include groups such as pyrrole, pyridine, imidazole, pyrazole, triazole, tetrazole, pyrazine, pyrimidine, pyridazine, triazine (1,2,3-, 1,2,4- and 1,3,5-isomers), thiophene, furan, thiazole, isothiazole, oxazole, and isoxazole. The heteroaryl groups can also be fused to aromatic ring systems, such as a phenyl ring, to form members including, but not limited to, benzopyrroles such as indole and isoindole, benzopyridines such as quinoline and isoquinoline, benzopyrazine (quinoxaline), benzopyrimidine (quinazoline), benzopyridazines such as phthalazine and cinnoline, benzothiophene, and benzofuran. Other heteroaryl groups include heteroaryl rings linked by a bond, such as bipyridine. Heteroaryl groups can be substituted or unsubstituted. "Substituted heteroaryl" groups can be substituted with one or more groups selected from halo, hydroxy, amino, oxo (=O), alkylamino, amido, acyl, nitro, cyano, alkyl, and alkoxy.

Heteroaryl groups can be linked via any position on the ring. For example, pyrrole includes 1-, 2- and 3-pyrrole, pyridine includes 2-, 3- and 4-pyridine, imidazole includes 1-, 2-, 4- and 5-imidazole, pyrazole includes 1-, 3-, 4- and 5-pyrazole, triazole includes 1-, 4- and 5-triazole, tetrazole includes 1- and 5-tetrazole, pyrimidine includes 2-, 4-, 5- and 6-pyrimidine, pyridazine includes 3- and 4-pyridazine, 1,2,3-triazine includes 4- and 5-triazine, 1,2,4-triazine includes 3-, 5- and 6-triazine, 1,3,5-triazine includes 2-triazine, thiophene includes 2- and 3-thiophene, furan includes 2- and 3-furan, thiazole includes 2-, 4- and 5-thiazole, isothiazole includes 3-, 4- and 5-isothiazole, oxazole includes 2-, 4- and 5-oxazole, isoxazole includes 3-, 4- and 5-isoxazole, indole includes 1-, 2- and 3-indole, isoindole includes 1- and 2-isoindole, quinoline includes 2-, 3- and 4-quinoline, isoquinoline includes 1-, 3- and 4-isoquinoline, quinazoline includes 2- and 4-quinoazoline, cinnoline includes 3- and 4-cinnoline, benzothiophene includes 2- and 3-benzothiophene, and benzofuran includes 2- and 3-benzofuran.

"Heterocycloalkyl," by itself or as part of another substituent, refers to a saturated ring system having from 3 to 12 ring members and from 1 to 4 heteroatoms of N, O and S. Additional heteroatoms can also be useful, including, but not limited to, B, Al, Si and P. The heteroatoms can be oxidized to form moieties such as, but not limited to, -S(O)- and $-S(O)_2-$. Heterocycloalkyl groups can include any number of ring atoms, such as, 3 to 6, 4 to 6, 5 to 6, 3 to 8, 4 to 8, 5 to 8, 6 to 8, 3 to 9, 3 to 10, 3 to 11, or 3 to 12 ring members. Any suitable number of heteroatoms can be included in the heterocycloalkyl groups, such as 1, 2, 3, or 4, or 1 to 2, 1 to 3, 1 to 4, 2 to 3, 2 to 4, or 3 to 4. The heterocycloalkyl group can include groups such as aziridine, azetidine, pyrrolidine, piperidine, azepane, azocane, quinuclidine, pyrazolidine, imidazolidine, piperazine (1,2-, 1,3- and 1,4-isomers), oxirane, oxetane, tetrahydrofuran, oxane (tetrahydropyran), oxepane, thiirane, thietane, thiolane (tetrahydrothiophene), thiane (tetrahydrothiopyran), oxazolidine, isoxazolidine, thiazolidine, isothiazolidine, dioxolane, dithiolane, morpholine, thiomorpholine, dioxane, or dithiane. The heterocycloalkyl groups can also be fused to aromatic or non-aromatic ring systems to form members including, but not limited to, indoline. Heterocycloalkyl groups can be unsubstituted or substituted. "Substituted heterocycloalkyl" groups can be substituted with one or more groups selected from halo, hydroxy, amino, oxo (=O), alkylamino, amido, acyl, nitro, cyano, alkyl, and alkoxy.

Heterocycloalkyl groups can be linked via any position on the ring. For example, aziridine can be 1- or 2-aziridine, azetidine can be 1- or 2- azetidine, pyrrolidine can be 1-, 2- or 3-pyrrolidine, piperidine can be 1-, 2-, 3- or 4-piperidine,

pyrazolidine can be 1-, 2-, 3-, or 4-pyrazolidine, imidazolidine can be 1-, 2-, 3- or 4-imidazolidine, piperazine can be 1-, 2-, 3- or 4-piperazine, tetrahydrofuran can be 1- or 2-tetrahydrofuran, oxazolidine can be 2-, 3-, 4- or 5-oxazolidine, isoxazolidine can be 2-, 3-, 4- or 5-isoxazolidine, thiazolidine can be 2-, 3-, 4- or 5-thiazolidine, isothiazolidine can be 2-, 3-, 4- or 5- isothiazolidine, and morpholine can be 2-, 3- or 4-morpholine.

As used herein, the terms "halo" and "halogen," by themselves or as part of another substituent, refer to a fluorine, chlorine, bromine, or iodine atom.

As used herein, the term "carbonyl," by itself or as part of another substituent, refers to -C(O)-, i.e., a carbon atom double-bonded to oxygen and bound to two other groups in the moiety having the carbonyl.

As used herein, the term "amino" refers to a moiety $-NR_3$, wherein each R group is H or alkyl. An amino moiety can be ionized to form the corresponding ammonium cation.

As used herein, the term "hydroxy" refers to the moiety -OH.

As used herein, the term "cyano" refers to a carbon atom triple-bonded to a nitrogen atom (i.e., the moiety -C=N).

As used herein, the term "carboxy" refers to the moiety -C(O)OH. A carboxy moiety can be ionized to form the corresponding carboxylate anion.

As used herein, the term "amido" refers to a moiety -NRC(O)R or $-C(O)NR_2$, wherein each R group is H or alkyl.

As used herein, the term "nitro" refers to the moiety $-NO_2$.

As used herein, the term "oxo" refers to an oxygen atom that is double-bonded to a compound (i.e., O=).

As used herein, the terms "treat," "treatment," and "treating" refer to any indicia of success in the treatment or amelioration of an injury, pathology, condition, or symptom (e.g., cognitive impairment), including any objective or subjective parameter such as abatement; remission; diminishing of symptoms or making the symptom, injury, pathology or condition more tolerable to the patient; reduction in the rate of symptom progression; decreasing the frequency or duration of the symptom or condition; or, in some situations, preventing the onset of the symptom. The treatment or amelioration of symptoms can be based on any objective or subjective parameter; including, e.g., the result of a physical examination.

As used herein, the term "cancer" refers to conditions including solid cancers, lymphomas, and leukemias. Examples of different types of cancer include, but are not limited to, lung cancer (e.g., non-small cell lung cancer or NSCLC), ovarian cancer, prostate cancer, colorectal cancer, liver cancer (i.e., hepatocarcinoma), renal cancer (i.e., renal cell carcinoma), bladder cancer, breast cancer, thyroid cancer, pleural cancer, pancreatic cancer, uterine cancer, cervical cancer, testicular cancer, anal cancer, bile duct cancer, gastrointestinal carcinoid tumors, esophageal cancer, gall bladder cancer, appendix cancer, small intestine cancer, stomach (gastric) cancer, cancer of the central nervous system, skin cancer (e.g., melanoma), choriocarcinoma, head and neck cancer, blood cancer, osteogenic sarcoma, fibrosarcoma, neuroblastoma, glioma, melanoma, B-cell lymphoma, non-Hodgkin's lymphoma, Burkitt's lymphoma, Small Cell lymphoma, Large Cell lymphoma, monocytic leukemia, myelogenous leukemia, acute lymphocytic leukemia, acute myelocytic leukemia, and multiple myeloma.

As used herein the phrases "effective amount" and "therapeutically effective amount" refer to a dose of a substance such as an immunoconjugate that produces therapeutic effects for which it is administered. The particular dose will depend on the purpose of the treatment, and will be ascertainable by one skilled in the art using known techniques (see, e.g., Lieberman, Pharmaceutical Dosage Forms (volumes 1-3, 1992); Lloyd, The Art, Science and Technology of Pharmaceutical Compounding (1999); Pickar, Dosage Calculations (1999); Goodman & Gilman's The Pharmacological Basis of Therapeutics, 11th Edition, 2006, Brunton, ed., McGraw-Hill; and Remington: The Science and Practice of Pharmacy, 21st Edition, 2005, Hendrickson, Ed., Lippincott, Williams & Wilkins).

As used herein, the term "subject" refers to animals such as mammals, including, but not limited to, primates (e.g., humans), cows, sheep, goats, horses, dogs, cats, rabbits, rats, mice and the like. In certain embodiments, the subject is a human.

As used herein, the term "administering" refers to parenteral, intravenous, intraperitoneal, intramuscular, intratumoral, intralesional, intranasal or subcutaneous administration, oral administration, administration as a suppository, topical contact, intrathecal administration, or the implantation of a slow-release device, e.g., a mini-osmotic pump, to the subj ect.

The terms "about" and "around," as used herein to modify a numerical value, indicate a relatively close range surrounding that explicit value. If "X" were the value, "about X" or "around X" would indicate a value from 0.9X to 1. 1X, e.g., from 0.95X to 1.05X or from 0.99X to 1.01X. Any reference to "about X" or "around X" specifically indicates at least the values X, 0.95X, 0.96X, 0.97X, 0.98X, 0.99X, 1.01X, 1.02X, 1.03X, 1.04X, and 1.05X. Thus, "about X" and "around X" are intended to teach and provide written description support for a claim limitation of, e.g., "0.98X."

As used herein, the phrase "point of attachment of the linker to the adjuvant core" refers to a carbon atom or nitrogen atom present in the adjuvant core to which the linker is bound.

As used herein, the phrase "point of attachment of the hydrophobic substituent to the adjuvant core" refers to a carbon atom or nitrogen atom present in the adjuvant core to which the linker is bound.

Antibody Adjuvant Conjugates

In some embodiments, the immunoconjugates of the invention are of Formula Immunoconjugate A:

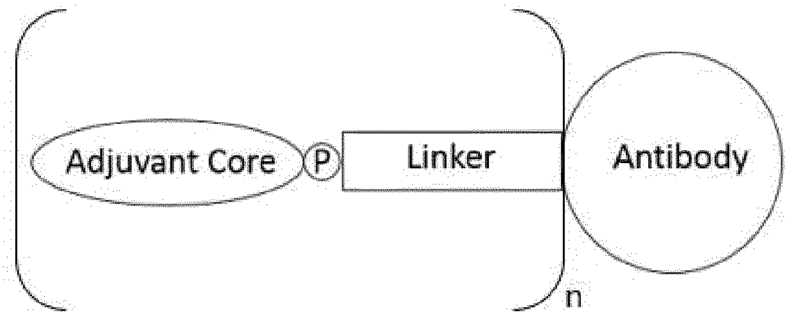


Immunoconjugate A,

wherein P represents the point of attachment of the linker to the adjuvant core, the adjuvant core comprises a 2-amino nitrogen moiety, and n is an integer from 1 to 8. The adjuvant core is optionally substituted such that the substitution pattern around the adjuvant core is not particularly limited, as long as the 2-amino nitrogen moiety remains unsubstituted.

The immunoconjugates of Formula Immunoconjugate A also can be defined by Formula Immunoconjugate B:

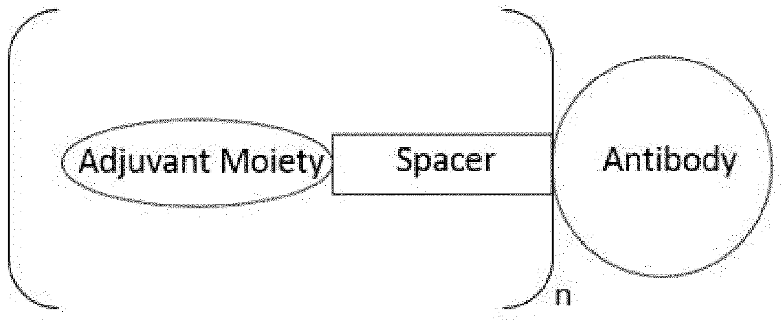


Immunoconjugate B;

wherein the adjuvant moiety comprises a 2-amino nitrogen moiety and a synthetic handle to attach the spacer, and n is an integer from 1 to 8. The adjuvant moiety is optionally substituted such that the substitution pattern around the adjuvant moiety is not particularly limited, as long as the 2-amino nitrogen moiety remains unsubstituted.

Accordingly, the adjuvant moiety can be of Formula Adjuvant Moiety A:

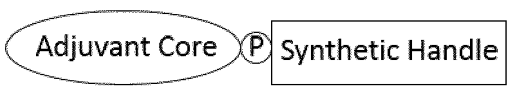


Adjuvant Moiety A,

and the linker can be of Formula Linker A:

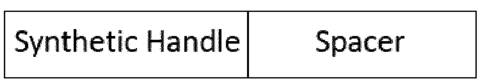


Linker A,

wherein P represents the point of attachment of the linker to the adjuvant core and the adjuvant core comprises a 2-amino

nitrogen moiety. The adjuvant core is optionally substituted such that the substitution pattern around the adjuvant core is not particularly limited, as long as the 2-amino nitrogen moiety remains unsubstituted. As used herein, the phrase "synthetic handle" refers to a chemical substituent that is a part of the adjuvant moiety and attaches the antibody via the spacer and the synthetic handle to the adjuvant core. Accordingly, when the antibody is attached via the spacer and the synthetic handle to the adjuvant core, the synthetic handle and the spacer become the linker such that the linker to bound to the adjuvant core at point of attachment "P." However, when considering adjuvant activity, the synthetic handle is considered part of the adjuvant moiety. Thus, the adjuvant moiety comprises the adjuvant core, the point of attachment, and the synthetic handle.

Thus, in some embodiments, the immunoconjugates of the invention are of the Formula Immunoconjugate C:

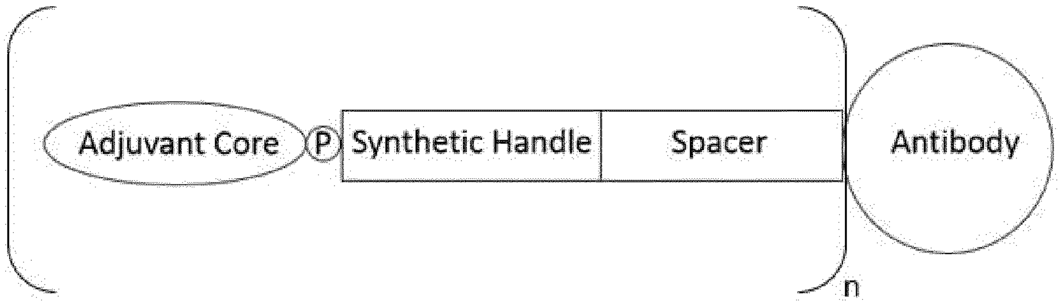


Immunoconjugate C;

wherein the synthetic handle and the spacer make up a linker, P represents the point of attachment of the linker to the adjuvant core, the adjuvant core and synthetic handle make up the adjuvant moiety, the adjuvant core comprises a 2-amino nitrogen moiety, and n is an integer from 1 to 8. The adjuvant core is optionally substituted such that the substitution pattern around the adjuvant core is not particularly limited, as long as the 2-amino nitrogen moiety remains unsubstituted.

The adjuvant moiety is a compound that elicits an immune response. In some immunoconjugates of the invention, the adjuvant moiety is a TLR agonist. TLR agonists include TLR1, TLR2, TLR3, TLR4, TLR5, TLR6, TLR7, TLR8, TLR9, TLR10, TLR11, or any combination thereof (e.g., TLR7/8 agonists). Any adjuvant capable of activating a TLR can be utilized in the immunoconjugates of the invention. TLRs are type-I transmembrane proteins that are responsible for initiation of innate immune responses in vertebrates. TLRs recognize a variety of pathogen-associated molecular patterns from bacteria, viruses, and fungi and act as a first line of defense against invading pathogens. TLRs elicit overlapping yet distinct biological responses due to differences in cellular expression and in the signaling pathways that they initiate within vertebrates. Once engaged (e.g., by a natural stimulus or a synthetic TLR agonist) TLRs initiate a signal transduction cascade leading to activation of NF-κB via the adapter protein myeloid differentiation primary response gene 88 (MyD88) and recruitment of the IL-1 receptor associated kinase (IRAK). Phosphorylation of IRAK then leads to recruitment of TNF-receptor associated factor (TRAF) 6 (TRAF6), which results in the phosphorylation of the NF-κB inhibitor I-xB. As a result, NF-κB enters the cell nucleus and initiates transcription of genes whose promoters contain NF-κB binding sites, such as cytokines. Additional modes of regulation for TLR signaling include TIR-domain containing adapter-inducing interferon-β (TRIF)-dependent induction of TRAF6 and activation of MyD88 independent pathways via TRIF and TRAF3, leading to the phosphorylation of interferon response factor (IRF) three (IRF3). Similarly, the MyD88 dependent pathway also activates several IRF family members, including IRF5 and IRF7 whereas the TRIF dependent pathway also activates the NF-κB pathway.

In certain embodiments, the adjuvant moiety is a TLR7 and/or TLR8 agonist. Any adjuvant capable of activating TLR7 and/or TLR8 can be utilized in the immunoconjugates of the invention. Examples of TLR7 agonists and TLR8 agonists are described, for example, by Vacchelli et al. (OncoImmunology, 2(8): e25238 (2013)) and Carson et al. (U.S. Patent Application Publication 2013/0165455). TLR7 and TLR8 are both expressed in monocytes and dendritic cells. In humans, TLR7 is also expressed in plasmacytoid dendritic cells (pDCs) and B cells. TLR8 is expressed mostly in cells of myeloid origin, i.e., monocytes, granulocytes, and myeloid dendritic cells. TLR7 and TLR8 are capable of detecting the presence of "foreign" single-stranded RNA within a cell as a means to respond to viral invasion. Treatment of TLR8-expressing cells with TLR8 agonists can result in production of high levels of IL-12, IFN-γ, IL-1, TNF-α, IL-6, and other inflammatory cytokines. Similarly, stimulation of TLR7-expressing cells, such as pDCs, with TLR7 agonists can result in production of high levels of IFN-α and other inflammatory cytokines. TLR7/TLR8 engagement and resulting cytokine production can activate dendritic cells and other antigen-presenting cells, driving diverse innate and acquired immune response mechanisms leading to tumor destruction.

In some embodiments, the immunoconjugates of the present disclosure are of Formula Immunoconjugate A1 or

Immunoconjugate A2:

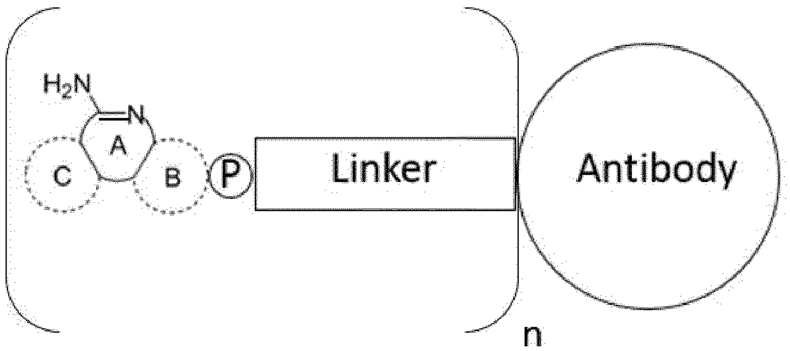

Immunoconjugate A1

or

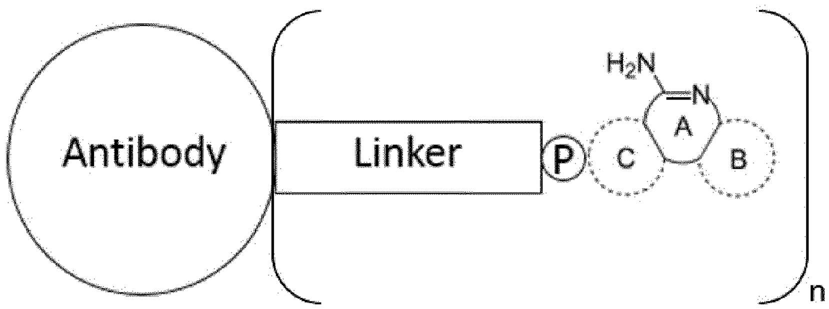

Immunoconjugate A2;

wherein the adjuvant core is represented by fused rings A, B, and C, and wherein A is present, B and C are optionally present, and A, B, and C denote 5-, 6-, 7-, 8-, or 9-membered rings, optionally comprising double bonds, optionally comprising heteroatoms (e.g., nitrogen, oxygen, and/or sulfur) in addition to the 2-amino nitrogen moiety, and optionally substituted, P represents the point of attachment of the linker to the B ring or the C ring, and n is an integer from 1 to 8. The adjuvant core is optionally substituted such that the substitution pattern around the adjuvant core is not particularly limited, as long as the 2-amino nitrogen moiety remains unsubstituted. In certain embodiments, the point of attachment of the linker to the B ring or the C ring occurs at the opposite face of the adjuvant core relative to the 2-amino nitrogen moiety. As used herein, the term "opposite face" refers to what would be the concave side and the ends of the adjuvant core of Immunoconjugate A1 and Immunoconjugate A2. Without wishing to be bound by any particular theory, it is believed that a point of attachment of the linker to the adjuvant core at the opposite face of the adjuvant core relative to the 2-amino nitrogen moiety allows for favorable steric and/or electronic interactions of the linker and the activity of the 2-amino nitrogen moiety is maintained. In certain embodiments, rings A, B, and C are present.

In some embodiments, the immunoconjugates of the present disclosure are of Formula Immunoconjugate C1 or Immunoconjugate C2:

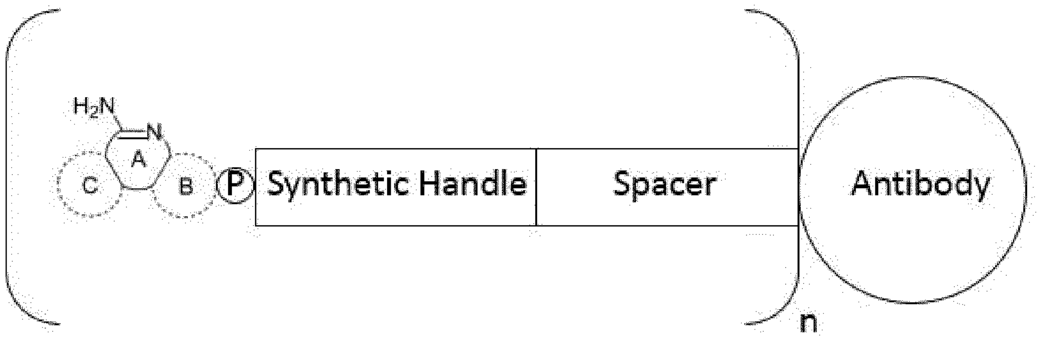


Immunoconjugate C1

or

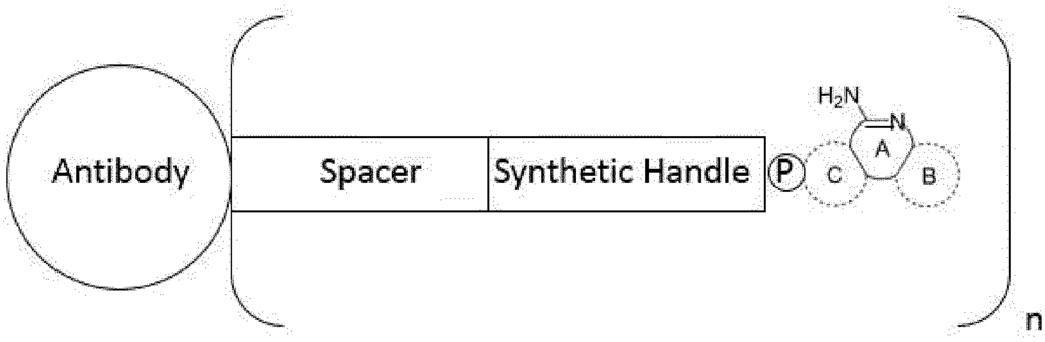


Immunoconjugate C2;

wherein the adjuvant core is represented by fused rings A, B, and C, and wherein A is present, B and C are optionally present, and A, B, and C denote 5-, 6-, 7-, 8-, or 9-membered rings, optionally comprising double bonds, optionally comprising heteroatoms (e.g., nitrogen, oxygen, and/or sulfur) in addition to the 2-amino nitrogen moiety, and optionally substituted, the synthetic handle and the spacer make up a linker, P represents the point of attachment of the linker to the B ring or the C ring, the adjuvant core and synthetic handle make up the adjuvant moiety, and n is an integer from 1 to 8. The adjuvant core is optionally substituted such that the substitution pattern around the adjuvant core is not particularly limited, as long as the 2-amino nitrogen moiety remains unsubstituted. In preferred embodiments, the point of attachment of the linker to the adjuvant core at the B ring or the C ring occurs at the opposite face of the adjuvant core relative to the 2-amino nitrogen moiety.

In some embodiments, the B ring and the C ring are not present. In such embodiments, the point of attachment of the linker to the adjuvant core can be on the A ring as long as the 2-amino nitrogen moiety remains unsubstituted. The adjuvant core is optionally substituted such that the substitution pattern around the adjuvant core is not particularly limited, as long as the 2-amino nitrogen moiety remains unsubstituted.

The immunoconjugates of the invention are of the formula:

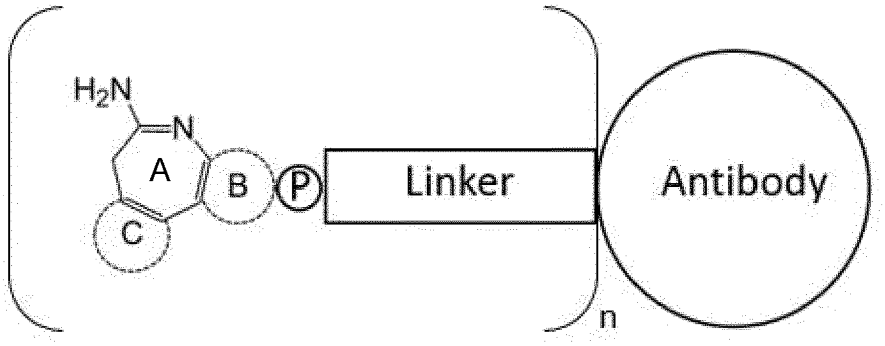

Immunoconjugate D2,

wherein the adjuvant core is represented by fused rings A, B and C, wherein A and B are present and C is optionally present, and B and C denote 5-, 6-, or 7-membered rings, optionally comprising double bonds, and optionally comprising heteroatoms in addition to the 2-amino nitrogen moiety, and the adjuvant core is optionally substituted such that the substitution pattern around the adjuvant core is not particularly limited, as long as the 2-amino nitrogen moiety remains unsubstituted, P represents the point of attachment of the linker to the B ring, and n is an integer from 1 to 8, or

the immunoconjugate is of formula:

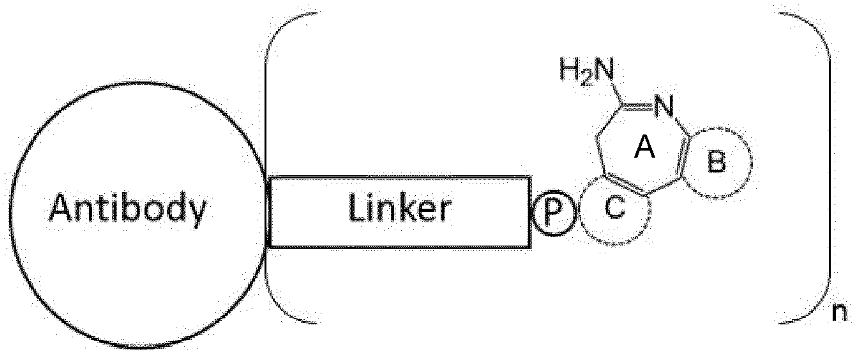

Immunoconjugate D4,

wherein the adjuvant core is represented by fused rings A, B and C, wherein A and C are present and B is optionally present, and B and C denote 5-, 6-, or 7-membered rings, optionally comprising double bonds, and optionally comprising heteroatoms in addition to the 2-amino nitrogen moiety, and the adjuvant core is optionally substituted such that the substitution pattern around the adjuvant core is not particularly limited, as long as the 2-amino nitrogen moiety remains unsubstituted, P represents the point of attachment of the linker to the C ring, and n is an integer from 1 to 8.

Also disclosed herein, but not according to the appended claims, are immunoconjugates of formula:

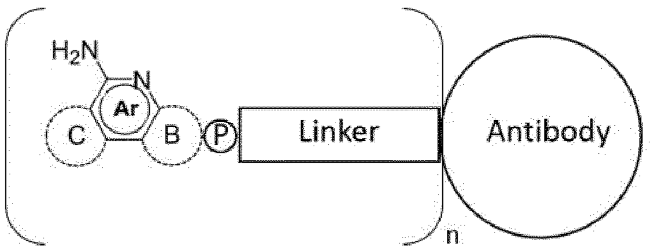

Immunoconjugate D1,

or

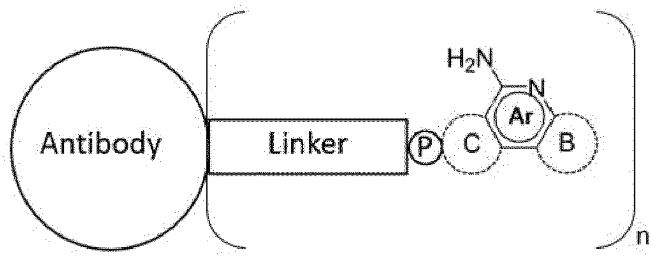

Immunoconjugate D3,

wherein the adjuvant core is represented by fused rings A, B, and C, and wherein A is present, B and C are optionally present, and A, B, and C denote 5-, 6-, 7-, 8-, or 9-membered rings, optionally comprising double bonds, optionally comprising heteroatoms (e.g., nitrogen, oxygen, and/or sulfur) in addition to the 2-amino nitrogen moiety, and optionally substituted, P represents the point of attachment of the linker to the B ring or the C ring, and n is an integer from 1 to 8. The adjuvant core is optionally substituted such that the substitution pattern around the adjuvant core is not particularly limited, as long as the 2-amino nitrogen moiety remains unsubstituted.

In some embodiments, the A ring and C ring are considered to be in the hydrophobic pocket of the binding domain of the toll like receptor. In such embodiments, the point of attachment of the linker is on the B ring. In certain aspects of the invention, the A ring and/or C ring further comprise a substituent that provides an additional interaction between the A ring and/or C ring and the hydrophobic pocket. In some embodiments, the substituent that provides an additional interaction is a hydrophobic substituent with at least 1 carbon atom (e.g., at least 2 carbon atoms, at least 3 carbon atoms, at least 4 carbon atoms, or at 6 least carbon atoms). Generally, substituent that provides an additional interaction between the A ring and/or C ring and the hydrophobic pocket occurs at the same face of the adjuvant core relative to the 2-amino nitrogen moiety. As used herein, the phrase "same face" refers to what would be the convex side of the adjuvant core of Immunoconjugate A1 and Immunoconjugate A2.

Without wishing to be bound by any particular theory, it is believed that adjuvant activity is increased with each one or more of (i) the 2-amino nitrogen moiety remains unsubstituted, (ii) the point of attachment of the linker is at the B ring or C ring, and occasionally the A ring but only when the B ring and C ring are not present, and (iii) the A ring and/or C ring further comprise a substituent with at least 1 carbon atom (e.g., at least 2 carbon atoms, at least 3 carbon atoms, at least 4 carbon atoms, or at least 6 carbon atoms). However, it is important that the substituent of the substituent of the A ring and/or C ring is not so large as to result in unfavorable steric and/or electronic interactions.

In some embodiments, the immunoconjugates of the invention comprise an adjuvant moiety with an adjuvant core comprising a 2-amino nitrogen moiety with a pendant nitrogen atom, wherein when bound to the binding domain of a TLR8 comprising an aspartic acid residue, the pendant nitrogen atom of the 2-amino nitrogen moiety is less than about 5 Å (e.g., less than about 4.9 Å, less than about 4.8 Å, less than about 4.7 Å, less than about 4.6 Å less than about 4.5 Å, less than about 4.4, Å less than about 4.3 Å, less than about 4.2 Å, less than about 4.1 Å, less than about 4.0 Å, less than about 3.9 Å, less than about 3.8 Å, less than about 3.7 Å, less than about 3.6 Å, less than about 3.5 Å, less than about 3.4 Å, less than about 3.3 Å, less than about 3.2 Å, less than about 3.1 Å, or less than about 3 Å) from a carbonyl oxygen of an acidic side chain of the aspartic acid residue. Generally, the aspartic acid residue is Asp543 or Asp545. In certain embodiments, the aspartic acid residue is Asp543. In preferred embodiments, the pendant nitrogen atom of the 2-amino nitrogen moiety is less than about 3 Å from a carbonyl oxygen of an acidic side chain of the aspartic acid residue. Without wishing to be bound by any particular theory, it is believed that the pendant nitrogen atom of the 2-amino nitrogen moiety forms a hydrogen bond and/or a salt bridge with the acidic side chain of the aspartic acid residue (e.g., Asp543). The hydrogen bond and/or salt bridge is believed to be crucial to maintaining the activity of the adjuvant moiety.

In some embodiments, the immunoconjugates of the invention comprise an adjuvant moiety with an adjuvant core comprising a 2-amino nitrogen moiety with a pendant nitrogen atom, wherein when bound to the binding domain of a TLR7 comprising an aspartic acid residue, the pendant nitrogen atom of the 2-amino nitrogen moiety is less than about 5 Å (e.g., less than about 4.9 Å, less than about 4.8 Å, less than about 4.7 Å, less than about 4.6 Å less than about 4.5 Å, less than about 4.4 Å, less than about 4.3 Å, less than about 4.2 Å, less than about 4.1 Å, less than about 4.0 Å, less than about 3.9 Å, less than about 3.8 Å, less than about 3.7 Å, less than about 3.6 Å, less than about 3.5 Å, less than about 3.4 Å, less than about 3.3 Å, less than about 3.2 Å, less than about 3.1 Å, or less than about 3 Å) from a carbonyl oxygen of an acidic side chain of the aspartic acid residue. Generally, the aspartic acid residue is Asp548 or Asp555. In certain embodiments, the aspartic acid residue is Asp555. In preferred embodiments, the pendant nitrogen atom of the 2-amino nitrogen moiety is less than about 3 Å from a carbonyl oxygen of an acidic side chain of the aspartic acid residue. Without wishing to be

bound by any particular theory, it is believed that the pendant nitrogen atom of the 2-amino nitrogen moiety forms a hydrogen bond and/or a salt bridge with the acidic side chain of the aspartic acid residue (e.g., Asp555). The hydrogen bond and/or salt bridge is believed to be crucial to maintaining the activity of the adjuvant moiety.

In some embodiments, the immunoconjugates of the invention comprise an adjuvant moiety with an adjuvant core comprising a point of attachment of a linker to the adjuvant core, wherein when bound to the binding domain of a TLR8 comprising an arginine and serine residue, the point of attachment of the linker to the adjuvant core is from about 3 Å to about 10 Å (e.g., from about 3 Å to about 9 Å, from about 3 Å to about 8 Å, from about 3 Å to about 7 Å, or from about 3 Å to about 6 Å) from an oxygen atom of a side chain of the serine residue and/or the point of attachment of the linker to the adjuvant core is from about 3 Å to about 10 Å (e.g., from about 3 Å to about 9 Å, from about 3 Å to about 8 Å, from about 3 Å to about 7 Å, or from about 3 Å to about 6 Å) from a nitrogen atom of a side chain of the arginine residue. In certain embodiments, the serine residue is Ser352 and the arginine residue is Arg429. In preferred embodiments, the point of attachment of the linker to the adjuvant core is less than about 7 Å from the oxygen atom of the side chain of the serine residue and the point of attachment of the linker to the adjuvant core is less than about 7 Å from the nitrogen atom of the side chain of the arginine residue. Without wishing to be bound by any particular theory, it is believed that the point of attachment of the linker to the adjuvant core must be close to the opening of the binding domain of the TLR8 to avoid steric and/or electronic interactions between the linker and the binding domain. Accordingly, the point of attachment of the linker to the adjuvant core must be close to the opening of the binding domain (e.g., Ser352 and Arg429).

In some embodiments, the immunoconjugates of the invention comprise an adjuvant moiety with an adjuvant core comprising a point of attachment of a linker to the adjuvant core, wherein when bound to the binding domain of a TLR7 comprising a lysine and valine residue, the point of attachment of the linker to the adjuvant core is from about 3 Å to about 10 Å (e.g., from about 3 Å to about 9 Å, from about 3 Å to about 8 Å, from about 3 Å to about 7 Å, or from about 3 Å to about 6 Å) from a methine carbon atom of a side chain of the valine residue and/or the point of attachment of the linker to the adjuvant core is from about 3 Å to about 10 Å (e.g., from about 3 Å to about 9 Å, from about 3 Å to about 8 Å, from about 3 Å to about 7 Å, or from about 3 Å to about 6 Å) from a nitrogen atom of a side chain of the lysine residue. In certain embodiments, the valine residue is Val355 and the lysine residue is Lys432. In preferred embodiments, the point of attachment of the linker to the adjuvant core is from about 3 Å to about 7 Å from the methine carbon atom of the side chain of the valine residue and the point of attachment of the linker to the adjuvant core is from about 3 Å to about 7 Å from the nitrogen atom of the side chain of the lysine residue. Without wishing to be bound by any particular theory, it is believed that the point of attachment of the linker to the adjuvant core must be close to the opening of the binding domain of the TLR7 to avoid steric and/or electronic interactions between the linker and the binding domain. Accordingly, the point of attachment of the linker to the adjuvant core must be close to the opening of the binding domain (e.g., Val355 and Lys432).

In preferred embodiments, when bound to the binding domain of a TLR8 comprising aspartic acid, arginine, and serine residues, the pendant nitrogen atom of the 2-amino nitrogen moiety is less than about 5 Å from the carbonyl oxygen of the acidic side chain of the aspartic acid residue and the point of attachment of the linker to the adjuvant core is from about 3 Å to about 10 Å from the oxygen atom of the side chain of the serine residue and/or the point of attachment of the linker to the adjuvant core from about 3 Å to about 10 Å from the nitrogen atom of the side chain of the arginine residue. In certain embodiments, the serine residue is Ser352, the arginine residue is Arg429, and the aspartic acid residue is Asp543.

In preferred embodiments, when bound to the binding domain of a TLR7 comprising aspartic acid, lysine, and valine residues, the pendant nitrogen atom of the 2-amino nitrogen moiety is less than about 5 Å from the carbonyl oxygen of the acidic side chain of the aspartic acid residue and the point of attachment of the linker to the adjuvant core is from about 3 Å to about 10 Å from the methine carbon atom of the side chain of the valine residue and/or the point of attachment of the linker to the adjuvant core from about 3 Å to about 10 Å from the nitrogen atom of the side chain of the lysine residue. In certain embodiments, the valine residue is Val355, the lysine residue is Lys432, and the aspartic acid residue is Asp555.

Without wishing to be bound by any particular theory, it is believed that the adjuvant core must be linked at a certain position to facilitate the orientation of the adjuvant moiety in the binding domain of the TLR such that the pendant nitrogen atom of the 2-amino nitrogen moiety maintains a hydrogen bond and/or salt bridge in the binding pocket. It is believed that when the point of attachment of the linker to the adjuvant core is not close to the opening of the binding domain, the adjuvant moiety is forced to shift, thereby disrupting the hydrogen bond and/or salt bridge of the 2-amino nitrogen moiety. See for example, Examples 1 and 2.

The distance between atoms can be measured by any suitable means. In some embodiments, the distance between atoms can be measured theoretically using PyMol v1.8.0.1 Enhanced for Mac OS X, Copyright © Schrodinger LLC. In some embodiments, the distance between atoms can be measured from a crystal structure of molecule, complex, or protein.

## Antibodies

The immunoconjugates of the invention comprise an antibody construct comprising (i) an antigen binding domain and (ii) an Fc domain. In some embodiments, the antibody construct further comprises a targeting binding domain. In

certain embodiments, the antibody construct is an antibody. In certain embodiments, the antibody construct is a fusion protein.

The antibodies in the immunoconjugates can be allogeneic antibodies. The phrase "allogeneic antibody" and term "alloantibody" refer to an antibody that is not from the individual in question (e.g., an individual with a tumor and seeking treatment), but is from the same species, or is from a different species, but has been engineered to reduce, mitigate, or avoid recognition as a xeno-antibody (e.g., non-self). For example, the "allogeneic antibody" can be a humanized antibody. One skilled in the art is knowledgeable regarding how to engineer a non-human antibody to avoid recognition as a xeno-antibody. Unless specifically stated otherwise, "antibody" and "allogeneic antibodies" as used herein refer to immunoglobulin G (IgG) or immunoglobulin A (IgA).

If a cancer cell of a human individual is contacted with an antibody that was not generated by that same person (e.g., the antibody was generated by a second human individual, the antibody was generated by another species such as a mouse, the antibody is a humanized antibody that was generated by another species, etc.), then the antibody is considered to be allogeneic (relative to the first individual). A humanized mouse monoclonal antibody that recognizes a human antigen (e.g., a cancer-specific antigen, an antigen that is enriched in and/or on cancer cells, etc.) is considered to be an "alloantibody" (an allogeneic antibody).

In some embodiments, the antibody is a polyclonal allogeneic IgG antibody. In some embodiments, the antibody is present in a mixture of polyclonal IgG antibodies with a plurality of binding specificities. In some embodiments, the antibodies of the mixture specifically bind to different target molecules, and in some cases the antibodies of the mixture specifically bind to different epitopes of the same target molecule. Thus, a mixture of antibodies can in some cases include more than one immunoconjugate of the invention (e.g., adjuvant moieties can be covalently bonded to antibodies of a mixture, e.g., a mixture of polyclonal IgG antibodies, resulting in a mixture of antibody-adjuvant conjugates of the invention). A mixture of antibodies can be pooled from 2 or more individuals (e.g., 3 or more individuals, 4 or more individuals, 5 or more individuals, 6 or more individuals, 7 or more individuals, 8 or more individuals, 9 or more individuals, 10 or more individuals, etc.). In some cases, pooled serum is used as a source of alloantibody, where the serum can come from any number of individuals, none of whom are the first individual (e.g., the serum can be pooled from 2 or more individuals, 3 or more individuals, 4 or more individuals, 5 or more individuals, 6 or more individuals, 7 or more individuals, 8 or more individuals, 9 or more individuals, 10 or more individuals, etc.). In some cases, the antibodies are isolated or purified from serum prior to use. The purification can be conducted before or after pooling the antibodies from different individuals.

In some cases where the antibodies in the immunoconjugates comprise IgGs from serum, the target antigens for some (e.g., greater than 0% but less than 50%), half, most (greater than 50% but less than 100%), or even all of the antibodies (i.e., IgGs from the serum) will be unknown. However, the chances are high that at least one antibody in the mixture will recognize the target antigen of interest because such a mixture contains a wide variety of antibodies specific for a wide variety of target antigens.

In some embodiments, the antibody is a polyclonal allogeneic IgA antibody. In some embodiments, the antibody is present in a mixture of polyclonal IgA antibodies with a plurality of binding specificities. In some cases, the antibodies of the mixture specifically bind to different target molecules, and in some cases the antibodies of the mixture specifically bind to different epitopes of the same target molecule. Thus, a mixture of antibodies can in some cases include more than one immunoconjugate of the invention (e.g., adjuvant moieties can be covalently bonded to antibodies of a mixture, e.g., a mixture of polyclonal IgA antibodies, resulting in a mixture of antibody-adjuvant conjugates of the invention). A mixture of antibodies can be pooled from 2 or more individuals (e.g., 3 or more individuals, 4 or more individuals, 5 or more individuals, 6 or more individuals, 7 or more individuals, 8 or more individuals, 9 or more individuals, 10 or more individuals, etc.). In some cases, pooled serum is used as a source of alloantibody, where the serum can come from any number of individuals, none of whom are the first individual (e.g., the serum can be pooled from 2 or more individuals, 3 or more individuals, 4 or more individuals, 5 or more individuals, 6 or more individuals, 7 or more individuals, 8 or more individuals, 9 or more individuals, 10 or more individuals, etc.). In some cases, the antibodies are isolated or purified from serum prior to use. The purification can be conducted before or after pooling the antibodies from different individuals.

In some cases where the antibodies in the immunoconjugates comprise IgAs from serum, the target antigens for some (e.g., greater than 0% but less than 50%), half, most (greater than 50% but less than 100%), or even all of the antibodies (i.e., IgAs from the serum) will be unknown. However, the chances are high that at least one antibody in the mixture will recognize the target antigen of interest because such a mixture contains a wide variety of antibodies specific for a wide variety of target antigens.

In some cases, the antibody in the immunoconjugates includes intravenous immunoglobulin (IVIG) and/or antibodies from (e.g., enriched from, purified from, e.g., affinity purified from) IVIG. IVIG is a blood product that contains IgG (immunoglobulin G) pooled from the plasma (e.g., in some cases without any other proteins) from many (e.g., sometimes over 1,000 to 60,000) normal and healthy blood donors. IVIG is commercially available. IVIG contains a high percentage of native human monomeric IVIG, and has low IgA content. When administered intravenously, IVIG ameliorates several disease conditions. Therefore, the United States Food and Drug Administration (FDA) has approved the use of IVIG for a

number of diseases including (1) Kawasaki disease; (2) immune-mediated thrombocytopenia; (3) primary immunodeficiencies; (4) hematopoietic stem cell transplantation (for those older than 20 years); (5) chronic B-cell lymphocytic leukemia; and (6) pediatric HIV type 1 infection. In 2004, the FDA approved the Cedars-Sinai IVIG Protocol for kidney transplant recipients so that such recipients could accept a living donor kidney from any healthy donor, regardless of blood type (ABO incompatible) or tissue match. These and other aspects of IVIG are described, for example, in U.S. Patent Application Publications 2010/0150942; 2004/0101909; 2013/0177574; 2013/0108619; and 2013/0011388.

In some cases, the antibody is a monoclonal antibody of a defined sub-class (e.g., $IgG_1$, $IgG_2$, $IgG_3$, $IgG_4$, $IgA_1$, or $IgA_2$). If combinations of antibodies are used, the antibodies can be from the same subclass or from different subclasses. For example, the antibodies can be $IgG_1$ antibodies. Various combinations of different subclasses, in different relative proportions, can be obtained by those of skill in the art. In some cases, a specific subclass, or a specific combination of different subclasses can be particularly effective at cancer treatment or tumor size reduction. Accordingly, some embodiments of the invention provide immunoconjugates wherein the antibody is a monoclonal antibody. In some embodiments, the monoclonal antibody is humanized.

In some embodiments, the antibody binds to an antigen of a cancer cell. For example, the antibody can bind to a target antigen that is present at an amount of at least 10; 100; 1,000; 10,000; 100,000; 1,000,000; $2.5 \times 10^6$; $5 \times 10^6$; or $1 \times 10^7$ copies or more on the surface of a cancer cell.

In some embodiments, the antibody binds to an antigen on a cancer or immune cell at a higher affinity than a corresponding antigen on a non-cancer cell. For example, the antibody may preferentially recognize an antigen containing a polymorphism that is found on a cancer or immune cell as compared to recognition of a corresponding wild-type antigen on the non-cancer or non-immune cell. In some cases, the antibody binds a cancer or immune cell with greater avidity than a non-cancer or non-immune cell. For example, the cancer or immune cell can express a higher density of an antigen, thus providing for a higher affinity binding of a multivalent antibody to the cancer or immune cell.

In some cases, the antibody does not significantly bind non-cancer antigens (e.g., the antibody binds one or more non-cancer antigens with at least 10; 100; 1,000; 10,000; 100,000; or 1,000,000-fold lower affinity (higher Kd) than the target cancer antigen). In some cases, the target cancer antigen to which the antibody binds is enriched on the cancer cell. For example, the target cancer antigen can be present on the surface of the cancer cell at a level that is at least 2, 5, 10; 100; 1,000; 10,000; 100,000; or 1,000,000-fold higher than a corresponding non-cancer cell. In some cases, the corresponding non-cancer cell is a cell of the same tissue or origin that is not hyperproliferative or otherwise cancerous. In general, a subject IgG antibody that specifically binds to an antigen (a target antigen) of a cancer cell preferentially binds to that particular antigen relative to other available antigens. However, the target antigen need not be specific to the cancer cell or even enriched in cancer cells relative to other cells (e.g., the target antigen can be expressed by other cells). Thus, in the phrase "an antibody that specifically binds to an antigen of a cancer cell," the term "specifically" refers to the specificity of the antibody and not to the uniqueness of the antigen in that particular cell type.

In some embodiments, the antibodies in the immunoconjugates contain a modified Fc region, wherein the modification modulates the binding of the Fc region to one or more Fc receptors.

In some embodiments, the antibodies contain one or more modifications (e.g., amino acid insertion, deletion, and/or substitution) in the Fc region that results in modulated binding (e.g., increased binding or decreased binding) to one or more Fc receptors (e.g., FcyRI (CD64), FcγRIIA (CD32A), FcγRIIB (CD32B), FcγRIIIA (CD16a), and/or FcyRIIIB (CD16b)) as compared to the native antibody lacking the mutation in the Fc region. In some embodiments, the antibodies contain one or more modifications (e.g., amino acid insertion, deletion, and/or substitution) in the Fc region that reduce the binding of the Fc region of the antibody to FcyRIIB. In some embodiments, the antibodies contain one or more modifications (e.g., amino acid insertion, deletion, and/or substitution) in the Fc region of the antibody that reduce the binding of the antibody to FcyRIIB while maintaining the same binding or having increased binding to FcγRI (CD64), FcγRIIA (CD32A), and/or FcRγIIIA (CD16a) as compared to the native antibody lacking the mutation in the Fc region. In some embodiments, the antibodies contain one of more modifications in the Fc region that increase the binding of the Fc region of the antibody to FcyRIIB. In some embodiments, the modifications substantially reduce or eliminate antibody effector functions.

In some embodiments, the modulated binding is provided by mutations in the Fc region of the antibody relative to the native Fc region of the antibody. The mutations can be in a CH2 domain, a CH3 domain, or a combination thereof. A "native Fc region" is synonymous with a "wild-type Fc region" and comprises an amino acid sequence that is identical to the amino acid sequence of an Fc region found in nature or identical to the amino acid sequence of the Fc region found in the native antibody. Native sequence human Fc regions include a native sequence human IgG1 Fc region, native sequence human IgG2 Fc region, native sequence human IgG3 Fc region, and native sequence human IgG4 Fc region, as well as naturally occurring variants thereof. Native sequence Fc includes the various allotypes of Fcs (see, e.g., Jefferis et al., mAbs, 1(4): 332-338 (2009)).

In some embodiments, the mutations in the Fc region that result in modulated binding to one or more Fc receptors can include one or more of the following mutations: SD (S239D), SDIE (S239D/I332E), SE (S267E), SELF (S267E/L328F), SDIE (S239D/I332E), SDIEAL (S239D/I332E/A330L), GA (G236A), ALIE (A330L/I332E), GASDALIE

(G236A/S239D/A330L/I332E), V9 (G237D/P238D/P271G/A330R), and V11 (G237D/P238D/H268D/P271G/A330R), and/or one or more mutations at the following amino acids: E233, G237, P238, H268, P271, L328 and A330. Additional Fc region modifications for modulating Fc receptor binding are described in, for example, U.S. Patent Application Publication 2016/0145350 and U.S. Patents 7,416,726 and 5,624,821.

In some embodiments, the Fc region of the antibodies are modified to have an altered glycosylation pattern of the Fc region compared to the native non-modified Fc region.

Human immunoglobulin is glycosylated at the Asn297 residue in the Cγ2 domain of each heavy chain. This *N*-linked oligosaccharide is composed of a core heptasaccharide, N-acetylglucosamine4Mannose3 (GlcNAc4Man3). Removal of the heptasaccharide with endoglycosidase or PNGase F is known to lead to conformational changes in the antibody Fc region, which can significantly reduce antibody-binding affinity to activating FcγR and lead to decreased effector function. The core heptasaccharide is often decorated with galactose, bisecting GlcNAc, fucose, or sialic acid, which differentially impacts Fc binding to activating and inhibitory FcyR. Additionally, it has been demonstrated that α2,6-sialyation enhances anti-inflammatory activity *in vivo*, while defucosylation leads to improved FcyRIIIa binding and a 10-fold increase in antibody-dependent cellular cytotoxicity and antibody-dependent phagocytosis. Specific glycosylation patterns, therefore, can be used to control inflammatory effector functions.

In some embodiments, the modification to alter the glycosylation pattern is a mutation. For example, a substitution at Asn297. In some embodiments, Asn297 is mutated to glutamine (N297Q). Methods for controlling immune response with antibodies that modulate FcyR-regulated signaling are described, for example, in U.S. Patent 7,416,726 and U.S. Patent Application Publications 2007/0014795 and 2008/0286819.

In some embodiments, the antibodies are modified to contain an engineered Fab region with a non-naturally occurring glycosylation pattern. For example, hybridomas can be genetically engineered to secrete afucosylated mAb, desialylated mAb or deglycosylated Fc with specific mutations that enable increased FcRyIIIa binding and effector function. In some embodiments, the antibodies are engineered to be afucosylated.

In some embodiments, the entire Fc region of an antibody is exchanged with a different Fc region, so that the Fab region of the antibody is conjugated to a non-native Fc region. For example, the Fab region of atezolizumab, which normally comprises an IgG1 Fc region, can be conjugated to IgG2, IgG3, IgG4, or IgA, or the Fab region of nivolumab, which normally comprises an IgG4 Fc region, can be conjugated to IgG1, IgG2, IgG3, IgA1, or IgG2. In some embodiments, the Fc modified antibody with a non-native Fc domain also comprises one or more amino acid modification, such as the S228P mutation within the IgG4 Fc, that modulate the stability of the Fc domain described. In some embodiments, the Fc modified antibody with a non-native Fc domain also comprises one or more amino acid modifications described herein that modulate Fc binding to FcR.

In some embodiments, the modifications that modulate the binding of the Fc region to FcR do not alter the binding of the Fab region of the antibody to its antigen when compared to the native non-modified antibody. In other embodiments, the modifications that modulate the binding of the Fc region to FcR also increase the binding of the Fab region of the antibody to its antigen when compared to the native non-modified antibody.

In some embodiments, the Fc region is modified by attachment or inclusion of a transforming growth factor beta 1 (TGFβ1) receptor, or a fragment thereof, that is capable of binding TGFβ1. For example, the receptor can be TGFβ receptor II (TGFβII) (see U.S. Patent 9,676,863. In some embodiments, the TGFβ receptor is a human TGFβ receptor. In some embodiments, the Fc region (e.g., IgG) has a C-terminal fusion to a TGFβ receptor (e.g., TGFβRII) extracellular domain (ECD; e.g., amino acids 24-159 of SEQ ID NO: 9 of U.S. Patent 9,676,863). An "Fc linker" may be used to attach the IgG to the TGFβR extracellular domain, for example, a $G_4S_4G$ Fc linker. The Fc linker may be a short, flexible peptide that allows for the proper three-dimensional folding of the molecule while maintaining the binding-specificity to the targets. In some embodiments, the N-terminus of the TGFβ receptor is fused to the Fc region (with or without an Fc linker). In some embodiments, the C-terminus of the immunoglobulin heavy chain is fused to the TGFβ receptor (with or without an Fc linker). In some embodiments, the C-terminal lysine residue of the antibody heavy chain is mutated to alanine. In some embodiments, the antibody includes SEQ ID NO: 168.

Targets

In some embodiments, the antigen binding domain or antibody is capable of binding one or more targets or antigens selected from (e.g., specifically binds to a target selected from) 5T4, ABL, ABCF1, ACVR1, ACVR1B, ACVR2, ACVR2B, ACVRL1, ADORA2A, AFP, Aggrecan, AGR2, AICDA, AIF1, AIGI, AKAP1, AKAP2, ALCAM, ALK, AMH, AMHR2, ANGPT1, ANGPT2, ANGPTL3, ANGPTL4, ANPEP, APC, APOCl, AR, aromatase, ASPH, ATX, AX1, AXL, AZGP1 (zinc-a-glycoprotein), B4GALNT1, B7, B7.1, B7.2, B7-H1, B7-H3, B7-H4, B7-H6, BAD, BAFF, BAG1, BAI1, BCR, BCL2, BCL6, BCMA, BDNF, BLNK, BLR1 (MDR15), BlyS, BMP1, BMP2, BMP3B (GDFIO), BMP4, BMP6, BMP8, BMP10, BMPR1A, BMPR1B, BMPR2, BPAG1 (plectin), BRCA1, C19orflO (IL27w), C3, C4A, C5, C5R1, CA6, CA9, CANT1, CAPRIN-1, CASP1, CASP4, CAV1, CCBP2 (D6/JAB61), CCL1 (1-309), CCLI1 (eotaxin), CCL13 (MCP-4), CCL15 (MIP-Id), CCL16 (HCC-4), CCL17 (TARC), CCL18 (PARC), CCL19 (MIP-3b), CCL2 (MCP-1), MCAF, CCL20 (MIP-3a), CCL21

(MEP-2), SLC, exodus-2, CCL22(MDC/STC-I), CCL23 (MPIF-I), CCL24 (MPIF-2/eotaxin-2), CCL25 (TECK), CCL26(eotaxin-3), CCL27 (CTACK/ILC), CCL28, CCL3 (MIP-Ia), CCL4 (MIPIb), CCL5(RANTES), CCL7 (MCP-3), CCL8 (mcp-2), CCNA1, CCNA2, CCND1, CCNE1, CCNE2, CCR1 (CKR1/HM145), CCR2 (mcp-IRB/RA), CCR3 (CKR3/CMKBR3), CCR4, CCR5(CMKBR5/ChemR13), CCR6 (CMKBR6/CKR-L3/STRL22/DRY6), CCR7 (CKR7/EBI1), CCR8 or CDw198 (CMKBR8/TERI/CKR-L 1), CCR9 (GPR-9-6), CCRL1 (VSHK1), CCRL2 (L-CCR), CD13, CD164, CD19, CDH6, CDIC, CD2, CD20, CD21, CD200, CD22, CD23, CD24, CD27, CD28, CD29, CD3, CD33, CD35, CD37, CD38, CD3E, CD3G, CD3Z, CD4, CD40, CD40L, CD44, CD45RB, CD47, CD52, CD56, CD69, CD70, CD72, CD74, CD79A, CD79B, CD8, CD80, CD81, CD83, CD86, CD97, CD99, CD117, CD125, CD137, CD147, CD179b, CD223, CD279, CD152, CD274, CDH1 (E-cadherin), CDH10, CDH12, CDH13, CDH18, CDH19, CDH2O, CDH3, CDH5, CDH7, CDH8, CDH9, CDH17, CDK2, CDK3, CDK4, CDK5, CDK6, CDK7, CDK9, CDKN1A (p21Wap1/Cip1), CDKN1B (p27Kip1), CDKN1C, CDKN2A (p16INK4a), CDKN2B, CDKN2C, CDKN3, CEA, CEACAM5, CEACAM6, CEBPB, CERI, CFC1B, CHGA, CHGB, Chitinase, CHST1O, CIK, CKLFSF2, CKLFSF3, CKLFSF4, CKLFSF5, CKLFSF6, CKLFSF7, CKLFSF8, CLDN3, CLDN6, CLDN7 (claudin-7), CLDN18, CLEC5A, CLEC6A, CLEC11A, CLEC14A, CLN3, CLU (clusterin), CMKLR1, CMKOR1 (RDC1), CNR1, C-MET, COL18A1, COLIA1, COL4A3, COL6A1, CR2, Cripto, CRP, CSF1 (M-CSF), CSF2 (GM-CSF), CSF3 (GCSF), CTAG1B (NY-ESO-1), CTLA4, CTL8, CTNNB1 (b-catenin), CTSB (cathepsin B), CX3CL1 (SCYD1), CX3CR1 (V28), CXCL1 (GRO1), CXCL1O (IP-IO), CXCLI1 (1-TAC/IP-9), CXCL12 (SDF1), CXCL13, CXCL14, CXCL16, CXCL2 (GRO2), CXCL3 (GRO3), CXCL5 (ENA-78/LIX), CXCL6 (GCP-2), CXCL9 (MIG), CXCR3 (GPR9/CKR-L2), CXCR4, CXCR6 (TYMSTR/STRL33Bonzo), CYB5, CYC1, CYSLTR1, DAB2IP, DES, DKFZp451J0118, DLK1, DNCL1, DPP4, E2F1, Engel, Edge, Fennel, EFNA3, EFNB2, EGF, EGFR, ELAC2, ENG, Enola, ENO2, ENO3, EpCAM, EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6, EPHRIN-A1, EPHRIN-A2, EPHRINA3, EPHRIN-A4, EPHRIN-A5, EPHRIN-A6, EPHRIN-B1, EPHRIN-B2, EPHRIN-B3, EPHB4, EPG, ERBB2 (HER-2), ERBB3, ERBB4, EREG, ERK8, Estrogen receptor, Earl, ESR2, F3 (TF), FADD, FAP, farnesyltransferase, FasL, FASNf, FCER1A, FCER2, FCGR3A, FGF, FGF1 (aFGF), FGF10, FGF1 1, FGF12, FGF12B, FGF13, FGF14, FGF16, FGF17, FGF18, FGF19, FGF2 (bFGF), FGF20, FGF21, FGF22, FGF23, FGF3 (int-2), FGF4 (HST), FGF5, FGF6 (HST-2), FGF7 (KGF), FGF8, FGF9, FGFR1, FGFR2, FGFR3, FGFR4, FIGF (VEGFD), FIL1(EPSILON), FBL1 (ZETA), FLJ12584, FLJ25530, FLRT1 (fibronectin), FLT1, FLT-3, FOLR1, FOS, FOSL1(FRA-1), FR-alpha, FY (DARC), GABRP (GABAa), GAGEB1, GAGEC1, GALNAC4S-6ST, GATA3, GD2, GD3, GDF5, GFI1, GFRA1, GGT1, GM-CSF, GNAS1, GNRH1, GPC1, GPC3, GPNB, GPR2 (CCR10), GPR31, GPR44, GPR81 (FKSG80), GRCC10 (C10), GRP, GSN (Gelsolin), GSTP1, GUCY2C, HAVCR1, HAVCR2, HDAC, HDAC4, HDAC5, HDAC7A, HDAC9, Hedgehog, HER3, HGF, HIF1A, HIP1, histamine and histamine receptors, HLA-A, HLA-DR, HLA-DRA, HLA-E, HM74, HMOXI, HSP90, HUMCYT2A, ICEBERG, ICOSL, ID2, IFN-a, IFNA1, IFNA2, IFNA4, IFNA5, EFNA6, BFNA7, IFNB1, IFNgamma, IFNW1, IGBP1, IGF1, IGFIR, IGF2, IGFBP2, IGFBP3, IGFBP6, DL-1, ILIO, ILIORA, ILIORB, IL-1, IL1R1 (CD121a), IL1R2(CD121b), IL-IRA, IL-2, IL2RA (CD25), IL2RB(CD122), IL2RG(CD132), IL-4, IL-4R(CD123), IL-5, IL5RA(CD125), IL3RB(CD131), IL-6, IL6RA, (CD126), IR6RB(CD130), IL-7, IL7RA(CD127), IL-8, CXCR1 (IL8RA), CXCR2, (IL8RB/CD128), IL-9, IL9R(CD129), IL-10, IL10RA(CD210), IL10RB(CDW210B), IL-11, IL11RA, IL-12, IL-12A, IL-12B, IL-12RB1, IL-12RB2, IL-13, IL13RA1, IL13RA2, IL14, IL15, IL15RA, IL16, IL17, IL17A, IL17B, IL17C, IL17R, IL18, IL18BP, IL18R1, IL18RAP, IL19, ILIA, ILIB, ILIF10, ILIF5, IL1F6, ILIF7, IL1F8, DL1F9, ILIHYI, ILIR1, IL1R2, ILIRAP, ILIRAPLI, ILIRAPL2, ILIRL1, II,1RL2, ILIRN, IL2, IL20, IL20RA, IL21R, IL22, IL22R, IL22RA2, IL23, DL24, IL25, IL26, IL27, IL28A, IL28B, IL29, IL2RA, IL2RB, IL2RG, IL3, IL30, IL3RA, IL4, 1L4, IL6ST (glycoprotein 130), ILK, INHA, INHBA, INSL3, INSL4, IRAK1, IRAK2, ITGA1, ITGA2, ITGA3, ITGA6 (α6 integrin), ITGAV, ITGB3, ITGB4 (β4 integrin), JAG1, JAK1, JAK3, JTB, JUN, K6HF, KAI1, KDR, KIT, KITLG, KLF5 (GC Box BP), KLF6, KLK10, KLK12, KLK13, KLK14, KLK15, KLK3, KLK4, KLK5, KLK6, KLK9, KRT1, KRT19 (Keratin 19), KRT2A, KRTHB6(hair-specific type II keratin), L1CAM, LAG3, LAMA5, LAMP1, LEP (leptin), Lewis Y antigen ("LeY"), LILRB1, Lingo-p75, Lingo-Troy, LGALS3BP, LRRC15, LPS, LTA (TNF-b), LTB, LTB4R (GPR16), LTB4R2, LTBR, LY75, LYPD3, MACMARCKS, MAG or OMgp, MAGEA3, MAGEA6, MAP2K7 (c-Jun), MCP-1, MDK, MIB1, midkine, MIF, MISRII, MJP-2, MLSN, MK, MKI67 (Ki-67), MMP2, MMP9, MS4A1, MSMB, MT3 (metallothionectin-UI), mTOR, MTSS1, MUC1 (mucin), MUC16, MYC, MYD88, NCK2, NCR3LG1, neurocan, NFKBI, NFKB2, NGFB (NGF), NGFR, NgR-Lingo, NgRNogo66, (Nogo), NgR-p75, NgR-Troy, NMEI (NM23A), NOTCH, NOTCH1, NOTCH3, NOX5, NPPB, NROB1, NROB2, NRID1, NR1D2, NR1H2, NR1H3, NR1H4, NR112, NR113, NR2C1, NR2C2, NR2E1, NR2E3, NR2F1, NR2F2, NR2F6, NR3C1, NR3C2, NR4A1, NR4A2, NR4A3, NR5A1, NR5A2, NR6A1, NRP1, NRP2, NT5E, NTN4, NY-ESO1, ODZI, OPRDI, P2RX7, PAP, PART1, PATE, PAWR, P-cadherin, PCA3, PCD1, PD-L1, PCDGF, PCNA, PDGFA, PDGFB, PDGFRA, PDGFRB, PECAMI, L1-CAM, peg-asparaginase, PF4 (CXCL4), PGF, PGR, phosphacan, PIAS2, PI3 Kinase, PIK3CG, PLAU (uPA), PLG, PLXDCI, PKC, PKC-beta, PPBP (CXCL7), PPID, PR1, PRAME, PRKCQ, PRKD1, PRL, PROC, PROK2, PSAP, PSCA, PSMA, PTAFR, PTEN, PTHR2, PTGS2 (COX-2), PTN, PVRIG, RAC2 (P21Rac2), RANK, RANK ligand, RARB, RGS1, RGS13, RGS3, RNFI10 (ZNF144), Ron, ROBO2, ROR1, RXR, S100A2, SCGB 1D2 (lipophilin B), SCGB2A1 (mammaglobin 2), SCGB2A2 (mammaglobin 1), SCYE1 (endothelial Monocyteactivating cytokine), SDF2, SERPENA1, SERPINA3, SERPINB5 (maspin), SERPINEI (PAI-I), SERPINFI, SHIP-1, SHIP-2, SHB1, SHB2, SHBG, SfcAZ, SLAMF7, SLC2A2, SLC33A1, SLC43A1, SLC44A4, SLC34A2, SLIT2, SPP1, SPRR1B (Spr1), ST6GAL1, ST8SIA1, STAB1,

STATE, STEAP, STEAP2, TB4R2, TBX21, TCP1O, TDGF1, TEK, TGFA, TGFB1, TGFB1I1, TGFB2, TGFB3, TGFBI, TGFBR1, TGFBR2, TGFBR3, THIL, THBS1 (thrombospondin-1), THBS2, THBS4, THPO, TIE (Tie-1), TIMP3, tissue factor, TLR1, TLR2, TLR3, TLR4, TLR5, TLR6, TLR7, TLR8, TLR9, TLR10, TLR11, TNF, TNF-a, TNFAIP2 (B94), TNFAIP3, TNFRSFI1A, TNFRSF1A, TNFRSF1B, TNFRSF21, TNFRSF5, TNFRSF6 (Fas), TNFRSF7, TNFRSF8, TNFRSF9, TNFSF10 (TRAIL), TNFRSF10A, TNFRSF10B, TNFRSF12A, TNFRSF17, TNFSF1 1 (TRANCE), TNFSF12 (APO3L), TNFSF13 (April), TNFSF13B, TNFSF14 (HVEM-L), TNFRSF14 (HVEM), TNFSF15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll-like receptors, TOP2A (topoisomerase Iia), TP53, TPM1, TPM2, TRADD, TRAF1, TRAF2, TRAF3, TRAF4, TRAF5, TRAF6, TRKA, TREM1, TREM2, TROP2, TRPC6, TSLP, TWEAK, Tyrosinase, uPAR, VEGF, VEGFB, VEGFC, versican, VHL C5, VLA-4, WT1, Wnt-1, XCL1 (lymphotactin), XCL2 (SCM-Ib), XCRI (GPR5/CCXCR1), YY1, ZFPM2, CLEC4C (BDCA-2, DLEC, CD303, CDH6, CLECSF7), CLEC4D (MCL, CLECSF8), CLEC4E (Mincle), CLEC6A (Dectin-2), CLEC5A (MDL-1, CLECSF5), CLEC1B (CLEC-2), CLEC9A (DNGR-1), CLEC7A (Dectin-1), CLEC11A, PDGFRa, SLAMF7, GP6 (GPVI), LILRA1 (CD85I), LILRA2 (CD85H, ILT1), LILRA4 (CD85G, ILT7), LILRA5 (CD85F, ILT11), LILRA6 (CD85b, ILT8), LILRB1, NCR1 (CD335, LY94, NKp46), NCR3 (CD335, LY94, NKp46), NCR3 (CD337, NKp30), OSCAR, TARM1, CD30, CD300C, CD300E, CD300LB (CD300B), CD300LD (CD300D), KIR2DL4 (CD158D), KIR2DS, KLRC2 (CD159C, NKG2C), KLRK1 (CD314, NKG2D), NCR2 (CD336, NKp44), PILRB, SIGLEC1 (CD169, SN), SIGLEC5, SIGLEC6, SIGLEC7, SIGLEC8, SIGLEC9, SIGLEC10, SIGLEC11, SIGLEC12, SIGLEC14, SIGLEC15 (CD33L3), SIGLEC16, SIRPA, SIRPB1 (CD172B), TREM1 (CD354), TREM2, KLRF1 (NKp80), 17-1A, SLAM7, MSLN, CTAG1B/NY-ESO-1, MAGEA3/A6, ATP5I (Q06185), OAT (P29758), AIFM1 (Q9Z0X1), AOFA (Q64133), MTDC (P18155), CMC1 (Q8BH59), PREP (Q8K411), YMEL1 (O88967), LPPRC (Q6PB66), LONM (Q8CGK3), ACON (Q99KI0), ODO1 (Q60597), IDHP (P54071), ALDH2 (P47738), ATPB (P56480), AATM (P05202), TMM93 (Q9CQW0), ERGI3 (Q9CQE7), RTN4 (Q99P72), CL041 (Q8BQR4), ERLN2 (Q8BFZ9), TERA (Q01853), DAD1 (P61804), CALX (P35564), CALU (O35887), VAPA (Q9WV55), MOGS (Q80UM7), GANAB (Q8BHN3), ERO1A (Q8R180), UGGG1 (Q6P5E4), P4HA1 (Q60715), HYEP (Q9D379), CALR (P14211), AT2A2 (055143), PDIA4 (P08003), PDIA1 (P09103), PDIA3 (P27773), PDIA6 (Q922R8), CLH (Q68FD5), PPIB (P24369), TCPG (P80318), MOT4 (P57787), NICA (P57716), BASI (P18572), VAPA (Q9WV55), ENV2 (P11370), VAT1 (Q62465), 4F2 (P10852), ENOA (P17182), ILK (O55222), GPNMB (Q99P91), ENV1 (P10404), ERO1A (Q8R180), CLH (Q68FD5), DSG1A (Q61495), AT1A1 (Q8VDN2), HYOU1 (Q9JKR6), TRAP1 (Q9CQN1), GRP75 (P38647), ENPL (P08113), CH60 (P63038), and CH10 (Q64433). In the preceding list, accession numbers are shown in parentheses.

In some embodiments, the antibody is selected from the group consisting of an anti-PD-L1 antibody, an anti-HER2 antibody, ananti-EGFR antibody, and an anti-CEA antibody.

An embodiment of the invention provides antibody construct or antigen binding domain which specifically recognizes and binds to PD-L1 (SEQ ID NO: 1). The antibody construct or antigen binding domain may comprise one or more variable regions (e.g., two variable regions) of an antigen binding domain of an anti-PD-L1 antibody, each variable region comprising a CDR1, a CDR2, and a CDR3.

An embodiment of the invention provides an antibody construct or antigen binding domain comprising the CDR regions of atezolizumab. In this regard, the antibody construct or antigen binding domain may comprise a first variable region comprising a CDR1 comprising the amino acid sequence of SEQ ID NO: 2 (CDR1 of first variable region), a CDR2 comprising the amino acid sequence of SEQ ID NO: 3 (CDR2 of first variable region), and a CDR3 comprising the amino acid sequence of SEQ ID NO: 4 (CDR3 of first variable region), and a second variable region comprising a CDR1 comprising the amino acid sequence of SEQ ID NO: 5 (CDR1 of second variable region), a CDR2 comprising the amino acid sequence of SEQ ID NO: 6 (CDR2 of second variable region), and a CDR3 comprising the amino acid sequence of SEQ ID NO: 7 (CDR3 of second variable region). In this regard, the antibody construct can comprise (i) all of SEQ ID NOs: 2-4, (ii) all of SEQ ID NOs: 5-7, or (iii) all of SEQ ID NOs: 2-7. Preferably, the antibody construct or antigen binding domain comprises all of SEQ ID NOs: 2-7.

In an embodiment of the invention, the antibody construct or antigen binding domain comprising the CDR regions of atezolizumab further comprises the framework regions of the atezolizumab. In this regard, the antibody construct or antigen binding domain comprising the CDR regions of the atezolizumab further comprises the amino acid sequence of SEQ ID NO: 8 (framework region ("FR") 1 of first variable region), the amino acid sequence of SEQ ID NO: 9 (FR2 of first variable region), the amino acid sequence of SEQ ID NO: 10 (FR3 of first variable region), the amino acid sequence of SEQ ID NO: 11 (FR4 of first variable region), the amino acid sequence of SEQ ID NO: 12 (FR1 of second variable region), the amino acid sequence of SEQ ID NO: 13 (FR2 of second variable region), the amino acid sequence of SEQ ID NO: 14 (FR3 of second variable region), and the amino acid sequence of SEQ ID NO: 15 (FR4 of second variable region). In this regard, the antibody construct or antigen binding domain can comprise (i) all of SEQ ID NOs: 2-4 and 8-11, (ii) all of SEQ ID NOs: 5-7 and 12-15; or (iii) all of SEQ ID NOs: 2-7 and 8-15.

An embodiment of the invention provides an antibody construct or antigen binding domain comprising one or both variable regions of atezolizumab. In this regard, the first variable region may comprise SEQ ID NO: 44. The second variable region may comprise SEQ ID NO: 45. Accordingly, in an embodiment of the invention, the antibody construct or antigen

binding domain comprises SEQ ID NO: 44, SEQ ID NO: 45, or both SEQ ID NOs: 44 and 45. Preferably, the polypeptide comprises both of SEQ ID NOs: 44-45.

An embodiment of the invention provides an antibody construct or antigen binding domain comprising the CDR regions of durvalumab. In this regard, the antibody construct or antigen binding domain may comprise a first variable region comprising a CDR1 comprising the amino acid sequence of SEQ ID NO: 18 (CDR1 of first variable region), a CDR2 comprising the amino acid sequence of SEQ ID NO: 19 (CDR2 of first variable region), and a CDR3 comprising the amino acid sequence of SEQ ID NO: 20 (CDR3 of first variable region), and a second variable region comprising a CDR1 comprising the amino acid sequence of SEQ ID NO: 21 (CDR1 of second variable region), a CDR2 comprising the amino acid sequence of SEQ ID NO: 22 (CDR2 of second variable region), and a CDR3 comprising the amino acid sequence of SEQ ID NO: 23 (CDR3 of second variable region). In this regard, the antibody construct can comprise (i) all of SEQ ID NOs: 18-20, (ii) all of SEQ ID NOs: 21-23, or (iii) all of SEQ ID NOs: 18-23. Preferably, the antibody construct or antigen binding domain comprises all of SEQ ID NOs: 18-23.

In an embodiment of the invention, the antibody construct or antigen binding domain comprising the CDR regions of durvalumab further comprises the framework regions of the durvalumab. In this regard, the antibody construct or antigen binding domain comprising the CDR regions of the durvalumab further comprises the amino acid sequence of SEQ ID NO: 24 (framework region ("FR") 1 of first variable region), the amino acid sequence of SEQ ID NO: 25 (FR2 of first variable region), the amino acid sequence of SEQ ID NO: 26 (FR3 of first variable region), the amino acid sequence of SEQ ID NO: 27 (FR4 of first variable region), the amino acid sequence of SEQ ID NO: 28 (FR1 of second variable region), the amino acid sequence of SEQ ID NO: 29 (FR2 of second variable region), the amino acid sequence of SEQ ID NO: 30 (FR3 of second variable region), and the amino acid sequence of SEQ ID NO: 31 (FR4 of second variable region). In this regard, the antibody construct or antigen binding domain can comprise (i) all of SEQ ID NOs: 18-20 and 24-26, (ii) all of SEQ ID NOs: 21-23 and 27-31; or (iii) all of SEQ ID NOs: 18-21 and 24-31.

An embodiment of the invention provides an antibody construct or antigen binding domain comprising one or both variable regions of durvalumab. In this regard, the first variable region may comprise SEQ ID NO: 46. The second variable region may comprise SEQ ID NO: 47. Accordingly, in an embodiment of the invention, the antibody construct or antigen binding domain comprises SEQ ID NO: 46, SEQ ID NO: 47, or both SEQ ID NOs: 46 and 47. Preferably, the polypeptide comprises both of SEQ ID NOs: 46-47.

An embodiment of the invention provides an antibody construct or antigen binding domain comprising the CDR regions of avelumab. In this regard, the antibody construct or antigen binding domain may comprise a first variable region comprising a CDR1 comprising the amino acid sequence of SEQ ID NO: 30 (CDR1 of first variable region), a CDR2 comprising the amino acid sequence of SEQ ID NO: 31 (CDR2 of first variable region), and a CDR3 comprising the amino acid sequence of SEQ ID NO: 32 (CDR3 of first variable region), and a second variable region comprising a CDR1 comprising the amino acid sequence of SEQ ID NO: 33 (CDR1 of second variable region), a CDR2 comprising the amino acid sequence of SEQ ID NO: 34 (CDR2 of second variable region), and a CDR3 comprising the amino acid sequence of SEQ ID NO: 35 (CDR3 of second variable region). In this regard, the antibody construct can comprise (i) all of SEQ ID NOs: 30-32, (ii) all of SEQ ID NOs: 33-35, or (iii) all of SEQ ID NOs: 30-35. Preferably, the antibody construct or antigen binding domain comprises all of SEQ ID NOs: 30-35.

In an embodiment of the invention, the antibody construct or antigen binding domain comprising the CDR regions of avelumab further comprises the framework regions of the avelumab. In this regard, the antibody construct or antigen binding domain comprising the CDR regions of the avelumab further comprises the amino acid sequence of SEQ ID NO: 36 (framework region ("FR") 1 of first variable region), the amino acid sequence of SEQ ID NO: 37 (FR2 of first variable region), the amino acid sequence of SEQ ID NO: 38 (FR3 of first variable region), the amino acid sequence of SEQ ID NO: 39 (FR4 of first variable region), the amino acid sequence of SEQ ID NO: 40 (FR1 of second variable region), the amino acid sequence of SEQ ID NO: 41 (FR2 of second variable region), the amino acid sequence of SEQ ID NO: 42 (FR3 of second variable region), and the amino acid sequence of SEQ ID NO: 43 (FR4 of second variable region). In this regard, the antibody construct or antigen binding domain can comprise (i) all of SEQ ID NOs: 30-32 and 36-39, (ii) all of SEQ ID NOs: 33-35 and 40-43; or (iii) all of SEQ ID NOs: 30-35 and 36-43.

An embodiment of the invention provides an antibody construct or antigen binding domain comprising one or both variable regions of avelumab. In this regard, the first variable region may comprise SEQ ID NO: 48. The second variable region may comprise SEQ ID NO: 49. Accordingly, in an embodiment of the invention, the antibody construct or antigen binding domain comprises SEQ ID NO: 48, SEQ ID NO: 49, or both SEQ ID NOs: 48 and 49. Preferably, the polypeptide comprises both of SEQ ID NOs: 48-49.

An embodiment of the invention provides antibody construct or antigen binding domain which specifically recognizes and binds to HER2 (SEQ ID NO: 50). The antibody construct or antigen binding domain may comprise one or more variable regions (e.g., two variable regions) of an antigen binding domain of an anti-HER2 antibody, each variable region comprising a CDR1, a CDR2, and a CDR3.

An embodiment of the invention provides an antibody construct or antigen binding domain comprising the CDR regions of trastuzumab. In this regard, the antibody construct or antigen binding domain may comprise a first variable

region comprising a CDR1 comprising the amino acid sequence of SEQ ID NO: 51 (CDR1 of first variable region), a CDR2 comprising the amino acid sequence of SEQ ID NO: 52 (CDR2 of first variable region), and a CDR3 comprising the amino acid sequence of SEQ ID NO: 53 (CDR3 of first variable region), and a second variable region comprising a CDR1 comprising the amino acid sequence of SEQ ID NO: 54 (CDR1 of second variable region), a CDR2 comprising the amino acid sequence of SEQ ID NO: 55 (CDR2 of second variable region), and a CDR3 comprising the amino acid sequence of SEQ ID NO: 56 (CDR3 of second variable region). In this regard, the antibody construct can comprise (i) all of SEQ ID NOs: 51-53, (ii) all of SEQ ID NOs: 54-56, or (iii) all of SEQ ID NOs: 51-56. Preferably, the antibody construct or antigen binding domain comprises all of SEQ ID NOs: 51-56.

In an embodiment of the invention, the antibody construct or antigen binding domain comprising the CDR regions of trastuzumab further comprises the framework regions of the trastuzumab. In this regard, the antibody construct or antigen binding domain comprising the CDR regions of the trastuzumab further comprises the amino acid sequence of SEQ ID NO: 57 (framework region ("FR") 1 of first variable region), the amino acid sequence of SEQ ID NO: 58 (FR2 of first variable region), the amino acid sequence of SEQ ID NO: 59 (FR3 of first variable region), the amino acid sequence of SEQ ID NO: 60 (FR4 of first variable region), the amino acid sequence of SEQ ID NO: 61 (FR1 of second variable region), the amino acid sequence of SEQ ID NO: 62 (FR2 of second variable region), the amino acid sequence of SEQ ID NO: 63 (FR3 of second variable region), and the amino acid sequence of SEQ ID NO: 64 (FR4 of second variable region). In this regard, the antibody construct or antigen binding domain can comprise (i) all of SEQ ID NOs: 51-53 and 57-60, (ii) all of SEQ ID NOs: 54-56 and 61-64; or (iii) all of SEQ ID NOs: 57-59 and 65-68.

An embodiment of the invention provides an antibody construct or antigen binding domain comprising one or both variable regions of trastuzumab. In this regard, the first variable region may comprise SEQ ID NO: 65. The second variable region may comprise SEQ ID NO: 66. Accordingly, in an embodiment of the invention, the antibody construct or antigen binding domain comprises SEQ ID NO: 65, SEQ ID NO: 66, or both SEQ ID NOs: 65 and 66. Preferably, the polypeptide comprises both of SEQ ID NOs: 65-66.

An embodiment of the invention provides an antibody construct or antigen binding domain comprising the CDR regions of pertuzumab. In this regard, the antibody construct or antigen binding domain may comprise a first variable region comprising a CDR1 comprising the amino acid sequence of SEQ ID NO: 67 (CDR1 of first variable region), a CDR2 comprising the amino acid sequence of SEQ ID NO: 68 (CDR2 of first variable region), and a CDR3 comprising the amino acid sequence of SEQ ID NO: 69 (CDR3 of first variable region), and a second variable region comprising a CDR1 comprising the amino acid sequence of SEQ ID NO: 70 (CDR1 of second variable region), a CDR2 comprising the amino acid sequence of SEQ ID NO: 71 (CDR2 of second variable region), and a CDR3 comprising the amino acid sequence of SEQ ID NO: 72 (CDR3 of second variable region). In this regard, the antibody construct can comprise (i) all of SEQ ID NOs: 67-69, (ii) all of SEQ ID NOs: 70-72, or (iii) all of SEQ ID NOs: 67-72. Preferably, the antibody construct or antigen binding domain comprises all of SEQ ID NOs: 67-72.

In an embodiment of the invention, the antibody construct or antigen binding domain comprising the CDR regions of pertuzumab further comprises the framework regions of the pertuzumab. In this regard, the antibody construct or antigen binding domain comprising the CDR regions of the pertuzumab further comprises the amino acid sequence of SEQ ID NO: 73 (framework region ("FR") 1 of first variable region), the amino acid sequence of SEQ ID NO: 74 (FR2 of first variable region), the amino acid sequence of SEQ ID NO: 75 (FR3 of first variable region), the amino acid sequence of SEQ ID NO: 76 (FR4 of first variable region), the amino acid sequence of SEQ ID NO: 77 (FR1 of second variable region), the amino acid sequence of SEQ ID NO: 78 (FR2 of second variable region), the amino acid sequence of SEQ ID NO: 79 (FR3 of second variable region), and the amino acid sequence of SEQ ID NO: 80 (FR4 of second variable region). In this regard, the antibody construct or antigen binding domain can comprise (i) all of SEQ ID NOs: 67-69 and 73-76, (ii) all of SEQ ID NOs: 70-72 and 77-80; or (iii) all of SEQ ID NOs: 67-72 and 73-80.

An embodiment of the invention provides an antibody construct or antigen binding domain comprising one or both variable regions of pertuzumab. In this regard, the first variable region may comprise SEQ ID NO: 81. The second variable region may comprise SEQ ID NO: 82. Accordingly, in an embodiment of the invention, the antibody construct or antigen binding domain comprises SEQ ID NO: 81, SEQ ID NO: 82, or both SEQ ID NOs: 81 and 82. Preferably, the polypeptide comprises both of SEQ ID NOs: 81-82.

An embodiment of the invention provides antibody construct or antigen binding domain which specifically recognizes and binds to CEA (SEQ ID NO: 83). The antibody construct or antigen binding domain may comprise one or more variable regions (e.g., two variable regions) of an antigen binding domain of an anti-CEA antibody, each variable region comprising a CDR1, a CDR2, and a CDR3.

An embodiment of the invention provides an antibody construct or antigen binding domain comprising the CDR regions of labetuzumab. In this regard, the antibody construct or antigen binding domain may comprise a first variable region comprising a CDR1 comprising the amino acid sequence of SEQ ID NO: 84 (CDR1 of first variable region), a CDR2 comprising the amino acid sequence of SEQ ID NO: 85 (CDR2 of first variable region), and a CDR3 comprising the amino acid sequence of SEQ ID NO: 86 (CDR3 of first variable region), and a second variable region comprising a CDR1 comprising the amino acid sequence of SEQ ID NO: 87 (CDR1 of second variable region), a CDR2 comprising the amino

acid sequence of SEQ ID NO: 88 (CDR2 of second variable region), and a CDR3 comprising the amino acid sequence of SEQ ID NO: 89 (CDR3 of second variable region). In this regard, the antibody construct can comprise (i) all of SEQ ID NOs: 84-86, (ii) all of SEQ ID NOs: 87-89, or (iii) all of SEQ ID NOs: 84-89. Preferably, the antibody construct or antigen binding domain comprises all of SEQ ID NOs: 84-89.

In an embodiment of the invention, the antibody construct or antigen binding domain comprising the CDR regions of labetuzumab further comprises the framework regions of the labetuzumab. In this regard, the antibody construct or antigen binding domain comprising the CDR regions of the labetuzumab further comprises the amino acid sequence of SEQ ID NO: 90 (framework region ("FR") 1 of first variable region), the amino acid sequence of SEQ ID NO: 91 (FR2 of first variable region), the amino acid sequence of SEQ ID NO: 92 (FR3 of first variable region), the amino acid sequence of SEQ ID NO: 93 (FR4 of first variable region), the amino acid sequence of SEQ ID NO: 94 (FR1 of second variable region), the amino acid sequence of SEQ ID NO: 95 (FR2 of second variable region), the amino acid sequence of SEQ ID NO: 96 (FR3 of second variable region), and the amino acid sequence of SEQ ID NO: 97 (FR4 of second variable region). In this regard, the antibody construct or antigen binding domain can comprise (i) all of SEQ ID NOs: 84-86and 90-93, (ii) all of SEQ ID NOs: 87-89and 94-97; or (iii) all of SEQ ID NOs: 84--89 and 90-97.

An embodiment of the invention provides an antibody construct or antigen binding domain comprising one or both variable regions of labetuzumab. In this regard, the first variable region may comprise SEQ ID NO: 98. The second variable region may comprise SEQ ID NO: 99. Accordingly, in an embodiment of the invention, the antibody construct or antigen binding domain comprises SEQ ID NO: 98, SEQ ID NO: 99, or both SEQ ID NOs: 98 and 99. Preferably, the polypeptide comprises both of SEQ ID NOs: 98-99.

An embodiment of the invention provides an antibody construct or antigen binding domain comprising the CDR regions of PR1A3. In this regard, the antibody construct or antigen binding domain may comprise a first variable region comprising a CDR1 comprising the amino acid sequence of SEQ ID NO: 100 (CDR1 of first variable region), a CDR2 comprising the amino acid sequence of SEQ ID NO: 101 (CDR2 of first variable region), and a CDR3 comprising the amino acid sequence of SEQ ID NO: 102 (CDR3 of first variable region), and a second variable region comprising a CDR1 comprising the amino acid sequence of SEQ ID NO: 103 (CDR1 of second variable region), a CDR2 comprising the amino acid sequence of SEQ ID NO: 104 (CDR2 of second variable region), and a CDR3 comprising the amino acid sequence of SEQ ID NO: 105 (CDR3 of second variable region). In this regard, the antibody construct can comprise (i) all of SEQ ID NOs: 100-102, (ii) all of SEQ ID NOs: 103-105, or (iii) all of SEQ ID NOs: 100-105. Preferably, the antibody construct or antigen binding domain comprises all of SEQ ID NOs: 100-105.

In an embodiment of the invention, the antibody construct or antigen binding domain comprising the CDR regions of PR1A3 further comprises the framework regions of the PR1A3. In this regard, the antibody construct or antigen binding domain comprising the CDR regions of the PR1A3 further comprises the amino acid sequence of SEQ ID NO: 106 (framework region ("FR") 1 of first variable region), the amino acid sequence of SEQ ID NO: 107 (FR2 of first variable region), the amino acid sequence of SEQ ID NO: 108 (FR3 of first variable region), the amino acid sequence of SEQ ID NO: 109 (FR4 of first variable region), the amino acid sequence of SEQ ID NO: 110 (FR1 of second variable region), the amino acid sequence of SEQ ID NO: 111 (FR2 of second variable region), the amino acid sequence of SEQ ID NO: 112 (FR3 of second variable region), and the amino acid sequence of SEQ ID NO: 113 (FR4 of second variable region). In this regard, the antibody construct or antigen binding domain can comprise (i) all of SEQ ID NOs: 100-102 and 106-109, (ii) all of SEQ ID NOs: 103-105 and 110-113; or (iii) all of SEQ ID NOs: 100-103 and 106-113.

An embodiment of the invention provides an antibody construct or antigen binding domain comprising one or both variable regions of PR1A3. In this regard, the first variable region may comprise SEQ ID NO: 114. The second variable region may comprise SEQ ID NO: 115. Accordingly, in an embodiment of the invention, the antibody construct or antigen binding domain comprises SEQ ID NO: 114, SEQ ID NO: 115, or both SEQ ID NOs: 114 and 115. Preferably, the polypeptide comprises both of SEQ ID NOs: 114-115.

An embodiment of the invention provides an antibody construct or antigen binding domain comprising the CDR regions of MFE-23. In this regard, the antibody construct or antigen binding domain may comprise a first variable region comprising a CDR1 comprising the amino acid sequence of SEQ ID NO: 116 (CDR1 of first variable region), a CDR2 comprising the amino acid sequence of SEQ ID NO: 117 (CDR2 of first variable region), and a CDR3 comprising the amino acid sequence of SEQ ID NO: 118 (CDR3 of first variable region), and a second variable region comprising a CDR1 comprising the amino acid sequence of SEQ ID NO: 119 (CDR1 of second variable region), a CDR2 comprising the amino acid sequence of SEQ ID NO: 120 (CDR2 of second variable region), and a CDR3 comprising the amino acid sequence of SEQ ID NO: 121 (CDR3 of second variable region). In this regard, the antibody construct can comprise (i) all of SEQ ID NOs: 116118, (ii) all of SEQ ID NOs: 119-121, or (iii) all of SEQ ID NOs: 116-121. Preferably, the antibody construct or antigen binding domain comprises all of SEQ ID NOs: 116-121.

In an embodiment of the invention, the antibody construct or antigen binding domain comprising the CDR regions of MFE-23 further comprises the framework regions of the MFE-23. In this regard, the antibody construct or antigen binding domain comprising the CDR regions of the MFE-23 further comprises the amino acid sequence of SEQ ID NO: 122 (framework region ("FR") 1 of first variable region), the amino acid sequence of SEQ ID NO: 123 (FR2 of first variable

region), the amino acid sequence of SEQ ID NO: 124 (FR3 of first variable region), the amino acid sequence of SEQ ID NO: 125 (FR4 of first variable region), the amino acid sequence of SEQ ID NO: 126 (FR1 of second variable region), the amino acid sequence of SEQ ID NO: 127 (FR2 of second variable region), the amino acid sequence of SEQ ID NO: 128 (FR3 of second variable region), and the amino acid sequence of SEQ ID NO: 129 (FR4 of second variable region). In this regard, the antibody construct or antigen binding domain can comprise (i) all of SEQ ID NOs: 116-118 and 122-125, (ii) all of SEQ ID NOs: 119-121 and 126-129; or (iii) all of SEQ ID NOs: 116-121 and 122-129.

An embodiment of the invention provides an antibody construct or antigen binding domain comprising one or both variable regions of MFE-23. In this regard, the first variable region may comprise SEQ ID NO: 130. The second variable region may comprise SEQ ID NO: 131. Accordingly, in an embodiment of the invention, the antibody construct or antigen binding domain comprises SEQ ID NO: 130, SEQ ID NO: 131, or both SEQ ID NOs: 130 and 131. Preferably, the polypeptide comprises both of SEQ ID NOs: 130-131.

An embodiment of the invention provides an antibody construct or antigen binding domain comprising the CDR regions of SM3E. In this regard, the antibody construct or antigen binding domain may comprise a first variable region comprising a CDR1 comprising the amino acid sequence of SEQ ID NO: 132 (CDR1 of first variable region), a CDR2 comprising the amino acid sequence of SEQ ID NO: 133 (CDR2 of first variable region), and a CDR3 comprising the amino acid sequence of SEQ ID NO: 134 (CDR3 of first variable region), and a second variable region comprising a CDR1 comprising the amino acid sequence of SEQ ID NO: 135 (CDR1 of second variable region), a CDR2 comprising the amino acid sequence of SEQ ID NO: 136 (CDR2 of second variable region), and a CDR3 comprising the amino acid sequence of SEQ ID NO: 137 (CDR3 of second variable region). In this regard, the antibody construct can comprise (i) all of SEQ ID NOs: 132-134, (ii) all of SEQ ID NOs: 135-137, or (iii) all of SEQ ID NOs: 132-137. Preferably, the antibody construct or antigen binding domain comprises all of SEQ ID NOs: 132-137.

In an embodiment of the invention, the antibody construct or antigen binding domain comprising the CDR regions of SM3E further comprises the framework regions of the SM3E. In this regard, the antibody construct or antigen binding domain comprising the CDR regions of the SM3E further comprises the amino acid sequence of SEQ ID NO: 138 (framework region ("FR") 1 of first variable region), the amino acid sequence of SEQ ID NO: 139 (FR2 of first variable region), the amino acid sequence of SEQ ID NO: 140 (FR3 of first variable region), the amino acid sequence of SEQ ID NO: 141 (FR4 of first variable region), the amino acid sequence of SEQ ID NO: 142 (FR1 of second variable region), the amino acid sequence of SEQ ID NO: 143 (FR2 of second variable region), the amino acid sequence of SEQ ID NO: 144 (FR3 of second variable region), and the amino acid sequence of SEQ ID NO: 145 (FR4 of second variable region). In this regard, the antibody construct or antigen binding domain can comprise (i) all of SEQ ID NOs: 132-134and 138-53, (ii) all of SEQ ID NOs: 135-137 and 142-144; or (iii) all of SEQ ID NOs: 132-137 and 138-144.

An embodiment of the invention provides an antibody construct or antigen binding domain comprising one or both variable regions of SM3E. In this regard, the first variable region may comprise SEQ ID NO: 146. The second variable region may comprise SEQ ID NO: 147. Accordingly, in an embodiment of the invention, the antibody construct or antigen binding domain comprises SEQ ID NO: 146, SEQ ID NO: 147, or both SEQ ID NOs: 146 and 147. Preferably, the polypeptide comprises both of SEQ ID NOs: 146-147.

An embodiment of the invention provides an antibody construct or antigen binding domain comprising the CDR regions of the anti-EGFR antibody cetuximab. In this regard, the antibody construct or antigen binding domain may comprise a first variable region comprising a CDR1 comprising the amino acid sequence of SEQ ID NO: 148 (CDR1 of first variable region), a CDR2 comprising the amino acid sequence of SEQ ID NO: 149 (CDR2 of first variable region), and a CDR3 comprising the amino acid sequence of SEQ ID NO: 150 (CDR3 of first variable region), and a second variable region comprising a CDR1 comprising the amino acid sequence of SEQ ID NO: 151 (CDR1 of second variable region), a CDR2 comprising the amino acid sequence of SEQ ID NO: 152 (CDR2 of second variable region), and a CDR3 comprising the amino acid sequence of SEQ ID NO: 153 (CDR3 of second variable region). In this regard, the antibody construct or antigen binding domain can comprise (i) all of SEQ ID NOs: 148-150, (ii) all of SEQ ID NOs: 151-153, or (iii) all of SEQ ID NOs: 148-153. Preferably, the antibody construct or antigen binding domain comprises all of SEQ ID NOs: 148-153.

An embodiment of the invention provides antibody construct or antigen binding domain comprising the CDR regions of the anti-EGFR antibody panitumumab. In this regard, the antibody may comprise a first variable region comprising a CDR1 comprising the amino acid sequence of SEQ ID NO: 154 (CDR1 of first variable region), a CDR2 comprising the amino acid sequence of SEQ ID NO: 155 (CDR2 of first variable region), and a CDR3 comprising the amino acid sequence of SEQ ID NO: 156 (CDR3 of first variable region), and a second variable region comprising a CDR1 comprising the amino acid sequence of SEQ ID NO: 157 (CDR1 of second variable region), a CDR2 comprising the amino acid sequence of SEQ ID NO: 158 (CDR2 of second variable region), and a CDR3 comprising the amino acid sequence of SEQ ID NO: 159 (CDR3 of second variable region). In this regard, antibody construct or antigen binding domain can comprise (i) all of SEQ ID NOs: 154-156, (ii) all of SEQ ID NOs: 157-159, or (iii) all of SEQ ID NOs: 154-159. Preferably, antibody construct or antigen binding domain comprises all of SEQ ID NOs: 154-159.

An embodiment of the invention provides antibody construct or antigen binding domain comprising the CDR regions of the anti-EGFR antibody necitumumab. In this regard, the antibody construct or antigen binding domain may

comprise a first variable region comprising a CDR1 comprising the amino acid sequence of SEQ ID NO: 160 (CDR1 of first variable region), a CDR2 comprising the amino acid sequence of SEQ ID NO: 161 (CDR2 of first variable region), and a CDR3 comprising the amino acid sequence of SEQ ID NO: 162 (CDR3 of first variable region), and a second variable region comprising a CDR1 comprising the amino acid sequence of SEQ ID NO: 163 (CDR1 of second variable region), a CDR2 comprising the amino acid sequence of SEQ ID NO: 164 (CDR2 of second variable region), and a CDR3 comprising the amino acid sequence of SEQ ID NO: 165 (CDR3 of second variable region). In this regard, antibody construct or antigen binding domain can comprise (i) all of SEQ ID NOs: 160-162, (ii) all of SEQ ID NOs: 163-165, or (iii) all of SEQ ID NOs: 160-165. Preferably, antibody construct or antigen binding domain comprises all of SEQ ID NOs: 160-165.

An embodiment of the invention provides an antibody construct or antigen binding domain comprising one or both variable regions of the anti-EGFR antibody cetuximab. In this regard, the first variable region may comprise SEQ ID NO: 166. The second variable region may comprise SEQ ID NO: 167. Accordingly, in an embodiment of the invention, the antibody comprises SEQ ID NO: 166, SEQ ID NO: 167, or both SEQ ID NOs: 166 and 167. Preferably, the antibody comprises both of SEQ ID NOs: 166-167.

In addition to antibodies, alternative protein scaffolds may be used as part of the immunoconjugates. The phrase "alternative protein scaffold" refers to a non-immunoglobulin derived protein or peptide. Such proteins and peptides are generally amenable to engineering and can be designed to confer monospecificity against a given antigen, bispecificity, or multi specificity. Engineering of an alternative protein scaffold can be conducted using several approaches. A loop grafting approach can be used where sequences of known specificity are grafted onto a variable loop of a scaffold. Sequence randomization and mutagenesis can be used to develop a library of mutants, which can be screened using various display platforms (e.g., phage display) to identify a novel binder. Site-specific mutagenesis can also be used as part of a similar approach. Alternative protein scaffolds exist in a variety of sizes, ranging from small peptides with minimal secondary structure to large proteins of similar size to a full sized antibody. Examples of scaffolds include, but are not limited to, cystine knotted miniproteins (also known as knottins), cyclic cystine knotted miniproteins (also known as cyclotides), avimers, affibodies, the tenth type III domain of human fibronectin, DARPins (designed ankyrin repeats), and anticalins (also known as lipocalins). Naturally occurring ligands with known specificity can also be engineered to confer novel specificity against a given target. Examples of naturally occurring ligands that may be engineered include the EGF ligand and VEGF ligand. Engineered proteins can either be produced as monomeric proteins or as multimers, depending on the desired binding strategy and specificities. Protein engineering strategies can be used to fuse alternative protein scaffolds to Fc domains.

In some embodiments, the antibody binds to an FcRy-coupled receptor. In some embodiments, the FcRy-coupled receptor is selected from the group consisting of GP6 (GPVI), LILRA1 (CD85I), LILRA2 (CD85H, ILT1), LILRA4 (CD85G, ILT7), LILRA5 (CD85F, ILT11), LILRA6 (CD85b, ILT8), LILRB1, NCR1 (CD335, LY94, NKp46), NCR3 (CD335, LY94, NKp46), NCR3 (CD337, NKp30), OSCAR, and TARM1.

In some embodiments, the antibody binds to a DAP12-coupled receptor. In some embodiments, the DAP12-coupled receptor is selected from the group consisting of CD300C, CD300E, CD300LB (CD300B), CD300LD (CD300D), KIR2DL4 (CD158D), KIR2DS, KLRC2 (CD159C, NKG2C), KLRK1 (CD314, NKG2D), NCR2 (CD336, NKp44), PILRB, SIGLEC1 (CD169, SN), SIGLEC5, SIGLEC6, SIGLEC7, SIGLEC8, SIGLEC9, SIGLEC10, SIGLEC11, SIGLEC12, SIGLEC14, SIGLEC15 (CD33L3), SIGLEC16, SIRPB1 (CD172B), TREM1 (CD354), and TREM2.

In some embodiments, the antibody binds to a hemITAM-bearing receptor. In some embodiments, the hemITAM-bearing receptor is KLRF1 (NKp80).

In some embodiments, the antibody is capable of binding one or more targets selected from CLEC4C (BDCA-2, DLEC, CD303, CLECSF7), CLEC4D (MCL, CLECSF8), CLEC4E (Mincle), CLEC6A (Dectin-2), CLEC5A (MDL-1, CLECSF5), CLEC1B (CLEC-2), CLEC9A (DNGR-1), and CLEC7A (Dectin-1). In some embodiments, the antibody is capable of binding CLEC6A (Dectin-2) or CLEC5A. In some embodiments, the antibody is capable of binding CLEC6A (Dectin-2).

In some embodiments, the antibody is capable of binding one or more targets selected from (e.g., specifically binds to a target selected from): ATP5I (Q06185), OAT (P29758), AIFM1 (Q9Z0X1), AOFA (Q64133), MTDC (P18155), CMC1 (Q8BH59), PREP (Q8K411), YMEL1 (088967), LPPRC (Q6PB66), LONM (Q8CGK3), ACON (Q99KI0), ODO1 (Q60597), IDHP (P54071), ALDH2 (P47738), ATPB (P56480), AATM (P05202), TMM93 (Q9CQW0), ERGI3 (Q9CQE7), RTN4 (Q99P72), CL041 (Q8BQR4), ERLN2 (Q8BFZ9), TERA (Q01853), DAD1 (P61804), CALX (P35564), CALU (035887), VAPA (Q9WV55), MOGS (Q80UM7), GANAB (Q8BHN3), ERO1A (Q8R180), UGGG1 (Q6P5E4), P4HA1 (Q60715), HYEP (Q9D379), CALR (P14211), AT2A2 (055143), PDIA4 (P08003), PDIA1 (P09103), PDIA3 (P27773), PDIA6 (Q922R8), CLH (Q68FD5), PPIB (P24369), TCPG (P80318), MOT4 (P57787), NICA (P57716), BASI (P18572), VAPA (Q9WV55), ENV2 (P11370), VAT1 (Q62465), 4F2 (P10852), ENOA (P17182), ILK (055222), GPNMB (Q99P91), ENV1 (P10404), ERO1A (Q8R180), CLH (Q68FD5), DSG1A (Q61495), AT1A1 (Q8VDN2), HYOU1 (Q9JKR6), TRAP1 (Q9CQN1), GRP75 (P38647), ENPL (P08113), CH60 (P63038), and CH10 (Q64433). In the preceding list, accession numbers are shown in parentheses.

In some embodiments, the antibody binds to an antigen selected from CCR8, CDH1, CD19, CD20, CD29, CD30, CD38, CD40, CD47, EpCAM, MUC1, MUC16, EGFR, HER2, SLAMF7, and gp75. In some embodiments, the antigen is

selected from CCR8, CD19, CD20, CD47, EpCAM, MUC1, MUC16, EGFR, and HER2. In some embodiments, the antibody binds to an antigen selected from the Tn antigen and the Thomsen-Friedenreich antigen.

In some embodiments, the antibody or Fc fusion protein is selected from: abagovomab, abatacept (also known as ORENCIA™), abciximab (also known as REOPRO™, c7E3 Fab), adalimumab (also known as HUMIRA™), adecatumumab, alemtuzumab (also known as CAMPATH™, MabCampath or Campath-1H), altumomab, afelimomab, anatumomab mafenatox, anetumumab, anrukizumab, apolizumab, arcitumomab, aselizumab, atlizumab, atorolimumab, bapineuzumab, basiliximab (also known as SIMULECT™), bavituximab, bectumomab (also known as LYMPHOSCAN™), belimumab (also known as LYMPHO-STAT-B™), bertilimumab, besilesomab, bevacizumab (also known as AVASTIN™), biciromab brallobarbital, bivatuzumab mertansine, campath, canakinumab (also known as ACZ885), cantuzumab mertansine, capromab (also known as PROSTASCINT™), catumaxomab (also known as REMOVAB™), cedelizumab (also known as CIMZIA™), certolizumab pegol, cetuximab (also known as ERBITUX™), clenoliximab, dacetuzumab, dacliximab, daclizumab (also known as ZENAPAX™), denosumab (also known as AMG 162), detumomab, dorlimomab aritox, dorlixizumab, duntumumab, durimulumab, durmulumab, ecromeximab, eculizumab (also known as SOLIRIS™), edobacomab, edrecolomab (also known as Mab17-1A, PANOREX™), efalizumab (also known as RAPTIVA™), efungumab (also known as MYCOGRAB™), elotuzumab, elsilimomab, enlimomab pegol, epitumomab cituxetan, efalizumab, epitumomab, epratuzumab, erlizumab, ertumaxomab (also known as REXOMUN™), etanercept (also known as ENBREL™), etaracizumab (also known as etaratuzumab, VITAXIN™, ABEGRIN™), exbivirumab, fanolesomab (also known as NEUTROSPEC™), faralimomab, felvizumab, fontolizumab (also known as HUZAF™), galiximab, gantenerumab, gavilimomab (also known as ABXCBL™), gemtuzumab ozogamicin (also known as MYLOTARG™), golimumab (also known as CNTO 148), gomiliximab, ibalizumab (also known as TNX-355), ibritumomab tiuxetan (also known as ZEVALIN™), igovomab, imciromab, infliximab (also known as REMICADE™), inolimomab, inotuzumab ozogamicin, ipilimumab (also known as MDX-010, MDX-101), iratumumab, keliximab, labetuzumab, lemalesomab, lebrilizumab, lerdelimumab, lexatumumab (also known as, HGS-ETR2, ETR2-ST01), lexitumumab, libivirumab, lintuzumab, lucatumumab, lumiliximab, mapatumumab (also known as HGSETR1, TRM-1), maslimomab, matuzumab (also known as EMD72000), mepolizumab (also known as BOSATRIA™), metelimumab, milatuzumab, minretumomab, mitumomab, morolimumab, motavizumab (also known as NUMAX™), muromonab (also known as OKT3), nacolomab tafenatox, naptumomab estafenatox, natalizumab (also known as TYSABRI™, ANTEGREN™), nebacumab, nerelimomab, nimotuzumab (also known as THERACIM hR3™, THERA-CIM-hR3™, THERALOC™), nofetumomab merpentan (also known as VERLUMA™), obinutuzumab, ocrelizumab, odulimomab, ofatumumab, omalizumab (also known as XOLAIR™), oregovomab (also known as OVAREX™), otelixizumab, pagibaximab, palivizumab (also known as SYNAGIS™), panitumumab (also known as ABX-EGF, VECTIBIX™), pascolizumab, pemtumomab (also known as THERAGYN™), pertuzumab (also known as 2C4, OMNITARG™), pexelizumab, pintumomab, priliximab, pritumumab, ranibizumab (also known as LUCENTIS™), raxibacumab, regavirumab, reslizumab, rituximab (also known as RITUXAN™, MabTHERA™), rovelizumab, ruplizumab, satumomab, sevirumab, sibrotuzumab, siplizumab (also known as MEDI-507), sontuzumab, stamulumab (also known as MYO-029), sulesomab (also known as LEUKOSCAN™), tacatuzumab tetraxetan, tadocizumab, talizumab, taplitumomab paptox, tefibazumab (also known as AUREXIS™), telimomab aritox, teneliximab, teplizumab, ticilimumab, tocilizumab (also known as ACTEMRA™), toralizumab, tositumomab, trastuzumab (also known as HERCEPTIN™), tremelimumab (also known as CP-675,206), tucotuzumab celmoleukin, tuvirumab, urtoxazumab, ustekinumab (also known as CNTO 1275), vapaliximab, veltuzumab, vepalimomab, visilizumab (also known as NUVION™), volociximab (also known as M200), votumumab (also known as HUMASPECT™), zalutumumab, zanolimumab (also known as HuMAX-CD4), ziralimumab, zolimomab aritox, daratumumab, olaratumab, brentuximab vedotin, afibercept, abatacept, belatacept, afibercept, etanercept, romiplostim, SBT-040 (sequences listed in U.S. 2017/0158772. In some embodiments, the antibody is selected from the group consisting of olaratumab, obinutuzumab, trastuzumab, cetuximab, rituximab, pertuzumab, bevacizumab, daratumumab, etanercept, pembrolizumab, nivolumab, atezolizumab, ipilimumab, panitumumab, zalutumumab, nimotuzumab, matuzumab, and elotuzumab. In certain embodiments, the antibody is trastuzumab.

Checkpoint Inhibitors

Any suitable immune checkpoint inhibitor is contemplated for use with the immunoconjugates disclosed herein. In some embodiments, the immune checkpoint inhibitor reduces the expression or activity of one or more immune checkpoint proteins. In another embodiment, the immune checkpoint inhibitor reduces the interaction between one or more immune checkpoint proteins and their ligands. Inhibitory nucleic acids that decrease the expression and/or activity of immune checkpoint molecules can also be used in the methods disclosed herein.

Most checkpoint antibodies are designed not to have effector function as they are not trying to kill cells, but rather to block the signaling. Immunoconjugates of the invention can add back the "effector functionality" needed to activate myeloid immunity. Hence, for most checkpoint antibody inhibitors this discovery will be critical.

In some embodiments, the immune checkpoint inhibitor is cytotoxic T-lymphocyte antigen 4 (CTLA4, also known

as CD 152), T cell immunoreceptor with Ig and ITIM domains (TIGIT), glucocorticoid-induced TNFR-related protein (GITR, also known as TNFRSF18), inducible T cell costimulatory (ICOS, also known as CD278), CD96, poliovirus receptor-related 2 (PVRL2, also known as CD112R, programmed cell death protein 1 (PD-1, also known as CD279), programmed cell death 1 ligand 1 (PD-L1, also known as B7-H3 and CD274), programmed cell death ligand 2 (PD-L2, also known as B7-DC and CD273), lymphocyte activation gene-3 (LAG-3, also known as CD223), B7-H4, killer immunoglobulin receptor (KIR), Tumor Necrosis Factor Receptor superfamily member 4 (TNFRSF4, also known as OX40 and CD134) and its ligand OX40L (CD252), indoleamine 2,3-dioxygenase 1 (IDO-1), indoleamine 2,3-dioxygenase 2 (IDO-2), carcinoembryonic antigen-related cell adhesion molecule 1 (CEACAM1), B and T lymphocyte attenuator (BTLA, also known as CD272), T-cell membrane protein 3 (TIM3), the adenosine A2A receptor (A2Ar), and V-domain Ig suppressor of T cell activation (VISTA protein). In some embodiments, the immune checkpoint inhibitor is an inhibitor of CTLA4, PD-1, or PD-L1.

In some embodiments, the antibody is selected from ipilimumab (also known as YERVOY™ pembrolizumab (also known as KEYTRUDA™), nivolumab (also known as OPDIVO™), atezolizumab (also known as TECENTRIG™), avelumab (also known as BAVENCIO™), and durvalumab (also known as IMFINZI™). In some embodiments, the antibody is selected from ipilimumab (also known as YERVOY™), pembrolizumab (also known as KEYTRUDA™), nivolumab (also known as OPDIVO™), and atezolizumab (also known as TECENTRIG™).

Spacer

The adjuvant moieties and adjuvant cores in the conjugates can be covalently bonded to the antibodies using various chemistries for protein modification, and that the linkers or spacers described above result from the reaction of protein functional groups (i.e., amino acid side chains), with reagents having reactive linker groups. A wide variety of such reagents are known in the art. Examples of such reagents include, but are not limited to, N-hydroxysuccinimidyl (NHS) esters and N-hydroxysulfosuccinimidyl (sulfo-NHS) esters (amine reactive); carbodiimides (amine and carboxyl reactive); hydroxymethyl phosphines (amine reactive); maleimides (thiol reactive); halogenated acetamides such as *N*-iodoacetamides (thiol reactive); aryl azides (primary amine reactive); fluorinated aryl azides (reactive via carbon-hydrogen (C-H) insertion); pentafluorophenyl (PFP) esters (amine reactive); tetrafluorophenyl (TFP) esters (amine reactive); imidoesters (amine reactive); isocyanates (hydroxyl reactive); vinyl sulfones (thiol, amine, and hydroxyl reactive); pyridyl disulfides (thiol reactive); and benzophenone derivatives (reactive via C-H bond insertion). Further reagents include but are not limited to those described in Hermanson, Bioconjugate Techniques, 2nd Edition, Academic Press, 2008.

The linker can have any suitable length such that when the linker is covalently bound to the antibody construct and the adjuvant core, the function of the antibody construct and the adjuvant moiety is maintained. The linker can have a length of about 3 Å or more, for example, about 4 Å or more, about 5 Å or more, about 6 Å or more, about 7 Å or more, about 8 Å or more, about 9 Å or more, about 10 Å or more, or about 20 Å or more. Alternatively, or in addition to, the linker can have a length of about 100 Å or less, for example, about 90 Å or less, about 80 Å or less, about 70 Å or less, about 60 Å or less, about 50 Å or less, about 45 Å or less, about 40 Å or less, about 35 Å or less, about 30 Å or less, about 25 Å or less, about 20 Å or less, or about 15 Å or less. Thus, the linker can have a length bounded by any two of the aforementioned endpoints. The linker can have a length from about 3 Å to about 100 Å, for example, from about 3 Å to about 90 Å, from about 3 Å to about 80 Å, from about 3 Å to about 70 Å, from about 3 Å to about 60 Å, from about 3 Å to about 50 Å, from about 3 Å to about 45 Å, from about 3 Å to about 40 Å, from about 3 Å to about 35 Å, from about 3 Å to about 30 Å, from about 3 Å to about 25 Å, from about 3 Å to about 20 Å, from about 3 Å to about 15 Å, from about 5 Å to about 50 Å, from about 5 Å to about 25 Å, from about 5 Å to about 20 Å, from about 10 Å to about 50 Å, from about 10 Å to about 20 Å, from about 5 Å to about 30 Å, from about 5 Å to about 15 Å, from about 20 Å to about 100 Å, from about 20 Å to about 90 Å, from about 20 Å to about 80 Å, from about 20 Å to about 70 Å, from about 20 Å to about 60 Å, or from about 20 Å to about 50 Å. In certain embodiments, the linker has a length from about 20 Å to about 100 Å.

The spacer can have any suitable length such that when the spacer is covalently bound to the antibody construct and the adjuvant moiety, the function of the antibody construct and the adjuvant moiety is maintained. The spacer can have a length of about 3 Å or more, for example, about 4 Å or more, about 5 Å or more, about 6 Å or more, about 7 Å or more, about 8 Å or more, about 9 Å or more, about 10 Å or more, or about 20 Å or more. Alternatively, or in addition to, the spacer can have a length of about 80 Å or less, for example, about 70 Å or less, about 60 Å or less, about 50 Å or less, about 45 Å or less, about 40 Å or less, about 35 Å or less, about 30 Å or less, about 25 Å or less, about 20 Å or less, or about 15 Å or less. Thus, the spacer can have a length bounded by any two of the aforementioned endpoints. The spacer can have a length from about from about 3 Å to about 80 Å, for example, from about 3 Å to about 70 Å, from about 3 Å to about 60 Å, from about 3 Å to about 50 Å, from about 3 Å to about 45 Å, from about 3 Å to about 40 Å, from about 3 Å to about 35 Å, from about 3 Å to about 3 0 Å, from about 3 Å to about 25 Å, from about 3 Å to about 20 Å, from about 3 Å to about 15 Å, from about 5 Å to about 50 Å, from about 5 Å to about 25 Å, from about 5 Å to about 20 Å, from about 10 Å to about 50 Å, from about 10 Å to about 20 Å, from about 5 Å to about 30 Å, from about 5 Å to about 15 Å, from about 20 Å to about 80 Å, from about 20 Å to about 70 Å, from about 20 Å to about 60 Å, or from about 20 Å to about 50 Å. In certain embodiments, the spacer has a length from about 20 Å to about 80 Å.

In some embodiments, the linker is non-cleavable under physiological conditions. As used herein, the term "physiological conditions" refers to a temperature range of 20-40 degrees Celsius, atmospheric pressure (i.e., 1 atm), a pH of about 6 to about 8, and the one or more physiological enzymes, proteases, acids, and bases.

In some embodiments, the linker is cleavable under physiological conditions. For example, the linker can be cleaved by an enzymatic process or a metabolic process.

The spacer can be any suitable organic divalent linking moiety such that the desired length of the spacer and/or the linker can be achieved.

In some embodiments, the spacer is a divalent linking moiety comprising an ethylene glycol group or a glycine residue. The spacer preferably is bonded to the adjuvant moiety via an amide bond, a C-N single bond, a C-O single bond, or a C-C single bond, and to the antibody via an amide bond or a C-N single bond. In some embodiments, the spacer is bonded to a nitrogen group of the adjuvant moiety and a nitrogen group of the antibody. In such embodiments, the spacer is bonded to adjacent nitrogen groups via amide bonds, C-N single bonds, or a combination thereof.

In some embodiments, the spacer comprises a poly(ethylene glycol) group. In certain embodiments, the spacer comprises at least 2 ethylene glycol groups (e.g., at least 3 ethylene glycol groups, at least 4 ethylene glycol groups, at least 5 ethylene glycol groups, at least 6 ethylene glycol groups, at least 7 ethylene glycol groups, at least 8 ethylene glycol groups, at least 9 ethylene glycol groups, at least 10 ethylene glycol groups, at least 11 ethylene glycol groups, at least 12 ethylene glycol groups, at least 13 ethylene glycol groups, at least 14 ethylene glycol groups, at least 15 ethylene glycol groups, at least 16 ethylene glycol groups, at least 17 ethylene glycol groups, at least 18 ethylene glycol groups, at least 19 ethylene glycol groups, at least 20 ethylene glycol groups, at least 21 ethylene glycol groups, at least 22 ethylene glycol groups, at least 23 ethylene glycol groups, at least 24 ethylene glycol groups, or at least 25 ethylene glycol groups. In certain embodiments, the spacer comprises a di(ethylene glycol) group, a tri(ethylene glycol) group, or a tetra(ethylene glycol) group, 5 ethylene glycol groups, 6 ethylene glycol groups, 8 ethylene glycol groups, 12 ethylene glycol groups, 24 ethylene glycol groups, or 25 ethylene glycol groups.

In some embodiments, the spacer comprises a glycine residue. In certain embodiments, the spacer comprises at least 2 glycine residues (e.g., at least 3 glycine residues, at least 4 glycine residues, at least 5 glycine residues, at least 6 glycine residues, at least 7 glycine residues, at least 8 glycine residues, at least 9 glycine residues, at least 10 glycine residues, at least 11 glycine residues, at least 12 glycine residues, at least 13 glycine residues, at least 14 glycine residues, at least 15 glycine residues, at least 16 glycine residues, at least 17 glycine residues, at least 18 glycine residues, at least 19 glycine residues, at least 20 glycine residues, at least 21 glycine residues, at least 22 glycine residues, at least 23 glycine residues, at least 24 glycine residues, or at least 25 glycine residues. In certain embodiments, the spacer comprises 2 glycine residues, 3 glycine residues, 4 glycine residues, 5 glycine residues, 6 glycine residues, 8 glycine residues, 12 glycine residues, 24 glycine residues, or 25 glycine residues.

In some embodiments, the spacer further comprises a divalent cyclohexylene group.

In some embodiments, the spacer is selected from:

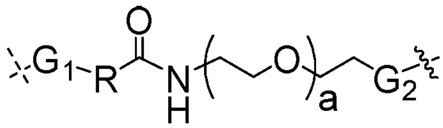
L1 ,

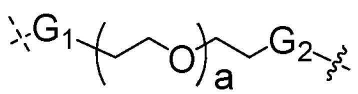
L2 ,

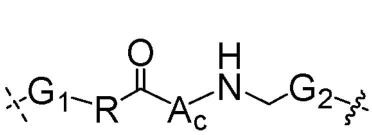
L3 ,

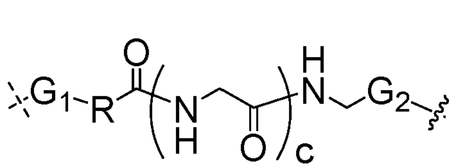
L4 ,

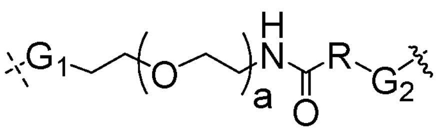
L5 ,

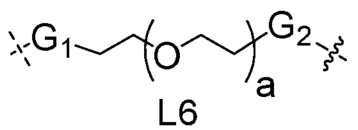
L6 ,

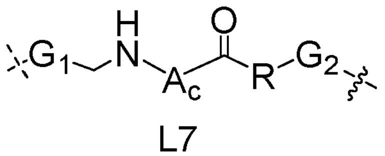, and 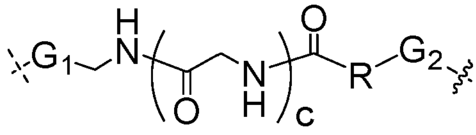 ;

L7 L8

wherein R is optionally present and is a linear or branched, cyclic or straight, saturated or unsaturated alkyl, heteroalkyl, aryl, or heteroaryl chain comprising from 1 to 8 carbon units; a is an integer from 1 to 40; each A is independently selected from any amino acid; subscript c is an integer from 1 to 25; $G_1$ is $CH_2$, C=O, or a bond, $G_2$ is $CH_2$, C=O, or a bond, the dashed line (" ") represents the point of attachment to the adjuvant moiety; and the wavy line (" ") represents the point of attachment to the antibody. In certain embodiments, a is an integer from 2 to 25. In certain embodiments, c is an integer from 2 to 8.

Adjuvants

Generally, the adjuvant moiety has an adjuvant core structure of Formula I:

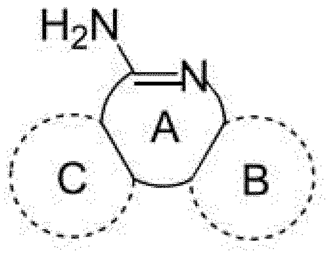

(I)

wherein B and C are optionally present, and A, B, and C denote 5-, 6-, 7-, 8-, or 9-membered rings, optionally comprising double bonds, optionally comprising heteroatoms (e.g., nitrogen, oxygen, and/or sulfur) in addition to the 2-amino nitrogen moiety, and optionally substituted. The double bonds and heteroatoms within the core structure of Formula I (i.e., other atoms about the aromatic ring) is not particularly limited, as long as the A ring has the 2-amino nitrogen moiety shown in the Formula I. Accordingly, as used herein, the phrase "adjuvant core" refers to a group of fused rings (i.e., 1, 2, or 3 rings) comprising a 2-amino nitrogen moiety. In preferred embodiments, the A ring denotes a cyclic aromatic or non-aromatic ring containing 4, 5, or 6 carbon atoms and at least one nitrogen atom (e.g., one nitrogen atom, two nitrogen atoms, or three nitrogen atoms). The substitution around the adjuvant core is not particularly limited, as long as the 2-amino nitrogen moiety remains unsubstituted. Without wishing to be bound by any particular theory, it is believed that the 2-amino nitrogen moiety is important for maintaining activity of the adjuvant.

In some embodiments, the adjuvant moiety has an adjuvant core structure of Formula I, wherein the B ring is present. In some embodiments, the adjuvant moiety has an adjuvant core structure of Formula I, wherein the C ring is present. In certain embodiments, the adjuvant moiety has an adjuvant core structure of Formula I, wherein the B ring and the C ring are present.

In certain embodiments disclosed herein, but not according to the appended claims, the adjuvant moiety has an adjuvant core of Formula IA:

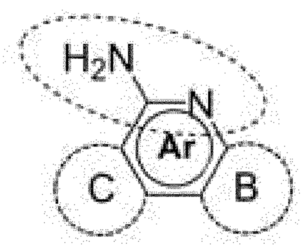

(IA)

,

wherein, Ar denotes that the ring is aromatic, optionally comprising other nitrogen atoms and optionally substituted, B and C are optionally present, and B, and C denote 5-, 6-, 7-, 8-, or 9-membered rings, optionally comprising double bonds, optionally comprising heteroatoms (e.g., nitrogen, oxygen, and/or sulfur) in addition to the 2-amino nitrogen moiety, and optionally substituted. The double bonds and heteroatoms within the core structure within the core structure of Formula IA (i.e., other atoms about the ring) is not particularly limited, as long as the ring has the 2-amino nitrogen moiety, which is marked by the dashed oval, is maintained.

In certain embodiments, the adjuvant moiety has an adjuvant core of Formula IB:

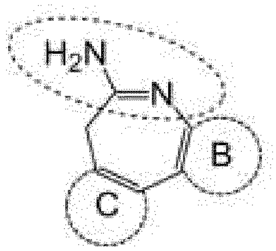

(IB)

,

wherein B and C are optionally present, and B, and C denote 5-, 6-, 7-, 8-, or 9-membered rings, optionally comprising double bonds, optionally comprising heteroatoms (e.g., nitrogen, oxygen, and/or sulfur) in addition to the 2-amino nitrogen moiety, and optionally substituted. The double bonds and heteroatoms within the core structure within the core structure of Formula IB (i.e., other atoms about the ring) is not particularly limited, as long as the ring has the 2-amino nitrogen moiety, which is marked by the dashed oval, is maintained.

In some embodiments, the immunoconjugates of the invention comprise an adjuvant moiety with an adjuvant core comprising a 2-amino nitrogen moiety with a pendant nitrogen atom and a point of attachment of a linker to the adjuvant core, wherein the distance between the pendant nitrogen atom and the point of attachment of the linker is greater than about 5 Å (e.g., greater than about 5.25 Å, greater than about 5.5 Å, greater than about 5.75 Å, or greater than about 6 Å). In certain embodiments, the distance between the pendant nitrogen atom and the point of attachment of the linker is greater than about 6 Å. Without wishing to be bound by any particular theory, it is believed that the pendant nitrogen atom of the 2-amino nitrogen moiety forms a hydrogen bond and/or a salt bridge with an amino acid on a side of the binding domain of a toll-like receptor opposite from the opening of the binding domain. Accordingly, the pendant nitrogen atom of the 2-amino nitrogen moiety and the point of attachment of the linker to the adjuvant core are typically on opposite sides of the adjuvant moiety. FIG. 1 shows an exemplary adjuvant moiety in relation to a binding domain of a TLR, wherein "" denotes the distance from the 2-amino nitrogen moiety to the point of attachment of the linker.

In some embodiments, the adjuvant moiety further comprises a hydrophobic substituent ("$R_H$") with at least 1 carbon atom (e.g., at least 2 carbon atoms, at least 3 carbon atoms, at least 4 carbon atoms, or at least 6 carbon atoms). The hydrophobic substituent can be any suitable alkyl, heteroalkyl, cycloalkyl, heterocycloalkyl, aryl, heteroaryl, or combination thereof so long as the hydrophobic substituent does not contain an $-NH_2$, -OH, or -SH substituent. In addition, it is important that the hydrophobic substituent is not so large as to result in unfavorable steric and/or electronic interactions. Thus, the hydrophobic substituent will have less than 8 non-hydrogen atoms (e.g., carbon, oxygen, nitrogen, sulfur, etc.).

Typically, when present, the hydrophobic substituent will have a point of attachment of the hydrophobic substituent to the adjuvant core that is a distance from the pendent nitrogen atom of the 2-amino nitrogen moiety of less than about 6 Å (e.g., less than about 5.75 Å, less than about 5.5 Å, less than about 5.25 Å, or less than about 5 Å). In certain embodiments, the distance between the pendant nitrogen atom and the point of attachment of the hydrophobic substituent to the adjuvant core is less than about 5 Å. Without wishing to be bound by any particular theory, it is believed that the pendant nitrogen atom of the 2-amino nitrogen moiety forms a hydrogen bond and/or a salt bridge with an amino acid on a side of the binding domain of a toll-like receptor in close proximity to a hydrophobic pocket region. Accordingly, the pendant nitrogen atom of the 2-amino nitrogen moiety and the point of attachment of the hydrophobic substituent to the adjuvant core are typically in close proximity. FIG. 1 shows an exemplary adjuvant moiety in relation to a binding domain of a TLR, wherein "" denotes the distance from the 2-amino nitrogen moiety to the hydrophobic substituent.

The adjuvant core is attached to a linker at a position that allows the adjuvant moiety to maintain its adjuvant activity. Accordingly, the linker cannot be attached to the 2-amino nitrogen moiety, as this chemical moiety must remain unsubstituted. In addition, the adjuvant moiety has one or more hydrophobic pocket regions ("$R_H$"), wherein the linker cannot be attached to the adjuvant core. As used herein, the phrase "hydrophobic pocket region" refers to an area of the adjuvant moiety that resides in a hydrophobic pocket and/or interferes with amino acid residues of the binding domain of

the adjuvant moiety's respective receptor, as determined by theoretical modeling of the receptor binding domain. Without wishing to be bound by any particular theory, it is believed that a linker attached at positions designated $R_H$ results in unfavorable steric and/or electronic interactions, thereby reducing the activity of the adjuvant. Accordingly, the adjuvant core must be attached to the linker at a specific location ("$R_C$") to maintain the activity of the adjuvant.

In some embodiments, the adjuvant moiety is of Formula Adj A:

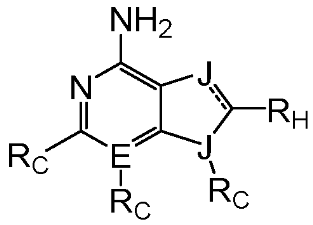

**Adj A**

wherein

each J independently is C, CH, $CH_2$, N, NH, O, or S,
E is C, CH, or N,
each $R_C$ is optionally present and independently is of the formula:

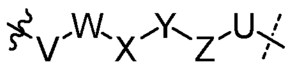 or 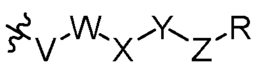,

except that at least one $R_C$ is present and is of the formula:

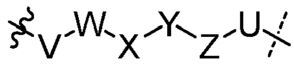,

$R_H$ is of the formula:

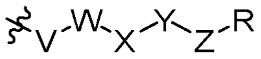,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

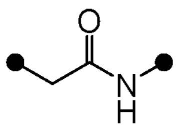,

each R independently is hydrogen, halogen (e.g., fluorine, chlorine, bromine, or iodine), nitrile, -COOH, or a linear or branched, saturated or unsaturated $C_1$-$C_4$ alkyl,
"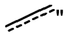" represents a single bond or a double bond,
the wavy line ("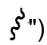") represents a point of attachment of $R_C$, and $R_H$,

the dot ("●") represents a point of attachment of U, and
the dashed line ("") represents a point of attachment of the adjuvant moiety to a spacer. Accordingly, the point of attachment of the linker to the adjuvant core ("P") is the atom in the adjuvant core to which $R_C$ is bound.

As used herein, the term "divalent" refers to a chemical moiety that is capable of binding to two other atoms, the same or different, at the same or different positions of the chemical moiety, even if the one of the other atoms is hydrogen. For example, divalent groups have two points of connection, the same or different, that can bind to carbon, nitrogen, oxygen, or hydrogen, as defined by V, W, X, Y, and Z.

In certain embodiments, the adjuvant moiety is of formula:

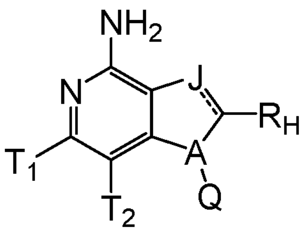

**Adj A-1a** ,

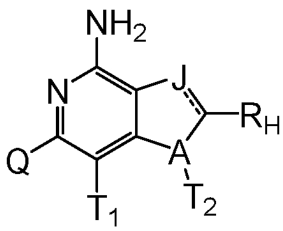

**Adj A-1b** , or

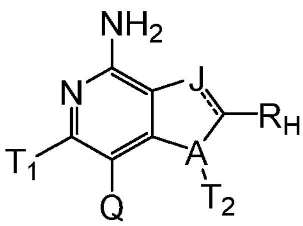

**Adj A-1c** ;

wherein

A is CH or N,
J is CH, $CH_2$, N, NH, O, or S,
Q is of the formula:

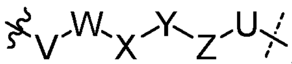

,

$T_1$, $T_2$, and $R_H$ independently are of the formula:

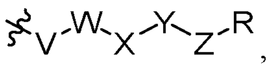

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

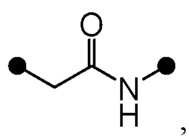

,

each R independently is hydrogen, halogen (e.g., fluorine, chlorine, bromine, or iodine), nitrile, -COOH, or a linear or branched, saturated or unsaturated $C_1$-$C_4$ alkyl,
"" represents a single bond or a double bond,
the wavy line ("") represents a point of attachment of Q, $T_1$, $T_2$, and $R_H$,
the dot ("●") represents a point of attachment of U, and

the dashed line ("") represents a point of attachment of the adjuvant moiety to a spacer. Accordingly, the point of attachment of the linker to the adjuvant core ("P") is the atom in the adjuvant core to which Q is bound.

In some instances, the adjuvant moiety is not of formula:

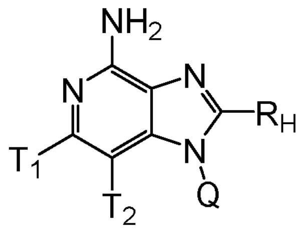

**Adj A-1a-i**

wherein

Q is of the formula:

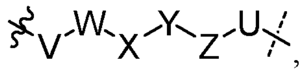

$T_1$, $T_2$, and $R_H$ independently are of the formula:

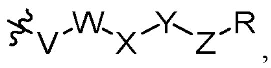

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

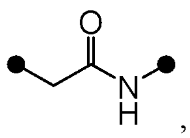

each R independently is hydrogen, halogen (e.g., fluorine, chlorine, bromine, or iodine), nitrile, -COOH, or a linear or branched, saturated or unsaturated $C_1$-$C_4$ alkyl,
"" represents a single bond or a double bond,
the wavy line ("") represents a point of attachment of Q, $T_1$, $T_2$, and $R_H$,
the dot ("●") represents a point of attachment of U, and
the dashed line ("") represents a point of attachment of the adjuvant moiety to a spacer.

In certain embodiments, the adjuvant moiety is of formula:

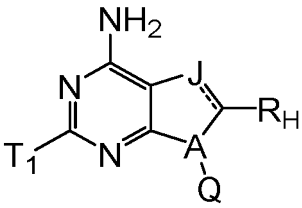 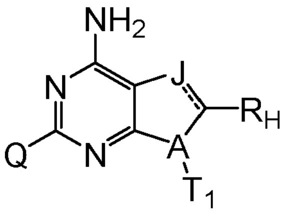

**Adj A-2a** or **Adj A-2b** ;

wherein

A is CH or N,
J is CH, $CH_2$, N, NH, O, or S,
Q is of the formula:

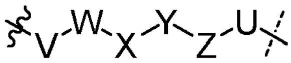,

$T_1$ and $R_H$ independently are of the formula:

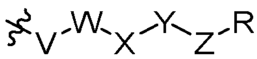,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

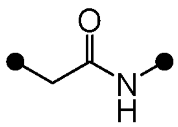,

each R independently is hydrogen, halogen (e.g., fluorine, chlorine, bromine, or iodine), nitrile, -COOH, or a linear or branched, saturated or unsaturated $C_1$-$C_4$ alkyl,
" " represents a single bond or a double bond,
the wavy line (" ") represents a point of attachment of Q, $T_1$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is not of formula:

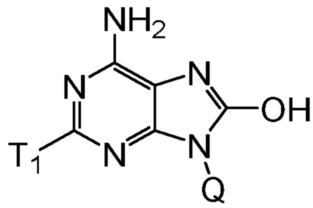

**Adj A-2a-i** ;

wherein

Q is of the formula:

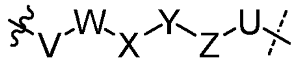

$T_1$ and $R_H$ independently are of the formula:

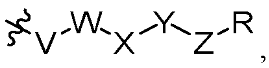

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

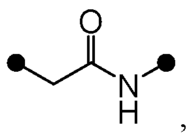

each R independently is hydrogen, halogen (e.g., fluorine, chlorine, bromine, or iodine), nitrile, -COOH, or a linear or branched, saturated or unsaturated $C_1$-$C_4$ alkyl,
" " represents a single bond or a double bond,
the wavy line (" ") represents a point of attachment of Q, $T_1$, and $R_H$,
the dot ("●") represents a point of attachment of U, and
the dashed line (" ") represents a point of attachment of the adjuvant moiety to a spacer.

In certain embodiments, the adjuvant moiety is not of formula:

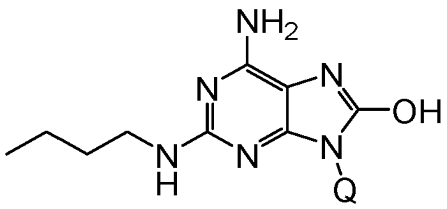

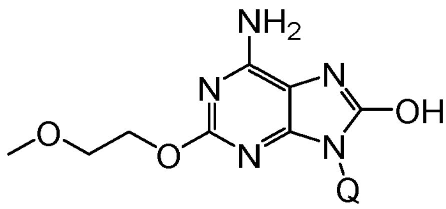

**Adj A-2a-ia** or **Adj A-2a-ib** .

In some embodiments, the adjuvant moiety is of Formula Adj B:

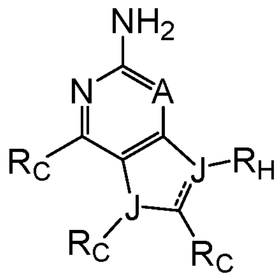

**Adj B** ;

wherein

each J independently is C, CH, $CH_2$, N, NH, O, or S,
A is CH, or N,
each $R_C$ is optionally present and independently is of the formula:

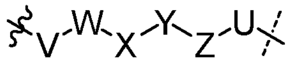 or 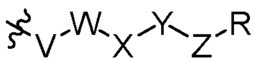 ,

except that at least one $R_C$ is present and is of the formula:

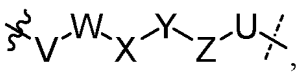 ,

$R_H$ is optionally present is of the formula:

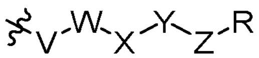 ;

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

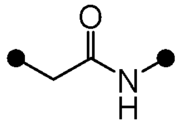,

each R independently is hydrogen, halogen (e.g., fluorine, chlorine, bromine, or iodine), nitrile, -COOH, or a linear or branched, saturated or unsaturated $C_1$-$C_4$ alkyl,

"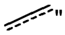" represents a single bond or a double bond,

the wavy line ("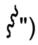") represents a point of attachment of $R_C$, and $R_H$,

the dot ("●") represents a point of attachment of U, and

the dashed line ("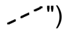") represents a point of attachment of the adjuvant moiety to a spacer. Accordingly, the point of attachment of the linker to the adjuvant core ("P") is the atom in the adjuvant core to which $R_C$ is bound.

In certain embodiments, the adjuvant moiety is of formula:

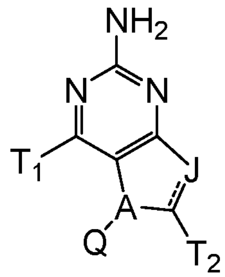

**Adj B-1a**,

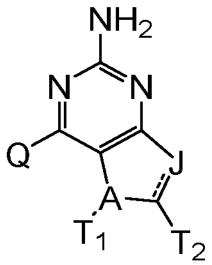

**Adj B-1b**,

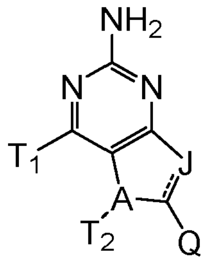

**Adj B-1c**,

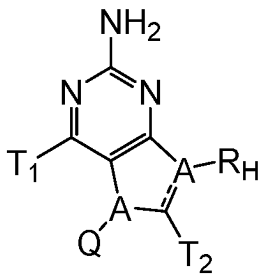

**Adj B-1d**,

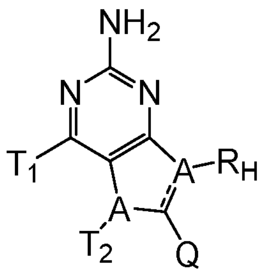

**Adj B-1e** or

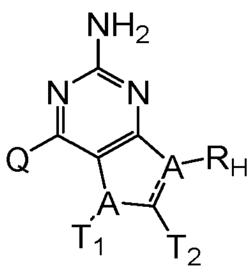

**Adj B-1f**;

wherein

A is CH or N,

J is CH, $CH_2$, N, NH, O, or S,

Q is of the formula:

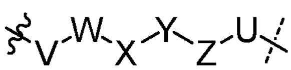,

$T_1$, $T_2$, and $R_H$ independently are of the formula:

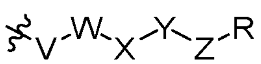,

each V is optionally present and independently is -O-, -S-, -NH-, -NR- or -CO-,

each W is optionally present and independently is a linear or branched, saturated or unsaturated, divalent $C_1$-$C_8$ alkyl,

each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,

each Z is optionally present and independently is -O-, -S-, -NH-, or -NR-,

U is optionally present and is

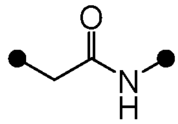

,

each R independently is hydrogen, halogen (e.g., fluorine, chlorine, bromine, or iodine), nitrile, -COOH, or a linear or branched, saturated or unsaturated $C_1$-$C_4$ alkyl,

" 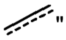 " represents a single bond or a double bond,

the wavy line (" 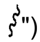 ") represents a point of attachment of Q, $T_1$, $T_2$, and $R_H$,

the dot ("●") represents a point of attachment of U, and

the dashed line (" 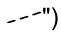 ") represents a point of attachment of the adjuvant moiety to a spacer. Accordingly, the point of attachment of the linker to the adjuvant core ("P") is the atom in the adjuvant core to which Q is bound.

In some instances, the adjuvant moiety is of formula:

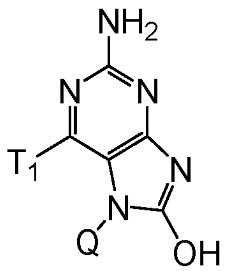

**Adj B-1a-i** .

In certain embodiments, the adjuvant moiety is of formula:

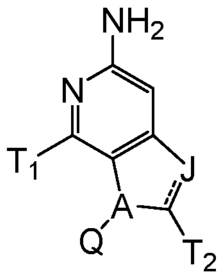

**Adj B-2a** ,

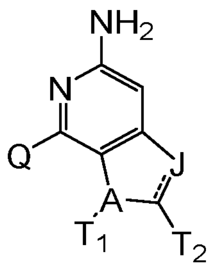

**Adj B-2b** ,

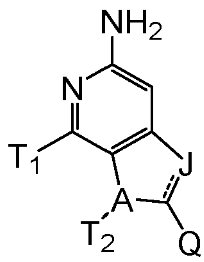

**Adj B-2c** ,

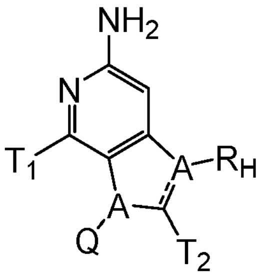 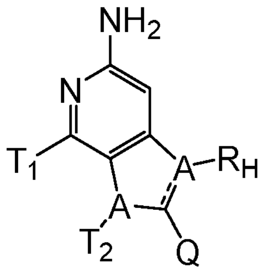 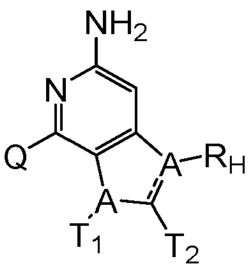

**Adj B-2d**, **Adj B-2e** or **Adj B-2f**;

wherein

A is CH or N,
J is CH, $CH_2$, N, NH, O, or S,
Q is of the formula:

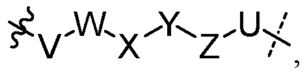

$T_1$, $T_2$, and $R_H$ independently are of the formula:

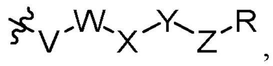

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

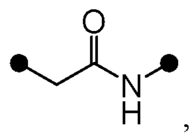

each R independently is hydrogen, halogen (e.g., fluorine, chlorine, bromine, or iodine), nitrile, -COOH, or a linear or branched, saturated or unsaturated $C_1$-$C_4$ alkyl,
" " represents a single bond or a double bond,
the wavy line (" ") represents a point of attachment of Q, $T_1$, $T_2$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In some embodiments, the adjuvant moiety is not of formula:

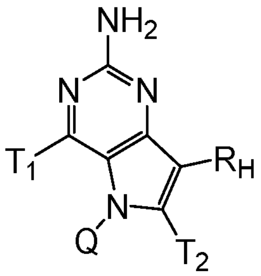

**Adj B-3**

;

wherein

Q is of the formula:

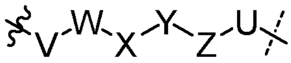,

$T_1$, $T_2$, and $R_H$ independently are of the formula:

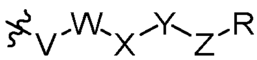,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

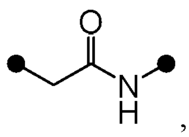,

each R independently is hydrogen, halogen (e.g., fluorine, chlorine, bromine, or iodine), nitrile, -COOH, or a linear or branched, saturated or unsaturated $C_1$-$C_4$ alkyl,
" " represents a single bond or a double bond,
the wavy line (" ") represents a point of attachment of Q, $T_1$, $T_2$, and $R_H$,
the dot ("●") represents a point of attachment of U, and
the dashed line (" ") represents a point of attachment of the adjuvant moiety to a spacer.

In some embodiments, the adjuvant moiety is of Formula Adj C:

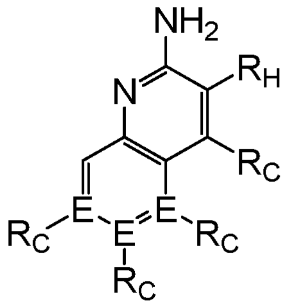

**Adj C** ;

wherein

each E independently is C, CH, or N,

each $R_C$ is optionally present and independently is of the formula:

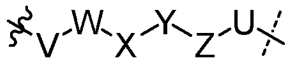 or 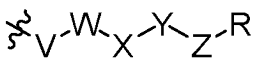,

except that at least one $R_C$ is present and is of the formula:

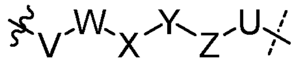,

$R_H$ is of the formula:

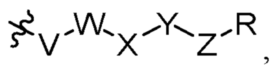,

each V is optionally present and independently is -O-, -S-, -NH-, -NR- or -CO-,

each W is optionally present and independently is a linear or branched, saturated or unsaturated, divalent $C_1$-$C_8$ alkyl,

each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,

each Z is optionally present and independently is -O-, -S-, -NH-, or -NR-,

U is optionally present and is

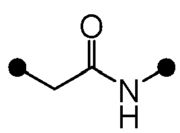,

each R independently is hydrogen, halogen (e.g., fluorine, chlorine, bromine, or iodine), nitrile, -COOH, or a linear or branched, saturated or unsaturated $C_1$-$C_4$ alkyl,

the wavy line (" ") represents a point of attachment of $R_C$, and $R_H$,

the dot ("●") represents a point of attachment of U, and

the dashed line (" ") represents a point of attachment of the adjuvant moiety to a spacer. Accordingly, the point of attachment of the linker to the adjuvant core ("P") is the atom in the adjuvant core to which $R_C$ is bound.

In certain embodiments, the adjuvant moiety is of formula:

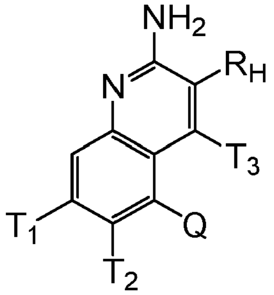 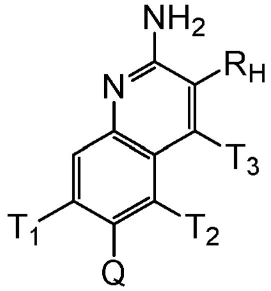 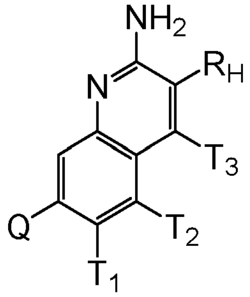 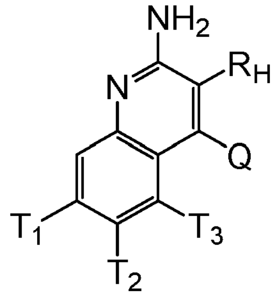

**Adj C-1a** , **Adj C-1b** , **Adj C-1c** , or **Adj C-1d** ;

wherein

Q is of the formula:

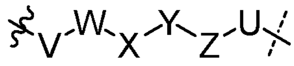

,

$T_1$, $T_2$, $T_3$, and $R_H$ independently are of the formula:

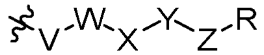

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

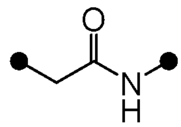

,

each R independently is hydrogen, halogen (e.g., fluorine, chlorine, bromine, or iodine), nitrile, -COOH, or a linear or branched, saturated or unsaturated $C_1$-$C_4$ alkyl,
the wavy line ("") represents a point of attachment of Q, $T_1$, $T_2$, $T_3$, and $R_H$,
the dot ("●") represents a point of attachment of U, and
the dashed line ("") represents a point of attachment of the adjuvant moiety to a spacer. Accordingly, the point of attachment of the linker to the adjuvant core ("P") is the atom in the adjuvant core to which $R_C$ is bound.

In certain embodiments, the adjuvant moiety is of formula:

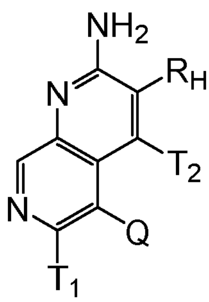

**Adj C-2a** ,

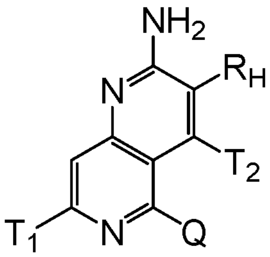

**Adj C-2b** ,

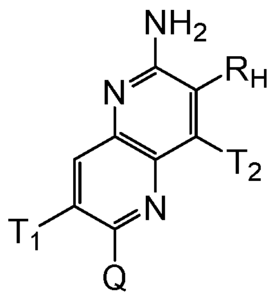

**Adj C-2c** ,

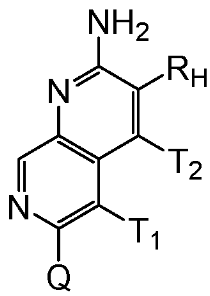

**Adj C-2d** ,

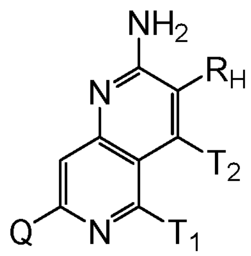

**Adj C-2e** ,

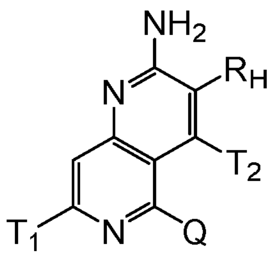

**Adj C-2f** ,

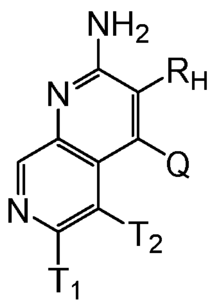

**Adj C-2g** ,

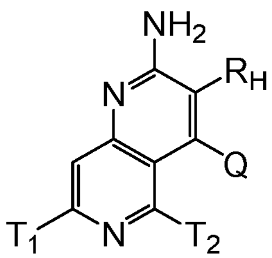

**Adj C-2h** , or

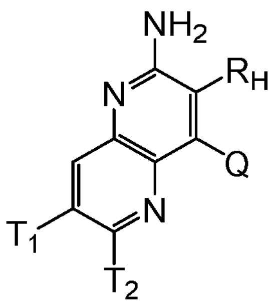

**Adj C-2i** ;

wherein

Q is of the formula:



$T_1$, $T_2$, and $R_H$ independently are of the formula:

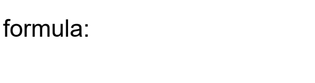

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or

heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

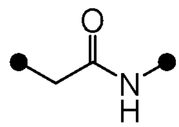

,

each R independently is hydrogen, halogen (e.g., fluorine, chlorine, bromine, or iodine), nitrile, -COOH, or a linear or branched, saturated or unsaturated $C_1$-$C_4$ alkyl,
the wavy line ("ξ") represents a point of attachment of Q, $T_1$, $T_2$, and $R_H$,
the dot ("●") represents a point of attachment of U, and
the dashed line ("- - -") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

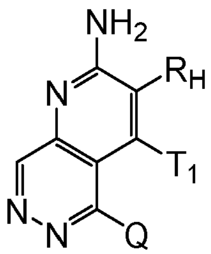

**Adj C-3a**,

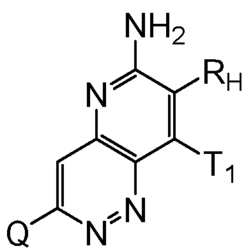

**Adj C-3b**,

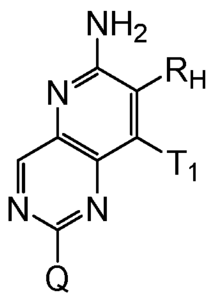

**Adj C-3c**,

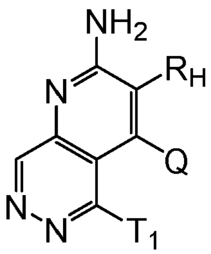

**Adj C-3d**,

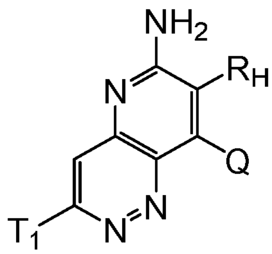

**Adj C-3e**, or

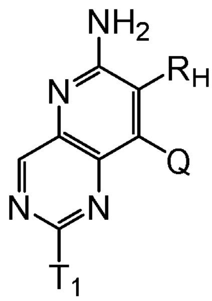

**Adj C-3f**;

wherein

Q is of the formula:

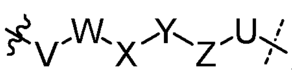

,

$T_1$ and $R_H$ independently are of the formula:

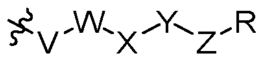,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

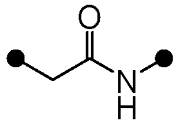,

each R independently is hydrogen, halogen (e.g., fluorine, chlorine, bromine, or iodine), nitrile, -COOH, or a linear or branched, saturated or unsaturated $C_1$-$C_4$ alkyl,
the wavy line (" ") represents a point of attachment of Q, $T_1$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In some embodiments, the adjuvant moiety is of Formula Adj D:

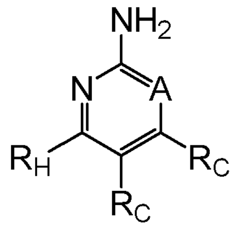

**Adj D** ;

wherein

A is CH, or N,
each $R_C$ is optionally present and independently is of the formula:

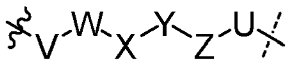 or 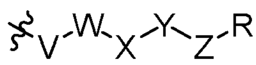,

except that at least one $R_C$ is present and is of the formula:

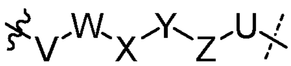,

$R_H$ is of the formula:

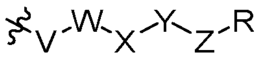,

wherein
each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

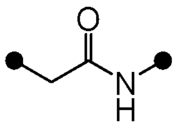,

each R independently is hydrogen, halogen (e.g., fluorine, chlorine, bromine, or iodine), nitrile, -COOH, or a linear or branched, saturated or unsaturated $C_1$-$C_4$ alkyl,

the wavy line (" ") represents a point of attachment of $R_C$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which $R_C$ is bound.

In certain embodiments, the adjuvant moiety is of formula:

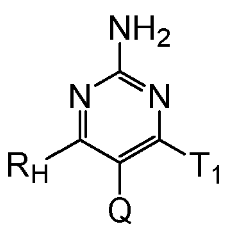

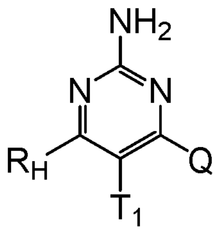

**Adj D-1a** or **Adj D-1b**;

wherein

Q is of the formula:

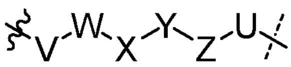,

$T_1$ and $R_H$ independently are of the formula:

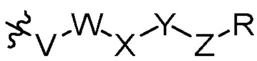,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

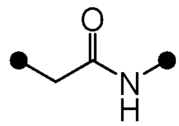
,

each R independently is hydrogen, halogen (e.g., fluorine, chlorine, bromine, or iodine), nitrile, -COOH, or a linear or branched, saturated or unsaturated $C_1$-$C_4$ alkyl,
the wavy line ("") represents a point of attachment of Q, $T_1$, and $R_H$,
the dot ("●") represents a point of attachment of U, and
the dashed line ("") represents a point of attachment of the adjuvant moiety to a spacer. Accordingly, the point of attachment of the linker to the adjuvant core ("P") is the atom in the adjuvant core to which Q is bound.

In some instances, the adjuvant moiety is of formula:

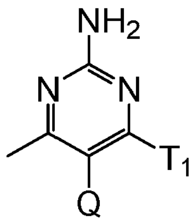

**Adj D-1a-i** .

In certain embodiments, the adjuvant moiety is not of formula:

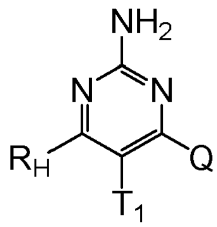

**Adj D-1b-i** ;

wherein

Q is of the formula:

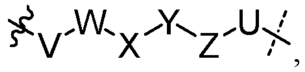

$T_1$ and $R_H$ independently are of the formula:

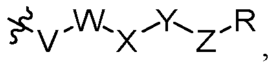

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

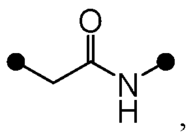

each R independently is hydrogen, halogen (e.g., fluorine, chlorine, bromine, or iodine), nitrile, -COOH, or a linear or branched, saturated or unsaturated $C_1$-$C_4$ alkyl,
the wavy line (" ") represents a point of attachment of Q, $T_1$, and $R_H$,
the dot ("●") represents a point of attachment of U, and
the dashed line (" ") represents a point of attachment of the adjuvant moiety to a spacer.

In certain embodiments, the adjuvant moiety is of formula:

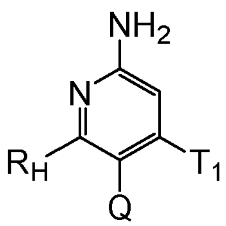

**Adj D-2a** or

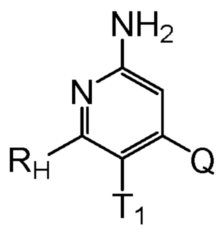

**Adj D-2b** ;

wherein

Q is of the formula:

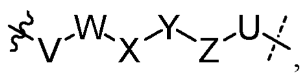

$T_1$ and $R_H$ independently are of the formula:

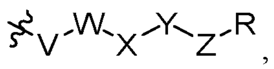

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

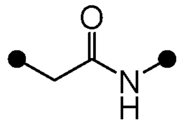
,

each R independently is hydrogen, halogen (e.g., fluorine, chlorine, bromine, or iodine), nitrile, -COOH, or a linear or branched, saturated or unsaturated $C_1$-$C_4$ alkyl,
the wavy line ("") represents a point of attachment of Q, $T_1$, and $R_H$,
the dot ("●") represents a point of attachment of U, and
the dashed line ("") represents a point of attachment of the adjuvant moiety to a spacer. Accordingly, the point of attachment of the linker to the adjuvant core ("P") is the atom in the adjuvant core to which Q is bound.

In some embodiments, the adjuvant moiety is of Formula Adj E:

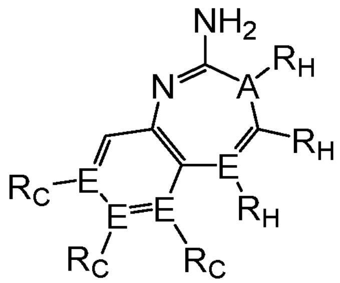

**Adj E** ;

wherein

each E independently is C, CH, or N,
A is CH or N,
each $R_C$ is optionally present and independently is of the formula:

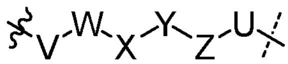 or 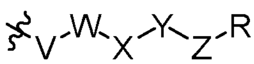 ,

except that at least one $R_C$ is present and is of the formula:

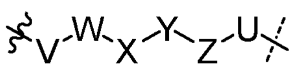 ,

each $R_H$ is optionally present and independently is of the formula:

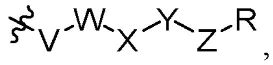

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

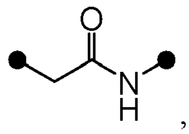

each R independently is hydrogen, halogen (e.g., fluorine, chlorine, bromine, or iodine), nitrile, -COOH, or a linear or branched, saturated or unsaturated $C_1$-$C_4$ alkyl,
the wavy line (" ") represents a point of attachment of $R_C$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which $R_C$ is bound.

In certain embodiments, the adjuvant moiety is of formula:

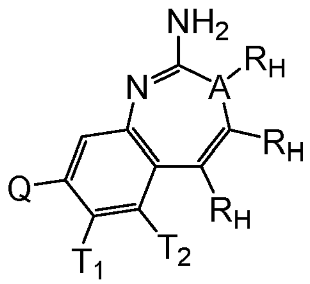

**Adj E-1a** ,

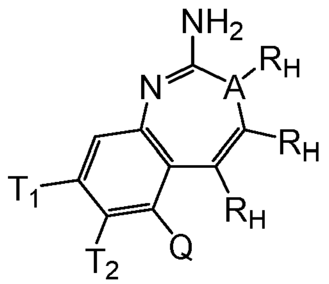

**Adj E-1b** , or

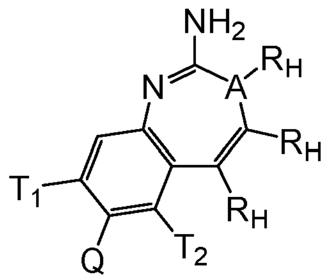

**Adj E-1c** ;

wherein

A is CH or N,
Q is of the formula:

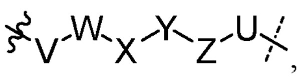

$T_1$, $T_2$, and each $R_H$ independently are of the formula:

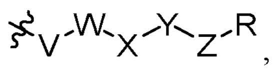

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or

heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

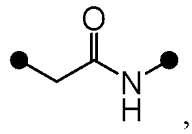

,

each R independently is hydrogen, halogen (e.g., fluorine, chlorine, bromine, or iodine), nitrile, -COOH, or a linear or branched, saturated or unsaturated $C_1$-$C_4$ alkyl,
the wavy line ("~") represents a point of attachment of Q, $T_1$, $T_2$, and $R_H$,
the dot ("●") represents a point of attachment of U, and
the dashed line ("- - -") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

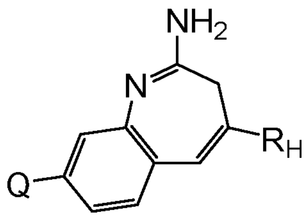

**Adj E-1ai** or

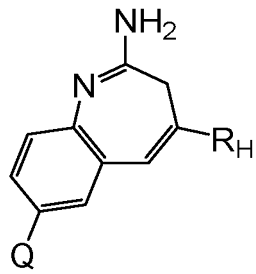

**Adj E-1ci** ;

wherein

Q is of the formula:

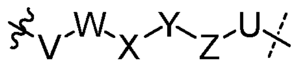

,

$R_H$ is of the formula:

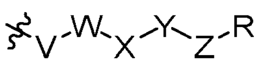

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

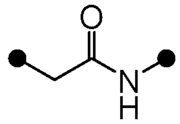

,

each R independently is hydrogen, halogen (e.g., fluorine, chlorine, bromine, or iodine), nitrile, -COOH, or a linear or branched, saturated or unsaturated $C_1$-$C_4$ alkyl,

the wavy line (" ") represents a point of attachment of Q and $R_H$,

the dot ("●") represents a point of attachment of U, and

the dashed line (" ") represents a point of attachment of the adjuvant moiety to a spacer. Accordingly, the point of attachment of the linker to the adjuvant core ("P") is the atom in the adjuvant core to which Q is bound.

In preferred embodiments, the adjuvant moiety is of formula:

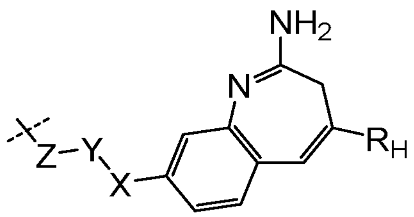

**Adj E-1aii** or

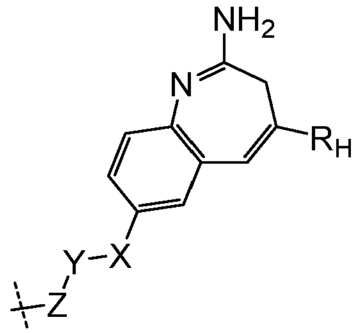

**Adj E-1cii** ;

wherein

$R_H$ is of the formula:

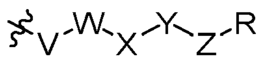

,

V is optionally present and independently is -O-, -S-, -NH-, -NR- or -CO-,

W is optionally present and independently is a linear or branched, saturated or unsaturated, divalent $C_1$-$C_8$ alkyl,

each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,

each Z is optionally present and independently is -O-, -S-, -NH-, or -NR-,

each R independently is hydrogen, halogen (e.g., fluorine, chlorine, bromine, or iodine), nitrile, -COOH, or a linear or branched, saturated or unsaturated $C_1$-$C_4$ alkyl,

the wavy line (" ") represents a point of attachment of $R_H$,

the dashed line (" ") represents a point of attachment of the adjuvant moiety to a spacer.

More preferably, the adjuvant moiety is of formula:

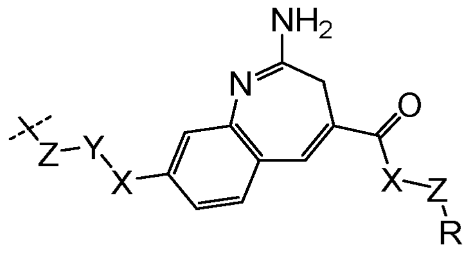

Adj E-1aiii

or

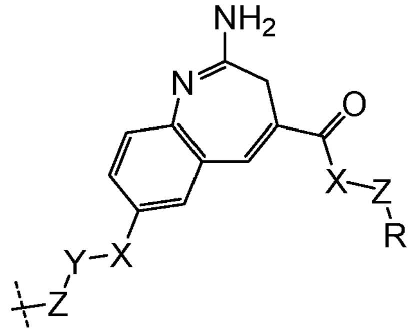

Adj E-1ciii

;

wherein

each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

Y is optionally present and independently is -CO- or a linear or branched, saturated or unsaturated, divalent $C_1$-$C_8$ alkyl,

each Z is optionally present and independently is -O-, -S-, -NH-, or -NR-,

each R independently is hydrogen, halogen (e.g., fluorine, chlorine, bromine, or iodine), nitrile, -COOH, or a linear or branched, saturated or unsaturated $C_1$-$C_4$ alkyl,

the dashed line (" - - - ") represents a point of attachment of the adjuvant moiety to a spacer.

In some embodiments, the adjuvant moiety is of formula:

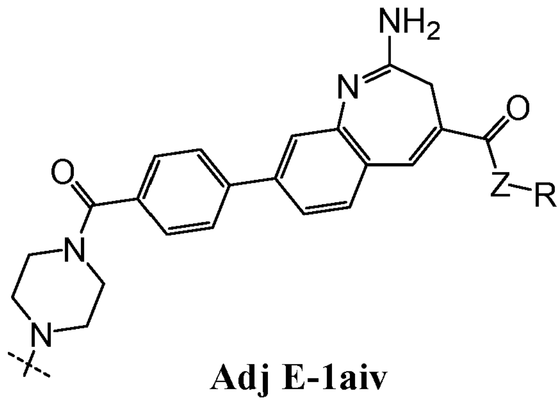

Adj E-1aiv

,

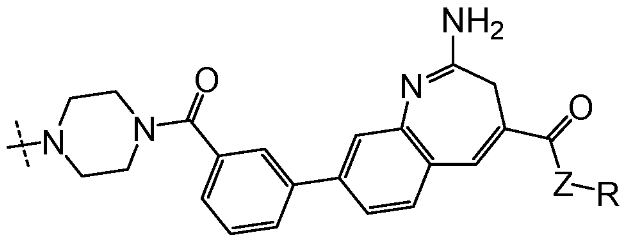

Adj E-1av

,

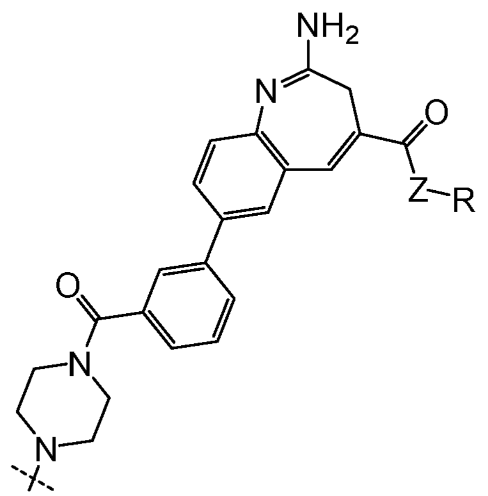

**Adj E-1civ** , or

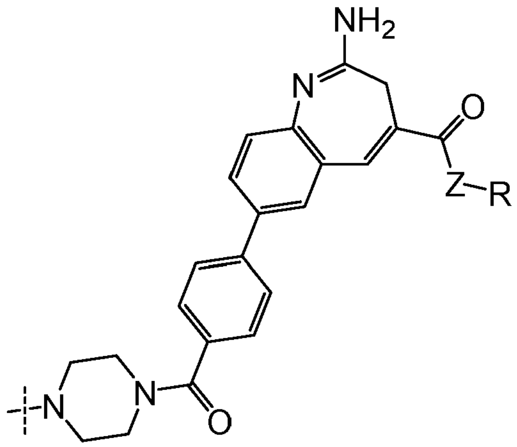

**Adj E-1cv** ;

wherein

Z is optionally present and independently is -O-, -S-, -NH-, or -NR-,
each R independently is hydrogen, halogen (e.g., fluorine, chlorine, bromine, or iodine), nitrile, -COOH, or a linear or branched, saturated or unsaturated $C_1$-$C_4$ alkyl,
the dashed line (" - - - ") represents a point of attachment of the adjuvant moiety to a spacer.

In certain embodiments, the adjuvant moiety is of formula:

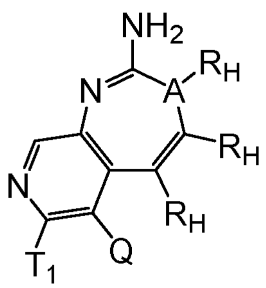

**Adj E-2a** ,

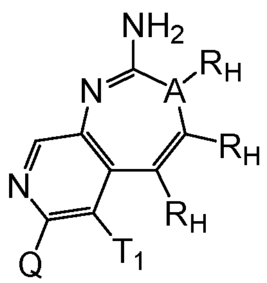

**Adj E-2b** ,

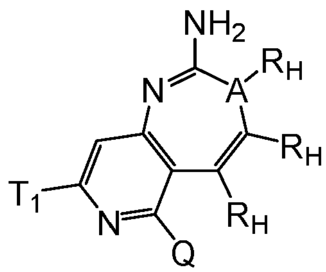

**Adj E-2c** ,

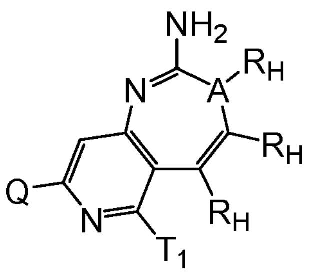

**Adj E-2d** ,

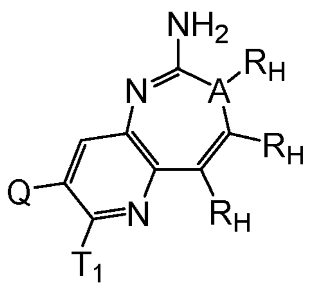

**Adj E-2e** ,

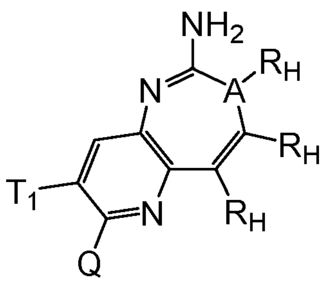

**Adj E-2f** ,

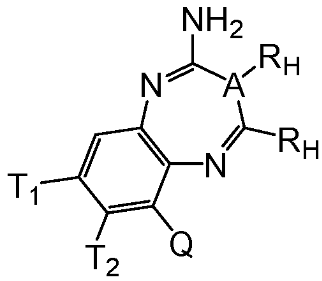

**Adj E-2g** ,

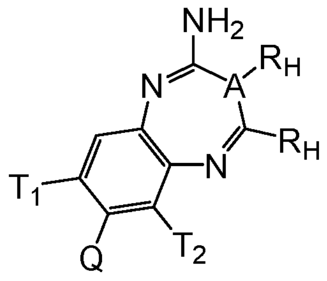

**Adj E-2h** , or

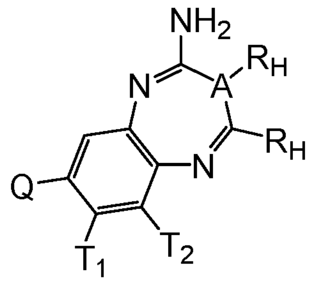

**Adj E-2i** ;

wherein

A is CH or N,

Q is of the formula:

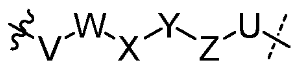

,

$T_1$, $T_2$, and each $R_H$ independently are of the formula:

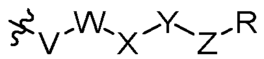

,

each V is optionally present and independently is -O-, -S-, -NH-, -NR- or -CO-,

each W is optionally present and independently is a linear or branched, saturated or unsaturated, divalent $C_1$-$C_8$ alkyl,

each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,

each Z is optionally present and independently is -O-, -S-, -NH-, or -NR-,

U is optionally present and is

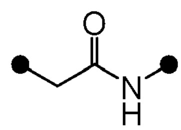

,

each R independently is hydrogen, halogen (e.g., fluorine, chlorine, bromine, or iodine), nitrile, -COOH, or a linear or branched, saturated or unsaturated $C_1$-$C_4$ alkyl,

the wavy line (" ") represents a point of attachment of Q, $T_1$, $T_2$, and $R_H$,

the dot ("●") represents a point of attachment of U, and

the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

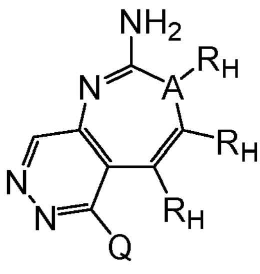

Adj E-3a ,

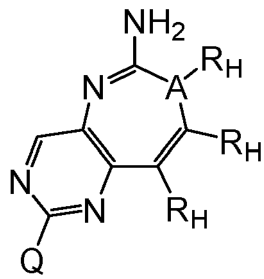

Adj E-3b ,

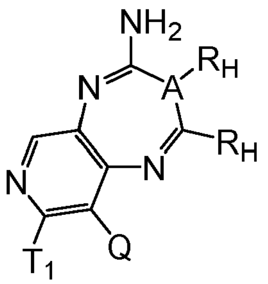

Adj E-3c ,

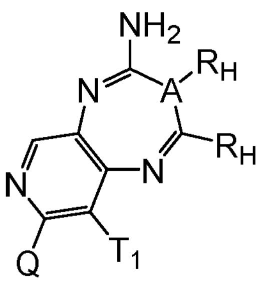

Adj E-3d ,

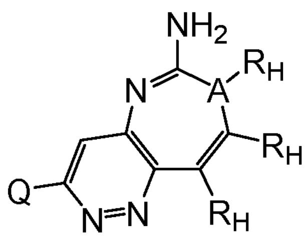

Adj E-3e ,

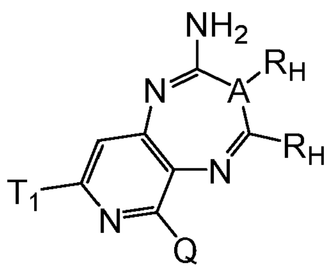

Adj E-3f ,

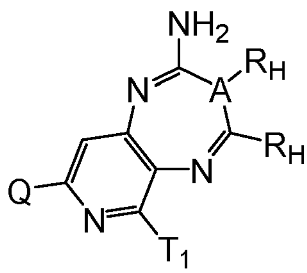

Adj E-3g ,

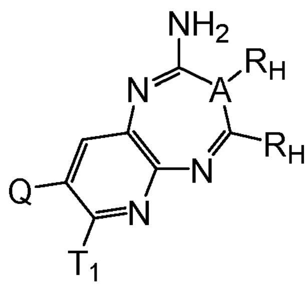

Adj E-3h , or

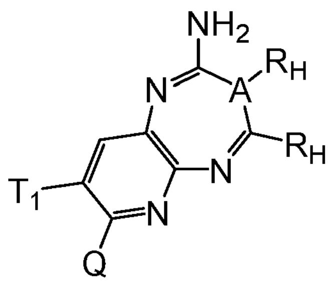

Adj E-3i ;

wherein

A is CH or N,
Q is of the formula:

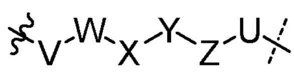

,

$T_1$, and each $R_H$ independently are of the formula:

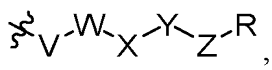

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is ,

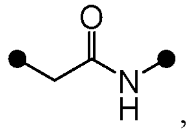
,

each R independently is hydrogen, halogen (e.g., fluorine, chlorine, bromine, or iodine), nitrile, -COOH, or a linear or branched, saturated or unsaturated $C_1$-$C_4$ alkyl
the wavy line (" ") represents a point of attachment of Q, $T_1$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

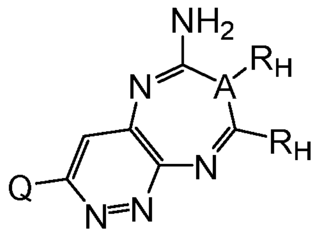
**Adj E-4a** ,
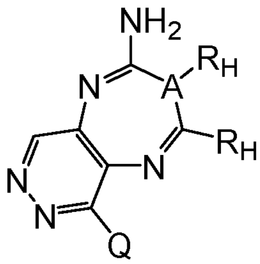
**Adj E-4b** , or
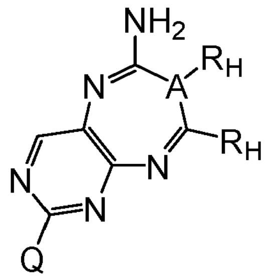
**Adj E-4c** ;

wherein

A is CH or N,
Q is of the formula:

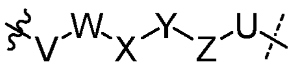
,

each $R_H$ independently is of the formula:

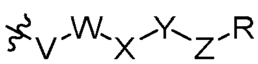
,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or linear or branched, saturated or unsaturated, divalent $C_1$-$C_8$ alkyl,
each Z is optionally present and independently is -O-, -S-, -NH-, or -NR-,
U is optionally present and is

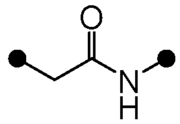,

each R independently is hydrogen, halogen (e.g., fluorine, chlorine, bromine, or iodine), nitrile, -COOH, or a linear or branched, saturated or unsaturated $C_1$-$C_4$ alkyl,
the wavy line ("~") represents a point of attachment of Q and $R_H$,
the dot ("●") represents a point of attachment of U, and
the dashed line ("- - -") represents a point of attachment of the adjuvant moiety to a spacer. Accordingly, the point of attachment of the linker to the adjuvant core ("P") is the atom in the adjuvant core to which Q is bound.

In some embodiments, the adjuvant moiety is of Formula Adj F:

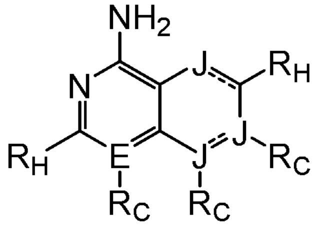

**Adj F** ;

wherein

each J independently is C, CH, $CH_2$, N, NH, O, or S,
E is C, CH, or N,
each $R_C$ is optionally present and independently is of the formula:

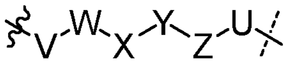 or 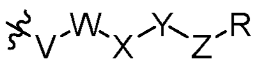,

except that at least one $R_C$ is present and is of the formula:

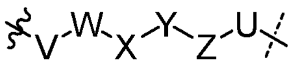,

each $R_H$ independently is of the formula:

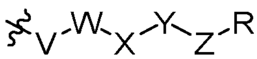,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

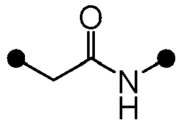,

each R independently is hydrogen, halogen (e.g., fluorine, chlorine, bromine, or iodine), nitrile, -COOH, or a linear or branched, saturated or unsaturated $C_1$-$C_4$ alkyl,

each " " independently represents a single bond or a double bond,

the wavy line (" ") represents a point of attachment of $R_C$, and $R_H$,

the dot ("●") represents a point of attachment of U, and

the dashed line (" ") represents a point of attachment of the adjuvant moiety to a spacer. Accordingly, the point of attachment of the linker to the adjuvant core ("P") is the atom in the adjuvant core to which $R_C$ is bound.

In certain embodiments, the adjuvant moiety is of formula:

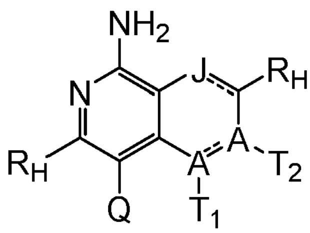

**Adj F-1a** ,

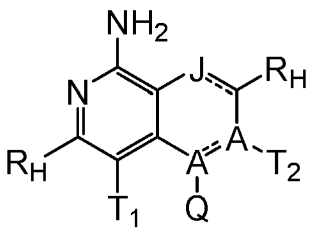

**Adj F-1b** , or

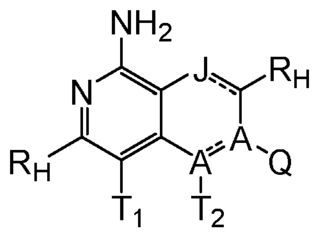

**Adj F-1c** ;

wherein

each A independently is CH or N,

J is CH, $CH_2$, N, NH, O, or S,

Q is of the formula:

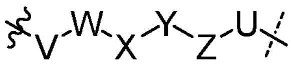,

$T_1$, $T_2$, and each $R_H$ independently are of the formula:

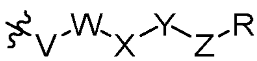,

each V is optionally present and independently is -O-, -S-, -NH-, -NR- or -CO-,

each W is optionally present and independently is a linear or branched, saturated or unsaturated, divalent $C_1$-$C_8$ alkyl,

each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,

each Z is optionally present and independently is -O-, -S-, -NH-, or -NR-,

U is optionally present and is

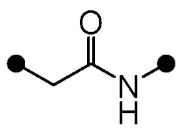,

each R independently is hydrogen, halogen (e.g., fluorine, chlorine, bromine, or iodine), nitrile, -COOH, or a linear or branched, saturated or unsaturated $C_1$-$C_4$ alkyl,

each " " independently represents a single bond or a double bond,

the wavy line (" ") represents a point of attachment of Q, $T_1$, $T_2$, and $R_H$,

the dot ("●") represents a point of attachment of U, and

the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

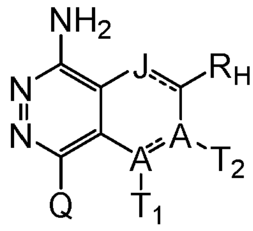

**Adj F-2a** ,

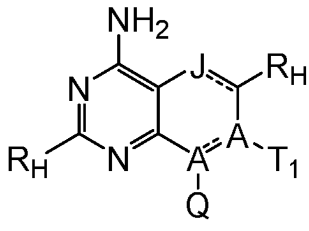

**Adj F-2b** , or

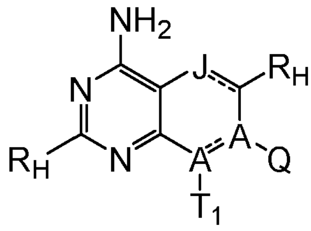

**Adj F-2c** ;

wherein

each A independently is CH or N,

J is CH, $CH_2$, N, NH, O, or S,

Q is of the formula:

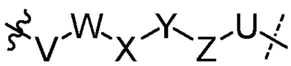

,

$T_1$, $T_2$, and each $R_H$ independently are of the formula:

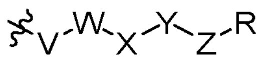

,

each V is optionally present and independently is -O-, -S-, -NH-, -NR- or -CO-,

each W is optionally present and independently is a linear or branched, saturated or unsaturated, divalent $C_1$-$C_8$ alkyl,

each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,

each Z is optionally present and independently is -O-, -S-, -NH-, or -NR-,

U is optionally present and is

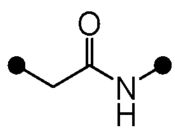

,

each R independently is hydrogen, halogen (e.g., fluorine, chlorine, bromine, or iodine), nitrile, -COOH, or a linear or branched, saturated or unsaturated $C_1$-$C_4$ alkyl,

each " " independently represents a single bond or a double bond,

the wavy line (" ") represents a point of attachment of Q, $T_1$, $T_2$, and $R_H$,

the dot ("●") represents a point of attachment of U, and
the dashed line (" - - - ") represents a point of attachment of the adjuvant moiety to a spacer. Accordingly, the point of attachment of the linker to the adjuvant core ("P") is the atom in the adjuvant core to which Q is bound.

In some embodiments, the adjuvant moiety is of Formula Adj G:

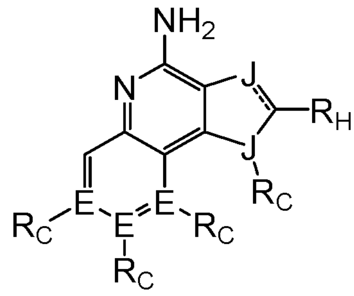

**Adj G** ;

wherein

each J independently is C, CH, $CH_2$, N, NH, O, or S,
each E independently is C, CH, or N,
each $R_C$ is optionally present and independently is of the formula:

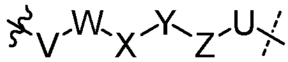 or 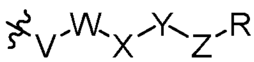 ,

except that at least one $R_C$ is present and is of the formula:

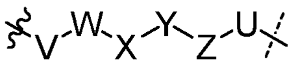 ,

$R_H$ is of the formula:

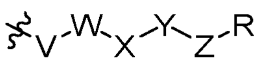 ,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

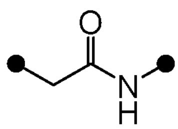 ,

each R independently is hydrogen, halogen (e.g., fluorine, chlorine, bromine, or iodine), nitrile, -COOH, or a linear or branched, saturated or unsaturated $C_1$-$C_4$ alkyl,

" " represents a single bond or a double bond,
the wavy line (" ") represents a point of attachment of $R_C$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which $R_C$ is bound.

In certain embodiments, the adjuvant moiety is of formula:

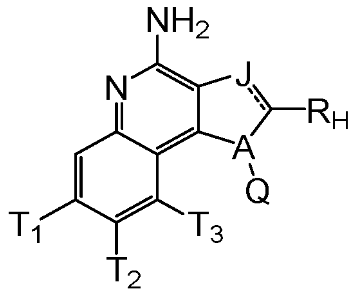

**Adj G-1a** ,

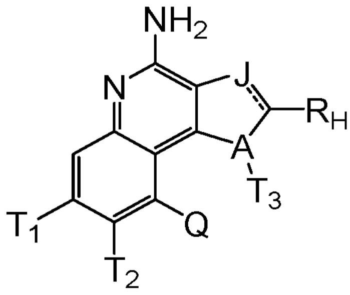

**Adj G-1b** ,

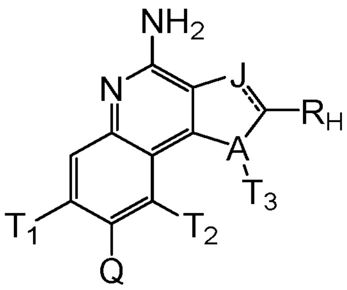

**Adj G-1c** , or

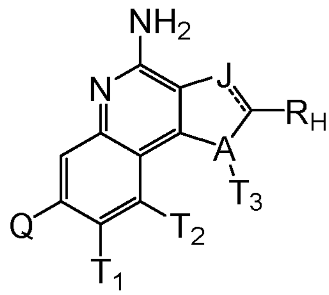

**Adj G-1d** ;

wherein

A is CH or N,
J is CH, $CH_2$, N, NH, O, or S,
Q is of the formula:

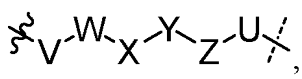

$T_1$, $T_2$, $T_3$, and $R_H$ independently are of the formula:

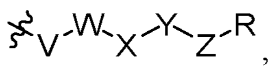

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,

each Z is optionally present and independently is -O-, -S-, -NH-, or -NR-,
U is optionally present and is

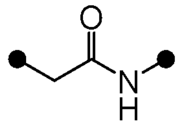,

each R independently is hydrogen, halogen (e.g., fluorine, chlorine, bromine, or iodine), nitrile, -COOH, or a linear or branched, saturated or unsaturated $C_1$-$C_4$ alkyl,
" " represents a single bond or a double bond,
the wavy line (" ") represents a point of attachment of Q, $T_1$, $T_2$, $T_3$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

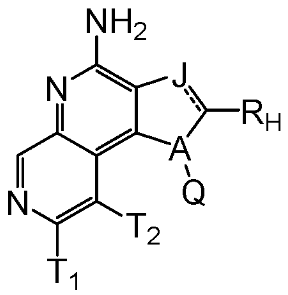

**Adj G-2a** ,

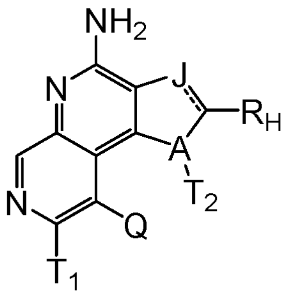

**Adj G-2b** ,

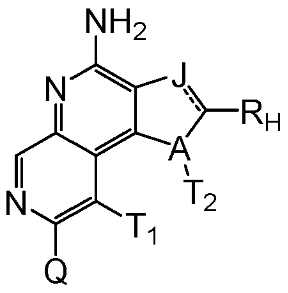

**Adj G-2c** ,

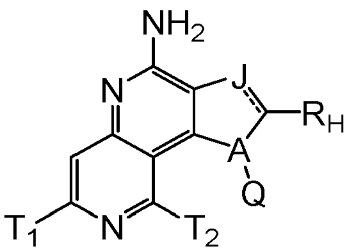

**Adj G-2d** ,

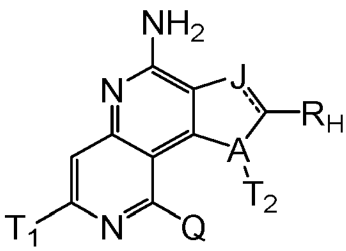

**Adj G-2e** ,

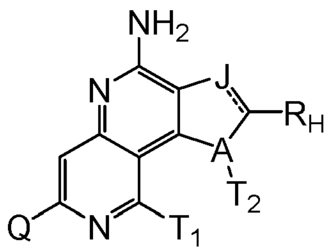

**Adj G-2f** ,

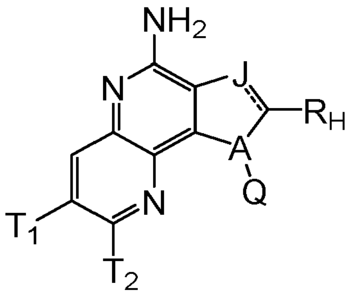

**Adj G-2g**,

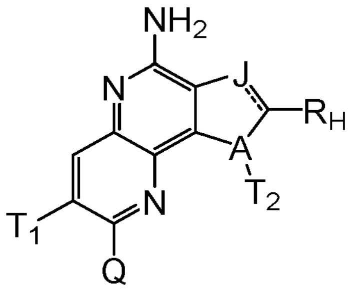

**Adj G-2h**, or

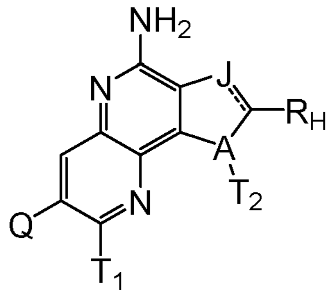

**Adj G-2i**;

wherein

A is CH or N,
J is CH, $CH_2$, N, NH, O, or S,
Q is of the formula:

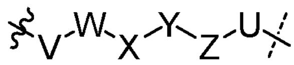

,

$T_1$, $T_2$, and $R_H$ independently are of the formula:

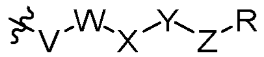

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

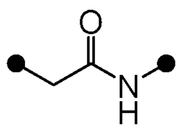

,

each R independently is hydrogen, halogen (e.g., fluorine, chlorine, bromine, or iodine), nitrile, -COOH, or a linear or branched, saturated or unsaturated $C_1$-$C_4$ alkyl,
" " represents a single bond or a double bond,
the wavy line (" ") represents a point of attachment of Q, $T_1$, $T_2$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In some embodiments, the adjuvant moiety is of formula:

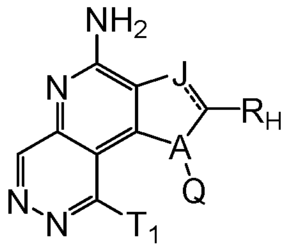

**Adj G-3a** ,

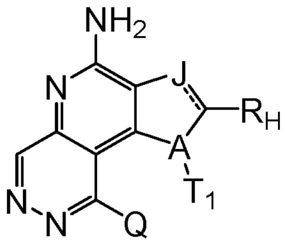

**Adj G-3b** ,

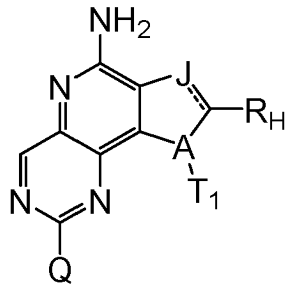

**Adj G-3c** ,

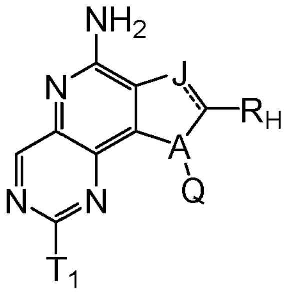

**Adj G-3d** ,

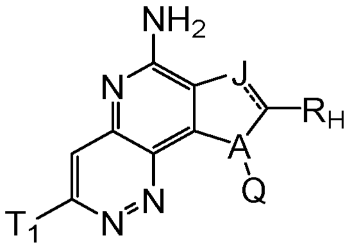

**Adj G-3e** , or

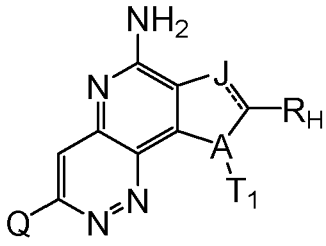

**Adj G-3f** ;

wherein

A is CH or N,
J is CH, $CH_2$, N, NH, O, or S,
Q is of the formula:

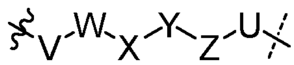

,

$T_1$ and $R_H$ independently are of the formula:

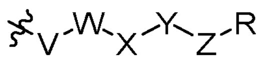

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

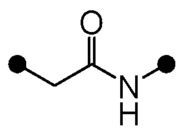

,

each R independently is hydrogen, halogen (e.g., fluorine, chlorine, bromine, or iodine), nitrile, -COOH, or a linear or branched, saturated or unsaturated $C_1$-$C_4$ alkyl; and " " represents a single bond or a double bond,
the wavy line (" ") represents a point of attachment of Q, $T_1$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

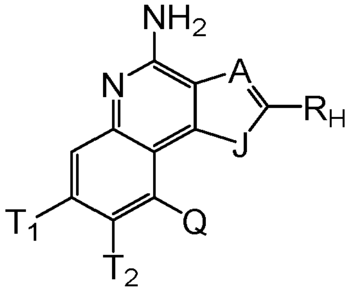

**Adj G-4a** ,

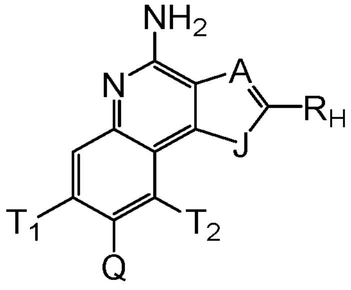

**Adj G-4b** , or

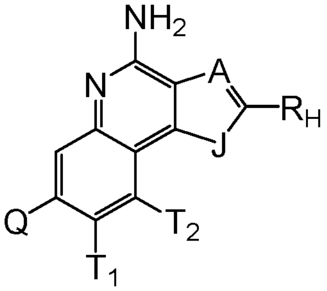

**Adj G-4c** ;

wherein

A is CH or N,
J is $CH_2$, NH, O, or S,
Q is of the formula:

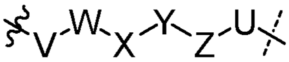
,

$T_1$, $T_2$, and $R_H$ independently are of the formula:

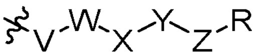
,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

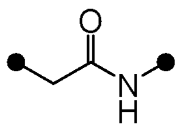
,

each R independently is hydrogen, halogen (e.g., fluorine, chlorine, bromine, or iodine), nitrile, -COOH, or a linear or branched, saturated or unsaturated $C_1$-$C_4$ alkyl,
the wavy line (" ") represents a point of attachment of Q, $T_1$, $T_2$, and $R_H$,
the dot ("●") represents a point of attachment of U, and

the dashed line ("") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

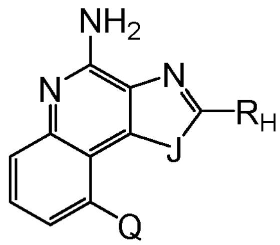

**Adj G-5a** ,

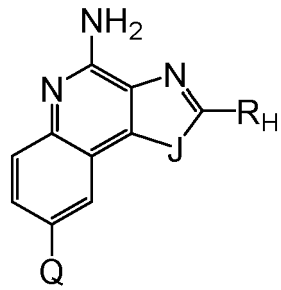

**Adj G-5b** ,

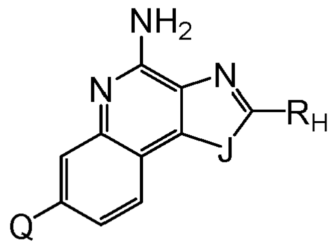

**Adj G-5c**

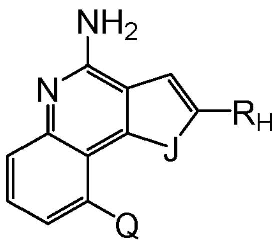

**Adj G-5d** ,

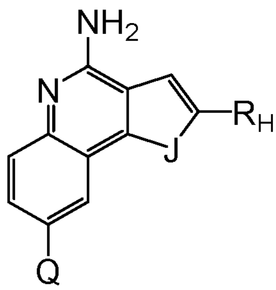

**Adj G-5e** , or

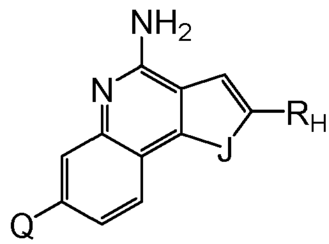

**Adj G-5f** ;

;

wherein

J is $CH_2$, NH, O, or S,
Q is of the formula:

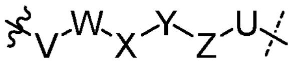

,

$R_H$ is of the formula:

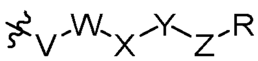

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

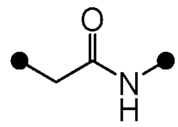,

each R independently is hydrogen, halogen (e.g., fluorine, chlorine, bromine, or iodine), nitrile, -COOH, or a linear or branched, saturated or unsaturated $C_1$-$C_4$ alkyl,

the wavy line (" ") represents a point of attachment of Q and $R_H$,

the dot ("●") represents a point of attachment of U, and

the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

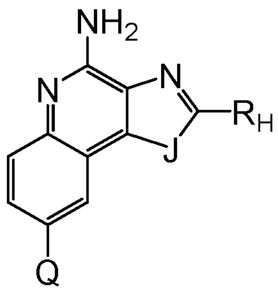

**Adj G-6a** ,

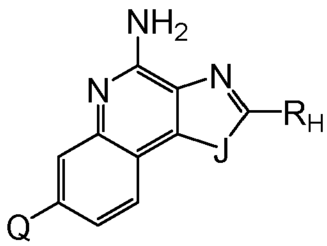

**Adj G-6b**

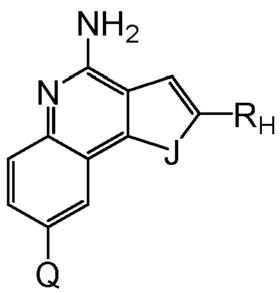

**Adj G-6c** , or

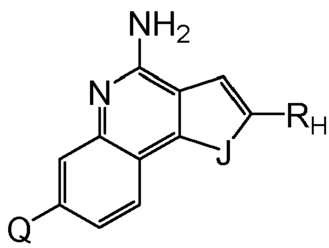

**Adj G-6d** ;

wherein

J is $CH_2$, NH, O, or S,

Q is of the formula:

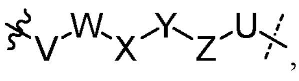,

$R_H$ is of the formula:

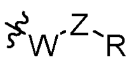,

V is optionally present and is -O-, -S-, -NH-, -NR- or -CO-,

each W is optionally present and independently is a linear or branched, saturated or unsaturated, divalent $C_1$-$C_8$ alkyl,

X is optionally present and is one, two, three, or four divalent cycloalkyl, heterocycloalkyl, aryl, or heteroaryl groups,

and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

Y is optionally present and is -CO- or a linear or branched, saturated or unsaturated, divalent $C_1$-$C_8$ alkyl,

each Z is optionally present and independently is -O-, -S-, -NH-, or -NR-,

U is optionally present and is

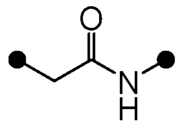

,

each R independently is hydrogen, halogen (e.g., fluorine, chlorine, bromine, or iodine), nitrile, -COOH, or a linear or branched, saturated or unsaturated $C_1$-$C_4$ alkyl,

the wavy line (" ") represents a point of attachment of Q and $R_H$,

the dot ("●") represents a point of attachment of U, and

the dashed line (" ") represents a point of attachment of the adjuvant moiety to a spacer. Accordingly, the point of attachment of the linker to the adjuvant core ("P") is the atom in the adjuvant core to which Q is bound.

In preferred embodiments, the adjuvant moiety is of formula:

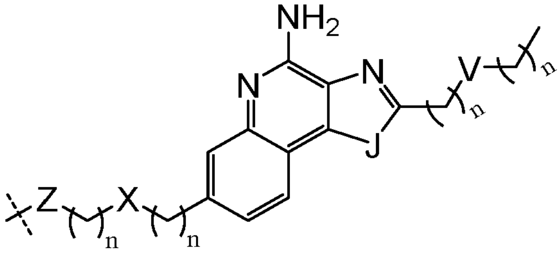

**Adj G-7a**

,

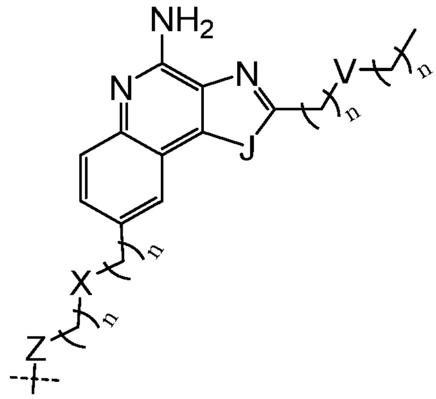

**Adj G-7b**

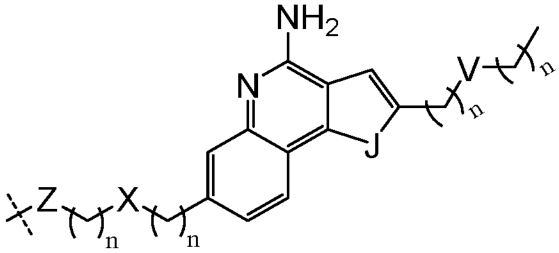

**Adj G-7c**

, or

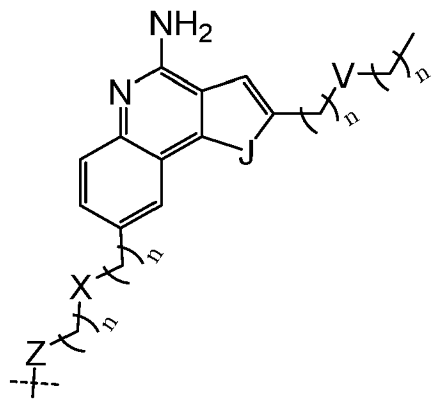

**Adj G-7d**

;

wherein

J is $CH_2$, NH, O, or S,

V is optionally present and is -O-, -S-, -NH-, -NR- or -CO-,

X is optionally present and is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
Z is optionally present and is -O-, -S-, -NH-, or -NR-,
provided that at least X or Z is present,
each R independently is hydrogen, halogen (e.g., fluorine, chlorine, bromine, or iodine), nitrile, -COOH, or a linear or branched, saturated or unsaturated $C_1$-$C_4$ alkyl,
each n independently is an integer from 0 to 4, and
the dashed line (" --- ") represents a point of attachment of the adjuvant moiety to a spacer.

More preferably, the adjuvant moiety is of formula:

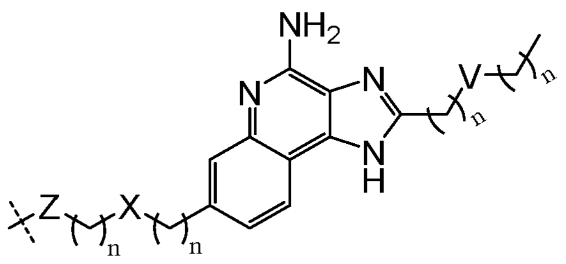

**Adj G-7ai**

or

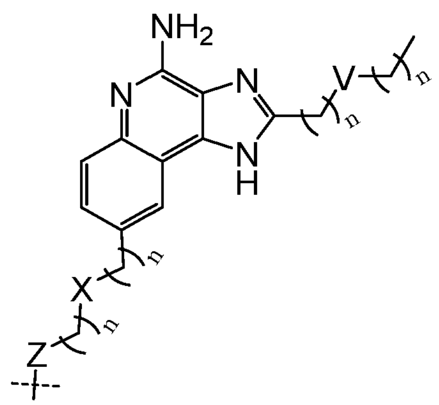

**Adj G-7bi**

wherein

V is optionally present and is -O-, -S-, -NH-, -NR- or -CO-,
X is optionally present and is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
Z is optionally present and is -O-, -S-, -NH-, or -NR-,
provided that at least X or Z is present,
each R independently is hydrogen, halogen (e.g., fluorine, chlorine, bromine, or iodine), nitrile, -COOH, or a linear or branched, saturated or unsaturated $C_1$-$C_4$ alkyl,
each n independently is an integer from 0 to 4, and
the dashed line (" --- ") represents a point of attachment of the adjuvant moiety to a spacer.

In some embodiments, the adjuvant moiety is of formula:

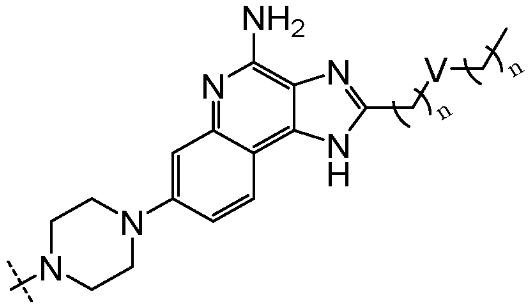

**Adj G-7aii** or

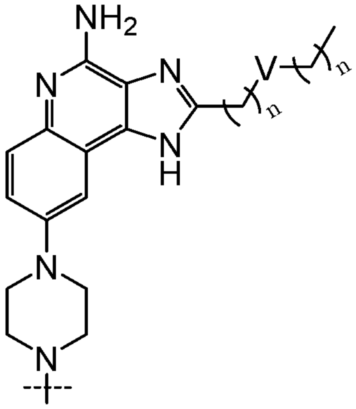

**Adj G-7bii**

wherein

V is optionally present and is -O-, -S-, -NH-, -NR- or -CO-,
R is hydrogen, halogen (e.g., fluorine, chlorine, bromine, or iodine), nitrile, -COOH, or a linear or branched, saturated or unsaturated $C_1$-$C_4$ alkyl,
each n independently is an integer from 0 to 4, and
the dashed line (" ⸺ ") represents a point of attachment of the adjuvant moiety to a spacer.

In some embodiments, the adjuvant moiety is not of formula:

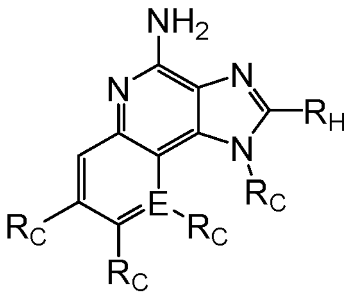

**Adj G-8a** or

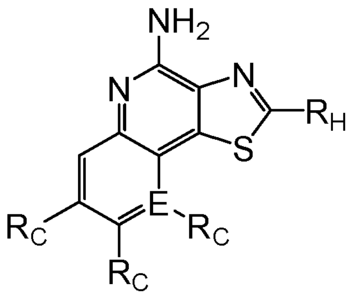

**Adj G-8b** ;

wherein

E is C, CH, or N,
each $R_C$ is optionally present and independently is of the formula:

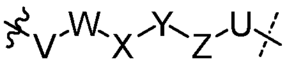 or 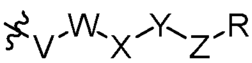,

except that at least one $R_C$ is present and is of the formula:

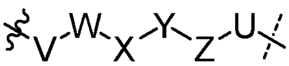,

$R_H$ is of the formula:

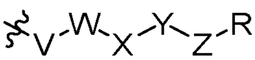,

each V is optionally present and independently is -O-, -S-, -NH-, -NR- or -CO-,

each W is optionally present and independently is a linear or branched, saturated or unsaturated, divalent $C_1$-$C_8$ alkyl,

each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,

each Z is optionally present and independently is -O-, -S-, -NH-, or -NR-,

U is optionally present and is

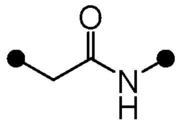,

each R independently is hydrogen, halogen (e.g., fluorine, chlorine, bromine, or iodine), nitrile, -COOH, or a linear or branched, saturated or unsaturated $C_1$-$C_4$ alkyl,

" " represents a single bond or a double bond,

the wavy line (" ") represents a point of attachment of $R_C$, and $R_H$,

the dot ("●") represents a point of attachment of U, and

the dashed line (" ") represents a point of attachment of the adjuvant moiety to a spacer.

In some embodiments, the adjuvant moiety is of Formula Adj H:

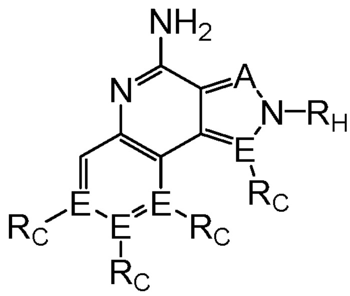

**Adj H** ;

wherein

A is CH or N,

each E independently is C, CH, or N,

each $R_C$ is optionally present and independently is of the formula:

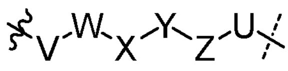 or 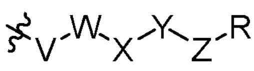,

except that at least one $R_C$ is present and is of the formula:

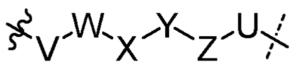,

$R_H$ is of the formula:

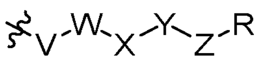,

each V is optionally present and independently is -O-, -S-, -NH-, -NR- or -CO-,

each W is optionally present and independently is a linear or branched, saturated or unsaturated, divalent $C_1$-$C_8$ alkyl,

each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,

each Z is optionally present and independently is -O-, -S-, -NH-, or -NR-,

U is optionally present and is

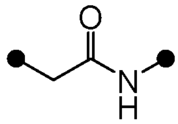
,

each R independently is hydrogen, halogen (e.g., fluorine, chlorine, bromine, or iodine), nitrile, -COOH, or a linear or branched, saturated or unsaturated $C_1$-$C_4$ alkyl,

" ═ " represents a single bond or a double bond,

the wavy line (" ≀ ") represents a point of attachment of $R_C$, and $R_H$,

the dot ("●") represents a point of attachment of U, and

the dashed line (" - - - ") represents a point of attachment of the adjuvant moiety to a spacer. Accordingly, the point of attachment of the linker to the adjuvant core ("P") is the atom in the adjuvant core to which $R_C$ is bound.

In certain embodiments, the adjuvant moiety is of formula:

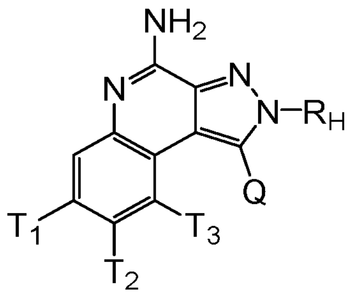

**Adj H-1a** ,

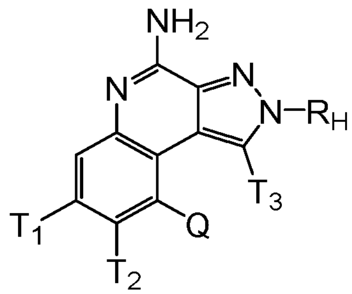

**Adj H-1b** ,

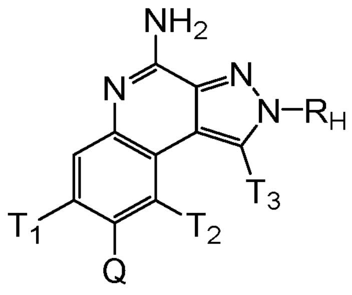

**Adj H-1c** , or

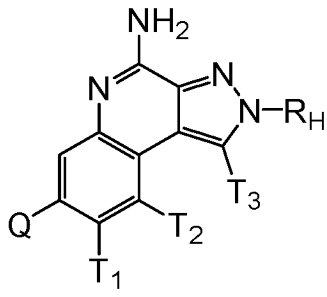

**Adj H-1d** ;

wherein

Q is of the formula:

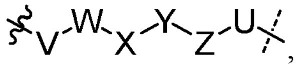,

$T_1$, $T_2$, $T_3$, and $R_H$ independently are of the formula:

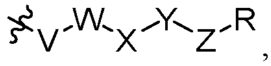,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

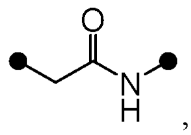,

each R independently is hydrogen, halogen (e.g., fluorine, chlorine, bromine, or iodine), nitrile, -COOH, or a linear or branched, saturated or unsaturated $C_1$-$C_4$ alkyl,
" " represents a single bond or a double bond,
the wavy line (" ") represents a point of attachment of Q, $T_1$, $T_2$, $T_3$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

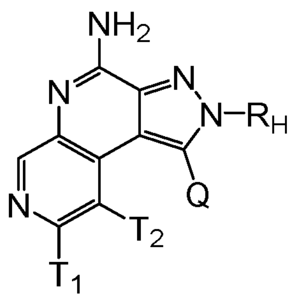

**Adj H-2a** ,

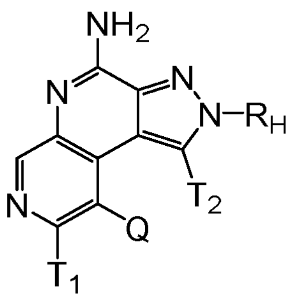

**Adj H-2b** ,

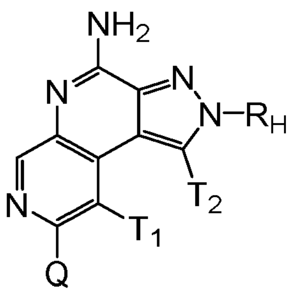

**Adj H-2c** ,

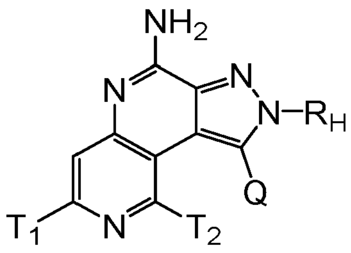

**Adj H-2d** ,

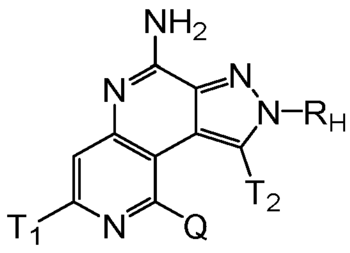

**Adj H-2e** ,

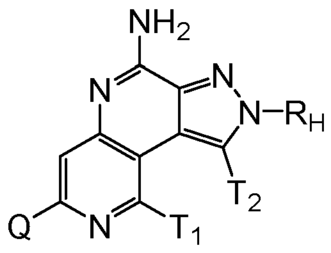

**Adj H-2f** ,

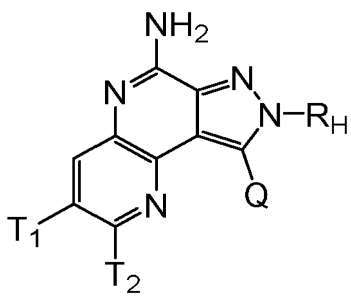

**Adj H-2g** ,

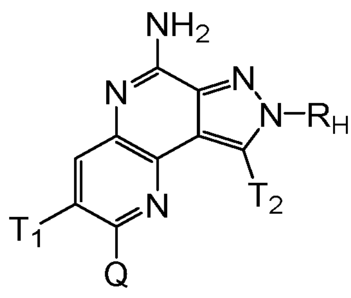

**Adj H-2h** , or

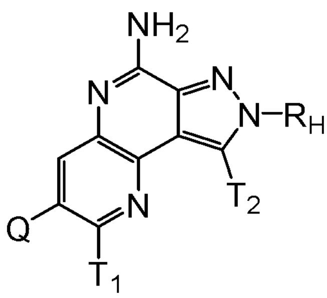

**Adj H-2i** ;

wherein

Q is of the formula:

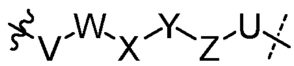
,

$T_1$, $T_2$, and $R_H$ independently are of the formula:

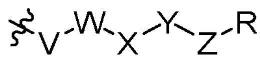
,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

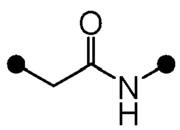
,

each R independently is hydrogen, halogen (e.g., fluorine, chlorine, bromine, or iodine), nitrile, -COOH, or a linear or branched, saturated or unsaturated $C_1$-$C_4$ alkyl,

" " represents a single bond or a double bond,
the wavy line (" ") represents a point of attachment of Q, $T_1$, $T_2$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

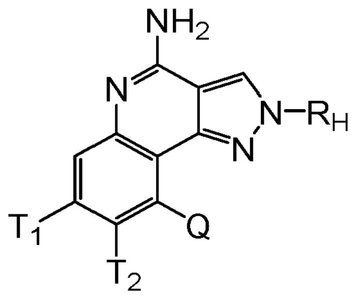

Adj H-3a ,

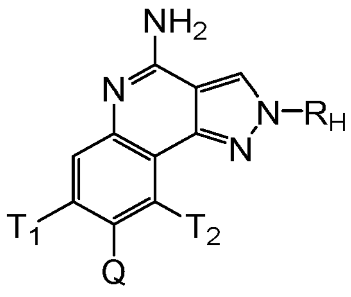

Adj H-3b , or

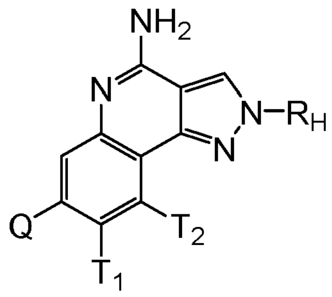

Adj H-3c ;

wherein

Q is of the formula:

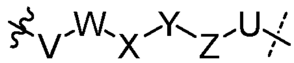

,

$T_1$, $T_2$, and $R_H$ independently are of the formula:

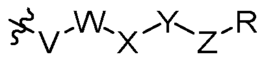

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

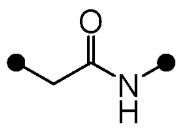

,

each R independently is hydrogen, halogen (e.g., fluorine, chlorine, bromine, or iodine), nitrile, -COOH, or a linear or branched, saturated or unsaturated $C_1$-$C_4$ alkyl,
" " represents a single bond or a double bond,
the wavy line (" ") represents a point of attachment of Q, $T_1$, $T_2$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

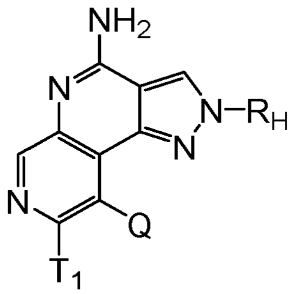

**Adj H-4a** ,

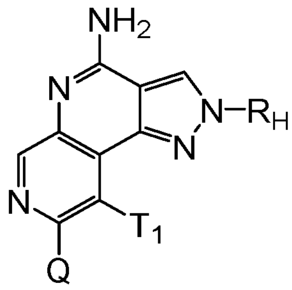

**Adj H-4b** ,

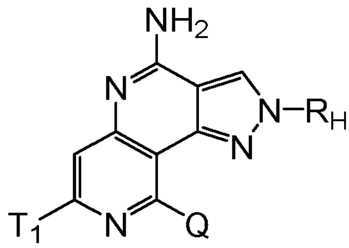

**Adj H-4c** ,

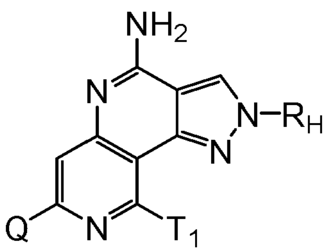

**Adj H-4d** ,

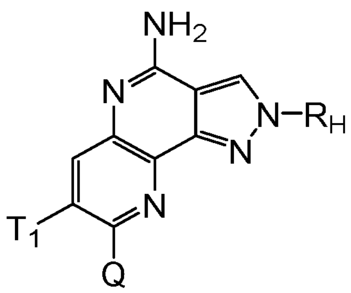

**Adj H-4e** , or

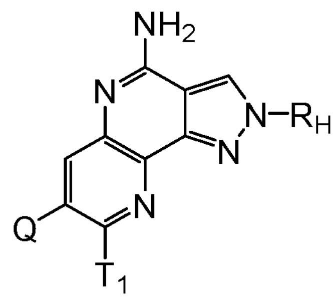

**Adj H-4f** ;

wherein

Q is of the formula:

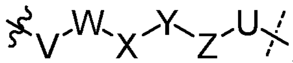

,

$T_1$ and $R_H$ independently are of the formula:

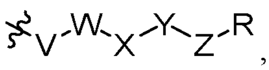

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

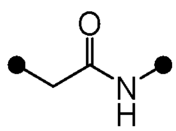

,

each R independently is hydrogen, halogen (e.g., fluorine, chlorine, bromine, or iodine), nitrile, -COOH, or a linear or branched, saturated or unsaturated $C_1$-$C_4$ alkyl,
" " represents a single bond or a double bond,
the wavy line (" ") represents a point of attachment of Q, $T_1$, and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

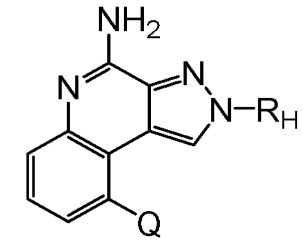

**Adj H-5a** ,

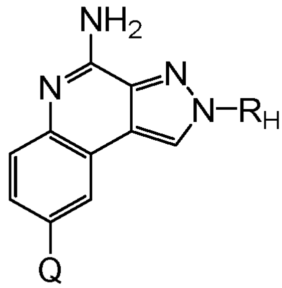

**Adj H-5b** ,

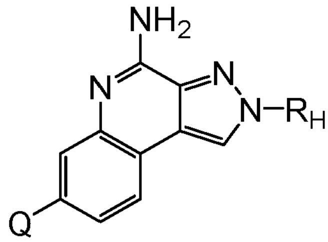

**Adj H-5c**

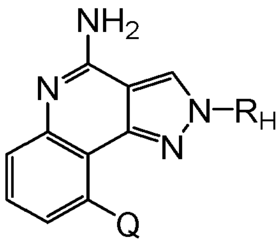

**Adj H-5d** ,

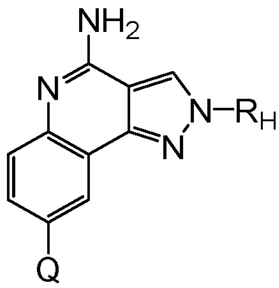

**Adj H-5e** , or

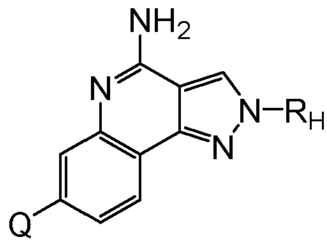

**Adj H-5f** ;

;

wherein

Q is of the formula:

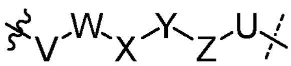

,

$R_H$ is of the formula:

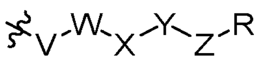

,

each V is optionally present and independently is -O-, -S-, -NH-, -NR- or -CO-,
each W is optionally present and independently is a linear or branched, saturated or unsaturated, divalent $C_1$-$C_8$ alkyl,
each X is optionally present and independently is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

each Y is optionally present and independently is -CO- or a linear or branched, saturated or unsaturated, divalent $C_1$-$C_8$ alkyl,
each Z is optionally present and independently is -O-, -S-, -NH-, or -NR-,
U is optionally present and is

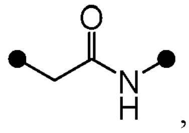
,

each R independently is hydrogen, halogen (e.g., fluorine, chlorine, bromine, or iodine), nitrile, -COOH, or a linear or branched, saturated or unsaturated $C_1$-$C_4$ alkyl,
the wavy line (" ") represents a point of attachment of Q and $R_H$,
the dot ("●") represents a point of attachment of U, and
the dashed line (" ") represents a point of attachment of the adjuvant moiety to a spacer. Accordingly, the point of attachment of the linker to the adjuvant core ("P") is the atom in the adjuvant core to which Q is bound.

In certain embodiments, the adjuvant moiety is of formula:

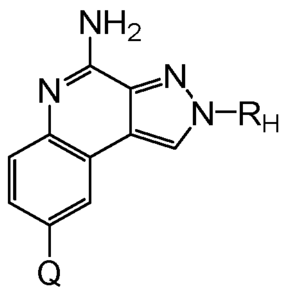

**Adj H-6a** ,

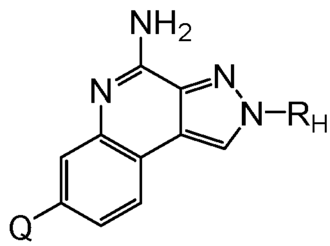

**Adj H-6b**

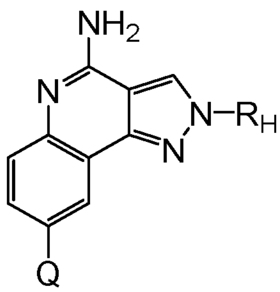

**Adj H-6c** , or

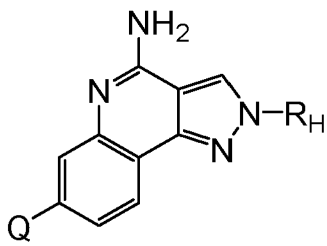

**Adj H-6d** ;

wherein

Q is of the formula:

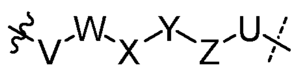
,

$R_H$ is of the formula:

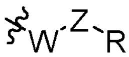
,

V is optionally present and is -O-, -S-, -NH-, -NR- or -CO-,

each W is optionally present and independently is a linear or branched, saturated or unsaturated, divalent $C_1$-$C_8$ alkyl,

X is optionally present and is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,

Y is optionally present and is -CO- or a linear or branched, saturated or unsaturated, divalent $C_1$-$C_8$ alkyl,

each Z is optionally present and independently is -O-, -S-, -NH-, or -NR-,

U is optionally present and is

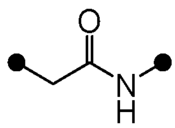,

each R independently is hydrogen, halogen (e.g., fluorine, chlorine, bromine, or iodine), nitrile, -COOH, or a linear or branched, saturated or unsaturated $C_1$-$C_4$ alkyl,

the wavy line ("⌇") represents a point of attachment of Q and $R_H$,

the dot ("●") represents a point of attachment of U, and

the dashed line ("- - -") represents a point of attachment of the adjuvant moiety to a spacer. Accordingly, the point of attachment of the linker to the adjuvant core ("P") is the atom in the adjuvant core to which Q is bound.

In preferred embodiments, the adjuvant moiety is of formula:

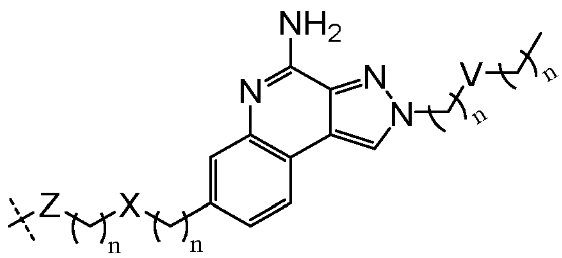

**Adj H-7a**,

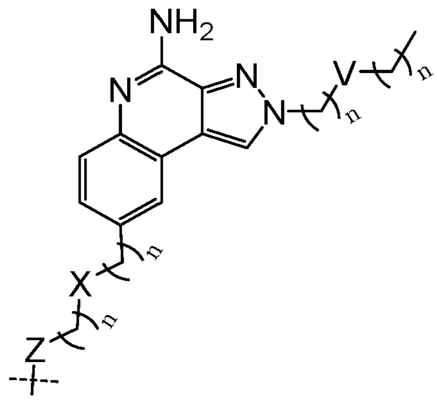

**Adj H-7b**

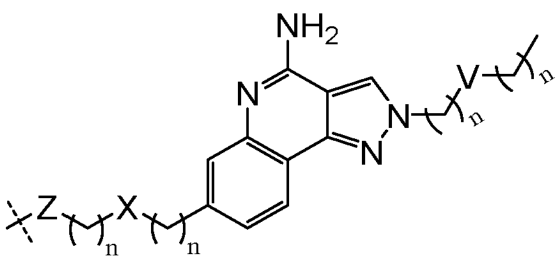

**Adj H-7c**, or

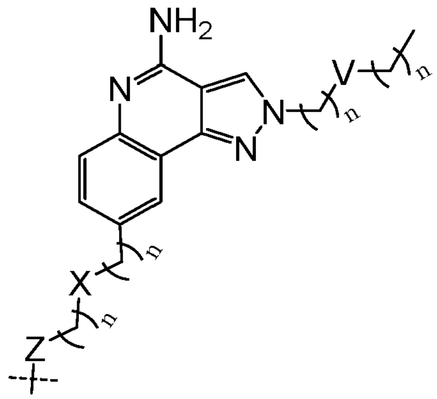

**Adj H-7d**;

wherein

V is optionally present and is -O-, -S-, -NH-, -NR- or -CO-,
X is optionally present and is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
Z is optionally present and is -O-, -S-, -NH-, or -NR-,
provided that at least X or Z is present,
each R independently is hydrogen, halogen (e.g., fluorine, chlorine, bromine, or iodine), nitrile, -COOH, or a linear or branched, saturated or unsaturated $C_1$-$C_4$ alkyl,
each n independently is an integer from 0 to 4, and
the dashed line ("---") represents a point of attachment of the adjuvant moiety to a spacer.

More preferably, the adjuvant moiety is of formula:

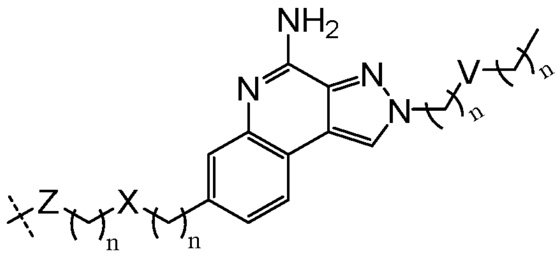

**Adj H-7ai**

or

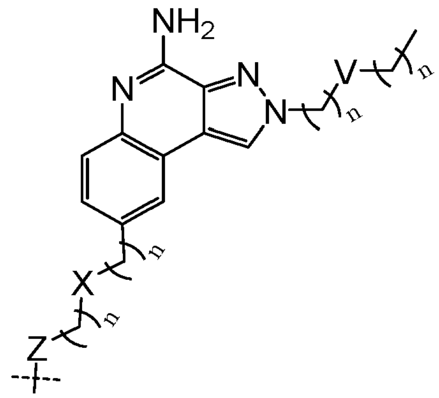

**Adj H-7bi**

wherein

V is optionally present and is -O-, -S-, -NH-, -NR- or -CO-,
X is optionally present and is one, two, three, or four divalent cycloalkyl, heterocycloalkyl, aryl, or heteroaryl groups, and when more than one divalent cycloalkyl, heterocycloalkyl, aryl, or heteroaryl group is present, the more than one divalent cycloalkyl, heterocycloalkyl, aryl, or heteroaryl groups are linked or fused, wherein linked divalent cycloalkyl, heterocycloalkyl, aryl, or heteroaryl groups are linked through a bond or -CO-,
Z is optionally present and is -O-, -S-, -NH-, or -NR-,
provided that at least X or Z is present,
each R independently is hydrogen, halogen (e.g., fluorine, chlorine, bromine, or iodine), nitrile, -COOH, or a linear or branched, saturated or unsaturated $C_1$-$C_4$ alkyl,
each n independently is an integer from 0 to 4, and
the dashed line ("---") represents a point of attachment of the adjuvant moiety to a spacer.

In some embodiments, the adjuvant moiety is of formula:

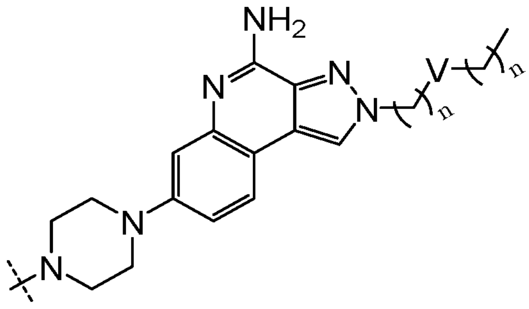

**Adj H-7aii** or

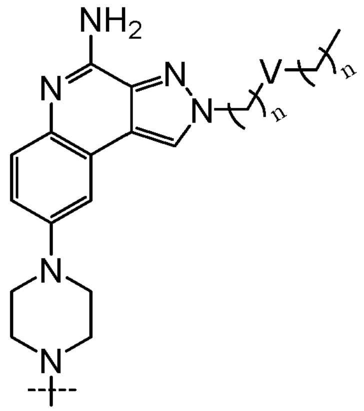

**Adj H-7bii**

wherein

V is optionally present and is -O-, -S-, -NH-, -NR- or -CO-,

R is hydrogen, halogen (e.g., fluorine, chlorine, bromine, or iodine), nitrile, -COOH, or a linear or branched, saturated or unsaturated $C_1$-$C_4$ alkyl,

each n independently is an integer from 0 to 4, and

the dashed line (" ‐‐‐") represents a point of attachment of the adjuvant moiety to a spacer.

In certain embodiments of the invention, one or more aromatic hydrogen atoms in formulas Adj E can be substituted with a halogen atom (e.g., fluorine, chlorine, bromine, iodine, or combinations thereof).

In some embodiments, X is one or more divalent groups selected from benzene, naphthalene, pyrrole, indole, isoindole, indolizine, furan, benzofuran, benzothiophene, thiophene, pyridine, acridine, naphthyridine, quinolone, isoquinoline, isoxazole, oxazole, benzoxazole, isothiazole, thiazole, benzthiazole, imidazole, thiadiazole, tetrazole, triazole, oxadiazole, benzimidazole, purine, pyrazole, pyrazine, pteridine, quinoxaline, phthalazine, quinazoline, triazine, phenazine, cinnoline, pyrimidine, pyridazine, cyclohexane, decahydronaphthalene, pyrrolidine, octahydroindole, octahydroisoindole, tetrahydrofuran, octahydrobenzofuran, octahydrobenzothiophene, tetrahydrothiophene, piperidine, tetradecahydroacridine, naphthyridine, decahydroquinoline, decahydroisoquinoline, isoxazolidine, oxazolidine, octahydrobenzooxazole, isothiazolidine, thiazolidine, octahydrobenzothiazole, imidazolidine, 1,2,3-thiadiazolidine, tetrazolidine, 1,2,3-triazolidine, 1,2,3-oxadiazolidine, octahydrobenzoimidazole, octahydropurine, pyrazolidine, piperazine, dechydropteridine, decahydroquinoxaline, dechydrophthalazine, dechydroquinazoline, 1,3,5-triazinane, tetradecahydrophenazine, decahydrocinnoline, hexhydropyrimidine, or hexahydropyridazine. In some embodiments, the one or more divalent groups of X are fused. In some embodiments, the one or more divalent groups of X are linked through a bond or - CO-.

In certain embodiments, X is of the formula:

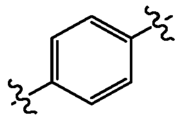 , 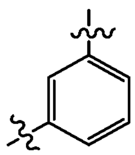 , 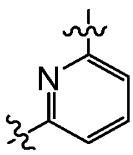 , 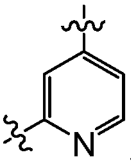 , 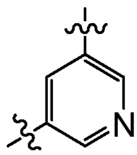 , 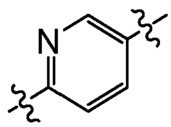 , 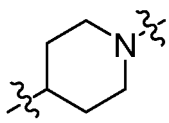 ,

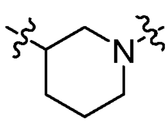 , 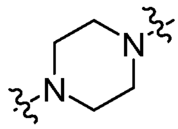 , 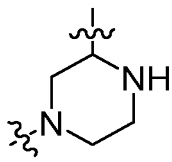 , 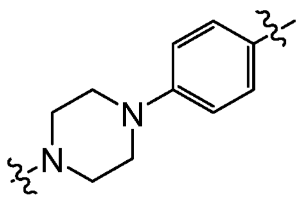 , 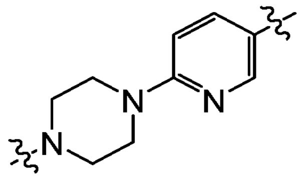 ,

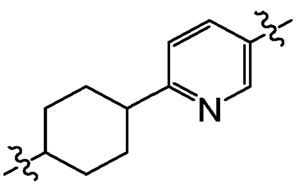, 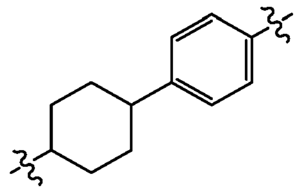, 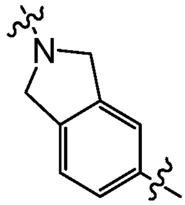, 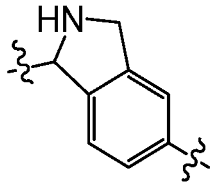,

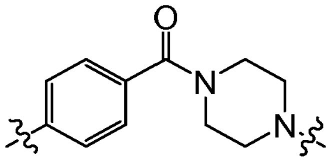, 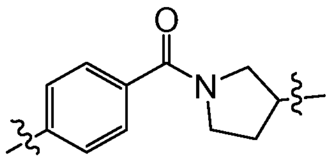, 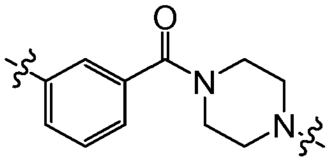, or

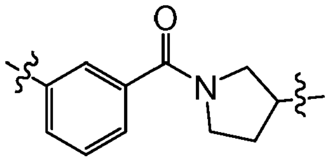,

wherein any of the above-referenced structures can be used bilaterally.

In certain embodiments, the adjuvant moiety is not S-27609, CL307, UC-IV150, imiquimod, gardiquimod, resiquimod, motolimod, VTS-1463GS-9620, GSK2245035, TMX-101, TMX-201, TMX-202, isatoribine, AZD8848, MEDI9197, 3M-051, 3M-852, 3M-052, 3M-854A, S-34240, KU34B, or CL663, ORN02, ORN06, CL075, CL097, CL264, or loxoribine.

In certain embodiments, the adjuvant ("Adj") is:

Table 1.

| Adj | Structure | Adj | Structure |
|---|---|---|---|
| 1 | 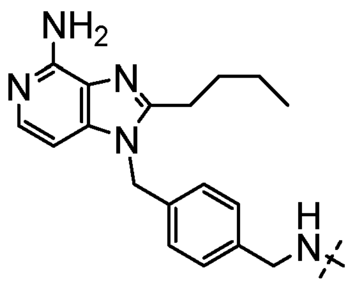 | 2 | 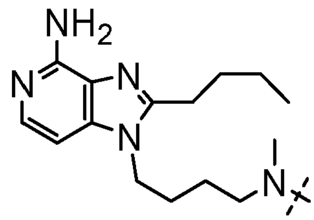 |
| 3 | 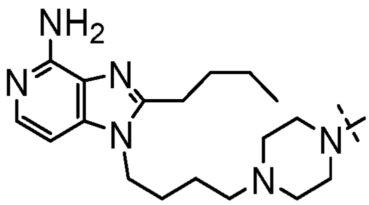 | 4 | 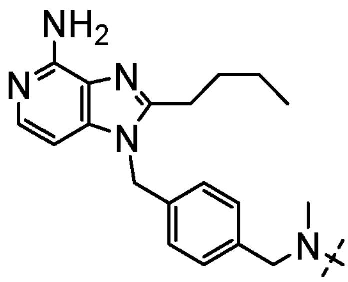 |
| 5 | 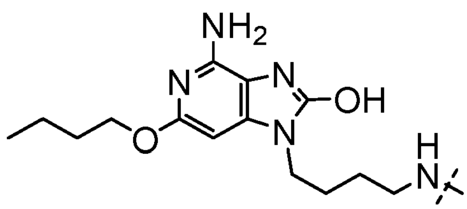 | 6 | 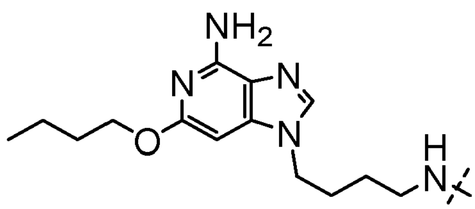 |

(continued)

| Adj | Structure | Adj | Structure |
|---|---|---|---|
| 7 | 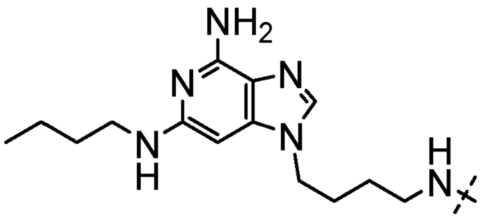 | 8 | 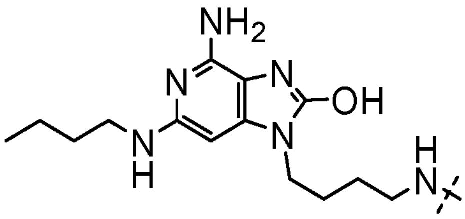 |
| 9 | 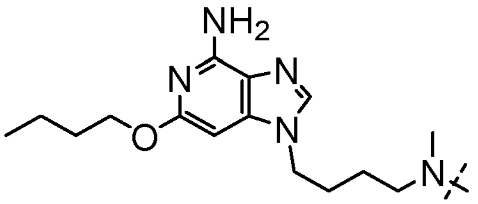 | 10 | 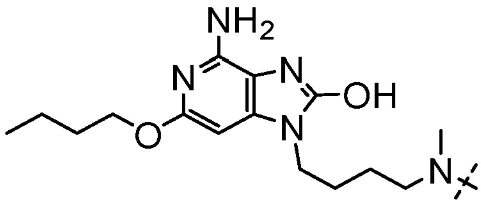 |
| 11 | 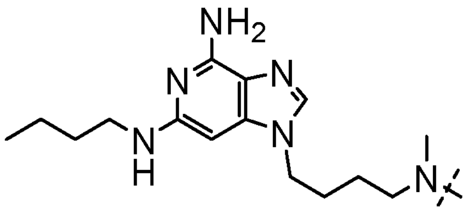 | 12 | 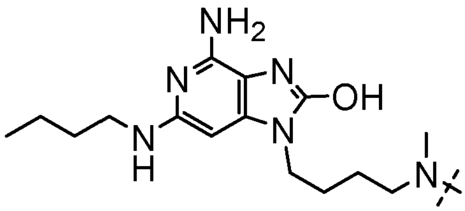 |
| 13 | 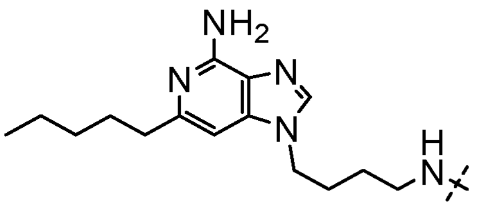 | 14 | 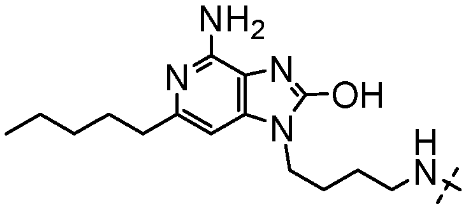 |
| 15 | 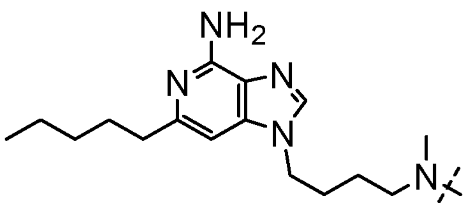 | 16 | 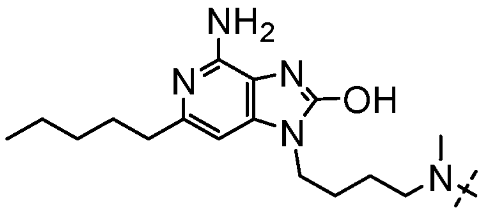 |
| 17 | 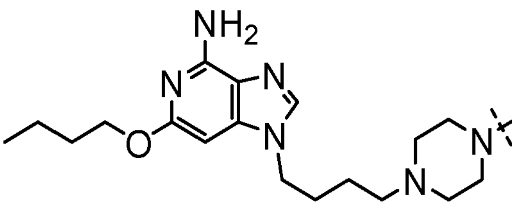 | 18 | 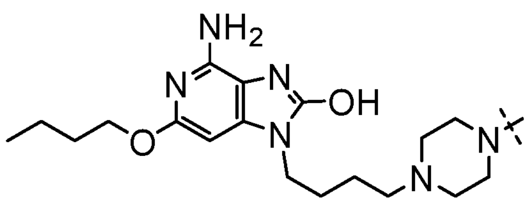 |
| 19 | 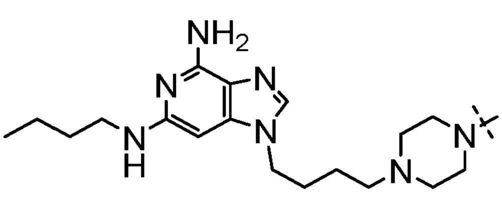 | 20 | 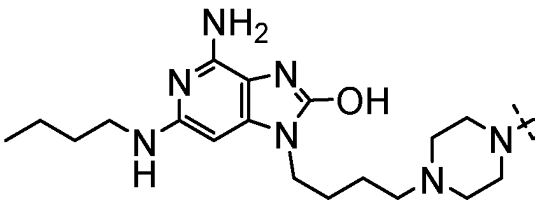 |

(continued)

| Adj | Structure | Adj | Structure |
|---|---|---|---|
| 21 | 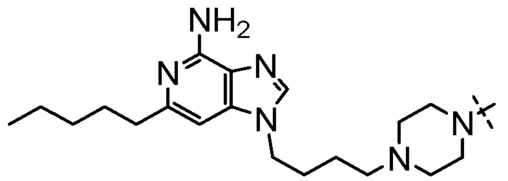 | 22 | 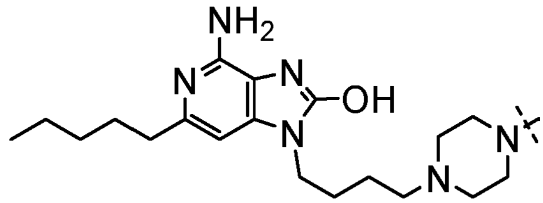 |
| 23 | 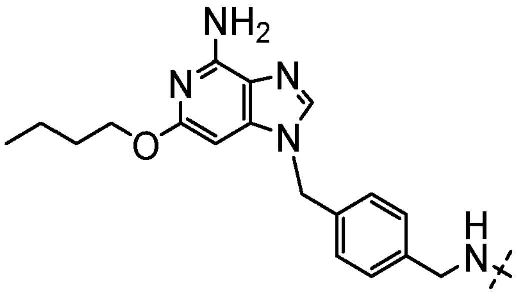 | 24 | 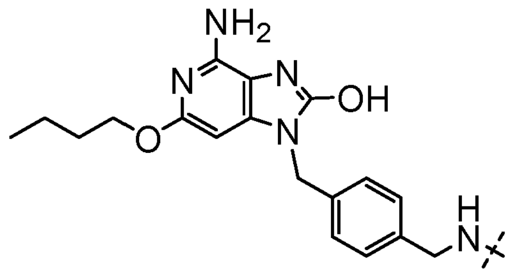 |
| 25 | 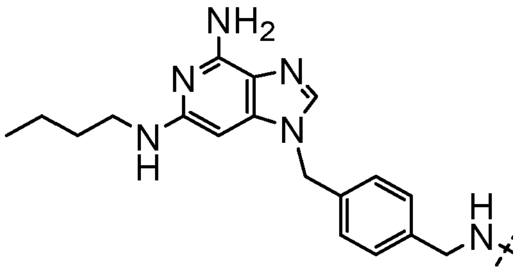 | 26 | 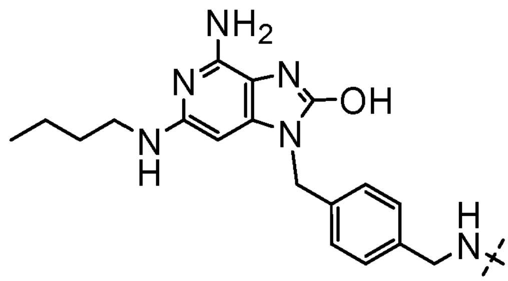 |
| 27 | 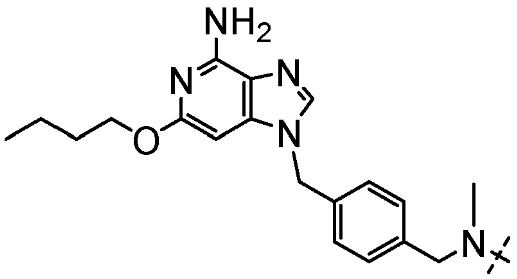 | 28 | 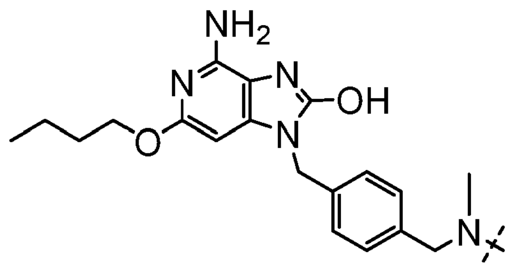 |
| 29 | 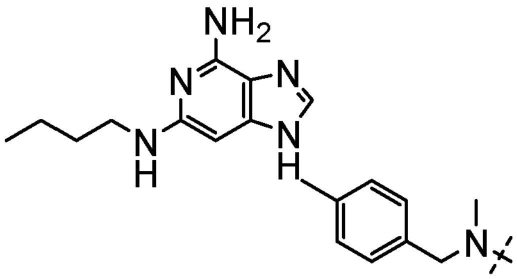 | 30 | 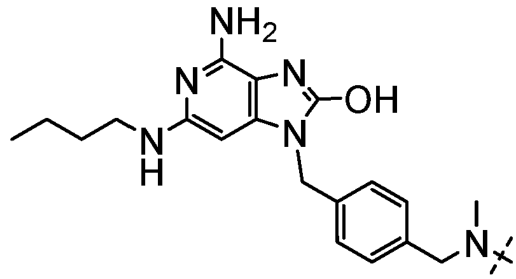 |

(continued)

| Adj | Structure | Adj | Structure |
|---|---|---|---|
| 31 | 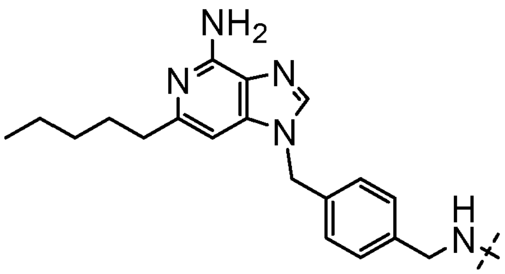 | 32 | 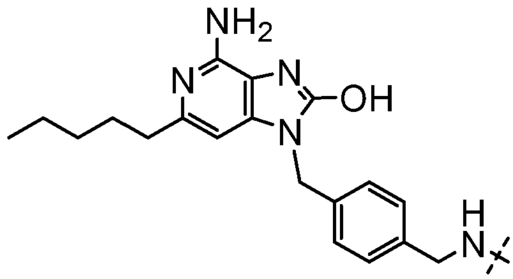 |
| 33 | 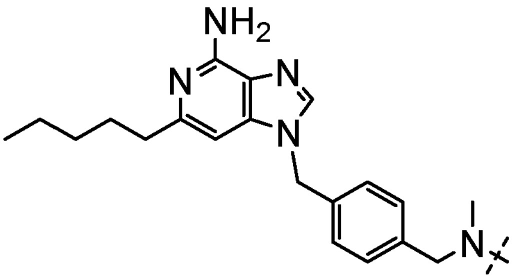 | 34 | 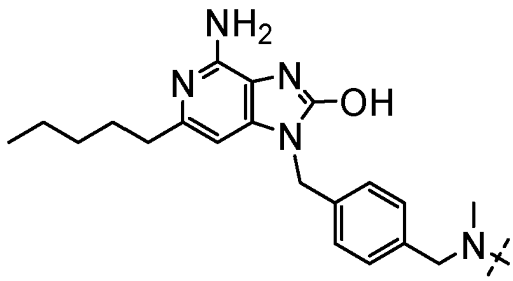 |
| 35 | 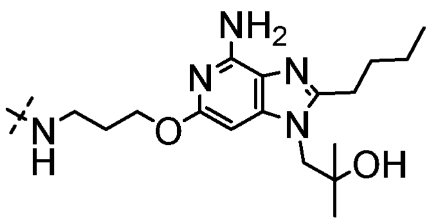 | 36 | 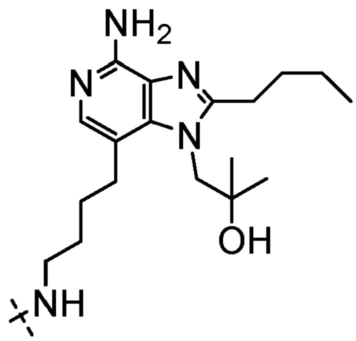 |
| 37 | 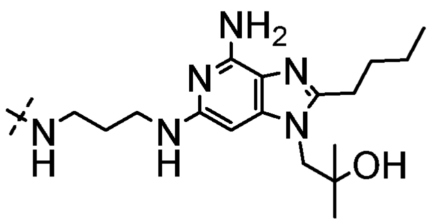 | 38 | 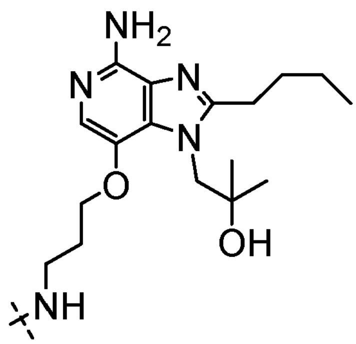 |
| 39 | 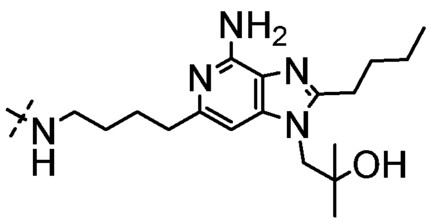 | 40 | 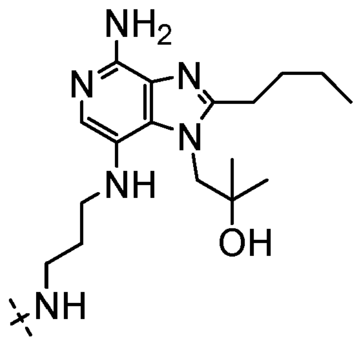 |

(continued)

| Adj | Structure | Adj | Structure |
|---|---|---|---|
| 41 | 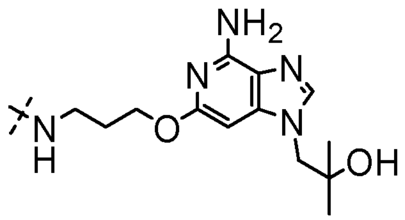 | 42 | 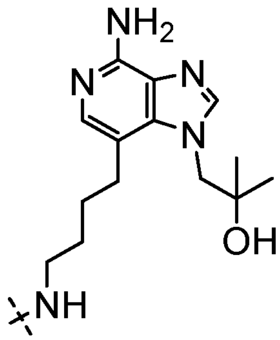 |
| 43 | 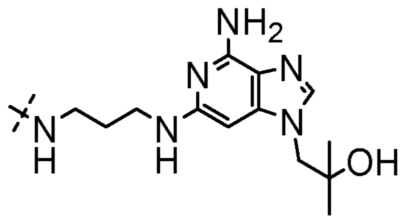 | 44 | 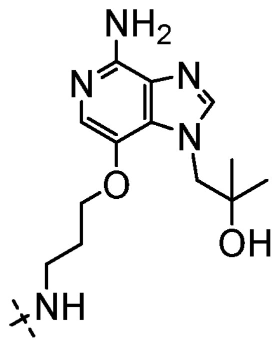 |
| 45 | 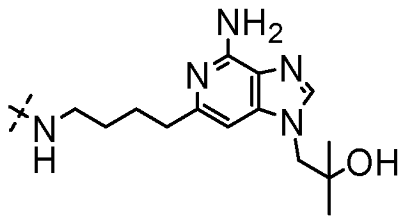 | 46 | 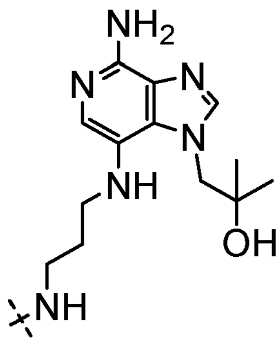 |
| 47 | 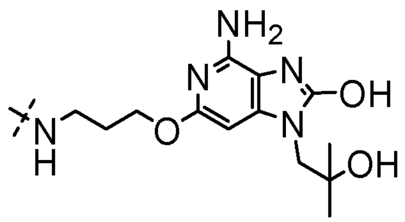 | 48 | 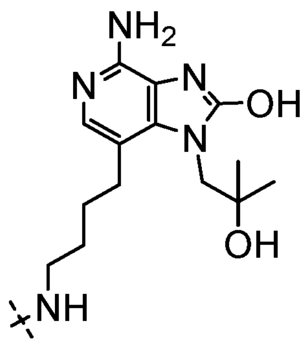 |

(continued)

| Adj | Structure | Adj | Structure |
|---|---|---|---|
| 49 | 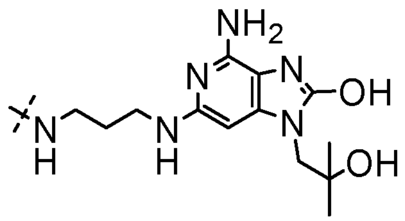 | 50 | 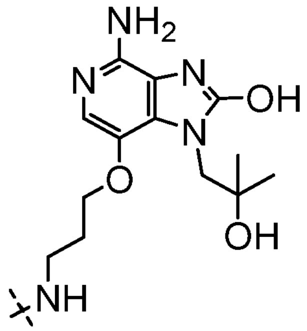 |
| 51 | 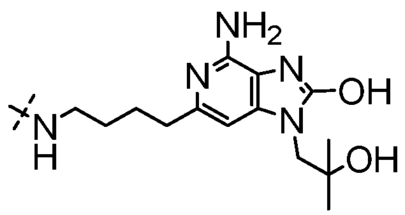 | 52 | 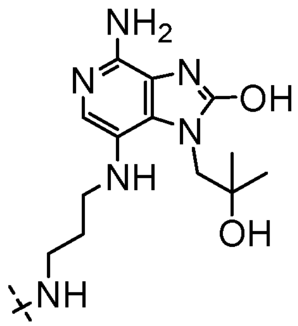 |
| 53 | 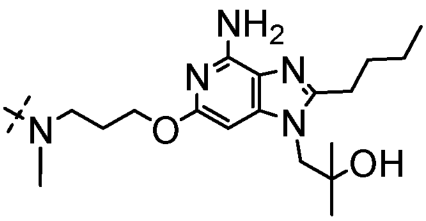 | 54 | 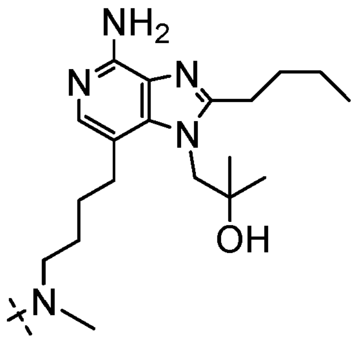 |
| 55 | 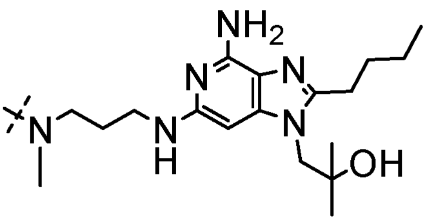 | 56 | 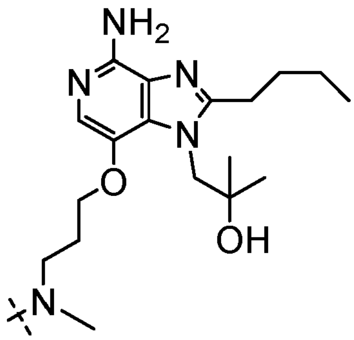 |

(continued)

| Adj | Structure | Adj | Structure |
|---|---|---|---|
| 57 | 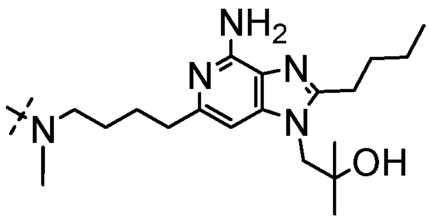 | 58 | 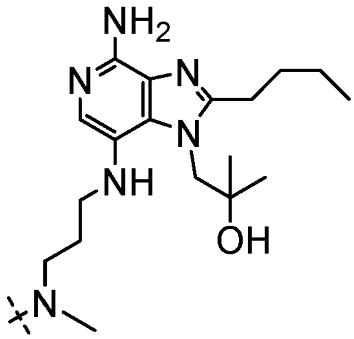 |
| 59 | 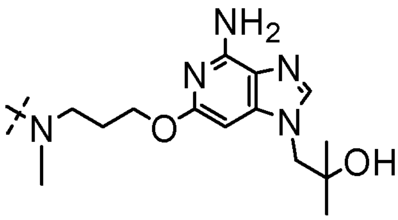 | 60 | 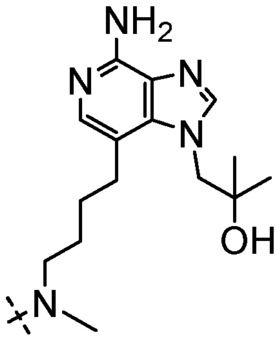 |
| 61 | 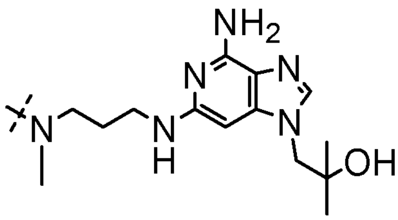 | 62 | 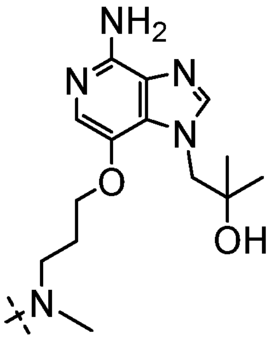 |
| 63 | 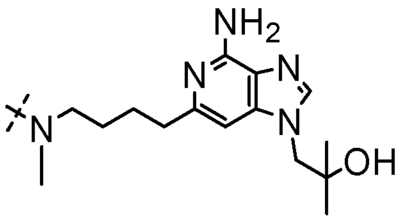 | 64 | 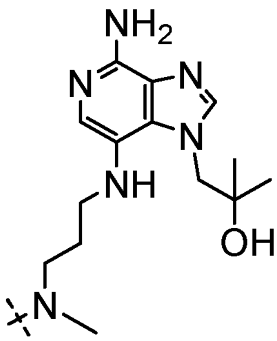 |

(continued)

| Adj | Structure | Adj | Structure |
|---|---|---|---|
| 65 | 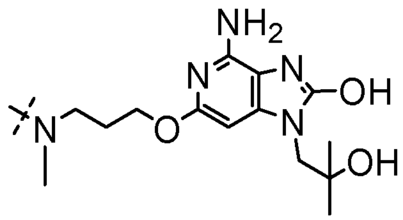 | 66 | 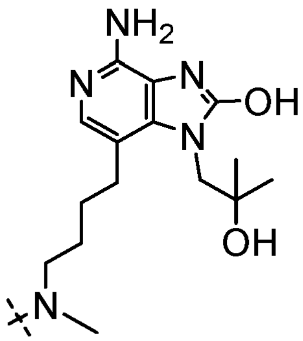 |
| 67 | 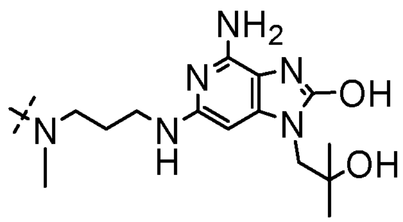 | 68 | 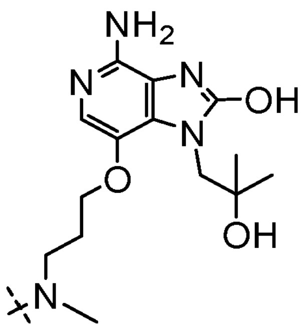 |
| 69 | 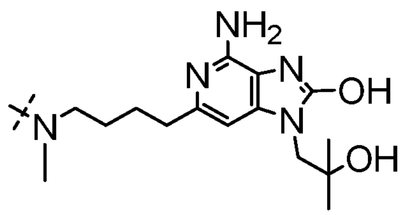 | 70 | 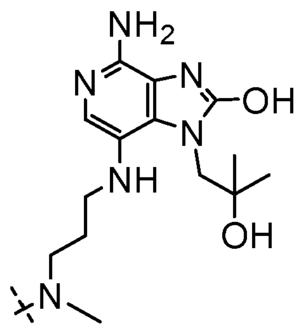 |
| 71 | 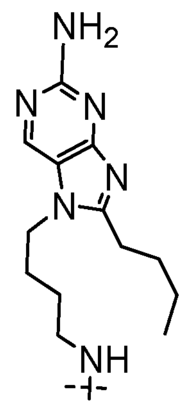 | 72 | 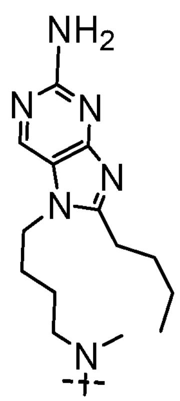 |

(continued)

| Adj | Structure | Adj | Structure |
|---|---|---|---|
| 73 | 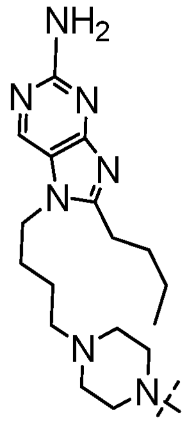 | 74 | 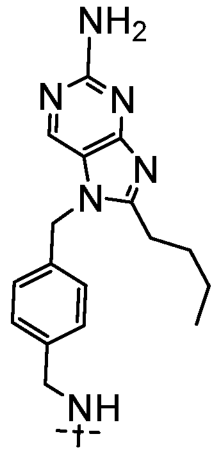 |
| 75 | 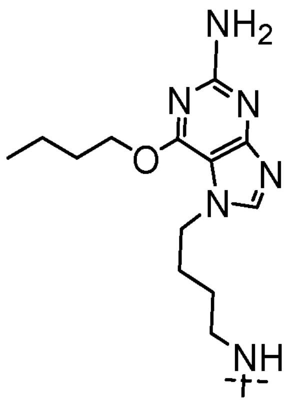 | 76 | 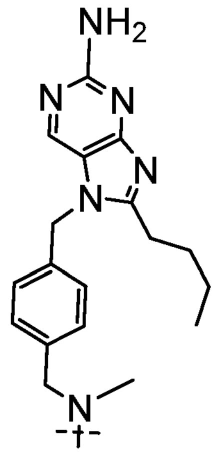 |
| 77 | 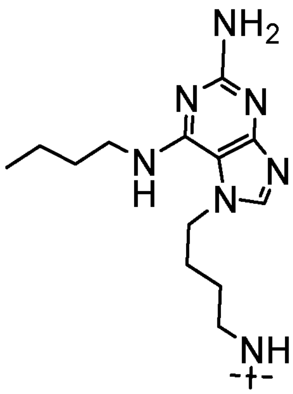 | 78 | 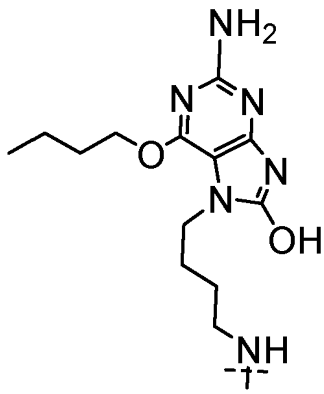 |

(continued)

| Adj | Structure | Adj | Structure |
|---|---|---|---|
| 79 | 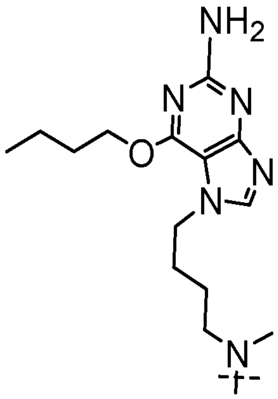 | 80 | 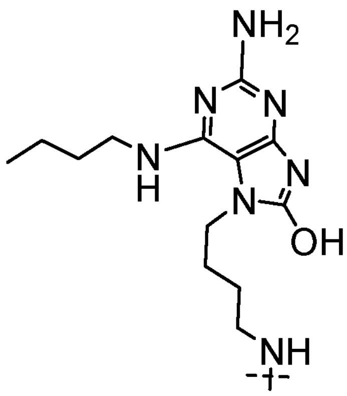 |
| 81 | 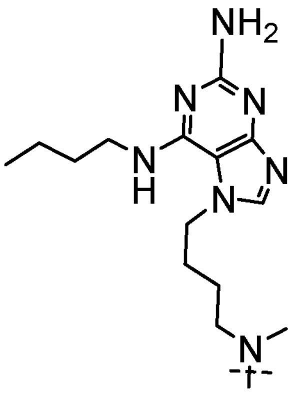 | 82 | 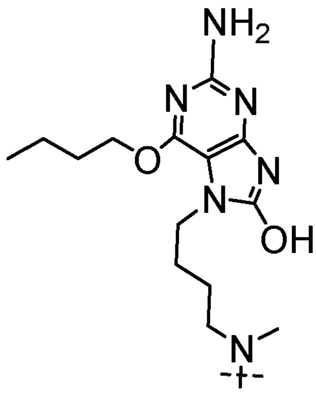 |
| 83 | 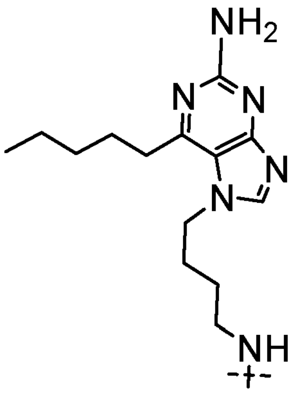 | 84 | 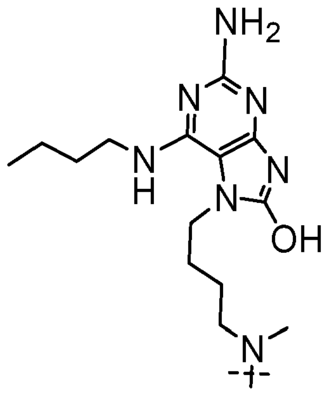 |
| 85 | 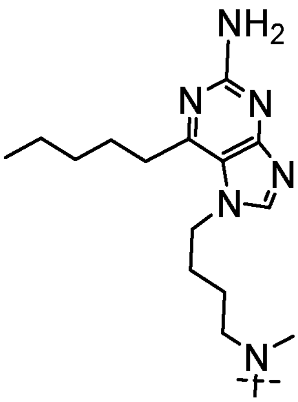 | 86 | 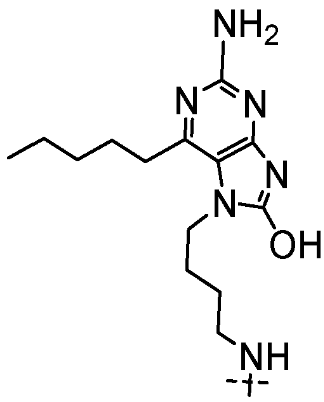 |

(continued)

| Adj | Structure | Adj | Structure |
|---|---|---|---|
| 87 | 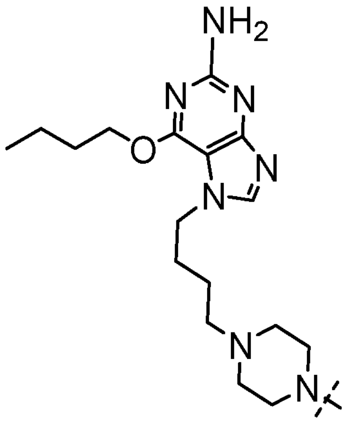 | 88 | 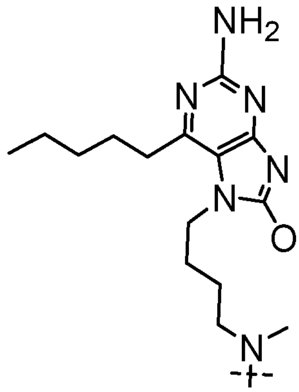 |
| 89 | 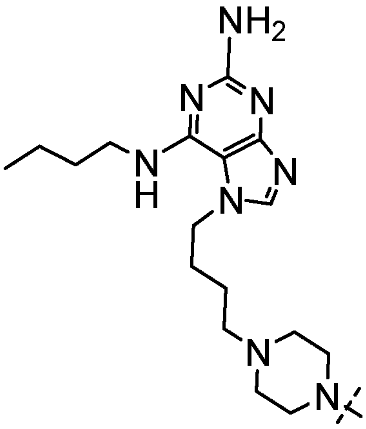 | 90 | 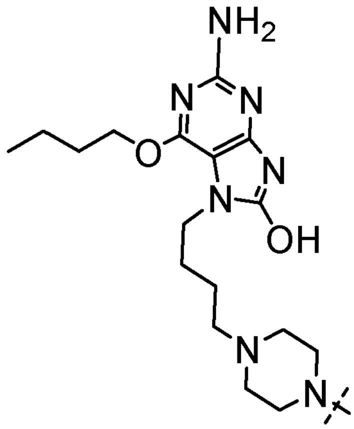 |
| 91 | 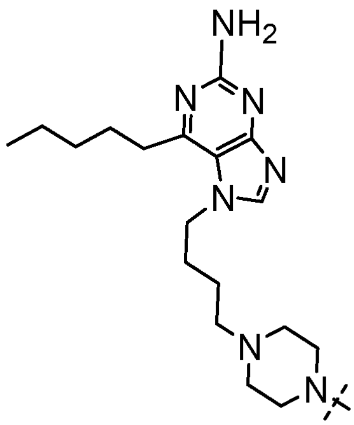 | 92 | 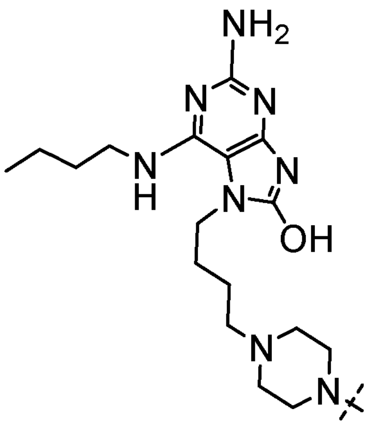 |

(continued)

| Adj | Structure | Adj | Structure |
|---|---|---|---|
| 93 | 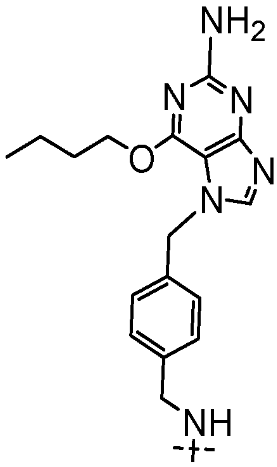 | 94 | 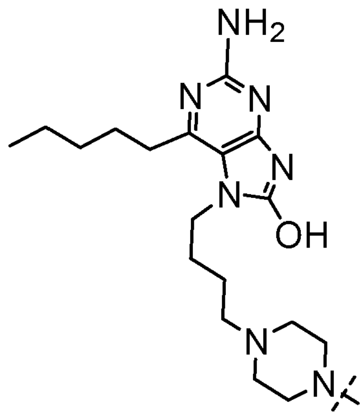 |
| 95 | 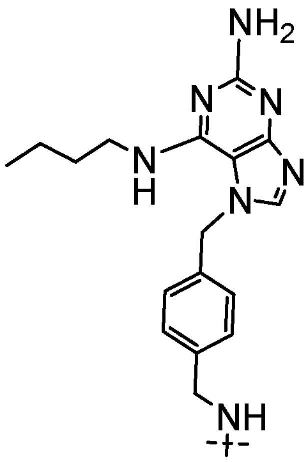 | 96 | 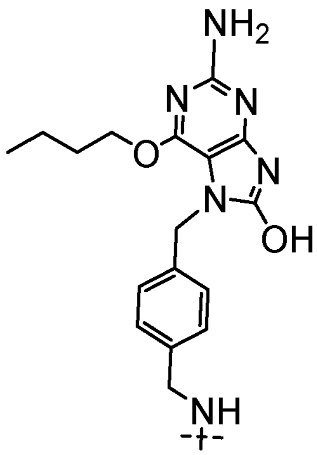 |
| 97 | 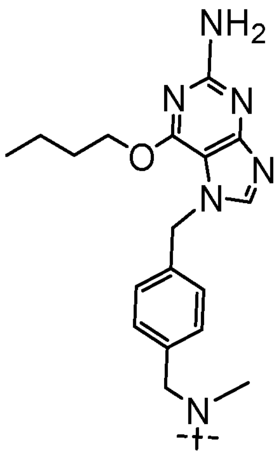 | 98 | 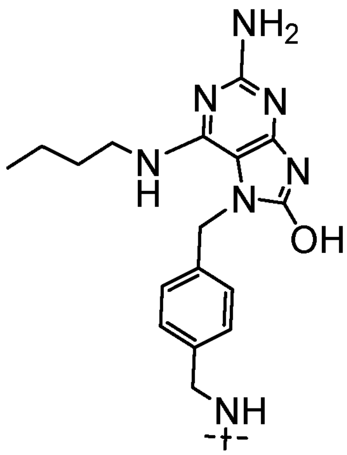 |

(continued)

| Adj | Structure | Adj | Structure |
|---|---|---|---|
| 99 | 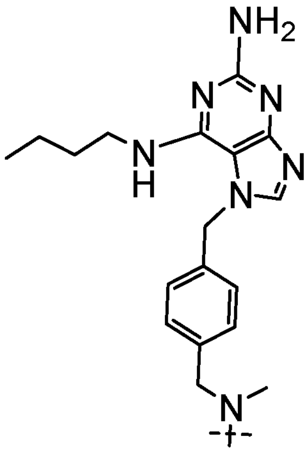 | 100 | 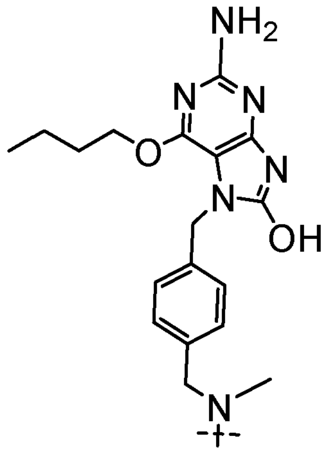 |
| 101 | 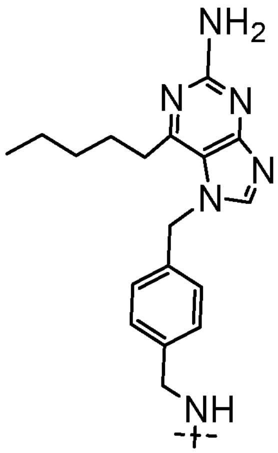 | 102 | 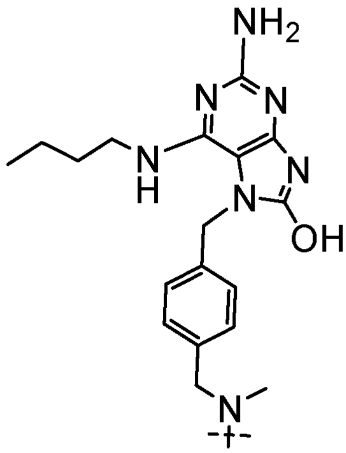 |
| 103 | 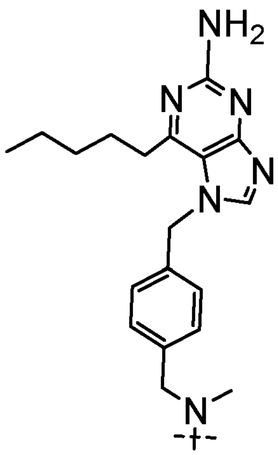 | 104 | 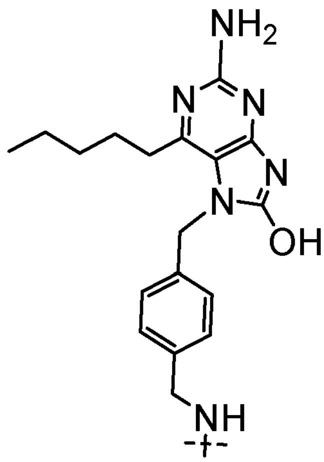 |

(continued)

| Adj | Structure | Adj | Structure |
|---|---|---|---|
| 105 | 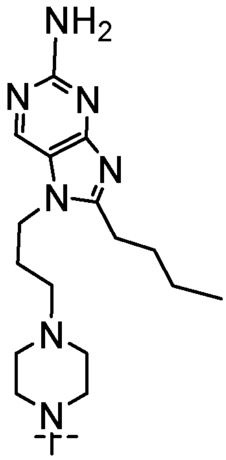 | 106 | 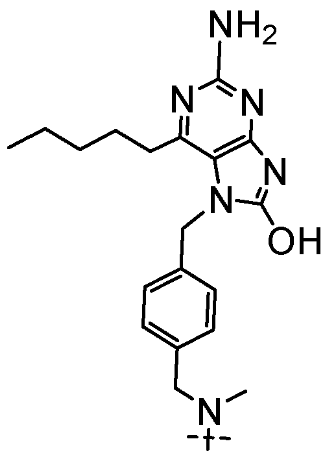 |
| 107 | 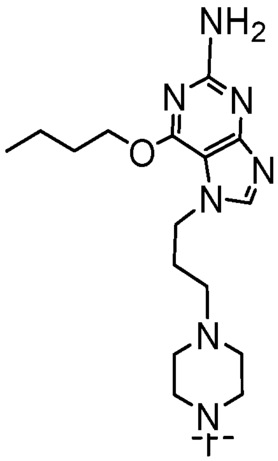 | 108 | 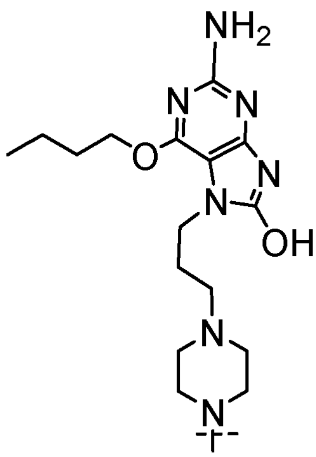 |
| 109 | 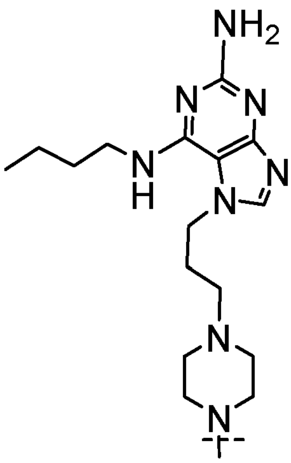 | 110 | 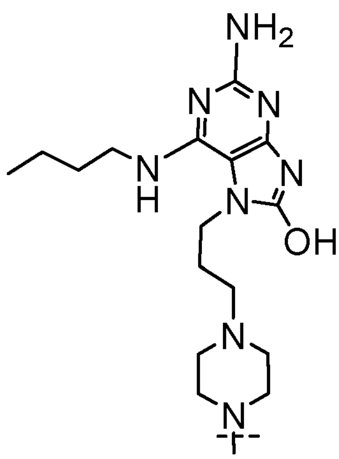 |

(continued)

| Adj | Structure | Adj | Structure |
|---|---|---|---|
| 111 | 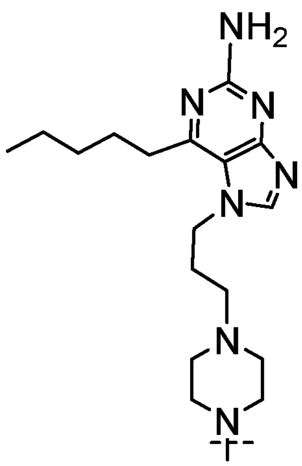 | 112 | 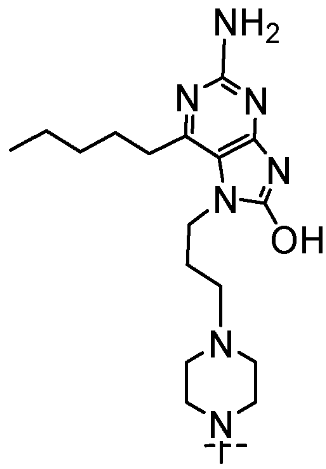 |
| 113 | 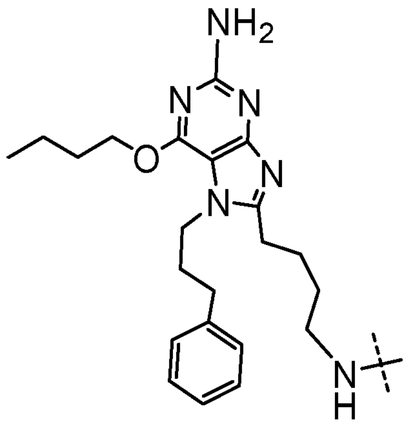 | 114 | 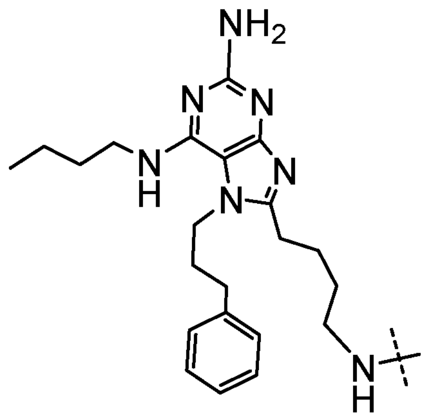 |
| 115 | 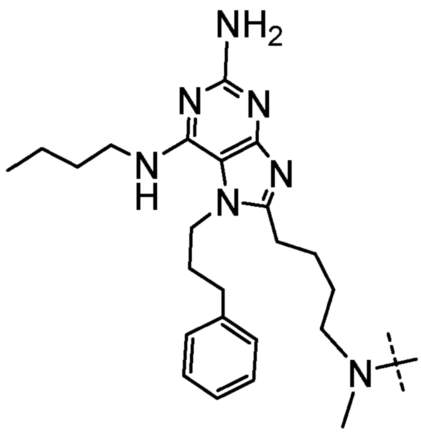 | 116 | 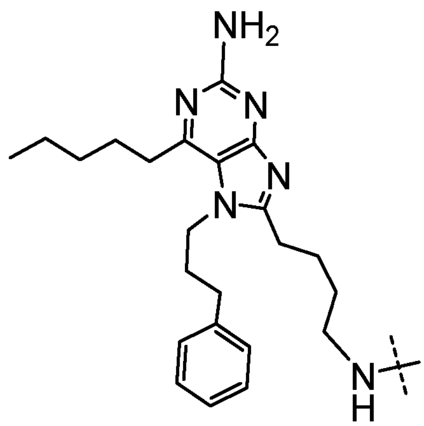 |

(continued)

| Adj | Structure | Adj | Structure |
|---|---|---|---|
| 117 | 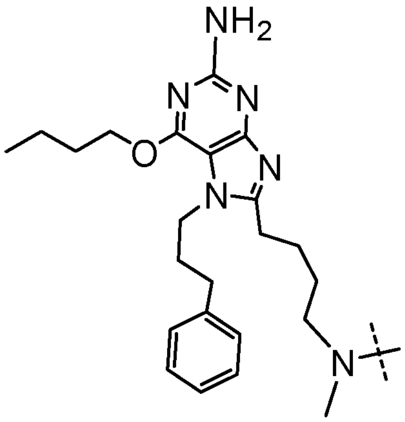 | 118 | 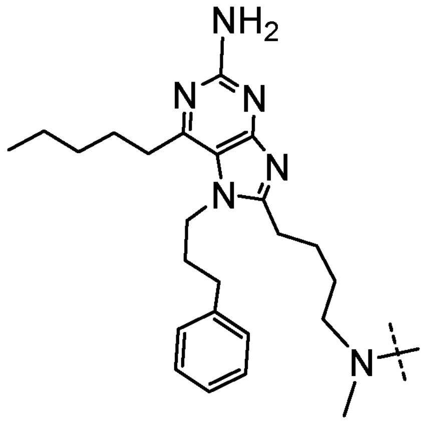 |
| 119 | 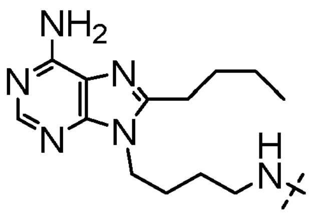 | 120 | 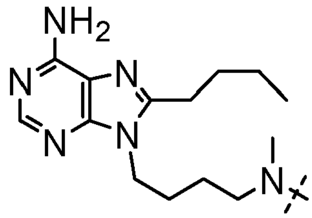 |
| 121 | 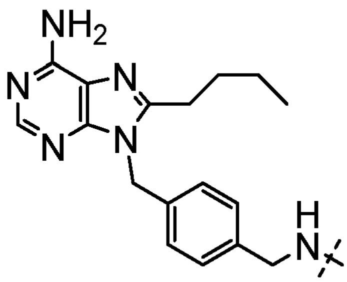 | 122 | 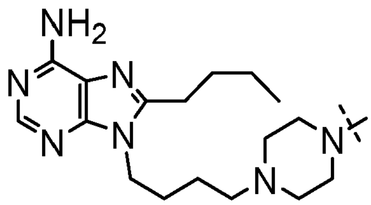 |
| 123 | 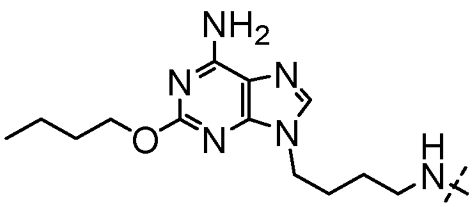 | 124 | 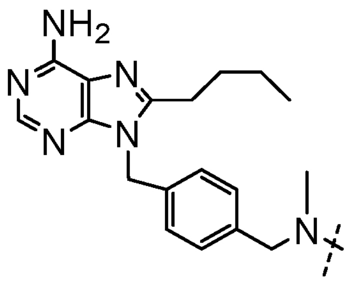 |
| 125 | 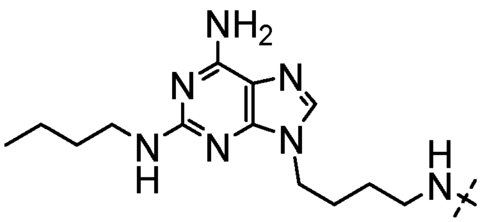 | 126 | 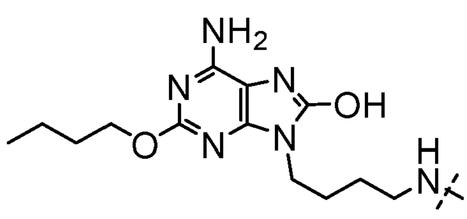 |

(continued)

| Adj | Structure | Adj | Structure |
|---|---|---|---|
| 127 | 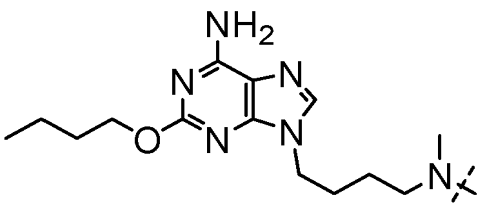 | 128 | 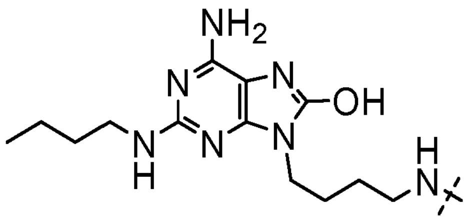 |
| 129 | 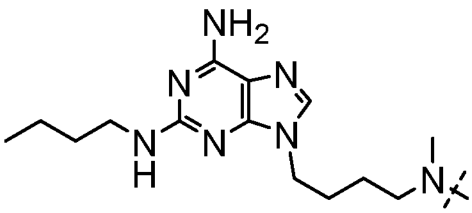 | 130 | 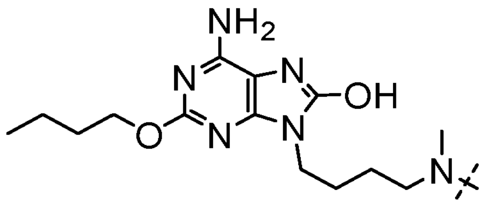 |
| 131 | 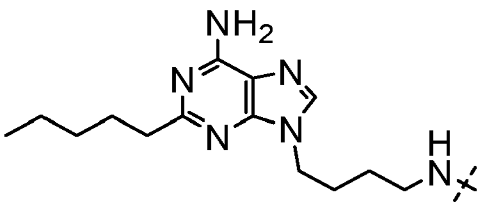 | 132 | 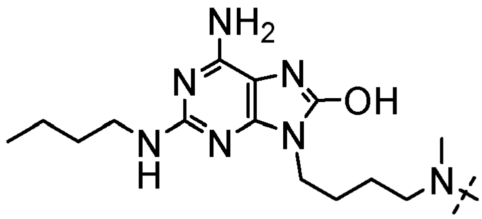 |
| 133 | 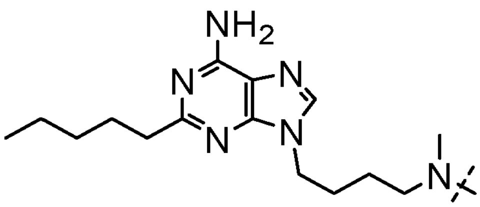 | 134 | 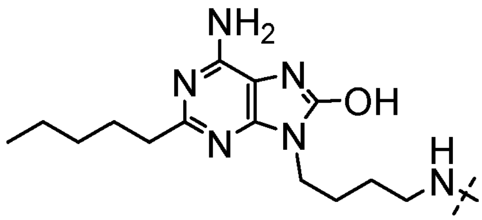 |
| 135 | 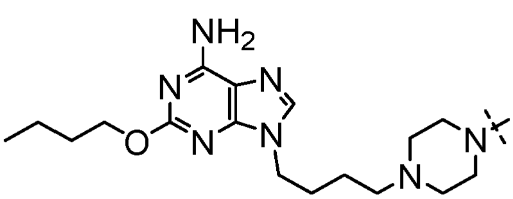 | 136 | 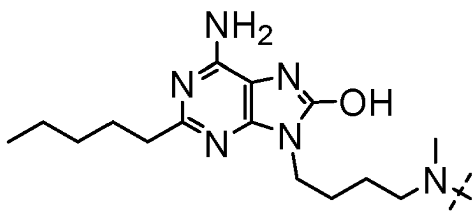 |
| 137 | 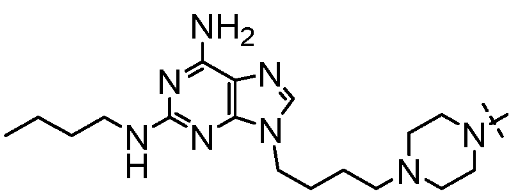 | 138 | 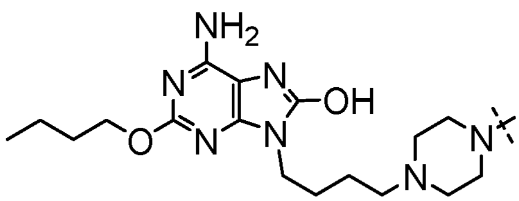 |
| 139 | 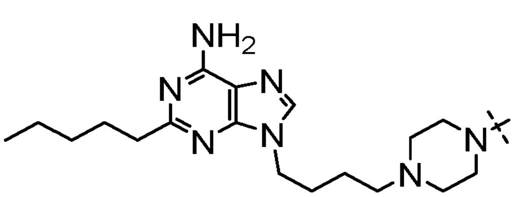 | 140 | 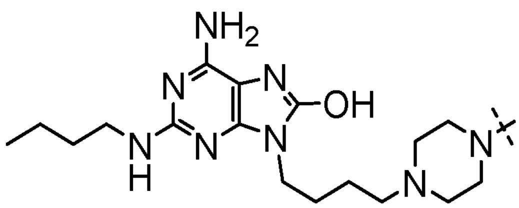 |

(continued)

| Adj | Structure | Adj | Structure |
|---|---|---|---|
| 141 | 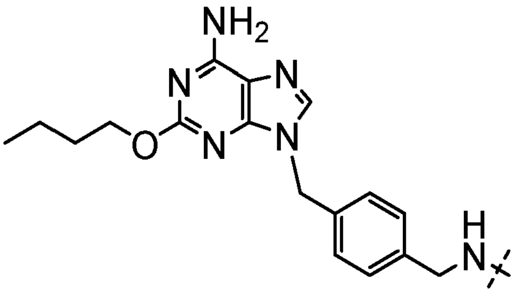 | 142 | 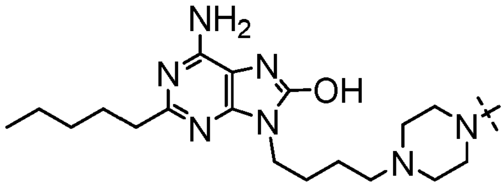 |
| 143 | 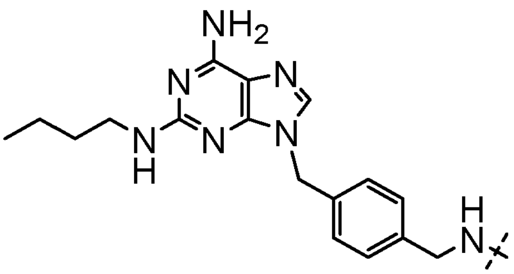 | 144 | 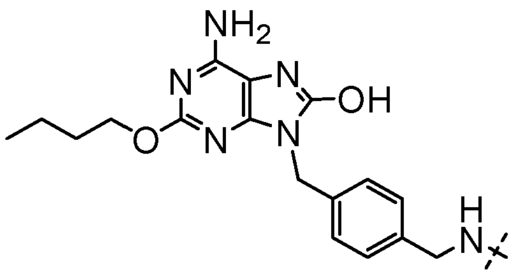 |
| 145 | 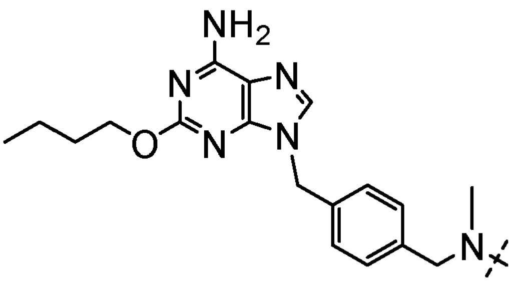 | 146 | 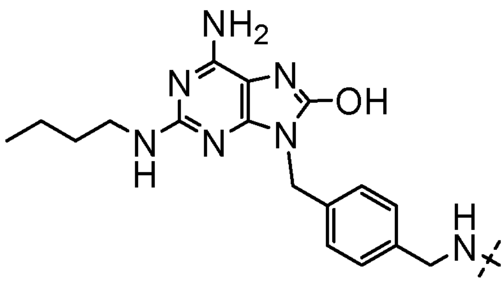 |
| 147 | 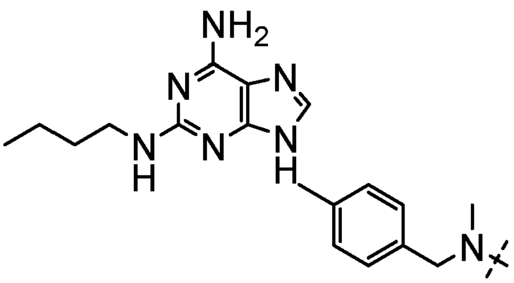 | 148 | 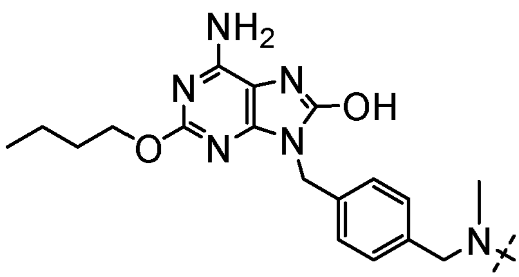 |
| 149 | 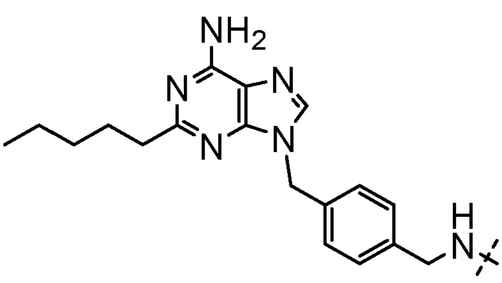 | 150 | 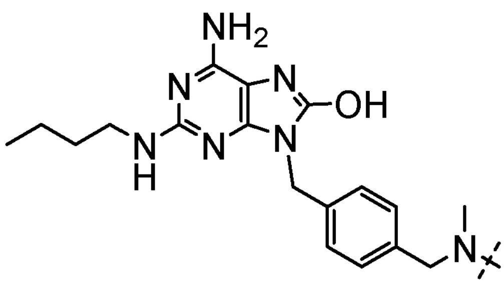 |

(continued)

| Adj | Structure | Adj | Structure |
|---|---|---|---|
| 151 | 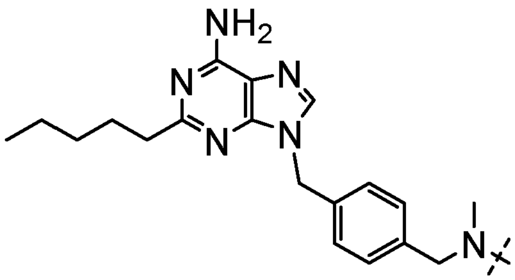 | 152 | 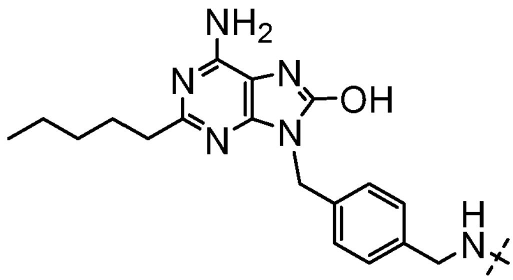 |
| 153 | 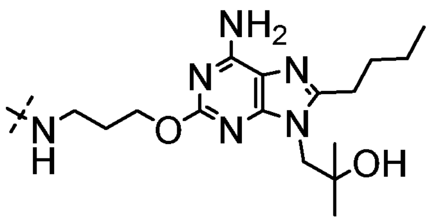 | 154 | 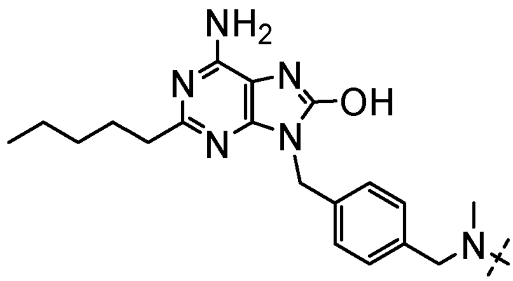 |
| 155 | 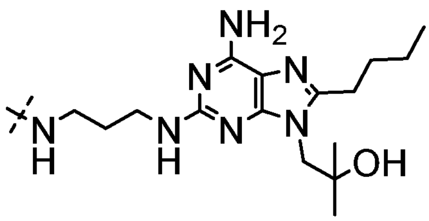 | 156 | 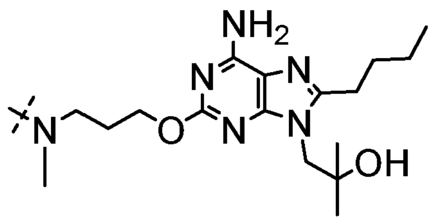 |
| 157 | 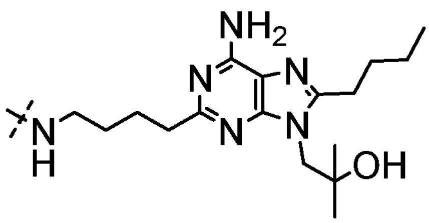 | 158 | 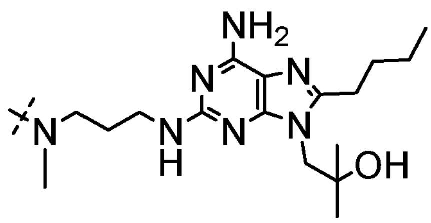 |
| 159 | 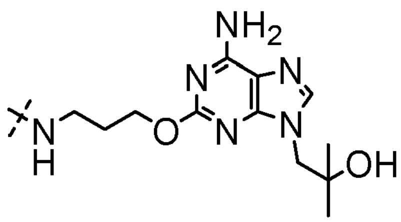 | 160 | 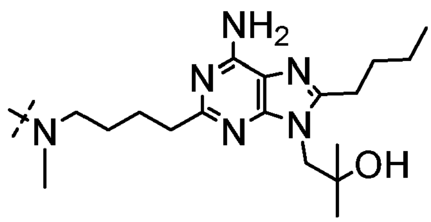 |
| 161 | 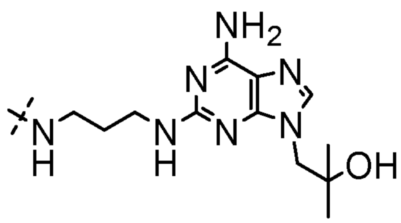 | 162 | 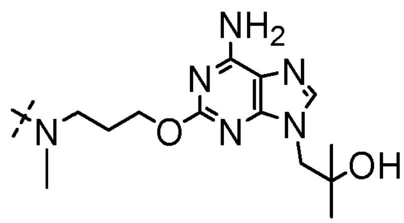 |

(continued)

| Adj | Structure | Adj | Structure |
|---|---|---|---|
| 163 | 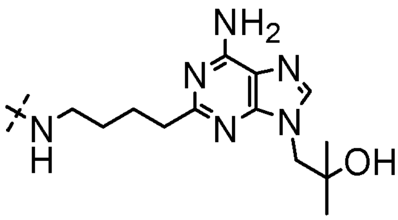 | 164 | 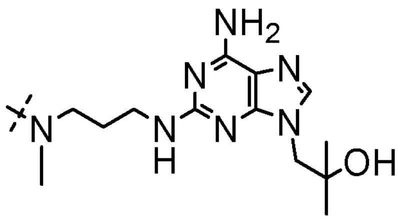 |
| 165 | 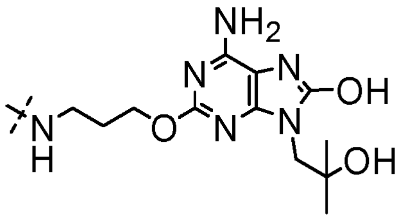 | 166 | 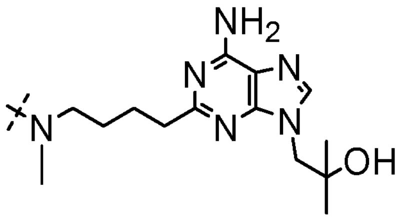 |
| 167 | 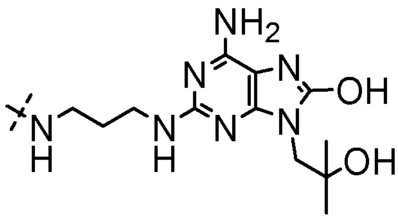 | 168 | 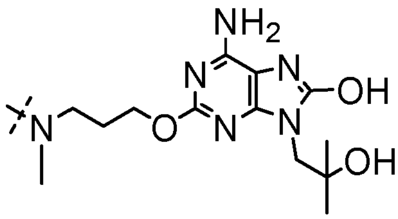 |
| 169 | 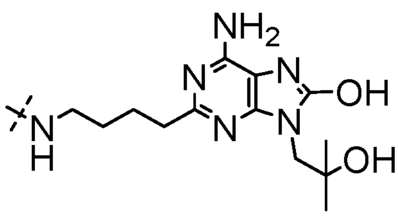 | 170 | 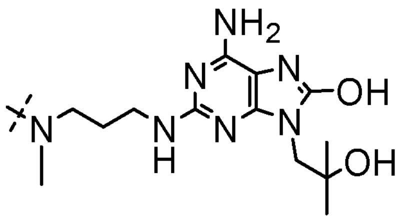 |
| 171 | 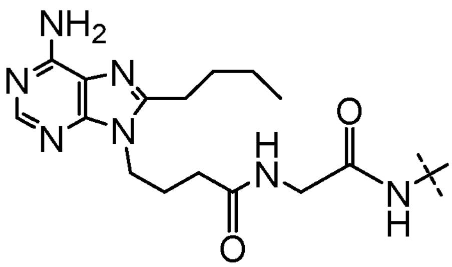 | 172 | 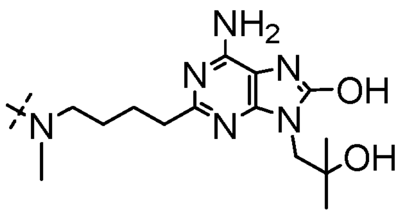 |
| 173 | 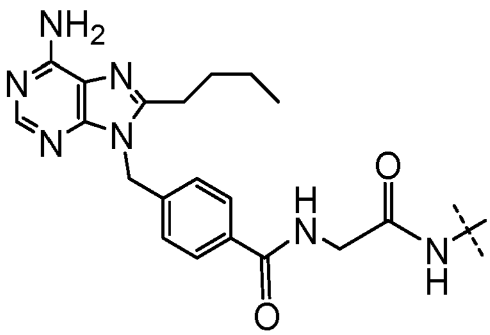 | 174 | 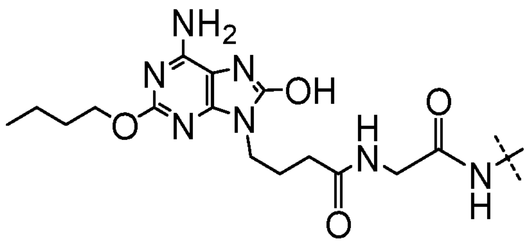 |

(continued)

| Adj | Structure | Adj | Structure |
|---|---|---|---|
| 175 | 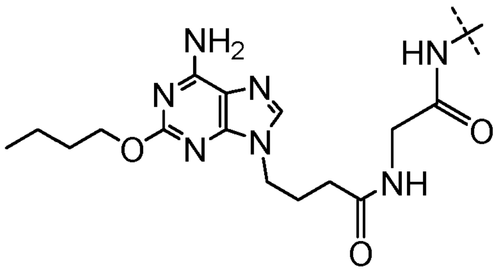 | 176 | 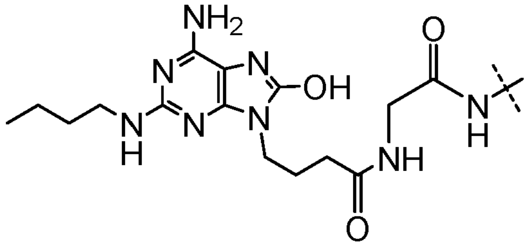 |
| 177 | 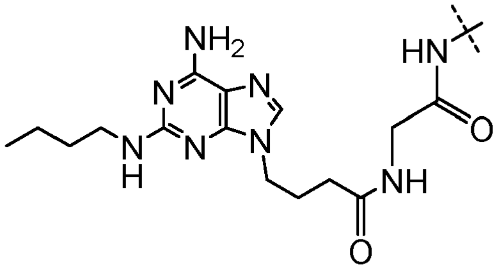 | 178 | 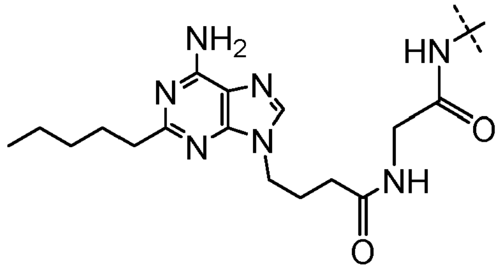 |
| 179 | 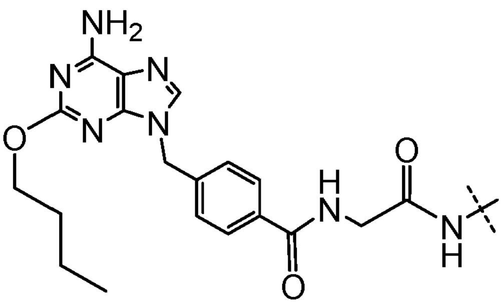 | 180 | 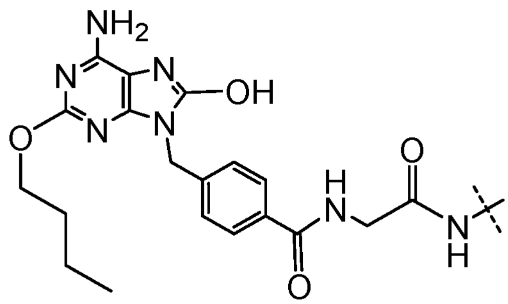 |
| 181 | 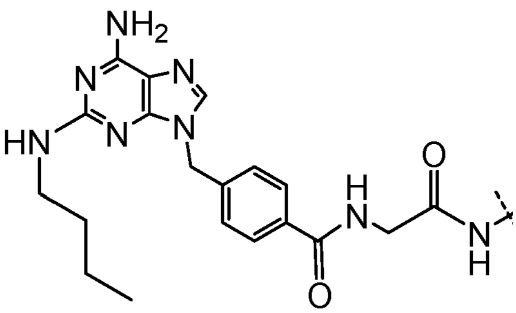 | 182 | 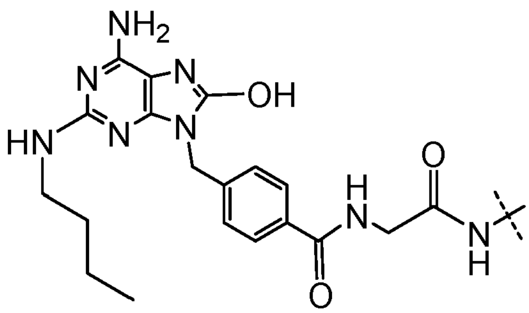 |
| 183 | 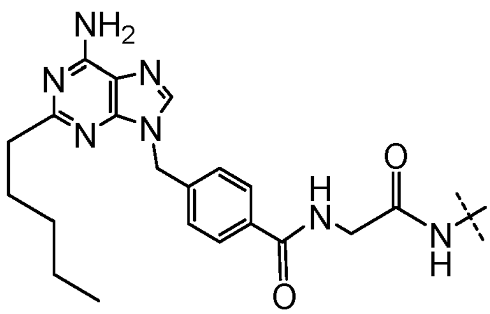 | 184 | 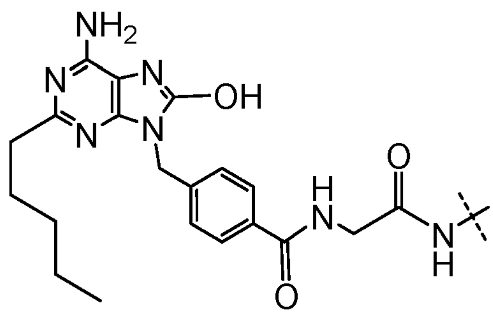 |

(continued)

| Adj | Structure | Adj | Structure |
|---|---|---|---|
| 185 | 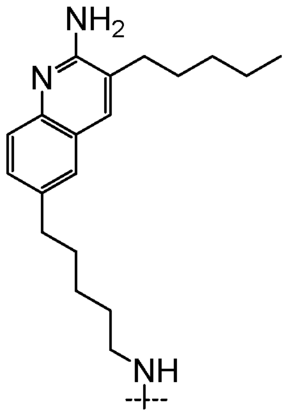 | 186 | 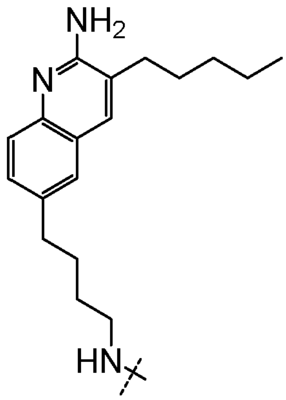 |
| 187 | 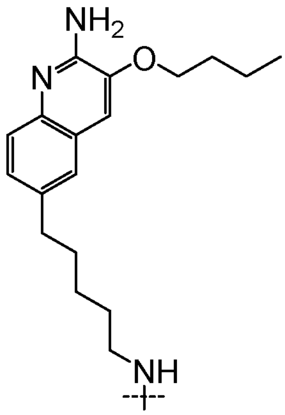 | 188 | 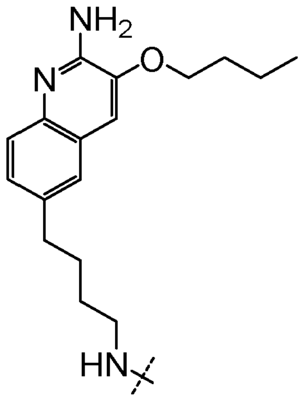 |
| 189 | 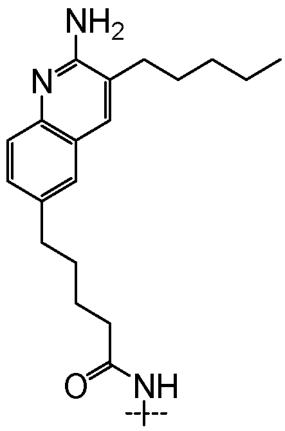 | 190 | 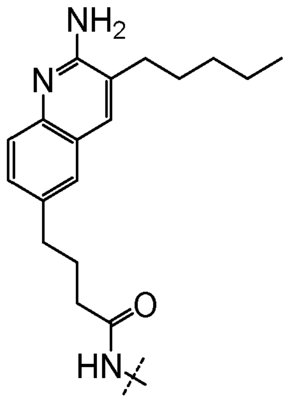 |

(continued)

| Adj | Structure | Adj | Structure |
|---|---|---|---|
| 191 | 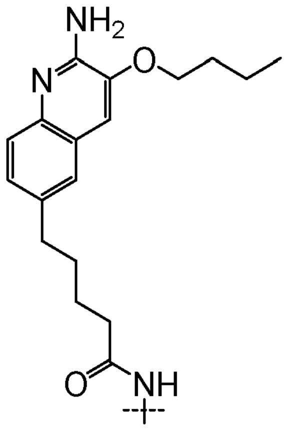 | 192 | 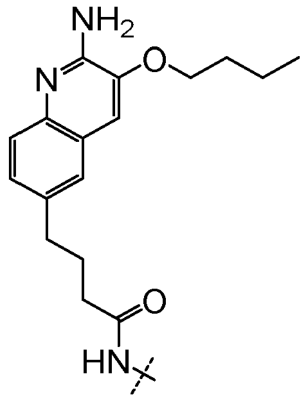 |
| 193 | 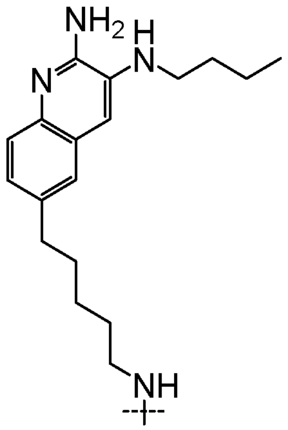 | 194 | 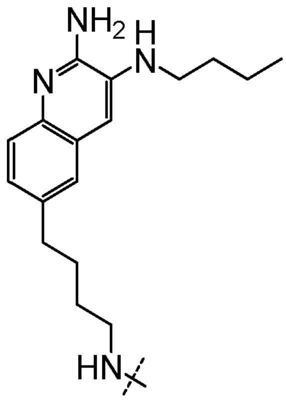 |
| 195 | 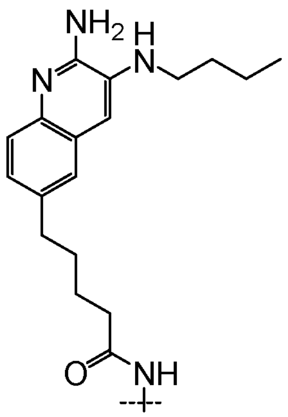 | 196 | 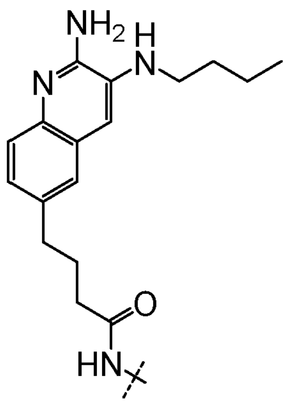 |

(continued)

| Adj | Structure | Adj | Structure |
|---|---|---|---|
| 197 | 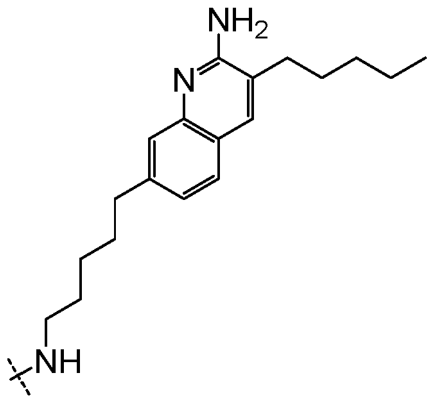 | 198 | 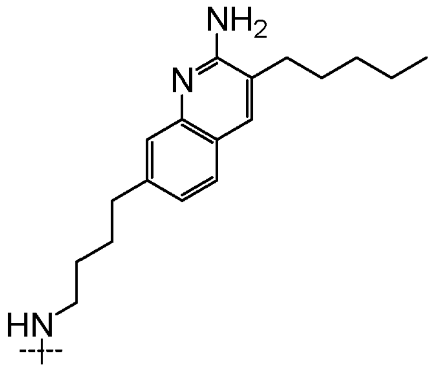 |
| 199 | 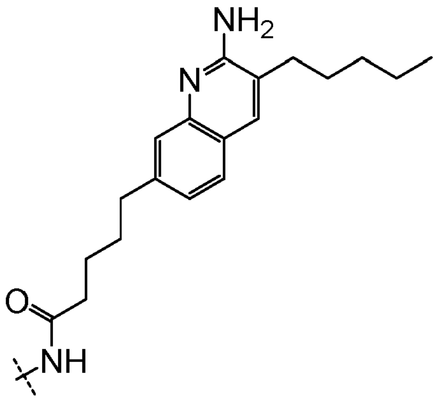 | 200 | 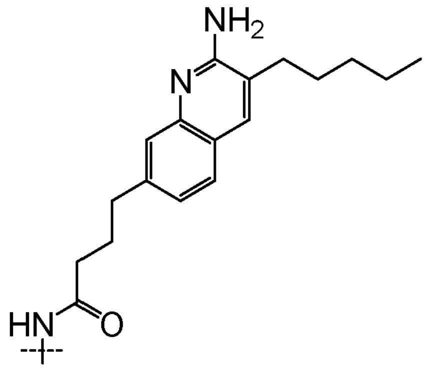 |
| 201 | 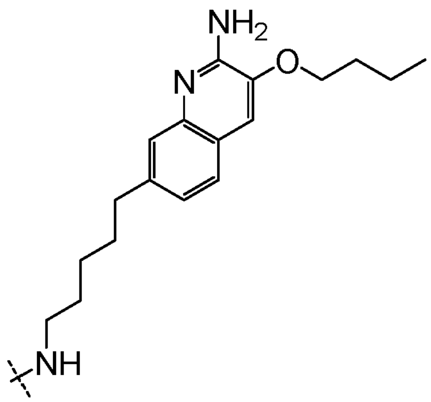 | 202 | 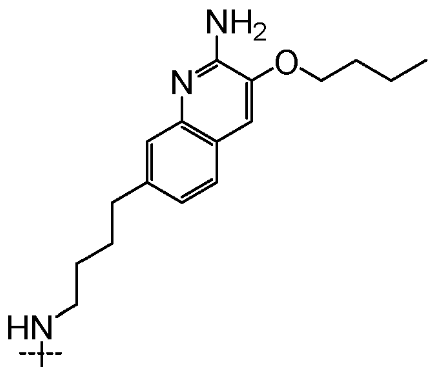 |
| 203 | 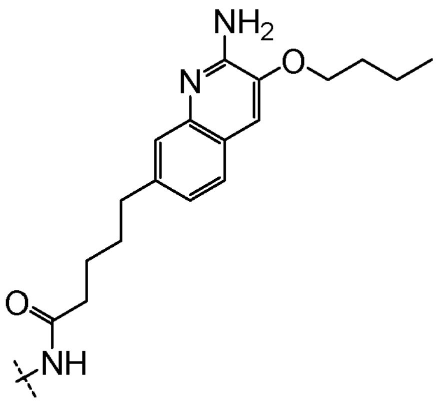 | 204 | 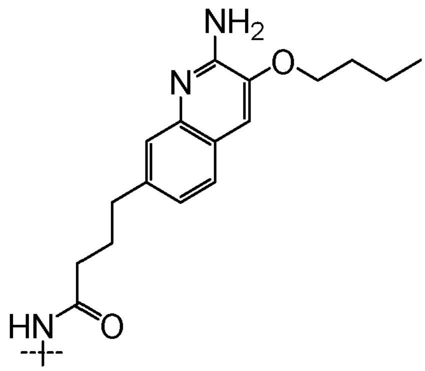 |

(continued)

| Adj | Structure | Adj | Structure |
|---|---|---|---|
| 205 | 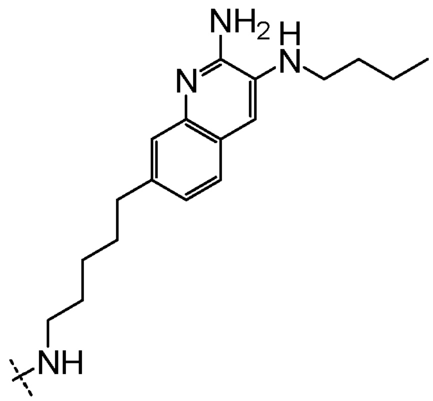 | 206 | 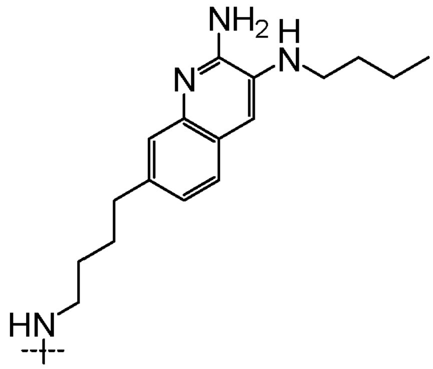 |
| 207 | 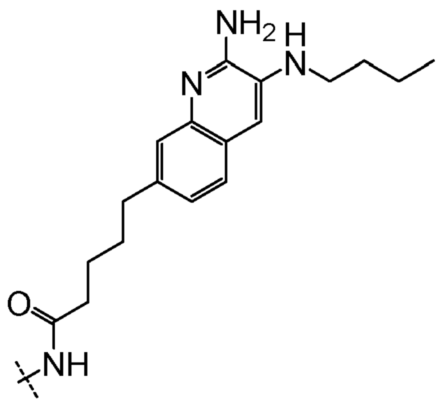 | 208 | 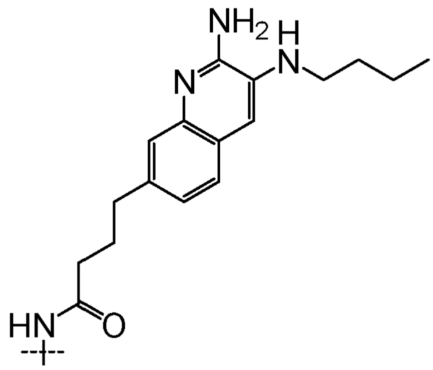 |
| 209 | 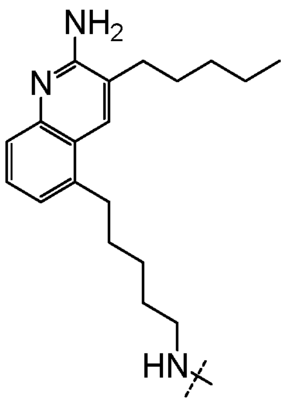 | 210 | 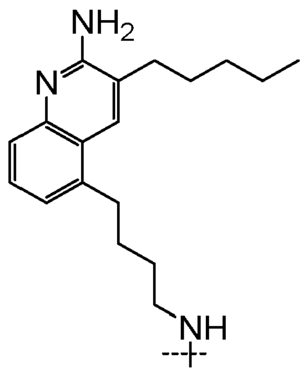 |
| 211 | 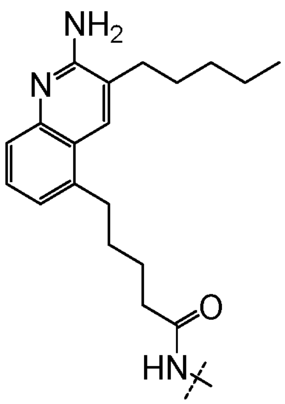 | 212 | 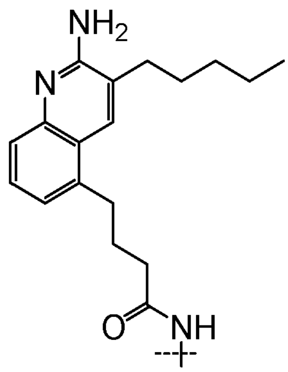 |

(continued)

| Adj | Structure | Adj | Structure |
|---|---|---|---|
| 213 | 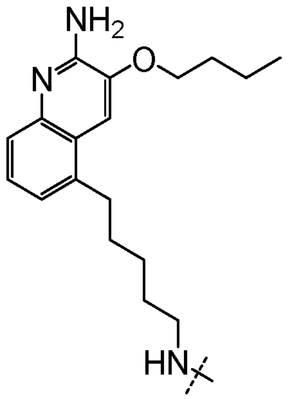 | 214 | 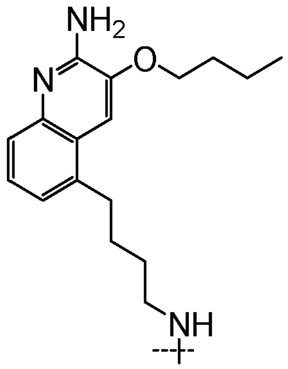 |
| 215 | 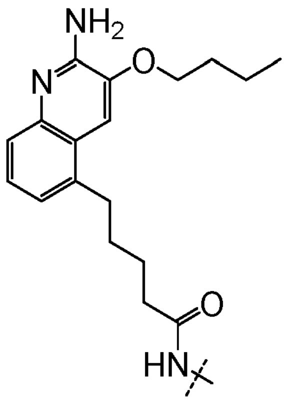 | 216 | 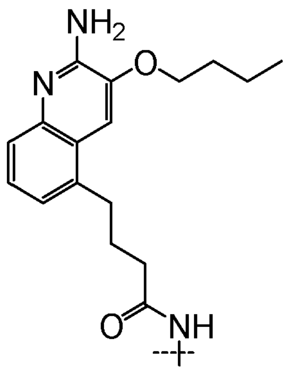 |
| 217 | 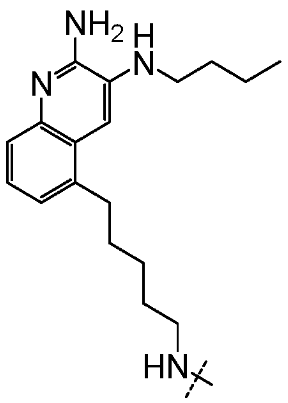 | 218 | 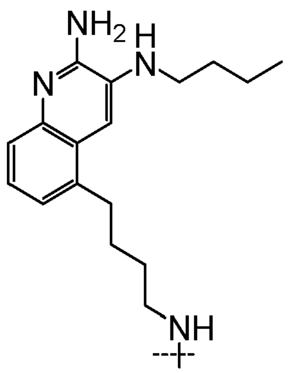 |
| 219 | 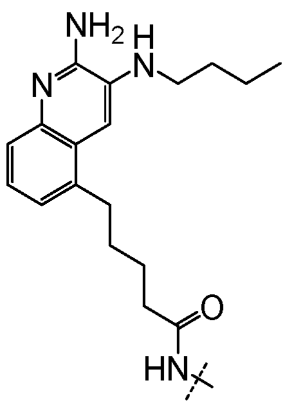 | 220 | 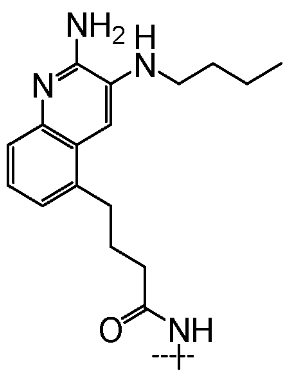 |

(continued)

| Adj | Structure | Adj | Structure |
|---|---|---|---|
| 221 | 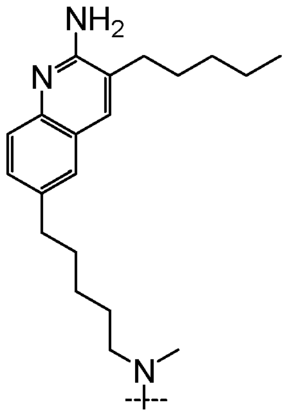 | 222 | 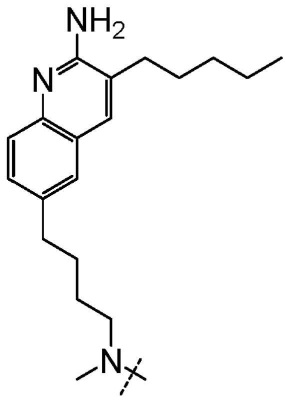 |
| 223 | 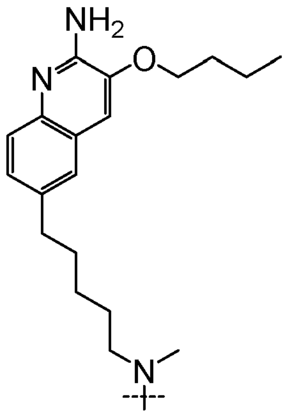 | 224 | 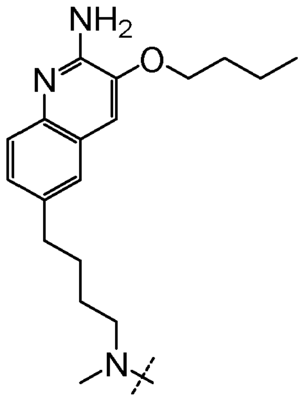 |
| 225 | 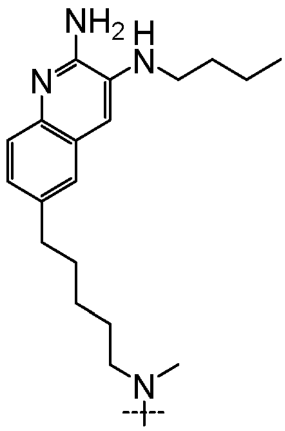 | 226 | 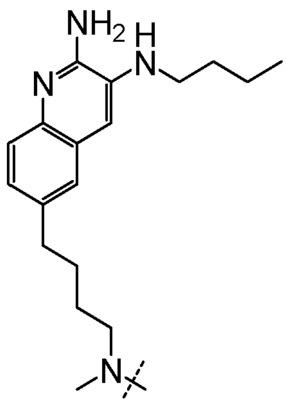 |
| 227 | 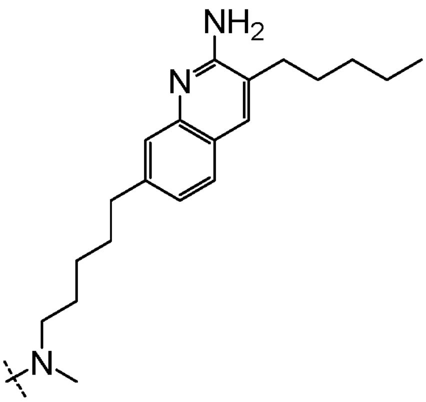 | 228 | 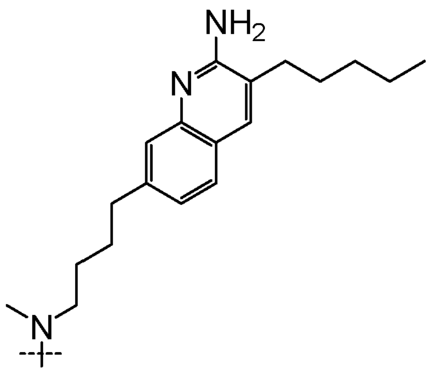 |

(continued)

| Adj | Structure | Adj | Structure |
|---|---|---|---|
| 229 | 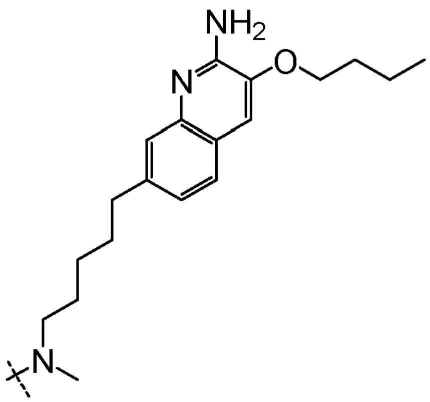 | 230 | 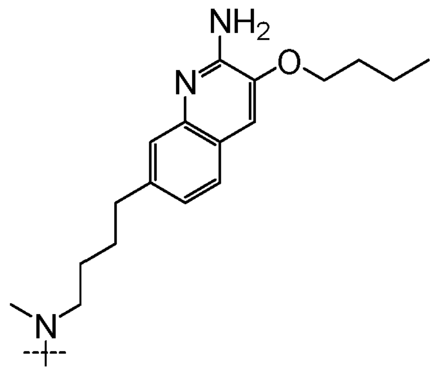 |
| 231 | 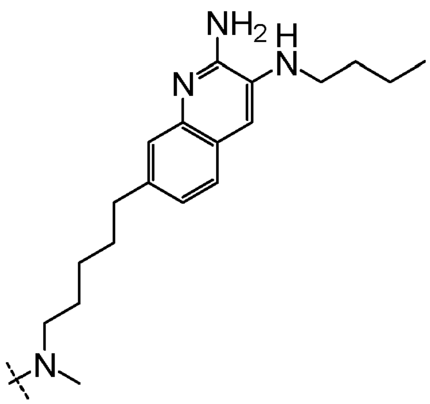 | 232 | 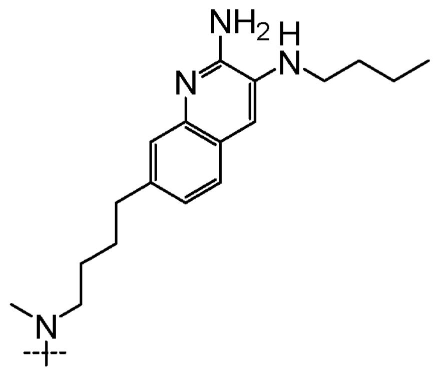 |
| 233 | 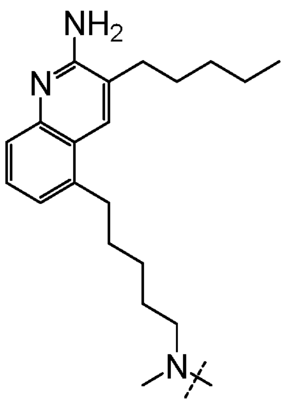 | 234 | 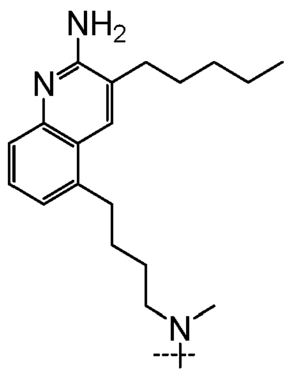 |
| 235 | 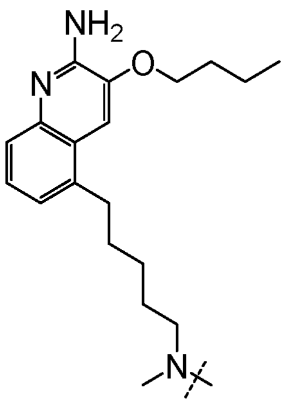 | 236 | 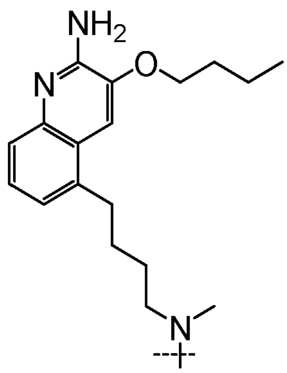 |

(continued)

| Adj | Structure | Adj | Structure |
|---|---|---|---|
| 237 | 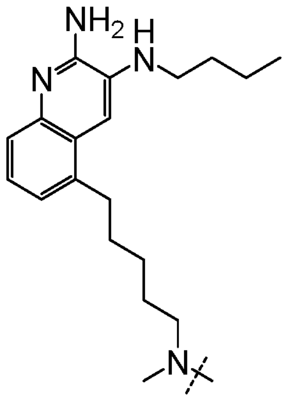 | 238 | 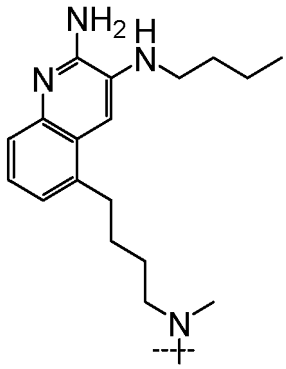 |
| 239 | 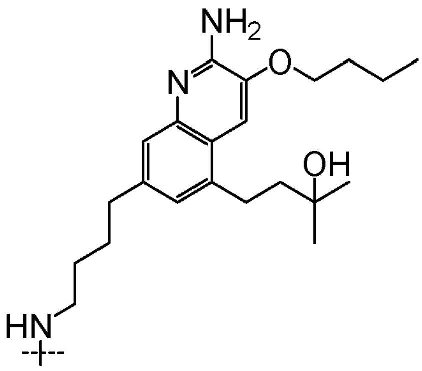 | 240 | 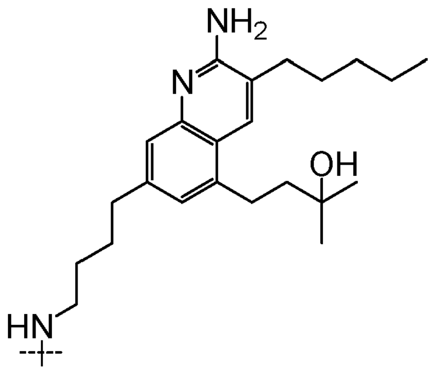 |
| 241 | 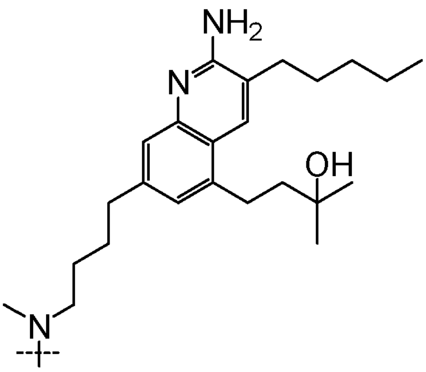 | 242 | 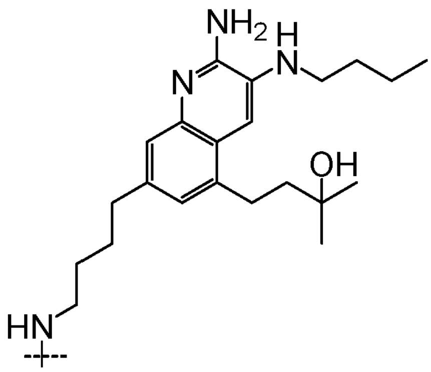 |
| 243 | 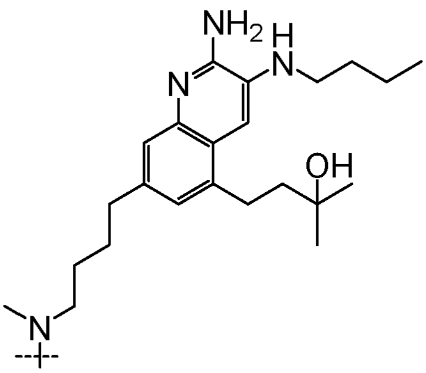 | 244 | 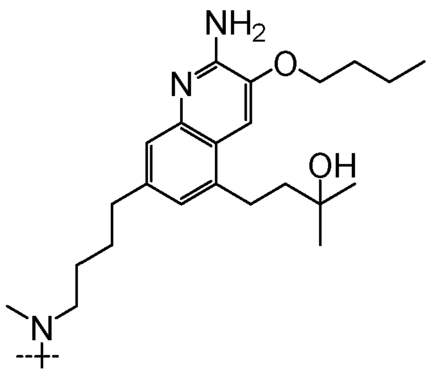 |

(continued)

| Adj | Structure | Adj | Structure |
|---|---|---|---|
| 245 | 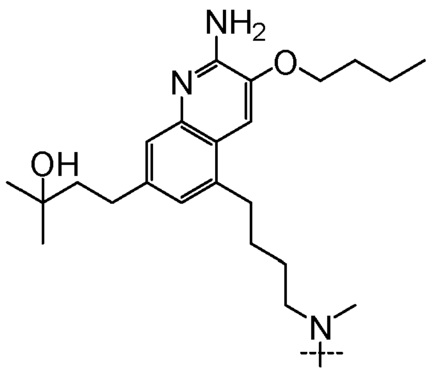 | 246 | 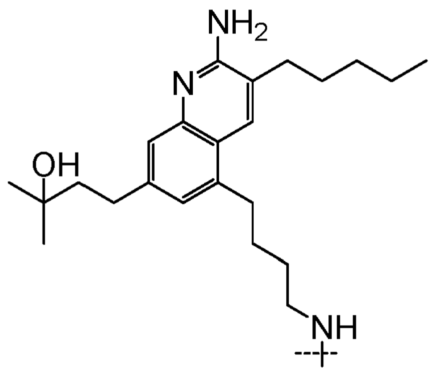 |
| 247 | 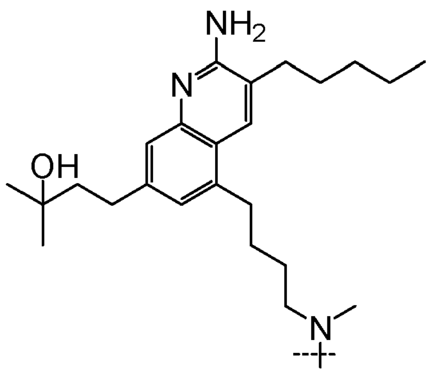 | 248 | 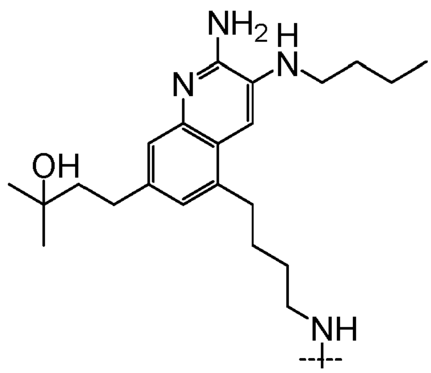 |
| 249 | 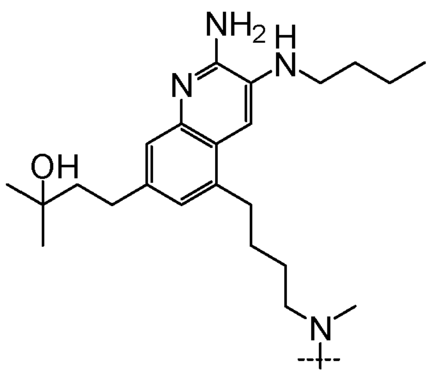 | 250 | 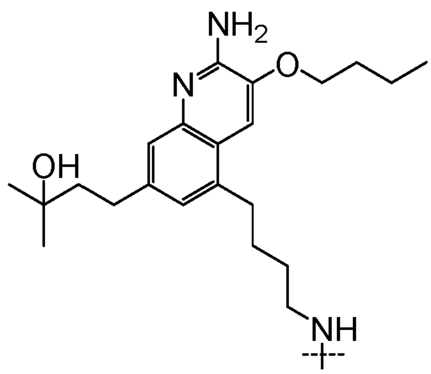 |
| 251 | 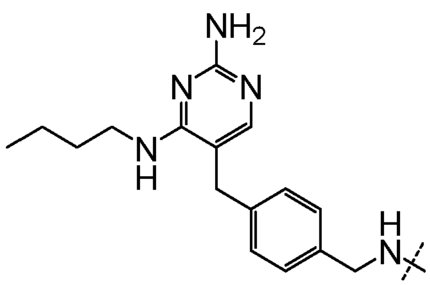 | 252 | 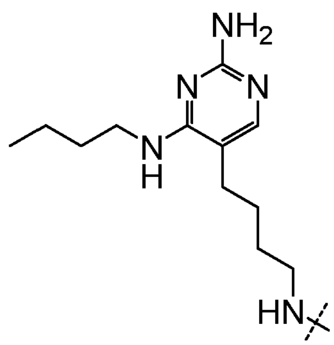 |

(continued)

| Adj | Structure | Adj | Structure |
|---|---|---|---|
| 253 | 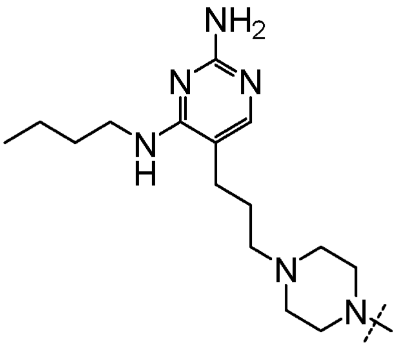 | 254 | 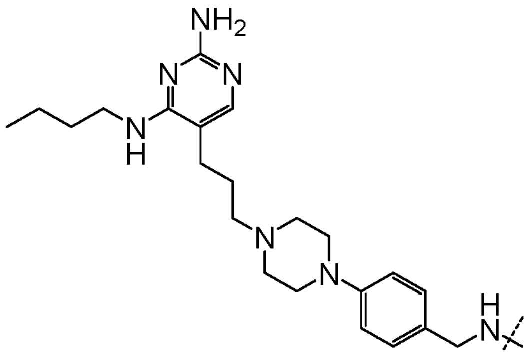 |
| 255 | 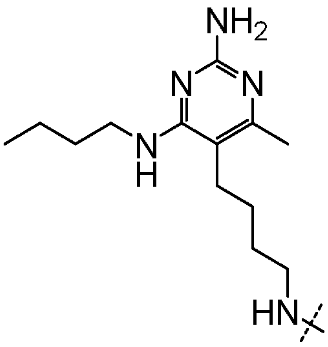 | 256 | 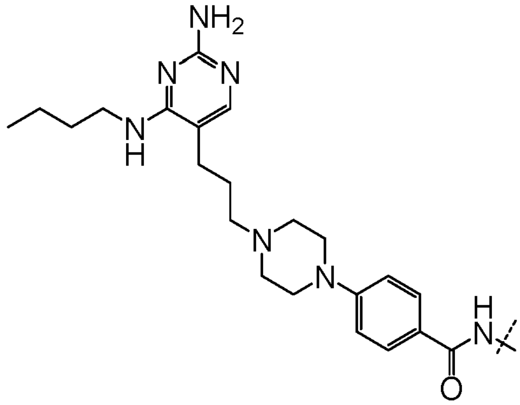 |
| 257 | 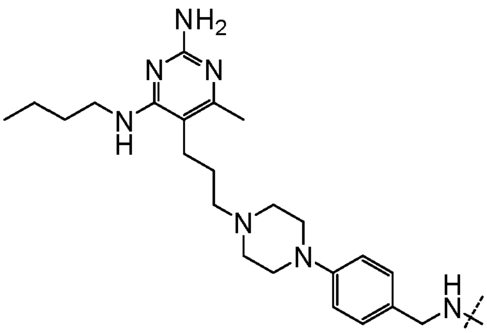 | 258 | 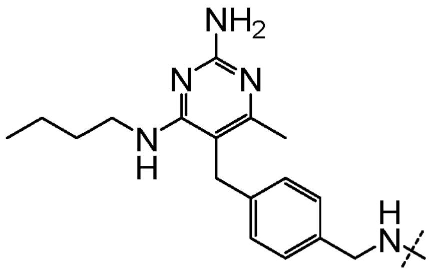 |
| 259 | 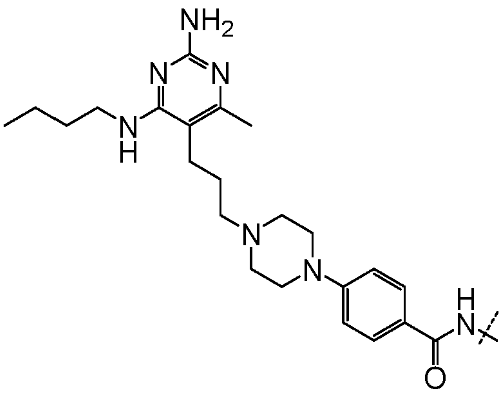 | 260 | 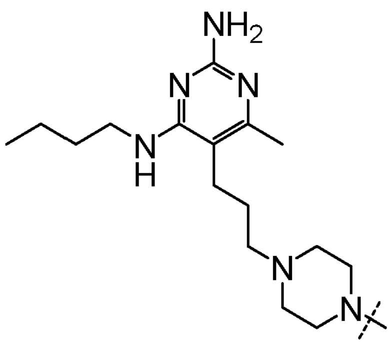 |

(continued)

| Adj | Structure | Adj | Structure |
|---|---|---|---|
| 261 | 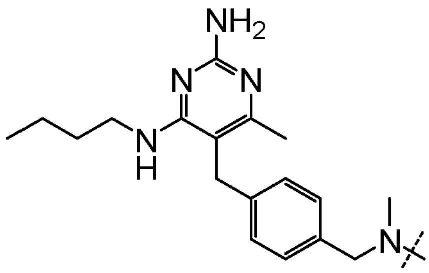 | 262 | 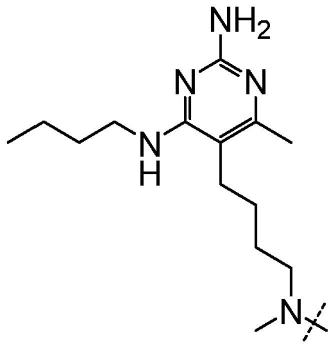 |
| 263 | 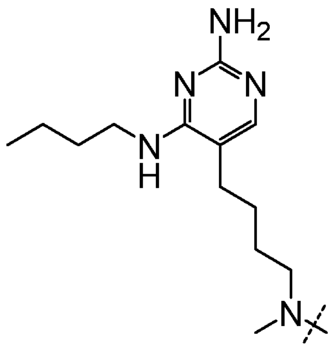 | 264 | 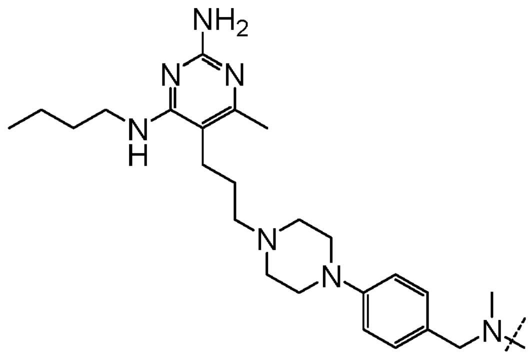 |
| 265 | 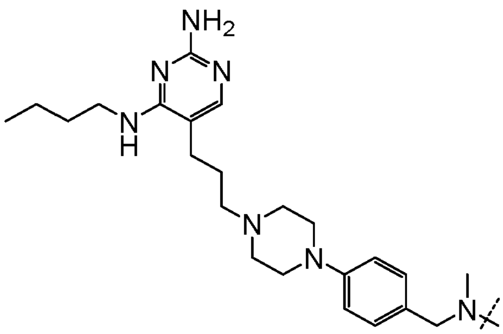 | 266 | 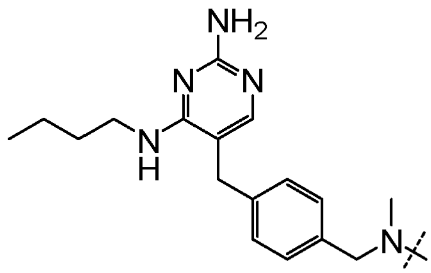 |
| 267 | 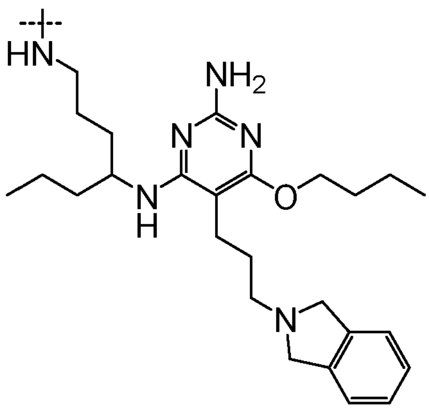 | 268 | 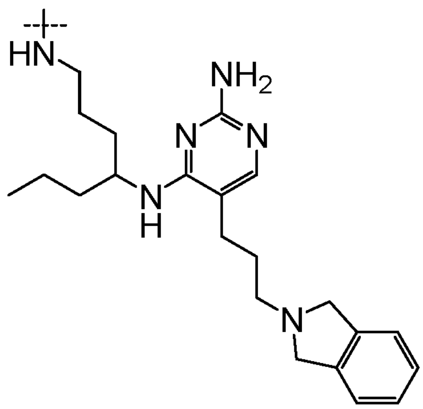 |

(continued)

| Adj | Structure | Adj | Structure |
|---|---|---|---|
| 269 | 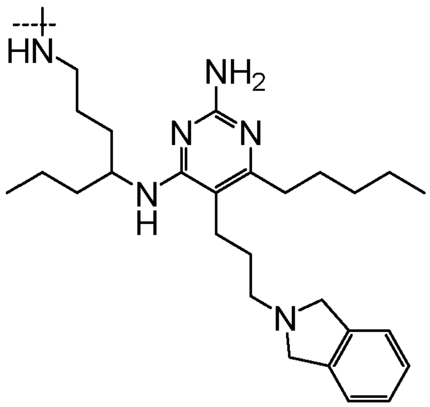 | 270 | 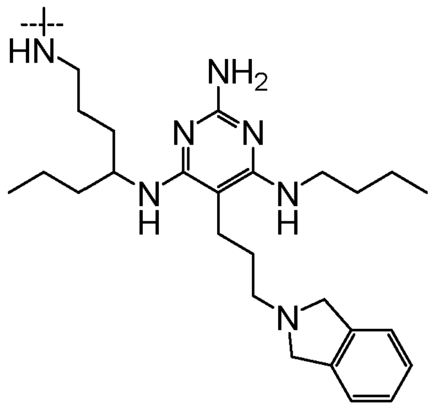 |
| 271 | 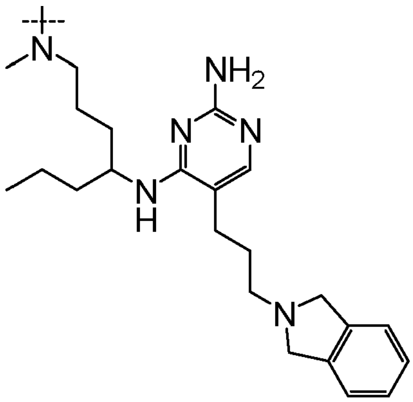 | 272 | 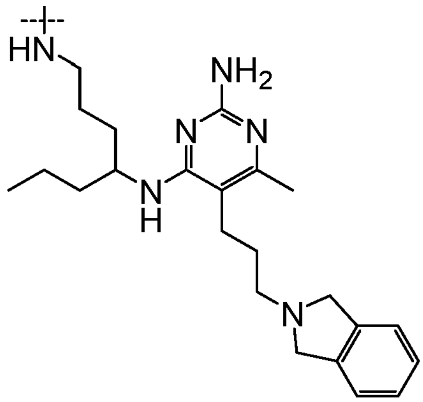 |
| 273 | 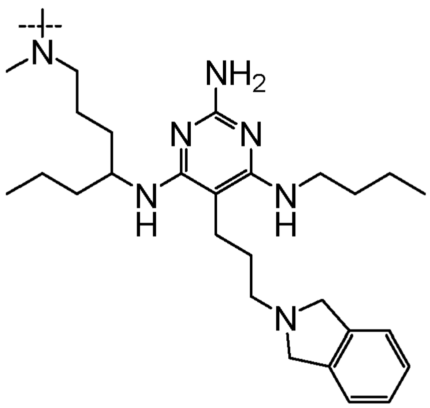 | 274 | 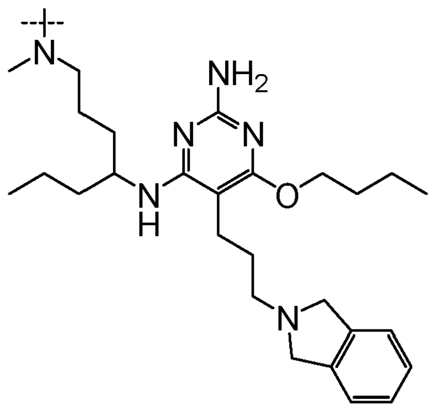 |
| 275 | 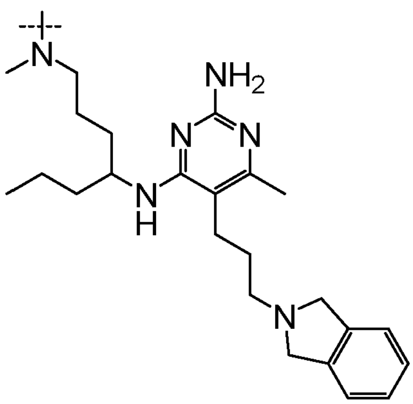 | 276 | 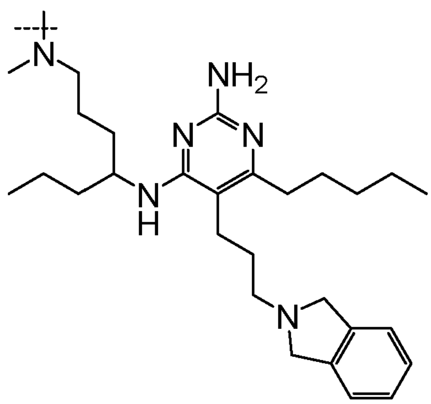 |

(continued)

| Adj | Structure | Adj | Structure |
|---|---|---|---|
| 277 | 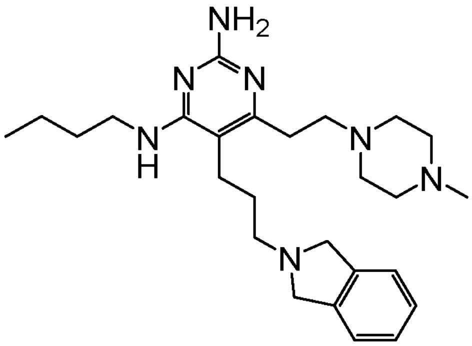 | 278 | 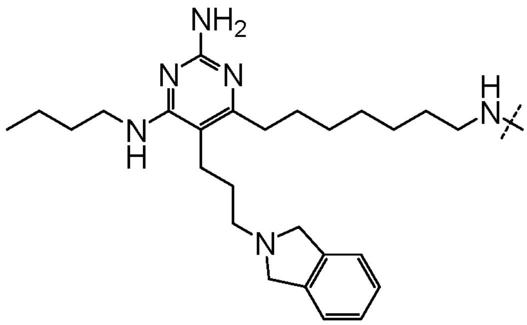 |
| 279 | 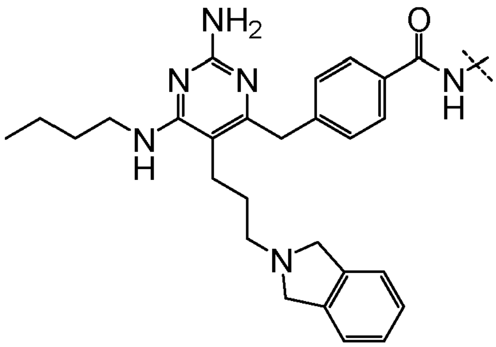 | 280 | 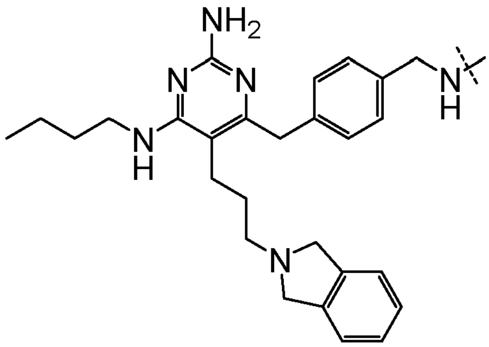 |
| 281 | 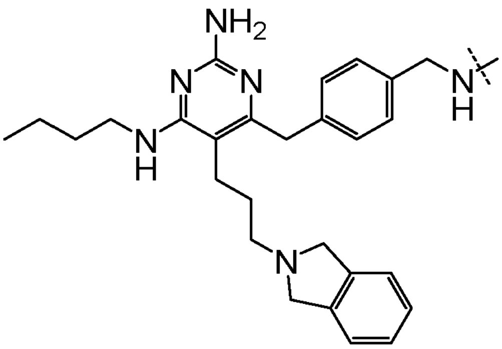 | 282 | 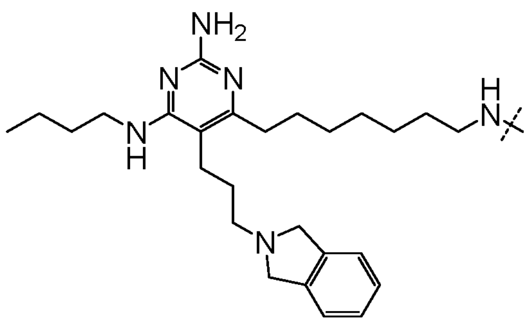 |
| 283 | 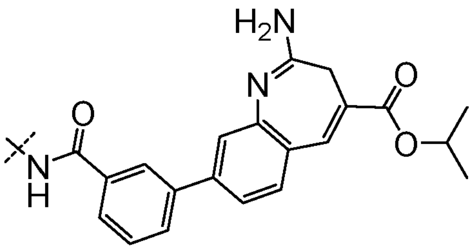 | 284 | 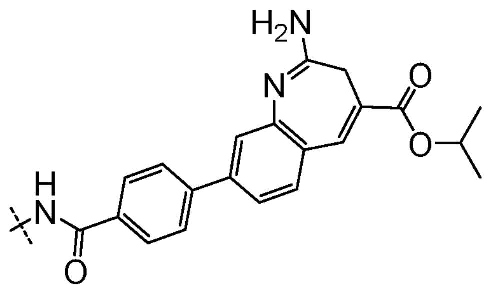 |
| 285 | 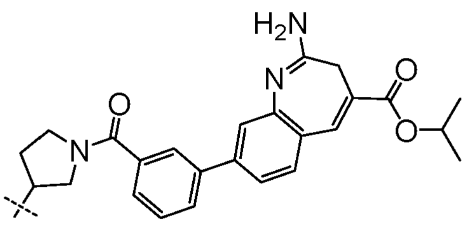 | 286 | 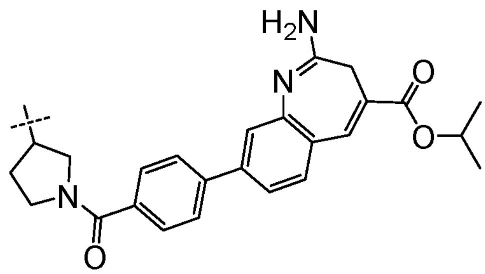 |

(continued)

| Adj | Structure | Adj | Structure |
|---|---|---|---|
| 287 | 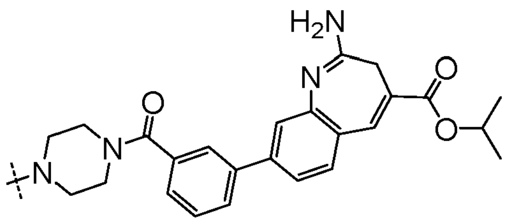 | 288 | 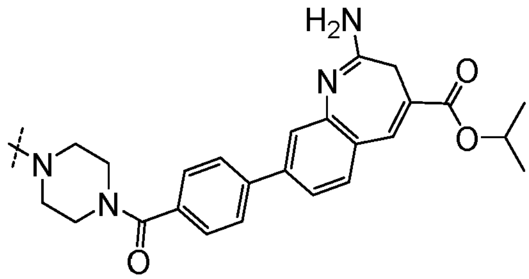 |
| 289 | 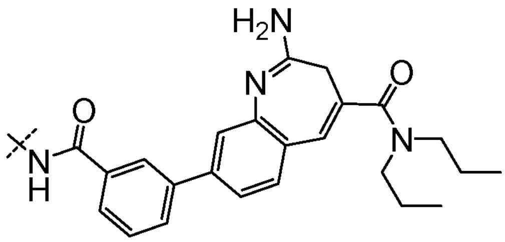 | 290 | 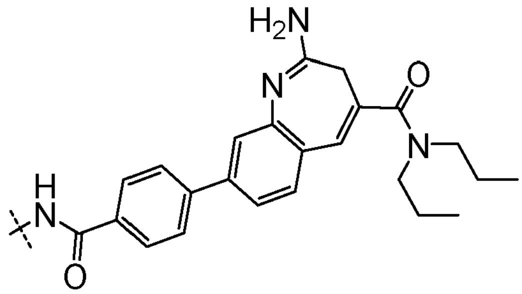 |
| 291 | 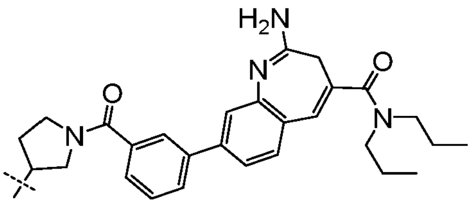 | 292 | 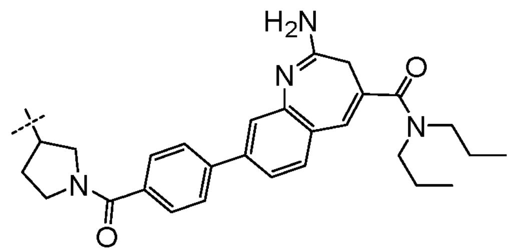 |
| 293 | 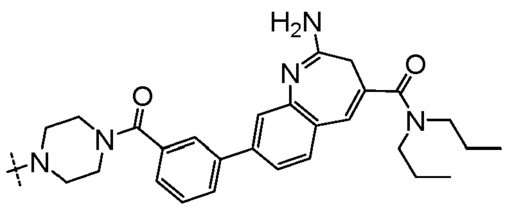 | 294 | 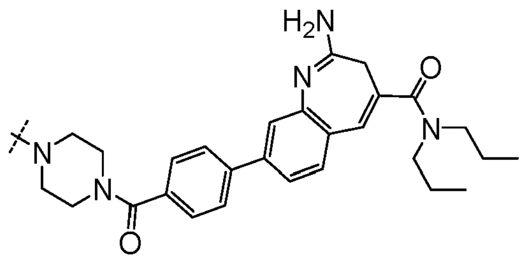 |
| 295 | 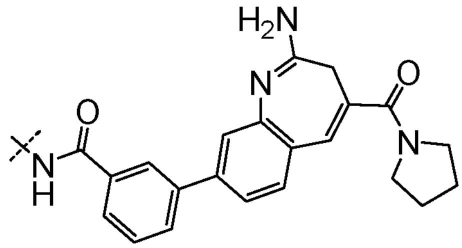 | 296 | 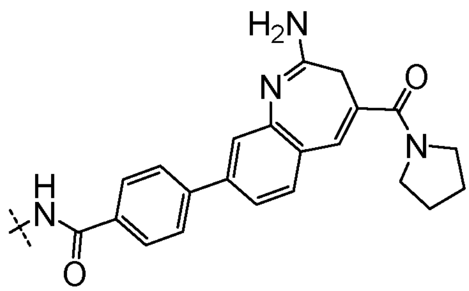 |

(continued)

| Adj | Structure | Adj | Structure |
|---|---|---|---|
| 297 | 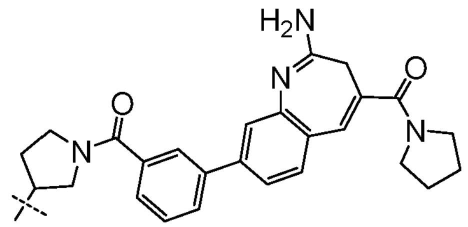 | 298 | 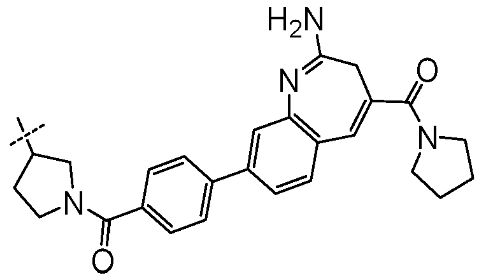 |
| 299 | 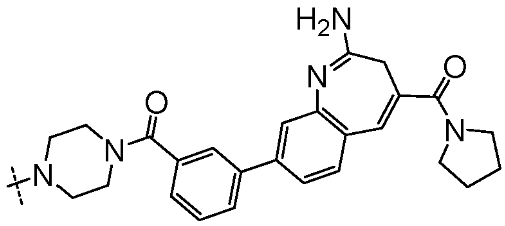 | 300 | 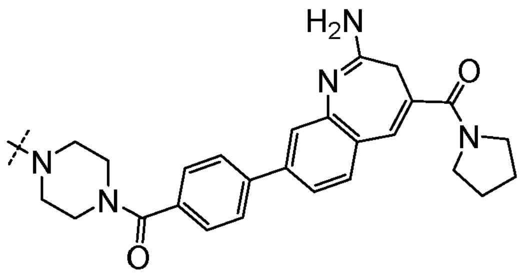 |
| 301 | 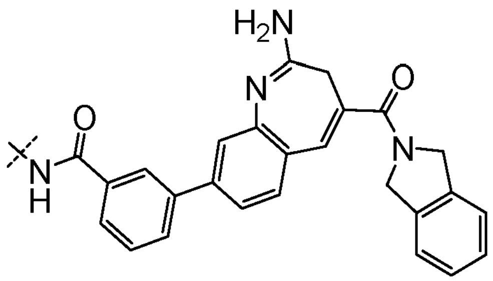 | 302 | 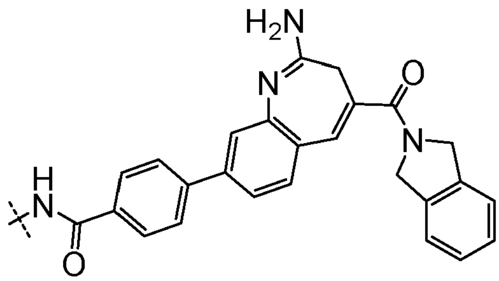 |
| 303 | 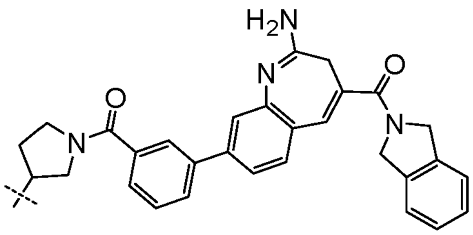 | 304 | 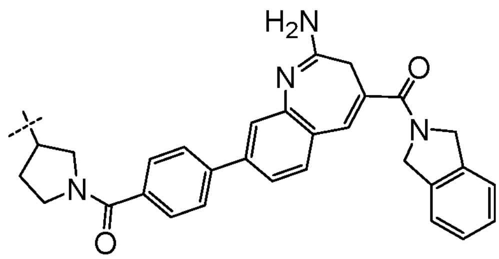 |
| 305 | 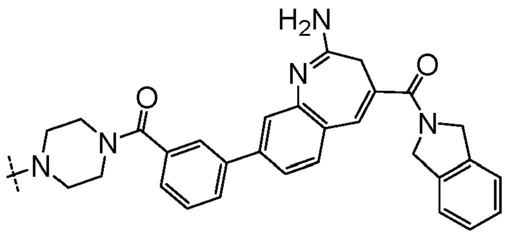 | 306 | 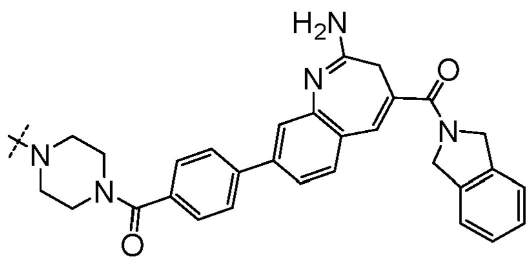 |
| 307 | 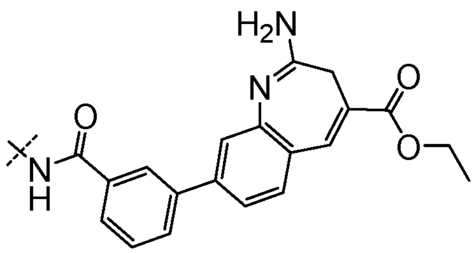 | 308 | 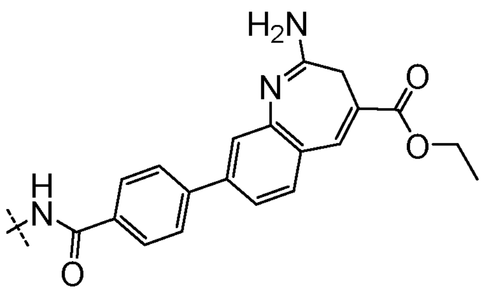 |

(continued)

| Adj | Structure | Adj | Structure |
|---|---|---|---|
| 309 | 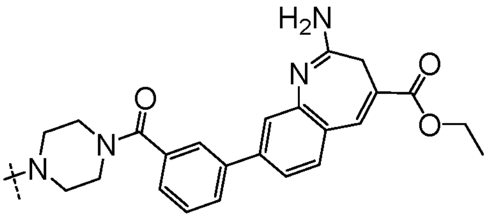 | 310 | 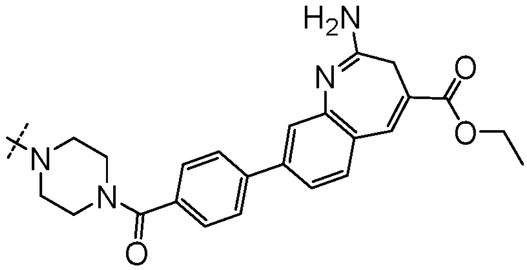 |
| 311 | 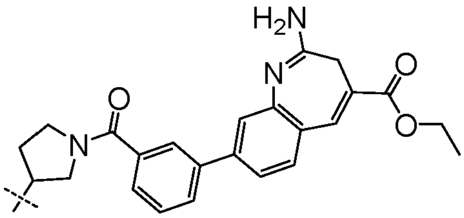 | 312 | 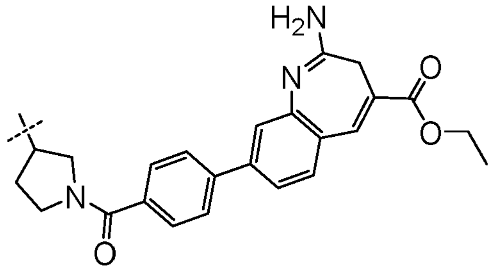 |
| 313 | 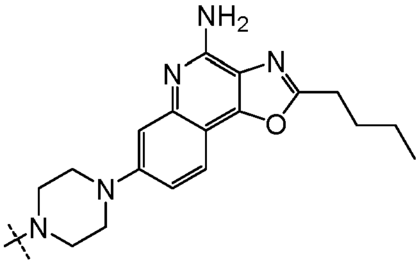 | 314 | 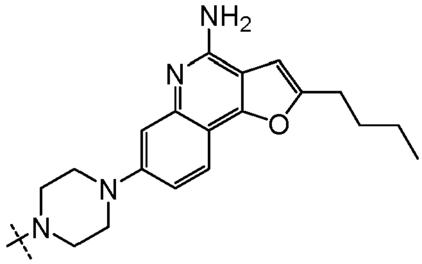 |
| 315 | 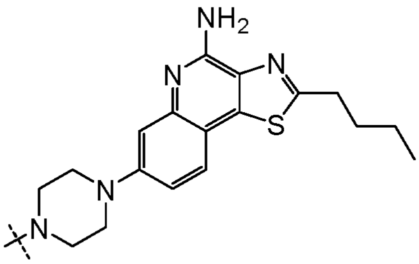 | 316 | 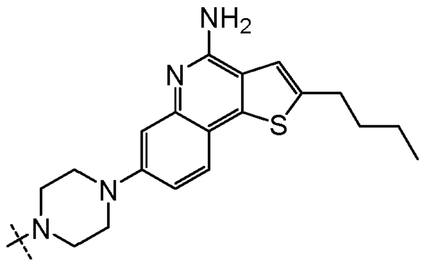 |
| 317 | 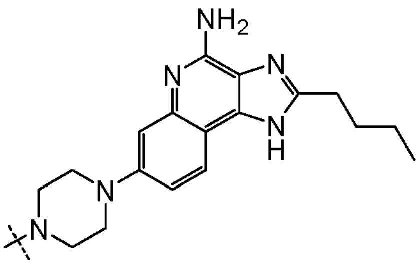 | 318 | 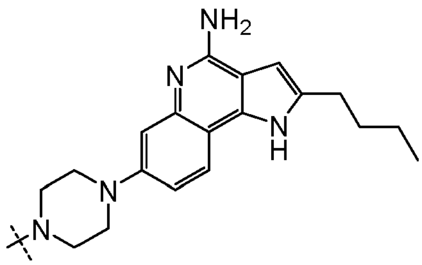 |
| 319 | 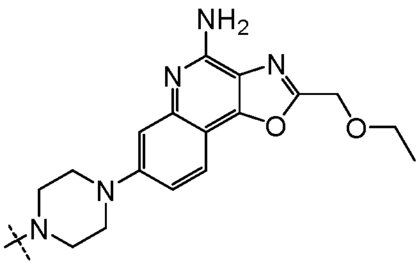 | 320 | 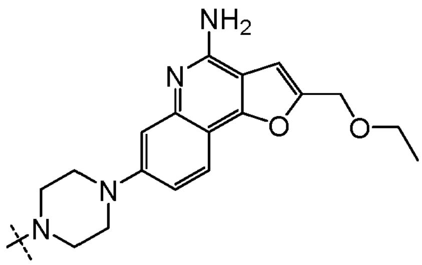 |

(continued)

| Adj | Structure | Adj | Structure |
|---|---|---|---|
| 321 | 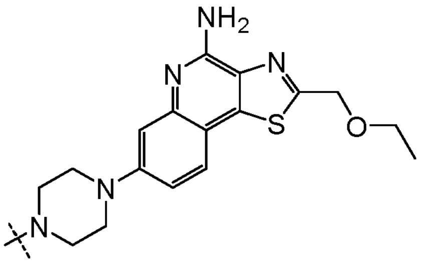 | 322 | 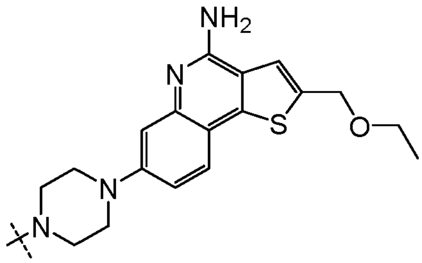 |
| 323 | 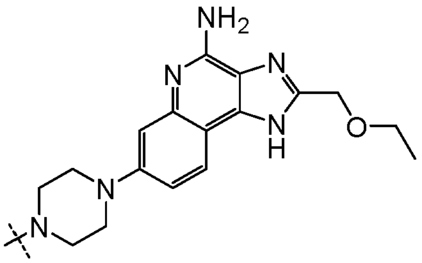 | 324 | 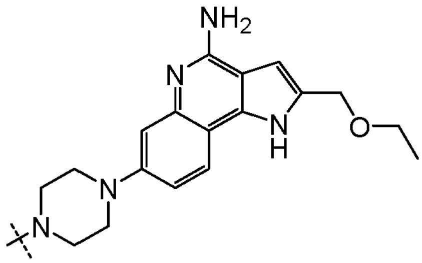 |
| 325 | 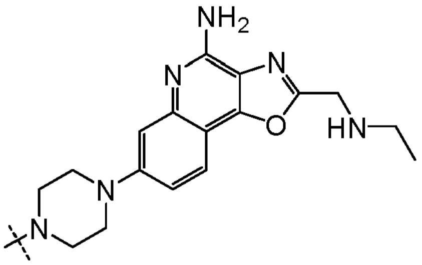 | 326 | 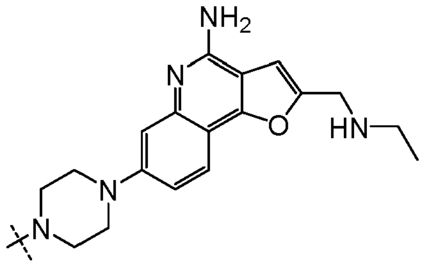 |
| 327 | 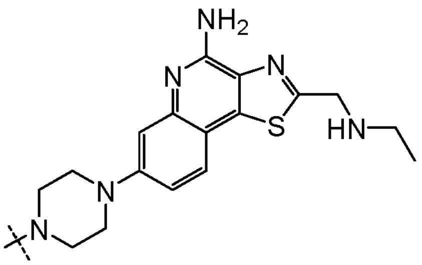 | 328 | 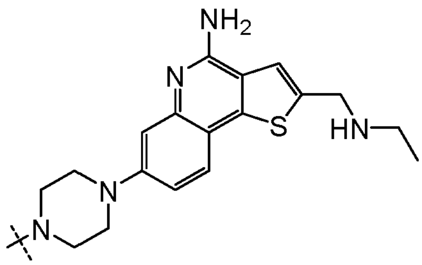 |
| 329 | 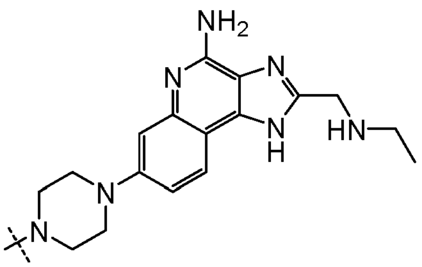 | 330 | 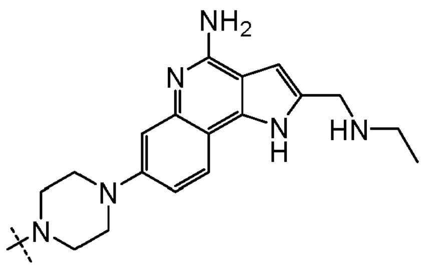 |
| 331 | 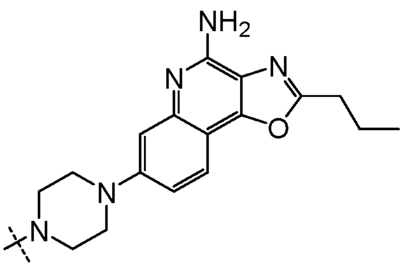 | 332 | 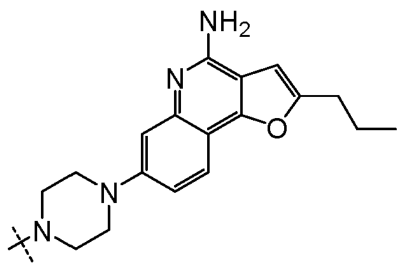 |

(continued)

| Adj | Structure | Adj | Structure |
|---|---|---|---|
| 333 | 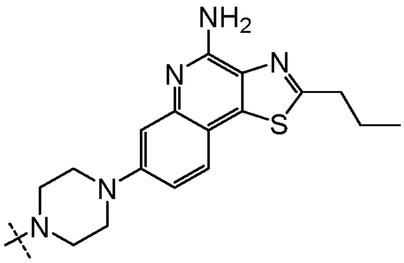 | 334 | 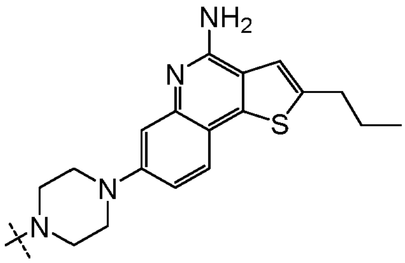 |
| 335 | 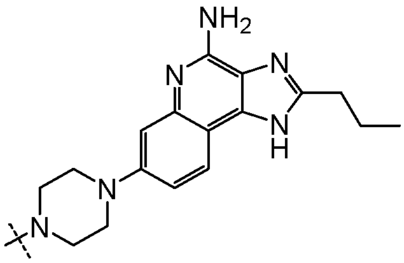 | 336 | 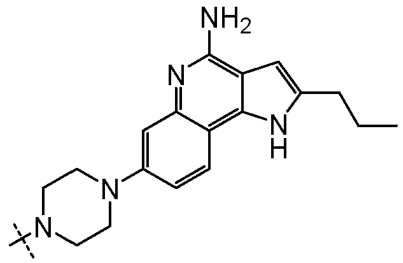 |
| 337 | 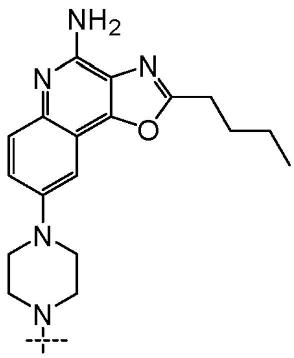 | 338 | 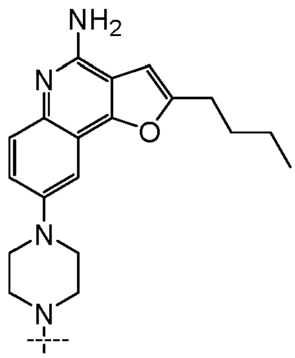 |
| 339 | 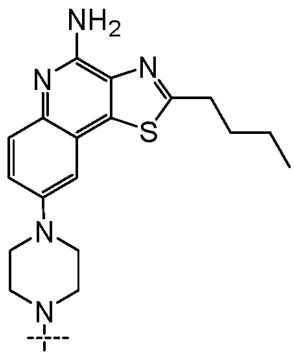 | 340 | 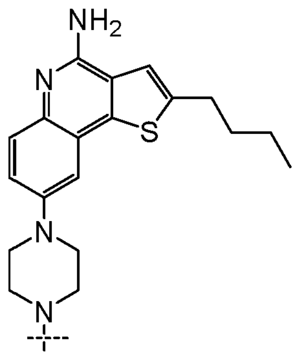 |
| 341 | 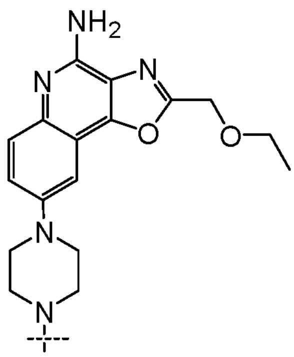 | 342 | 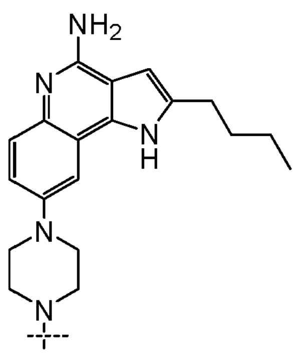 |

(continued)

| Adj | Structure | Adj | Structure |
|---|---|---|---|
| 343 | 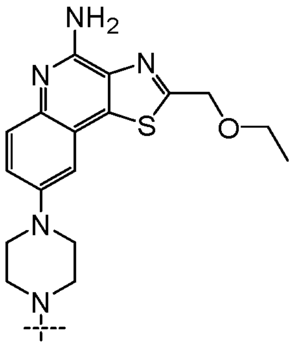 | 344 | 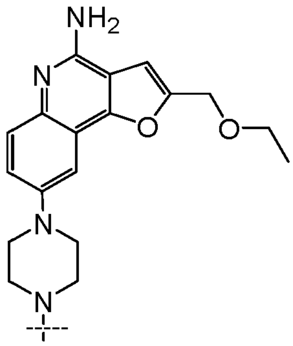 |
| 345 | 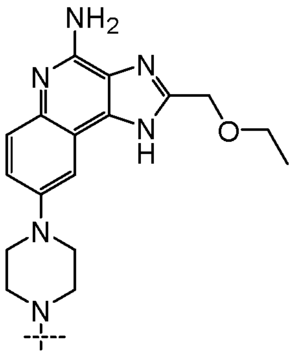 | 346 | 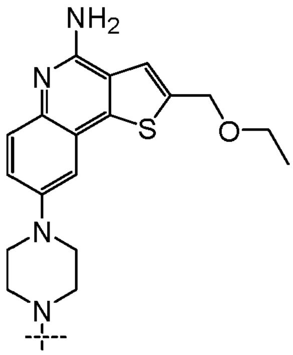 |
| 347 | 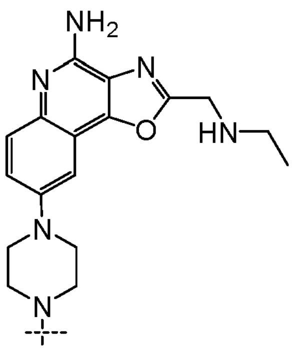 | 348 | 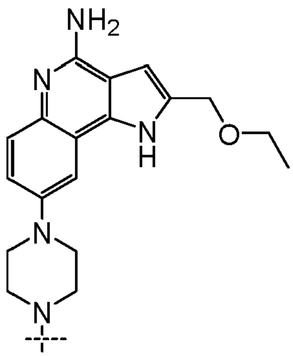 |
| 349 | 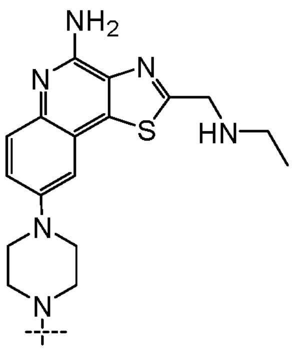 | 350 | 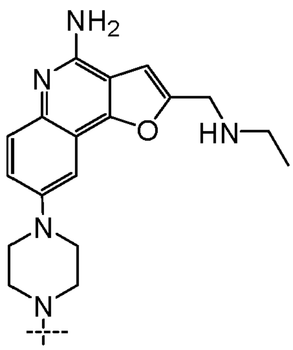 |

(continued)

| Adj | Structure | Adj | Structure |
|---|---|---|---|
| 351 | 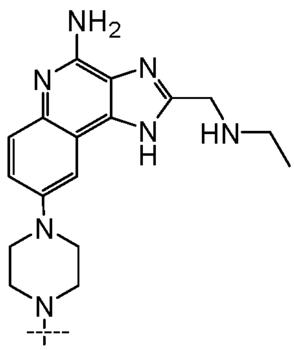 | 352 | 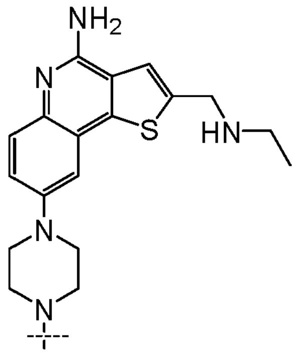 |
| 353 | 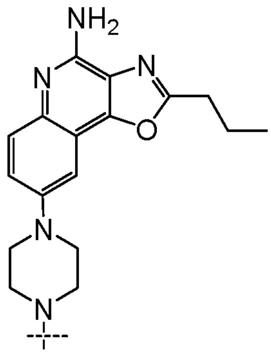 | 354 | 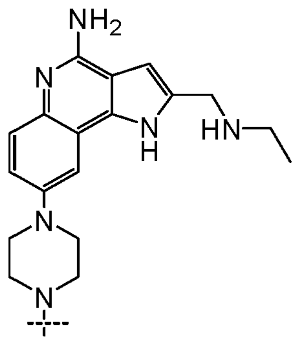 |
| 355 | 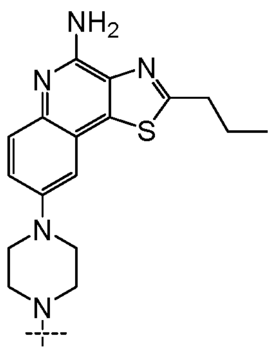 | 356 | 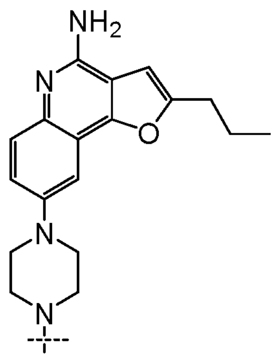 |
| 357 | 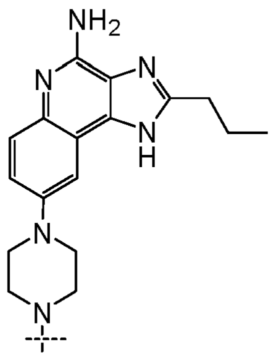 | 358 | 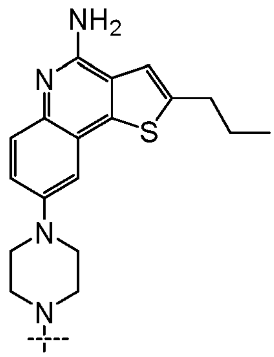 |

(continued)

| Adj | Structure | Adj | Structure |
|---|---|---|---|
| 359 | 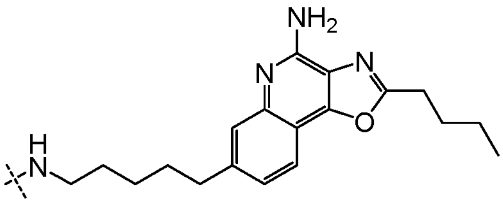 | 360 | 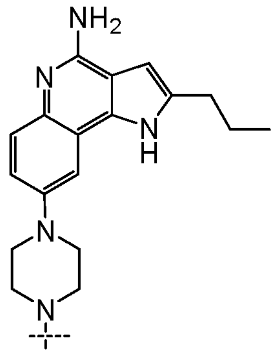 |
| 361 | 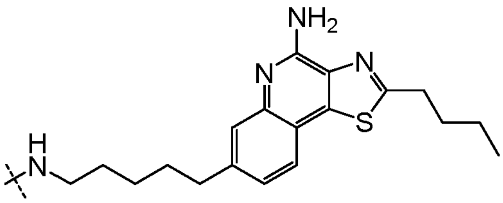 | 362 | 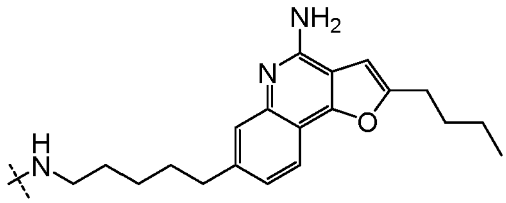 |
| 363 | 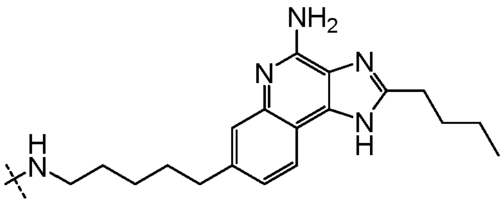 | 364 | 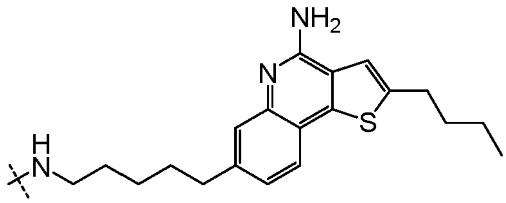 |
| 365 | 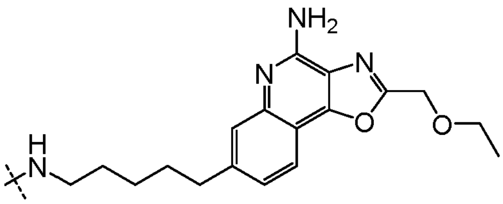 | 366 | 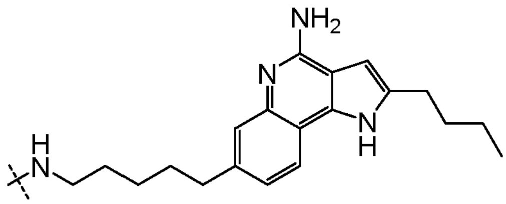 |
| 367 | 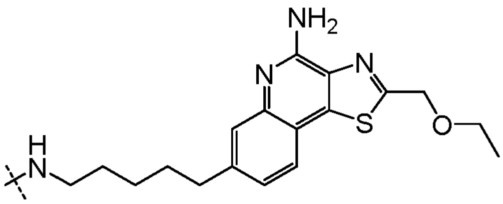 | 368 | 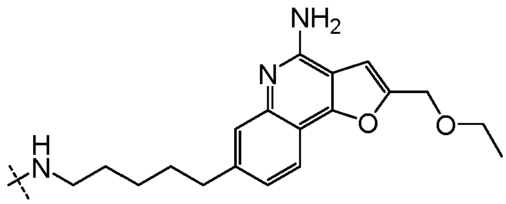 |
| 369 | 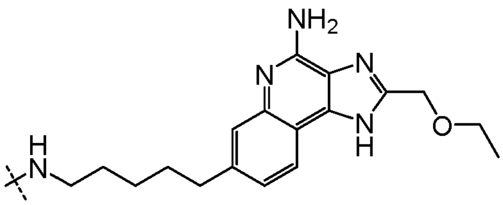 | 370 | 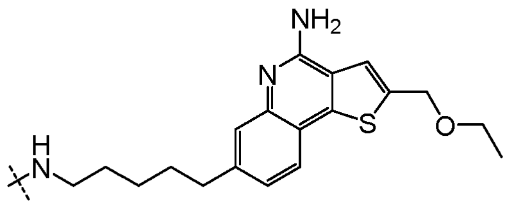 |
| 371 | 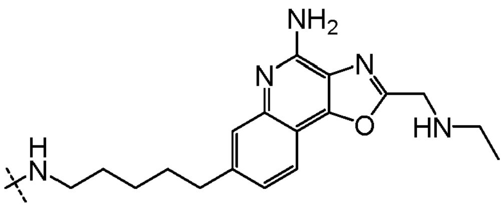 | 372 | 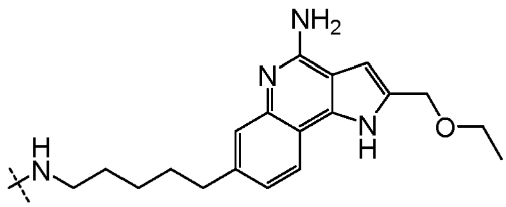 |

(continued)

| Adj | Structure | Adj | Structure |
|---|---|---|---|
| 373 | 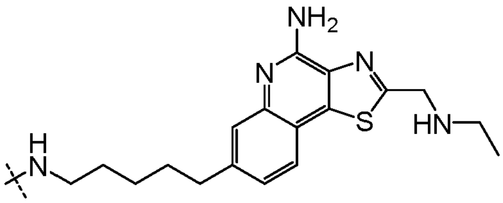 | 374 | 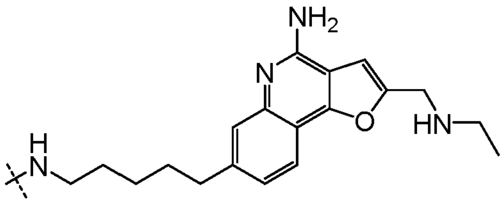 |
| 375 | 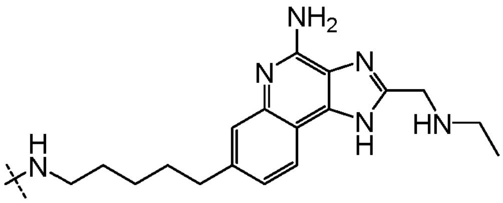 | 376 | 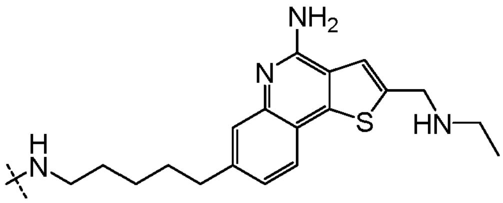 |
| 377 | 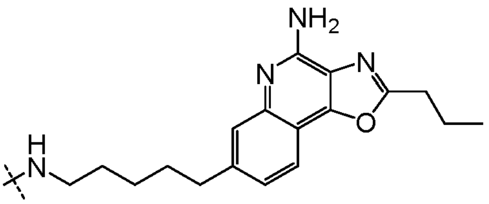 | 378 | 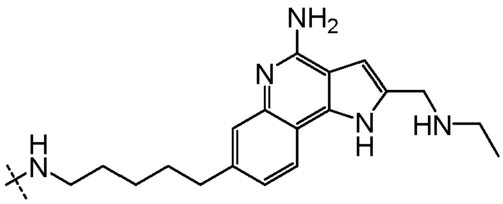 |
| 379 | 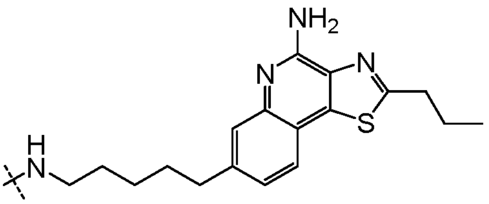 | 380 | 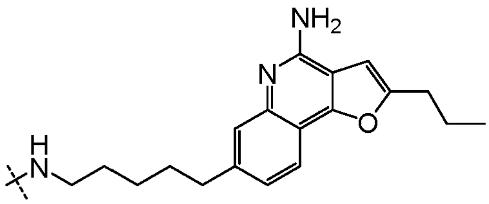 |
| 381 | 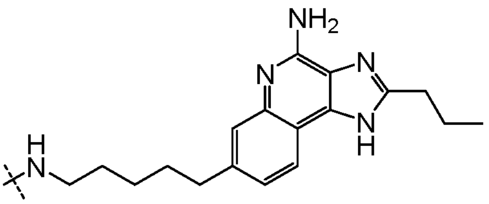 | 382 | 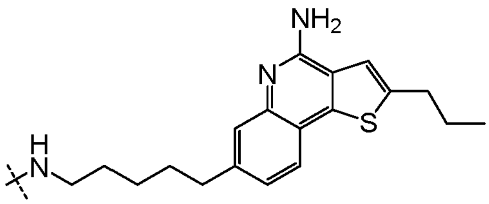 |
| 383 | 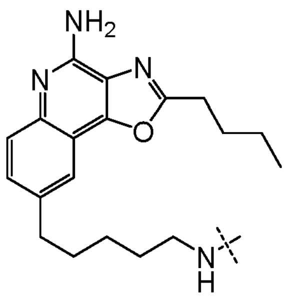 | 384 | 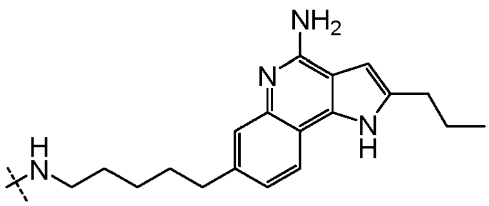 |
| 385 | 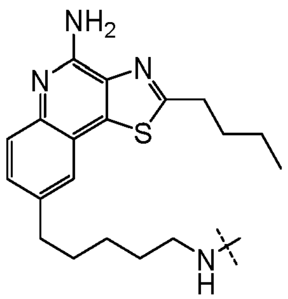 | 386 | 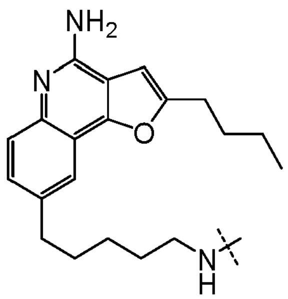 |

(continued)

| Adj | Structure | Adj | Structure |
|---|---|---|---|
| 387 | 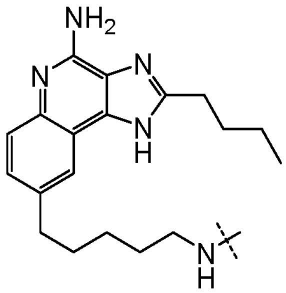 | 388 | 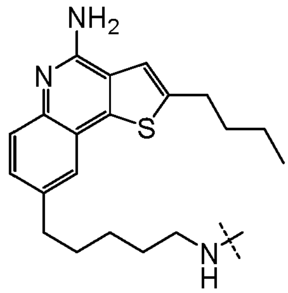 |
| 389 | 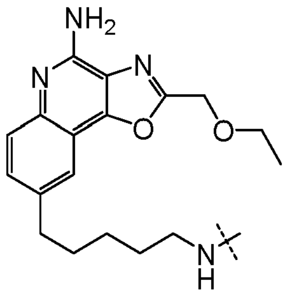 | 390 | 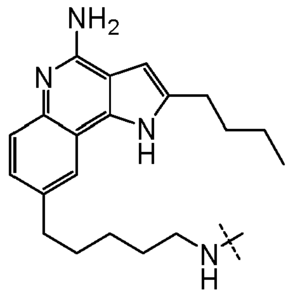 |
| 391 | 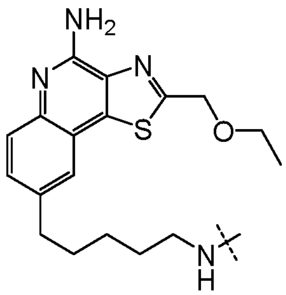 | 392 | 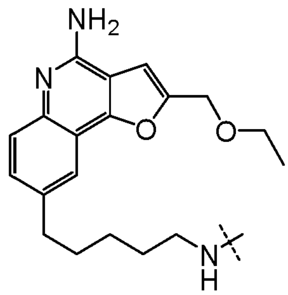 |
| 393 | 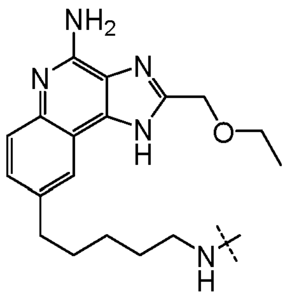 | 394 | 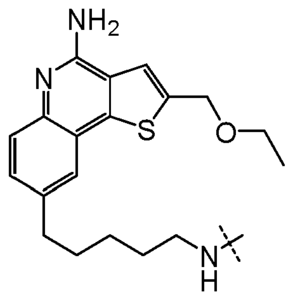 |
| 395 | 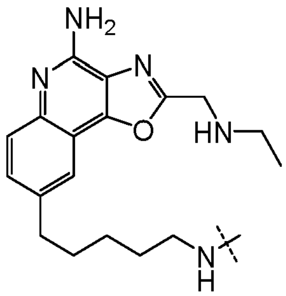 | 396 | 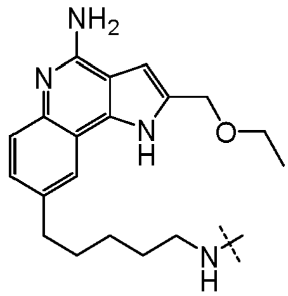 |

(continued)

| Adj | Structure | Adj | Structure |
|---|---|---|---|
| 397 | 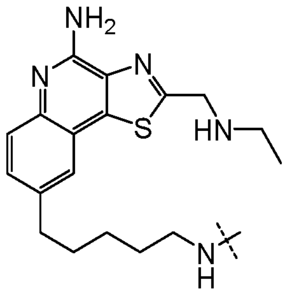 | 398 | 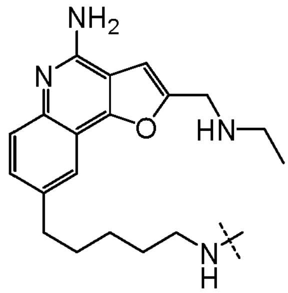 |
| 399 | 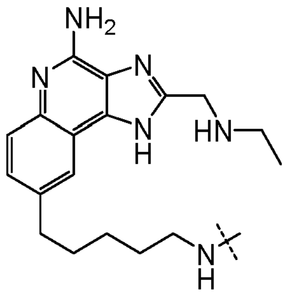 | 400 | 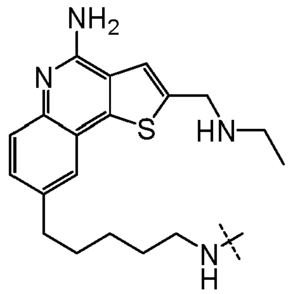 |
| 401 | 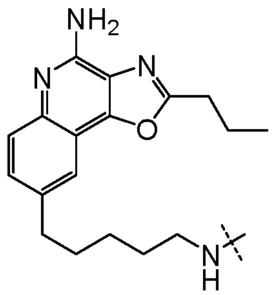 | 402 | 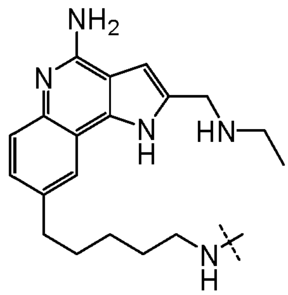 |
| 403 | 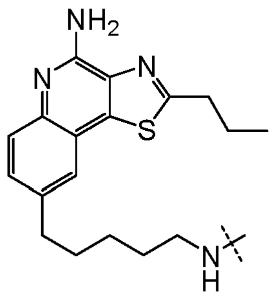 | 404 | 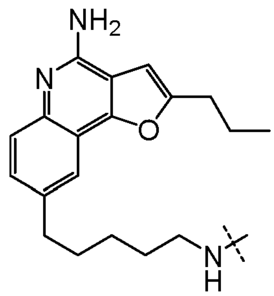 |
| 405 | 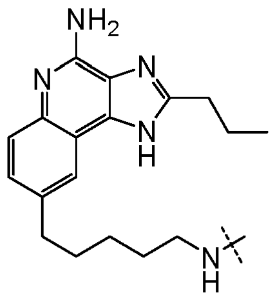 | 406 | 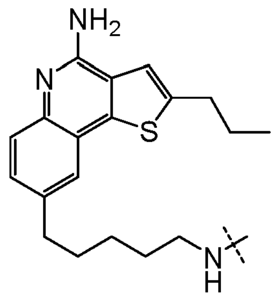 |

(continued)

| Adj | Structure | Adj | Structure |
|---|---|---|---|
| 407 | 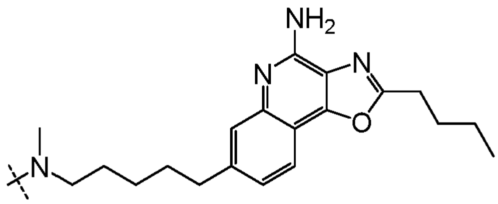 | 408 | 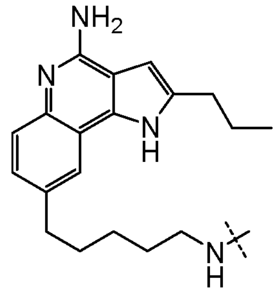 |
| 409 | 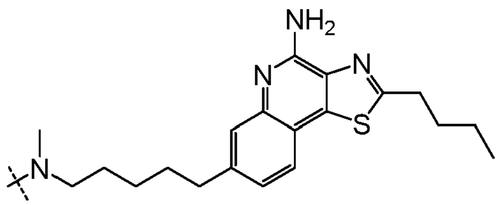 | 410 | 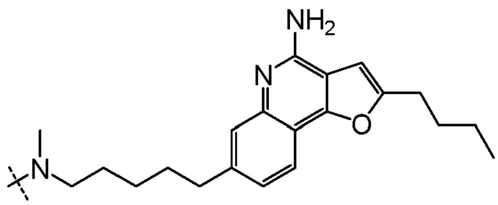 |
| 411 | 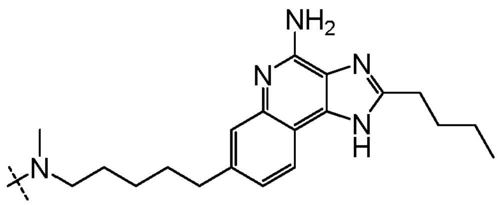 | 412 | 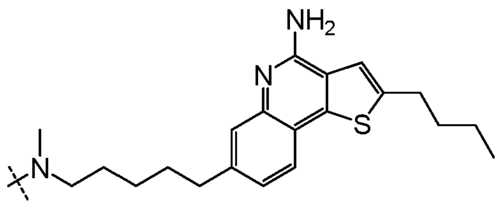 |
| 413 | 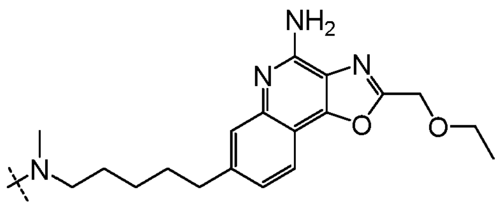 | 414 | 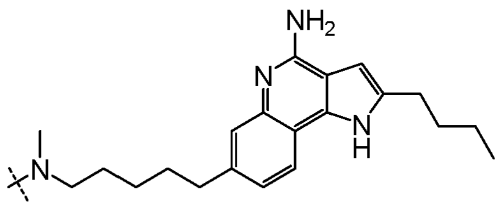 |
| 415 | 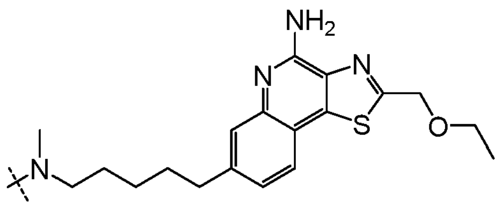 | 416 | 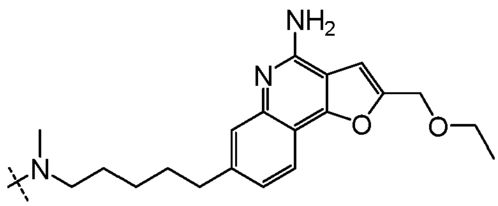 |
| 417 | 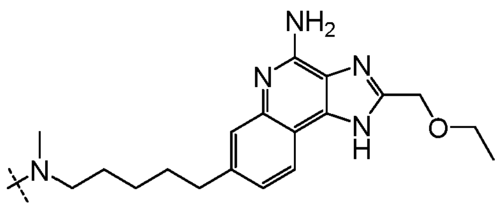 | 418 | 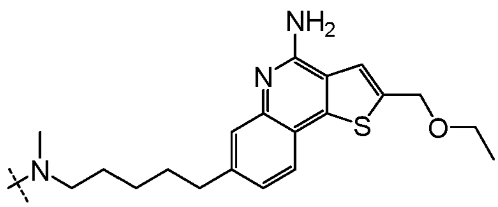 |
| 419 | 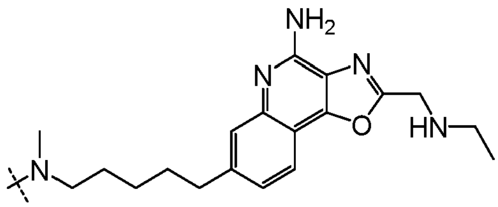 | 420 | 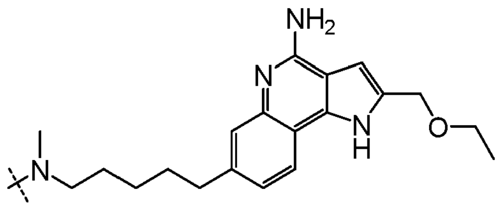 |

(continued)

| Adj | Structure | Adj | Structure |
|---|---|---|---|
| 421 | 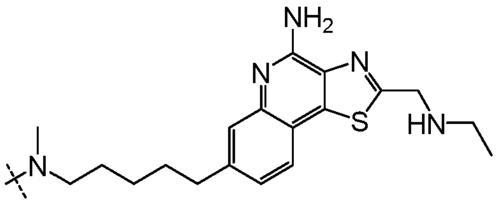 | 422 | 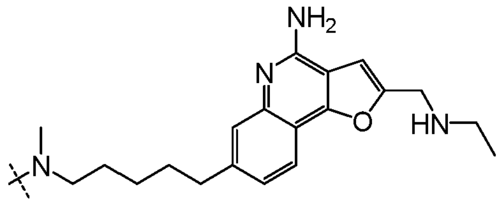 |
| 423 | 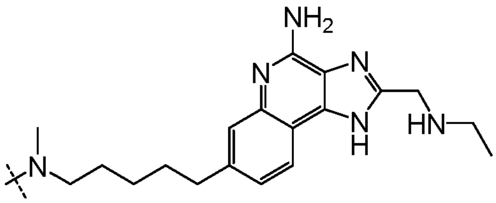 | 424 | 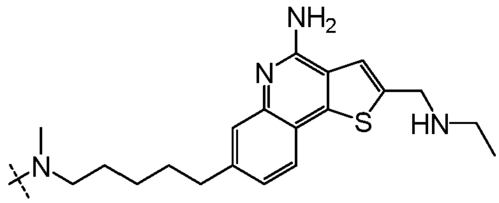 |
| 425 | 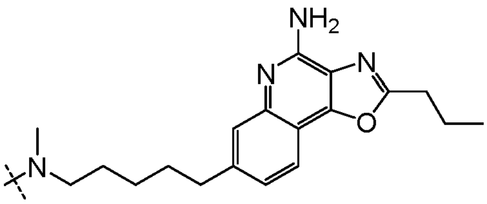 | 426 | 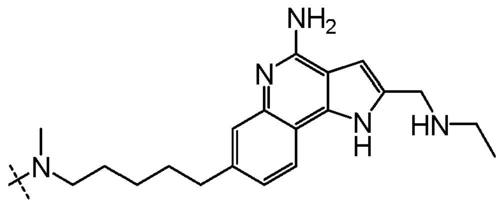 |
| 427 | 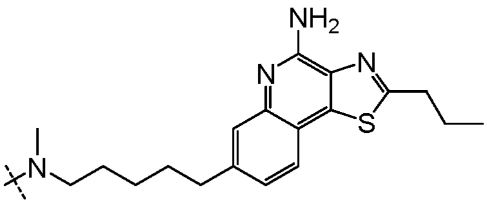 | 428 | 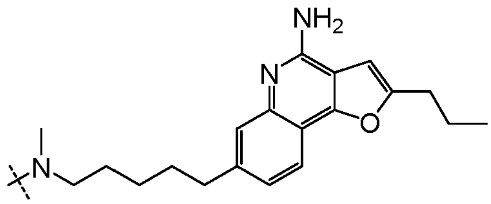 |
| 429 | 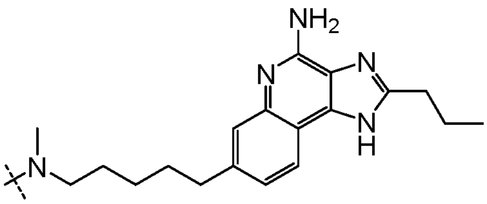 | 430 | 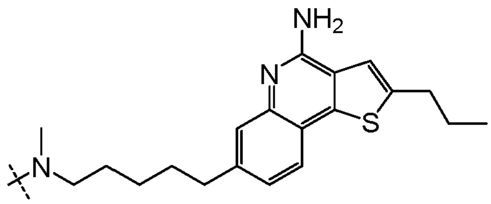 |
| 431 | 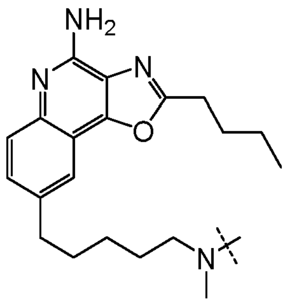 | 432 | 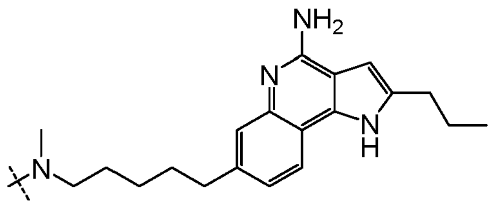 |

(continued)

| Adj | Structure | Adj | Structure |
|---|---|---|---|
| 433 | 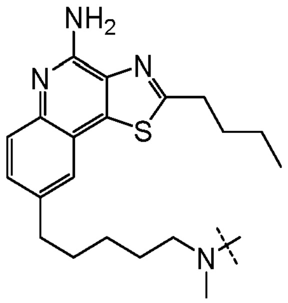 | 434 | 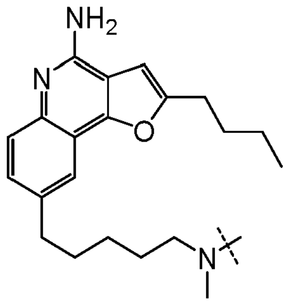 |
| 435 | 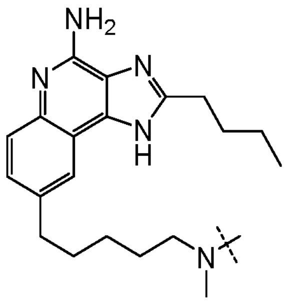 | 436 | 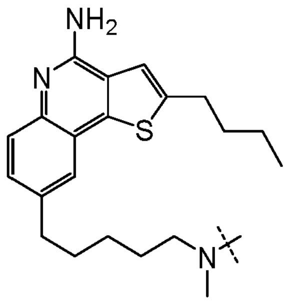 |
| 437 | 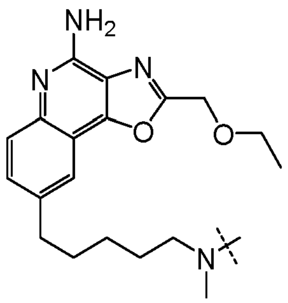 | 438 | 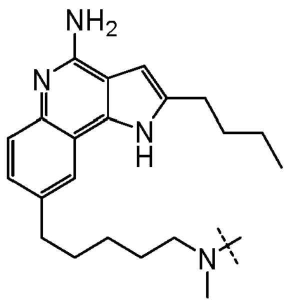 |
| 439 | 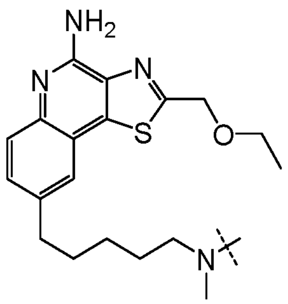 | 440 | 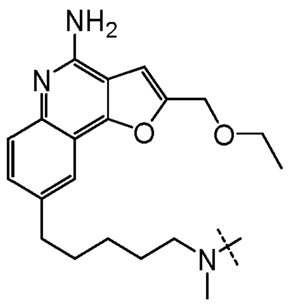 |
| 441 | 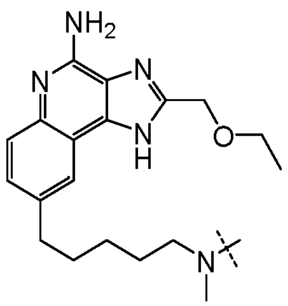 | 442 | 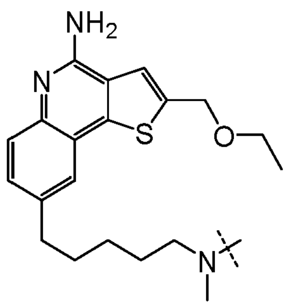 |

(continued)

| Adj | Structure | Adj | Structure |
|---|---|---|---|
| 443 | 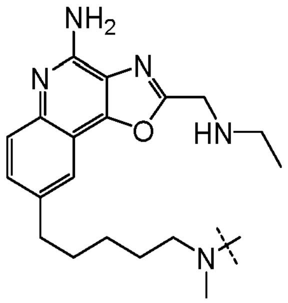 | 444 | 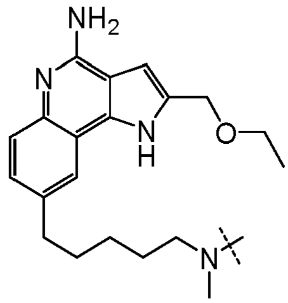 |
| 445 | 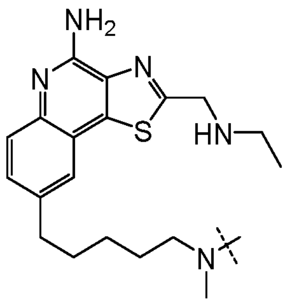 | 446 | 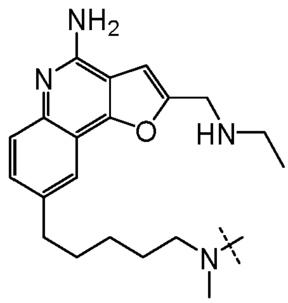 |
| 447 | 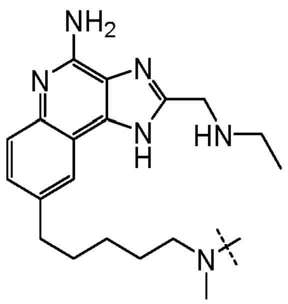 | 448 | 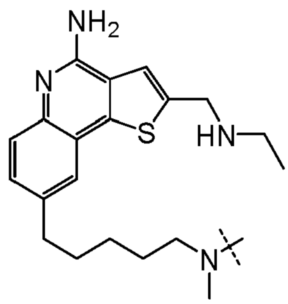 |
| 449 | 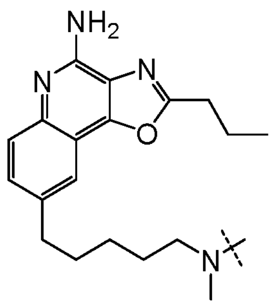 | 450 | 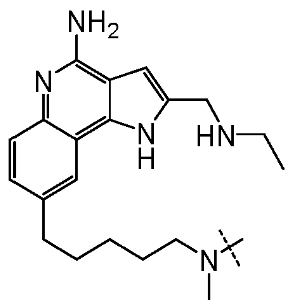 |
| 451 | 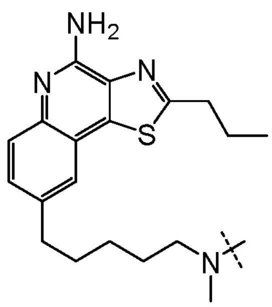 | 452 | 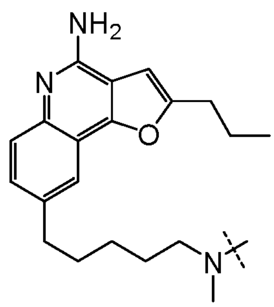 |

(continued)

| Adj | Structure | Adj | Structure |
|---|---|---|---|
| 453 | 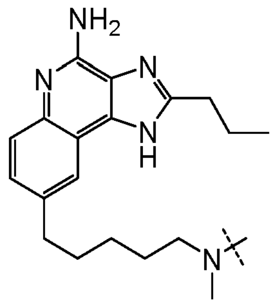 | 454 | 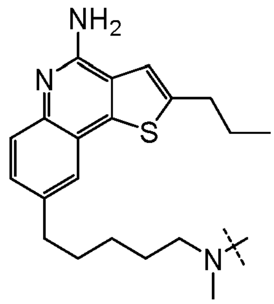 |
| 455 | 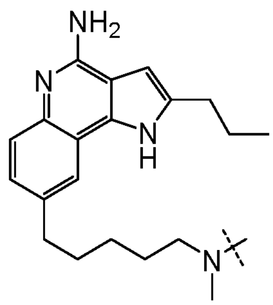 | 456 | 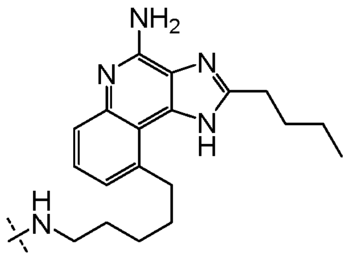 |
| 457 | 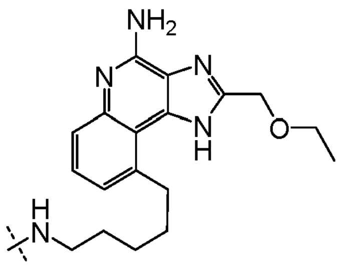 | 458 | 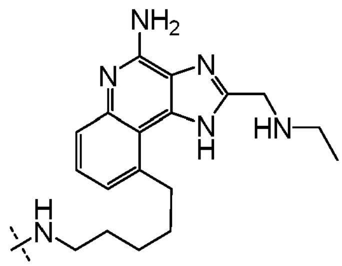 |
| 459 | 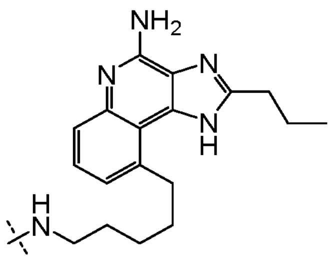 | 460 | 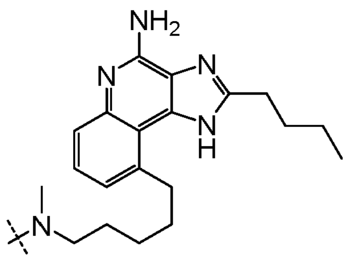 |
| 461 | 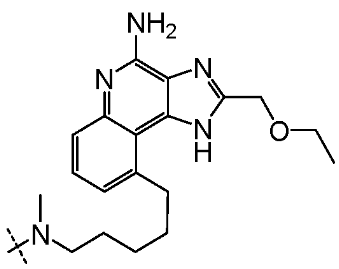 | 462 | 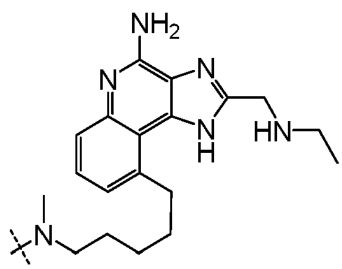 |

(continued)

| Adj | Structure | Adj | Structure |
|---|---|---|---|
| 463 | 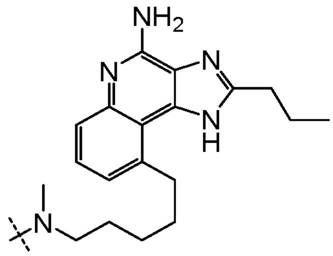 | 464 | 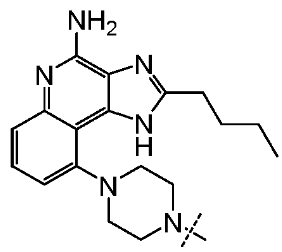 |
| 465 | 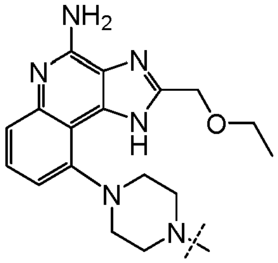 | 466 | 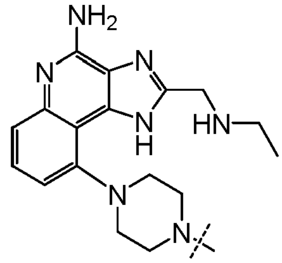 |
| 467 | 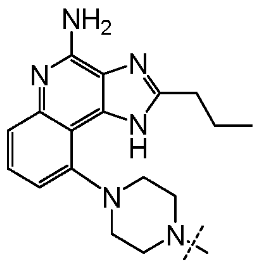 | 468 | 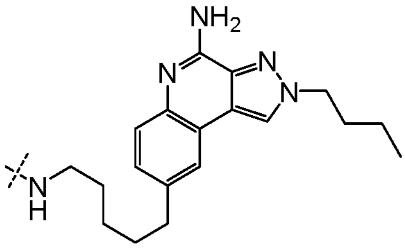 |
| 469 | 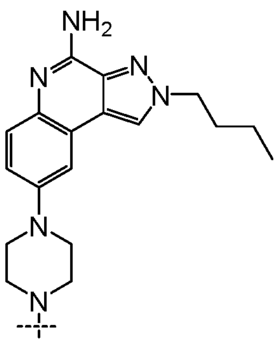 | 470 | 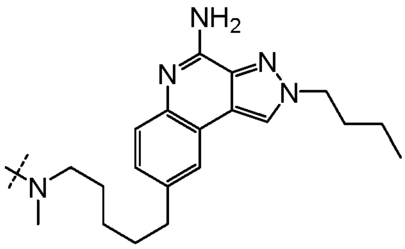 |
| 471 | 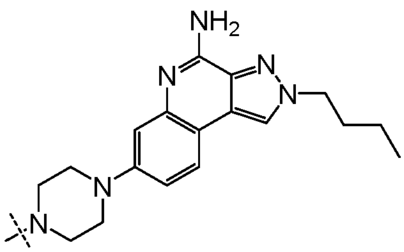 | 472 | 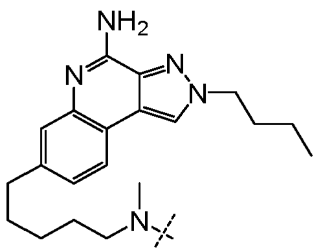 |
| 473 | 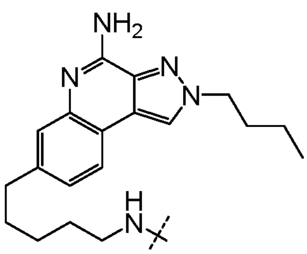 | 474 | 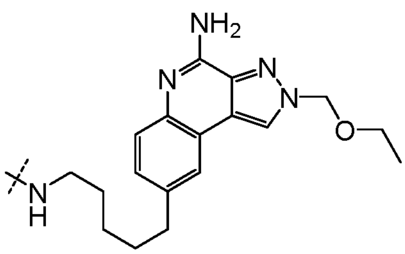 |

(continued)

| Adj | Structure | Adj | Structure |
|---|---|---|---|
| 475 | 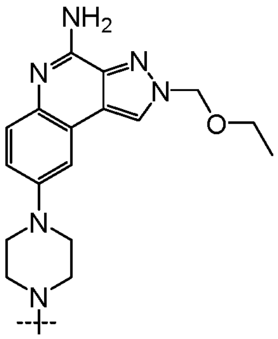 | 476 | 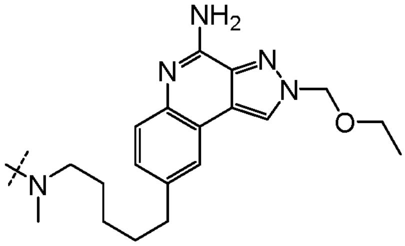 |
| 477 | 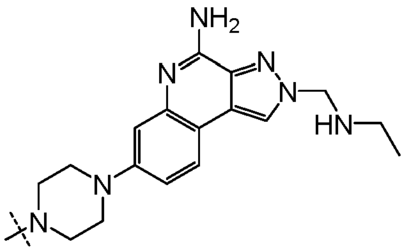 | 478 | 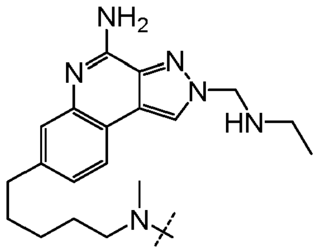 |
| 479 | 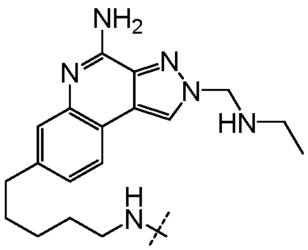 | 480 | 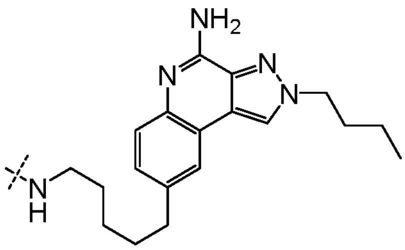 |
| 481 | 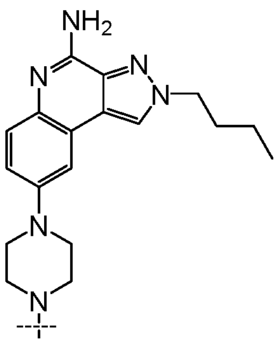 | 482 | 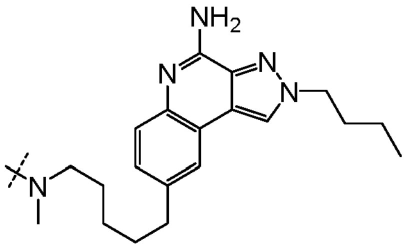 |
| 483 | 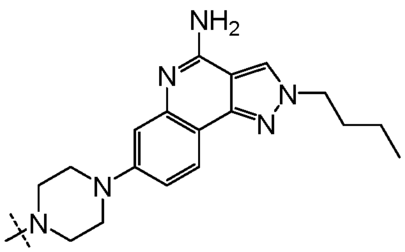 | 484 | 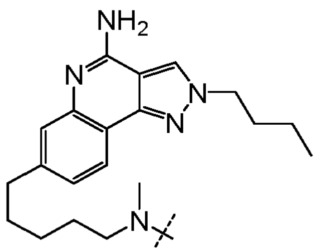 |

(continued)

| Adj | Structure | Adj | Structure |
|---|---|---|---|
| 485 | 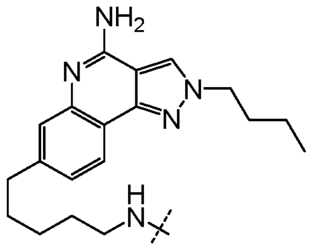 | 486 | 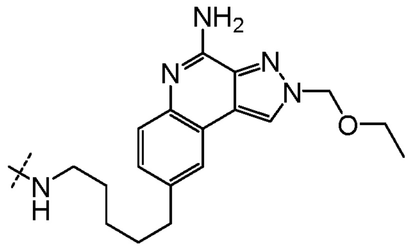 |
| 487 | 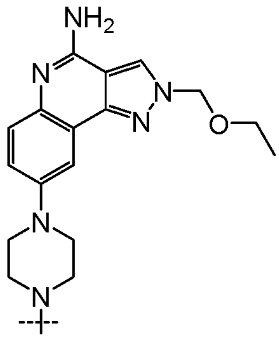 | 488 | 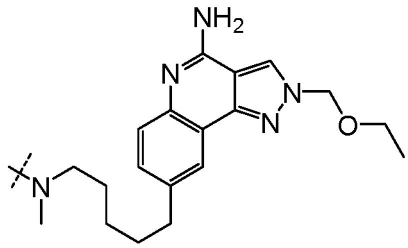 |
| 489 | 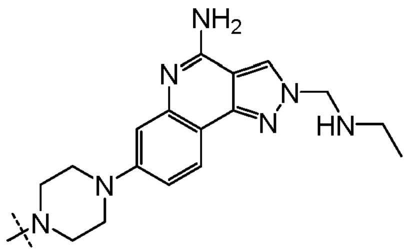 | 490 | 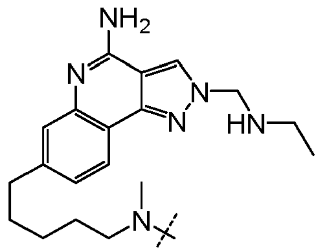 |
| 491 | 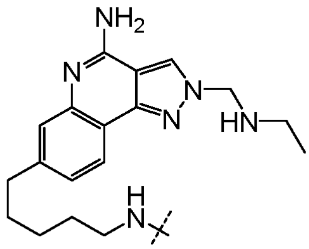 | | |

wherein the dashed line (" - - - ") represents the point of attachment of the adjuvant to the spacer.

In some embodiments, the invention provides an adjuvant as defined by the adjuvant moieties described herein, wherein the dashed line (" - - - ") represents a point of attachment of the adjuvant moiety to hydrogen. Without wishing to be bound by any particular theory, it is believed that if an adjuvant moiety has activity as a part of an immunoconjugate described herein, the adjuvant moiety will also be active as an adjuvant. Alternatively, the adjuvant moiety may not be active as a part of an immunoconjugate described herein but will be active as an adjuvant. Accordingly, the adjuvants can be used as a stand-alone therapeutic, or in combination therapies. For example, the adjuvants can be used in therapies targeting a toll-like receptor (e.g., TLR7 and/or TLR8).

In some embodiments, an immunoconjugate of the invention comprising and adjuvant moiety, wherein the adjuvant moiety contains an adjuvant core attached to a linker at location $R_C$, has an adjuvant activity of at least about 2 times, at least about 3 times, at least about 4 times, at least about 5 times, at least about 6 times, at least about 7 times, at least about 8 times, at least about 9 times, at least about 10 times, at least about 15 times, at least about 20 times, at least about 25 times, at least about 30 times, at least about 35 times, at least about 40 times, at least about 45 times, at least about 50 times, at least about 60 times, at least about 70 times, at least about 80 times, at least about 90 times, or at least about 100 times greater than the adjuvant activity of the same immunoconjugate except for the adjuvant core attached to the linker at the pendant amino group of the 2-amino nitrogen moiety under otherwise identical conditions. As used herein, "adjuvant activity" is a quantitative measure of the ability of the adjuvant moiety (i.e., comprising the adjuvant core and the

synthetic handle) of the immunoconjugate to bind to its receptor.

The adjuvant activity is determined using HEK293 cells that are co-transfected with human TLR7 or TLR8 or murine TLR7 and an inducible secreted embryonic alkaline phosphatase reporter gene under the control of the IFN-β minimal promoter fused to NF-κB and AP-1 binding sites. The cells are subsequently incubated with 2-fold serial dilutions of each the indicated adjuvants for 12 hours at 37 °C in the presence of an alkaline phosphatase substrate. Activity was measured by spectrophotometry (OD 650 nm).

In some embodiments, an immunoconjugate of the invention comprising and adjuvant moiety, wherein the adjuvant moiety contains an adjuvant core attached to a linker at location $R_C$, has an adjuvant activity of at least about 2 times, at least about 3 times, at least about 4 times, at least about 5 times, at least about 6 times, at least about 7 times, at least about 8 times, at least about 9 times, at least about 10 times, at least about 15 times, at least about 20 times, at least about 25 times, at least about 30 times, at least about 35 times, at least about 40 times, at least about 45 times, at least about 50 times, at least about 60 times, at least about 70 times, at least about 80 times, at least about 90 times, or at least about 100 times greater than the adjuvant activity of the same immunoconjugate except for the adjuvant core attached to the linker at location $R_H$ under otherwise identical conditions.

In some embodiments, an immunoconjugate of the invention comprising and adjuvant moiety, wherein the adjuvant moiety contains an adjuvant core attached to a linker at location $R_C$, has an adjuvant activity of at least about 60%, at least about 65%, at least about 70%, at least about 75%, at least about 80%, at least about 85%, at least about 90%, or at least about 95% of the adjuvant activity of the adjuvant moiety in the absence of an antibody and a linker under otherwise identical conditions.

In some embodiments, an immunoconjugate of the invention comprising and adjuvant moiety, wherein the adjuvant moiety contains an adjuvant core attached to a linker at location $R_C$, has an immunoconjugate activity of at least about 2 times, at least about 3 times, at least about 4 times, at least about 5 times, at least about 6 times, at least about 7 times, at least about 8 times, at least about 9 times, at least about 10 times, at least about 15 times, at least about 20 times, at least about 25 times, at least about 30 times, at least about 35 times, at least about 40 times, at least about 45 times, at least about 50 times, at least about 60 times, at least about 70 times, at least about 80 times, at least about 90 times, or at least about 100 times greater than the immunoconjugate activity of the same immunoconjugate except for the adjuvant core attached to the linker at the pendant amino group of the 2-amino nitrogen moiety under otherwise identical conditions. As used herein, "immunoconjugate activity" is a quantitative measure of the ability of the immunoconjugate to elicit an innate immune response.

The immunoconjugate activity is determined using human antigen presenting cells (APCs) that are negatively selected from human peripheral blood mononuclear cells obtained from healthy blood donors (Stanford Blood Center) by density gradient centrifugation using a RosetteSep Human Monocyte Enrichment Cocktail (Stem Cell Technologies) containing monoclonal antibodies against CD14, CD16, CD40, CD86, CD123, and HLA-DR. Immature APCs are subsequently purified to >97% purity via negative selection using an EasySep Human Monocyte Enrichment Kit without CD 16 depletion containing monoclonal antibodies against CD14, CD16, CD40, CD86, CD123, and HLA-DR. $2 \times 10^5$ APCs are incubated with or without $6.5 \times 10^5$ autologous or allogeneic CFSE-labeled tumor cells in 96-well plates (Corning) containing IMDM medium (Gibco) supplemented with 10% fetal bovine serum, 100 U/mL penicillin, 100 μg/mL streptomycin, 2 mM L-glutamine, sodium pyruvate, non-essential amino acids, 50 μM 2-ME and, where indicated, various concentrations of antibody. Cells and cell-free supernatants are analyzed after 18 hours via flow cytometry.

In some embodiments, an immunoconjugate of the invention comprising and adjuvant moiety, wherein the adjuvant moiety contains an adjuvant core attached to a linker at location $R_C$, has an immunoconjugate activity of at least about 2 times, at least about 3 times, at least about 4 times, at least about 5 times, at least about 6 times, at least about 7 times, at least about 8 times, at least about 9 times, at least about 10 times, at least about 15 times, at least about 20 times, at least about 25 times, at least about 30 times, at least about 35 times, at least about 40 times, at least about 45 times, at least about 50 times, at least about 60 times, at least about 70 times, at least about 80 times, at least about 90 times, or at least about 100 times greater than the immunoconjugate activity of the same immunoconjugate except for the adjuvant core attached to the linker at location $R_H$ under otherwise identical conditions.

In some embodiments, an immunoconjugate of the invention comprising and adjuvant moiety, wherein the adjuvant moiety contains an adjuvant core attached to a linker at location $R_C$, has an immunoconjugate activity of at least about 90%, at least about 100%, at least about 125%, at least about 150%, at least about 175%, at least about 200%, at least about 250%, or at least about 300% of the immunoconjugate activity of a mixture comprising the adjuvant moiety and the antibody in the absence of being linked under otherwise identical conditions.

In some examples, the disclosure provides an immunoconjugate of formula:

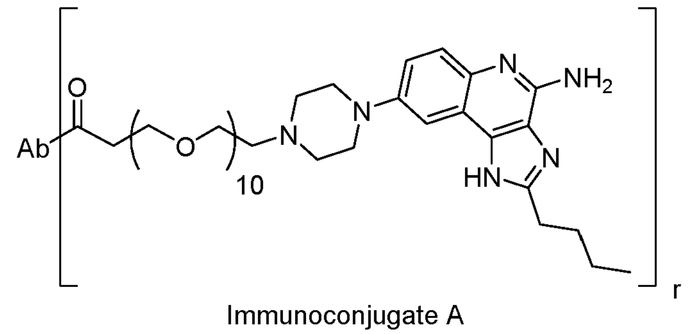

Immunoconjugate A

,

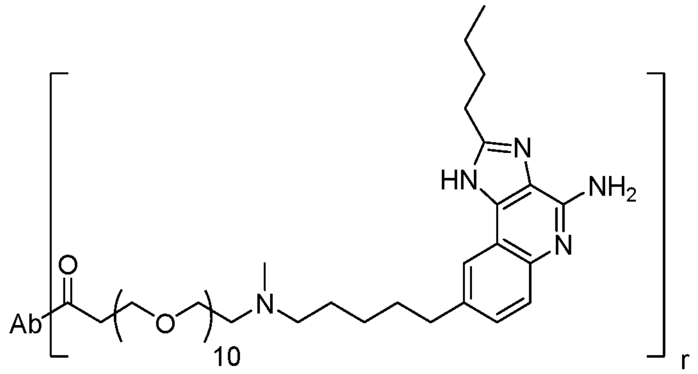

Immunoconjugate B

,

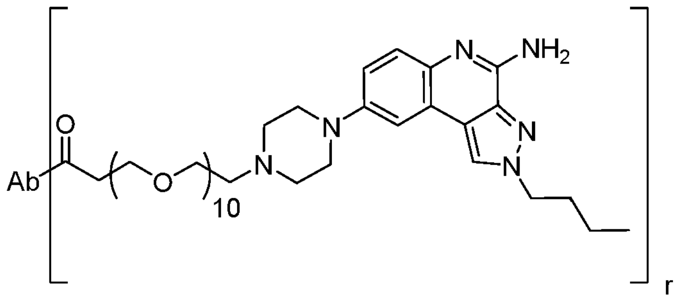

Immunoconjugate C

,

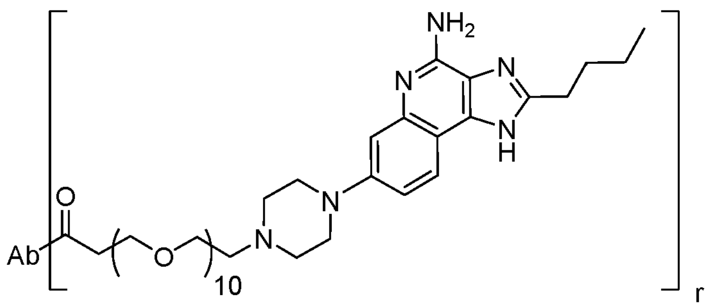

Immunoconjugate D

,

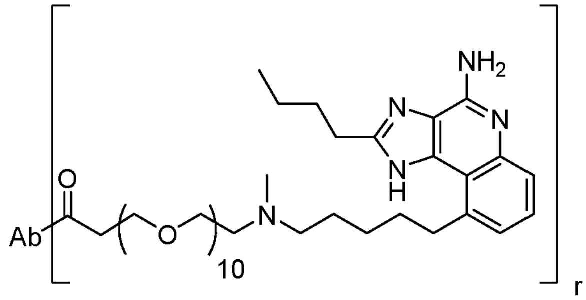

Immunoconjugate E

,

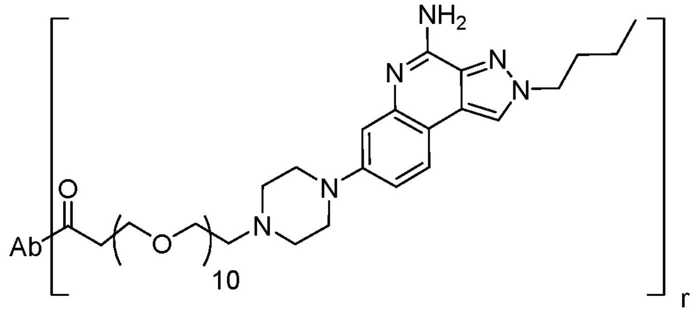

Immunoconjugate F

,

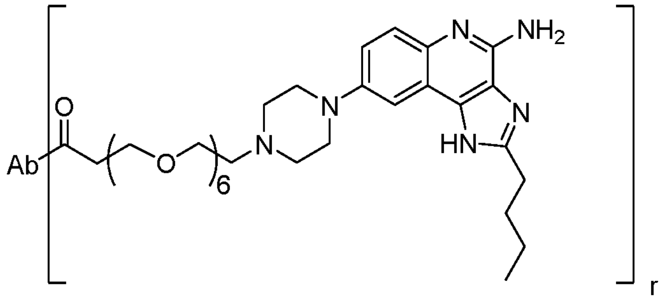

Immunoconjugate G

,

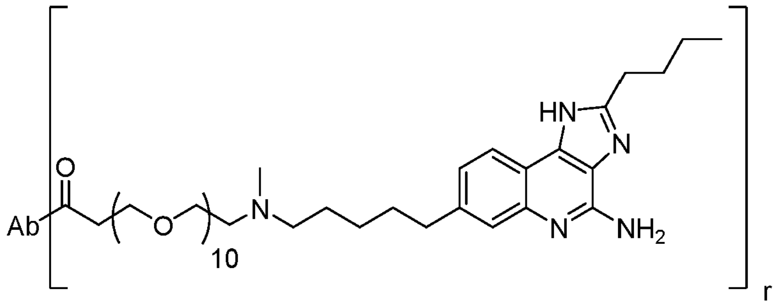

Immunoconjugate H

,

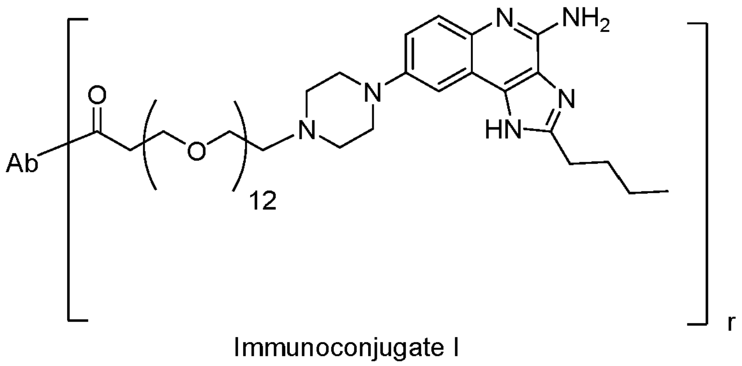

Immunoconjugate I

,

or

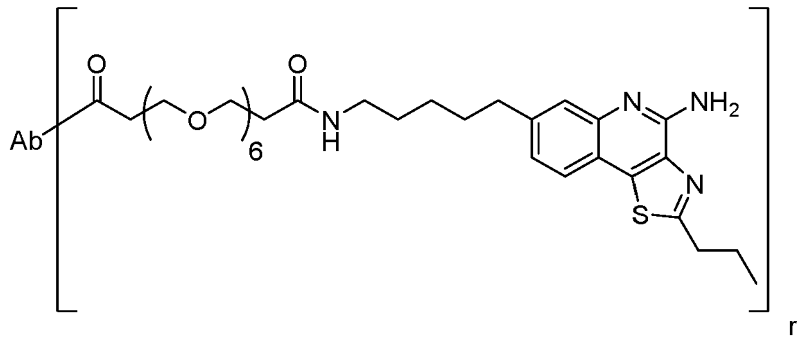

Immunoconjugate J

,

or a pharmaceutically acceptable salt thereof, wherein subscript r is an integer from 1 to 10 and "Ab" is an antibody construct described herein. In certain embodiments, subscript r is an integer from 1 to 4 (i.e., 1, 2, 3, or 4).

Formulation and administration of immunoconjugates

In a related aspect, the invention provides a composition comprising a plurality of immunoconjugates as defined in the appended claims. In some embodiments, the average number of adjuvant moieties per immunoconjugate ranges from about 1 to about 8. The average number of adjuvant moieties per immunoconjugate can range, for example, from about 1 to about 8, or from about 1 to about 6, or from about 1 to about 4. The average number of adjuvant moieties per immunoconjugate can be about 0.8, 1, 1.2, 1.4, 1.6, 1.8, 2, 2.2, 2.4, 2.6, 2.8, 3, 3.2, 3.4, 3.6, 3.8, 4.0, or 4.2. In some embodiments, the average number of adjuvant moieties per immunoconjugate is about 4. In some embodiments, the average number of adjuvant moieties per immunoconjugate is about 2. In some cases, the antibody is covalently bonded to a single adjuvant moiety. In some cases, the antibody is covalently bonded to 2 or more adjuvant moieties (e.g., 3 or more, 4 or more, or 5 or more adjuvant moieties) via a linker. In some cases, the antibody is covalently bonded to 1-8 adjuvant moieties (e.g., 1-5, 1-3, 2-8, 2-5, 2-3, or 3-8 adjuvant moieties) via a linker. In some cases, the antibody is covalently bonded to 2-8 adjuvant moieties (e.g., 2-5, 2-3, or 3-8 adjuvant moieties). In some cases in which the antibody is covalently bonded to more than one adjuvant moiety, the attached adjuvant moieties can be the same or different. For example, in some cases two or more of the adjuvant moieties can be the same (e.g., two different molecules of the same adjuvant moiety can each be attached to the antibody at a different site on the antibody). In some cases, the antibody is covalently bonded to 2 or more different adjuvant moieties (e.g., 3 or more, 4 or more, or 5 or more different adjuvant moieties). For example, when generating an immunoconjugate of the invention, one or more antibodies can be combined with a mixture that includes two or more (e.g., 3 or more, 4 or more, or 5 or more) different adjuvant-linker compounds such that amino acid sidechains in the one or more antibodies react with the adjuvant-linker compounds, thereby resulting in one or more immunoconjugates that are each covalently bonded to two or more different adjuvant moieties.

In some embodiments, the composition further comprises one or more pharmaceutically acceptable excipients. For example, the immunoconjugates of the invention can be formulated for parenteral administration, such as intravenous (IV) administration or administration into a body cavity or lumen of an organ. Alternatively, the immunoconjugates can be injected intra-tumorally. Formulations for injection will commonly comprise a solution of the immunoconjugate dissolved in a pharmaceutically acceptable carrier. Among the acceptable vehicles and solvents that can be employed are water and

Ringer's solution, an isotonic sodium chloride. In addition, sterile fixed oils can conventionally be employed as a solvent or suspending medium. For this purpose, any bland fixed oil can be employed including synthetic monoglycerides or diglycerides. In addition, fatty acids such as oleic acid can likewise be used in the preparation of injectables. These solutions are sterile and generally free of undesirable matter. These formulations can be sterilized by conventional, well known sterilization techniques. The formulations can contain pharmaceutically acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like. The concentration of the immunoconjugate in these formulations can vary widely, and will be selected primarily based on fluid volumes, viscosities, body weight, and the like, in accordance with the particular mode of administration selected and the patient's needs. In certain embodiments, the concentration of an immunoconjugate in a solution formulation for injection will range from about 0.1% (w/w) to about 10% (w/w).

In another aspect, the invention provides an immunconjugate of the invention or a composition of the invention for use in treating cancer. The use in treating cancer includes comprising administering a therapeutically effective amount of the immunoconjugate (e.g., as the composition as defined in the appended claims) to a subject in need thereof. For example, the use in treating cancer can include administering the immunoconjugate to provide a dose of from about 100 ng/kg to about 50 mg/kg to the subject. The immunoconjugate dose can range from about 5 mg/kg to about 50 mg/kg, from about 10 μg/kg to about 5 mg/kg, or from about 100 μg/kg to about 1 mg/kg. The immunoconjugate dose can be about 100, 200, 300, 400, or 500 μg/kg. The immunoconjugate dose can be about 1, 2, 3, 4, 5, 6, 7, 8, 9, or 10 mg/kg. The immunoconjugate dose can also lie outside of these ranges, depending on the particular conjugate as well as the type and severity of the cancer being treated. Frequency of administration can range from a single dose to multiple doses per week, or more frequently. In some embodiments, the immunoconjugate is administered from about once per month to about five times per week. In some embodiments, the immunoconjugate is administered once per week.

In another aspect, the invention provides an immunconjugate of the invention or a composition of the invention for use in preventing cancer. The use in preventing cancer includes comprising administering a therapeutically effective amount of the immunoconjugate (e.g., as the composition as defined in the appended claims) to a subject. In certain embodiments, the subject is susceptible to a certain cancer to be prevented. For example, the uses in preventing cancer can include administering the immunoconjugate to provide a dose of from about 100 ng/kg to about 50 mg/kg to the subject. The immunoconjugate dose can range from about 5 mg/kg to about 50 mg/kg, from about 10 μg/kg to about 5 mg/kg, or from about 100 μg/kg to about 1 mg/kg. The immunoconjugate dose can be about 100, 200, 300, 400, or 500 μg/kg. The immunoconjugate dose can be about 1, 2, 3, 4, 5, 6, 7, 8, 9, or 10 mg/kg. The immunoconjugate dose can also lie outside of these ranges, depending on the particular conjugate as well as the type and severity of the cancer being treated. Frequency of administration can range from a single dose to multiple doses per week, or more frequently. In some embodiments, the immunoconjugate is administered from about once per month to about five times per week. In some embodiments, the immunoconjugate is administered once per week.

Some embodiments of the invention provide an immunconjugate of the invention or a composition of the invention for use in treating cancer, wherein the cancer is a head and neck cancer. Head and neck cancer (as well as head and neck squamous cell carcinoma) refers to a variety of cancers characterized by squamous cell carcinomas of the oral cavity, pharynx and larynx, salivary glands, paranasal sinuses, and nasal cavity, as well as the lymph nodes of the upper part of the neck. Head and neck cancers account for approximately 3 to 5 percent of all cancers in the United States. These cancers are more common in men and in people over age 50. Tobacco (including smokeless tobacco) and alcohol use are the most important risk factors for head and neck cancers, particularly those of the oral cavity, oropharynx, hypopharynx and larynx. Eighty-five percent of head and neck cancers are linked to tobacco use. In the uses of the invention, the immunoconjugates can be used to target a number of malignant cells. For example, the immunoconjugates can be used to target squamous epithelial cells of the lip, oral cavity, pharynx, larynx, nasal cavity, or paranasal sinuses. The immunoconjugates can be used to target mucoepidermoid carcinoma cells, adenoid cystic carcinoma cells, adenocarcinoma cells, small-cell undifferentiated cancer cells, esthesioneuroblastoma cells, Hodgkin lymphoma cells, and Non-Hodgkin lymphoma cells. In some embodiments, uses for treating head and neck cancer include administering an immunoconjugate containing an antibody that is capable of binding EGFR (*e.g.,* cetuximab, panitumumab, matuzumab, and zalutumumab), PD-1 (*e.g.,* pembrolizumab), and/or MUC1.

Some embodiments of the invention provide an immunconjugate of the invention or a composition of the invention for use in treating cancer, wherein the cancer is breast cancer. Breast cancer can originate from different areas in the breast, and a number of different types of breast cancer have been characterized. For example, the immunoconjugates of the invention can be used for treating ductal carcinoma *in situ*; invasive ductal carcinoma (*e.g.,* tubular carcinoma; medullary carcinoma; mucinous carcinoma; papillary carcinoma; or cribriform carcinoma of the breast); lobular carcinoma *in situ*; invasive lobular carcinoma; inflammatory breast cancer; and other forms of breast cancer. In some embodiments, uses for treating breast cancer include administering an immunoconjugate containing an antibody that is capable of binding HER2 (*e.g.,* trastuzumab, margetuximab), glycoprotein NMB (*e.g.,* glembatumumab), and/or MUC1.

In some embodiments, the cancer is susceptible to an anti-inflammatory response induced by TLR7 and/or

TLR8.

Aspects, including embodiments, of the present subject matter described herein may be beneficial alone or in combination, with one or more other aspects or embodiments.

## EXAMPLES

The following examples further illustrate the invention but, of course, should not be construed as in any way limiting its scope.

### Example 1: Synthesis of Compound 2

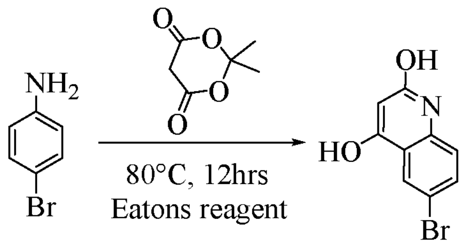


1 2

A mixture of 2,2-dimethyl-1,3-dioxane-4,6-dione (41.89 g, 290.66 mmol, 1 *eq)* and 4-bromoaniline (50 g, 290.66 mmol, 1 *eq*) was stirred (neat) at 80 °C for 12 hrs. Afterward, the small remaining amount of acetone was removed by vacuum. Eaton's reagent (415.15 g, 1.74 mol, 273.12 mL, 6 *eq)* was added to the mixture at 80 °C for 12 hrs. Water (1000 mL) was added to this mixture while stirring vigorously. The precipitate was filtered, washed with $H_2O$, and air dried to provide a solid. The solid was recrystallized from ethanol to afford 6-bromoquinoline-2,4-diol (26 g, 108.31 mmol, 37.26% yield) as off-white solid. $^1H$ NMR (dimethyl sulfoxide (DMSO)-$d_6$, 400 MHz) δ 11.53 (s, 1H), 11.33 (s, 1H), 7.85 (d, *J*= 2.4 Hz, 1H), 7.75 (dd, *J*= 8.0 Hz, 4.0 Hz, 1H), 7.18-7.24 (m, 1H), 5.75 (s, 1H).

### Example 2: Synthesis of Compound 3

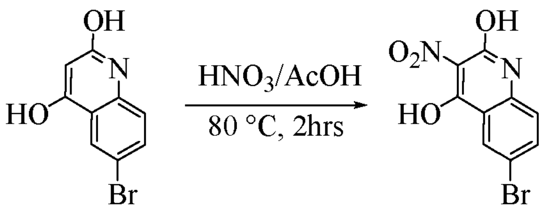


2 3

To a solution of nitric acid $HNO_3$ (13.65 g, 216.62 mmol, 9.75 mL, 2 *eq)* in AcOH (500 mL) was added 6-bromoquinoline-2,4-diol (26 g, 108.31 mmol, 1 *eq)* slowly at 15 °C. The mixture was stirred at 80 °C for 3 hours. The mixture was cooled and quenched by addition of water (1000 mL). The product was separated by filtration and washed by water (100 mL × 3), dried to give desired product. The crude product 6-bromo-3-nitro-quinoline-2,4-diol (30 g, 105.24 mmol, 97.17% yield) was obtained as a yellow solid and used into the next step without further purification. $^1H$ NMR (DMSO-$d_6$, 400 MHz) δ 11.92 (s, 1H), 8.13 (s, 1H), 7.76 (d, *J* = 8.4 Hz, 1H), 7.25 (d, *J* = 8.4 Hz, 1H).

### Example 3: Synthesis of Compound 4

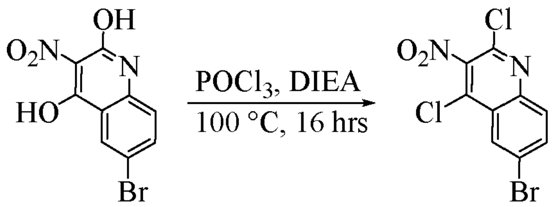


3 4

To a mixture of 6-bromo-3-nitro-quinoline-2,4-diol (30 g, 105.24 mmol, 1 *eq)* in $POCl_3$ (484.12 g, 3.16 mol, 293.41 mL, 30 *eq)* was added N,N-diisopropylethylamine (40.81 g, 315.73 mmol, 55.00 mL, 3 *eq)* slowly at 15°C. The mixture was stirred at 100 °C for 16 hrs. The mixture was concentrated in vacuum. The residue was poured into ice water (2000 mL), filtered and washed with $H_2O$ (500 mL × 3), and dried to provide 6-bromo-2,4-dichloro-3-nitro-quinoline (30 g, 93.18 mmol, 88.54% yield) as a yellow solid. $^1H$ NMR (DMSO-$d_6$, 400 MHz) δ 8.48 (d, *J* = 2.0 Hz, 1H), 8.25 (dd, *J* = 8.8, 2.0 Hz, 1H), 8.10 (d, *J* = 8.8 Hz, 1H).

Example 4: Synthesis of Compound 5

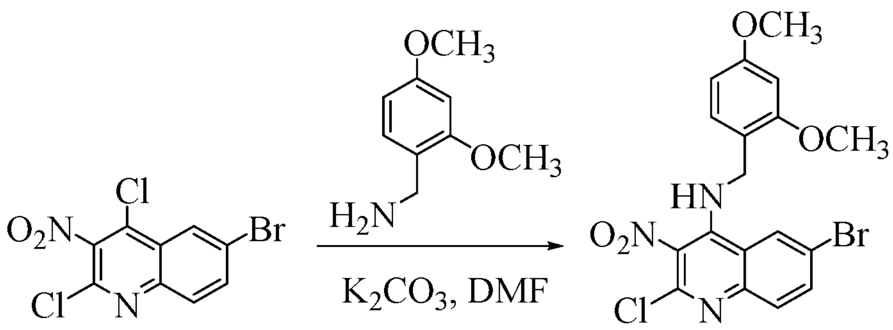


4 5

To a solution of 6-bromo-2,4-dichloro-3-nitroquinoline (5.6 g, 17.4 mmol, 1 eq.) and solid $K_2CO_3$ (3.6 g, 26 mmol, 1.5 eq.) in DMF (100 mL) at room temperature was added neat 2,4-dimethoxybenzylamine (3.5 g, 20.1 mmol, 1.2 eq.). The mixture was stirred for 15 minutes, water (300 mL) was added and the mixture was stirred for 5 additional minutes. The resultant solid was filtered and then dissolved in ethyl acetate (100 mL). The solution was washed with water (100 mL), brine (100 mL), separated, dried ($Na_2SO_4$), then filtered and concentrated in vacuo. The brown solid was triturated with 1:1 hexanes/diethyl ether (150 mL) and filtered to obtain 6-bromo-2-chloro-4-(2,4-dimethoxybenzyl)amino-3-nitroquinoline (6.9 g, 15.3 mmol, 88%) as a yellow solid. The compound was used without further purification.

Example 5: Synthesis of Compound 6

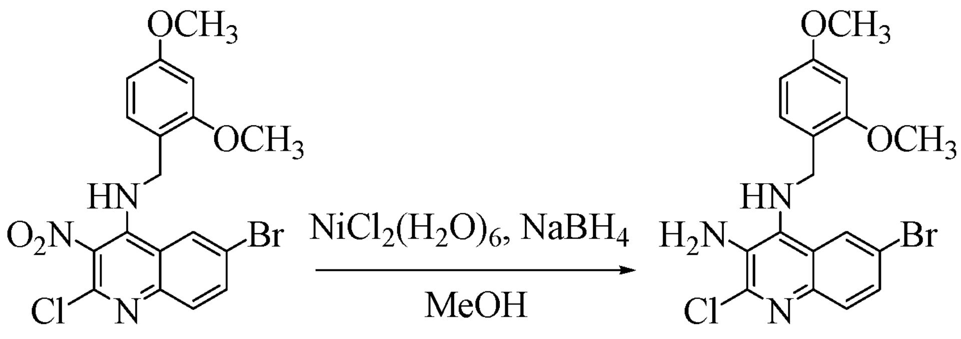


5 6

To 6-bromo-2-chloro-4-(2,4-dimethoxybenzyl)amino-3-nitroquinoline (6.9 g, 15.3 mmol, 88%) in methanol (200 mL) at 0 °C was added $NiCl_2 \cdot 6H_2O$ (0.36 g, 1.5 mmol, 0.1 eq). Sodium borohydride (pellets, 1.42 g, 38 mmol, 2.5 eq.) was added and reaction was stirred for 1 h at 0 °C then warmed to room temperature and allowed to stir for another 15 minutes. Glacial acetic acid (5 mL) was added until a pH of ~5 was obtained. The solvent was evaporated in vacuo and the crude solid was redissolved in ethyl acetate (150 mL) then filtered through a bed of diatomaceous earth to remove a black insoluble material. The ethyl acetate was removed in vacuo. The dark brown solid was triturated with ether (75 mL) then filtered to obtain 3-amino-6-bromo-2-chloro-4-(2,4-dimethoxybenzyl)aminoquinoline (5.81 g, 13.7 mmol, 90%) as a tan solid. The compound was used without further purification.

Example 6: Synthesis of Compound 7

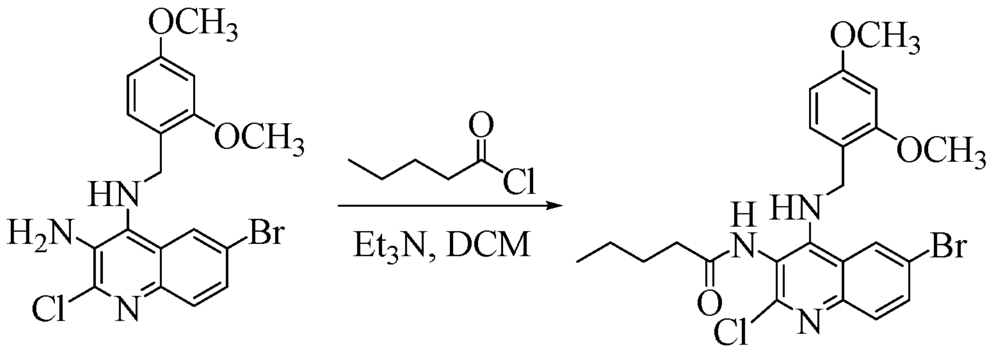


6 7

To a solution of 3-amino-6-bromo-2-chloro-4-(2,4-dimethoxybenzyl)aminoquinoline (5.75 g, 13.6 mmol, 1 eq.) in dichloromethane (100 mL) containing triethylamine (2.1 g, 2.8 mL, 20 mmol, 1.5 eq.) stirring at room temperature was added neat valeroyl chloride (2.0 mL, 2.0 g, 16 mmol, 1.2 eq). The mixture was washed with water (150 mL), brine (150 mL), separated, dried ($Na_2SO_4$), filtered, and concentrated. The solid was triturated with ether, filtered and dried under vacuum. N-(6-bromo-2-chloro-4-((2,4-dimethoxybenzyl)amino)quinolin-3-yl)pentanamide was obtained as a brown solid (5.8 g, 11.4 mmol, 84%). The compound was used without further purification.

Example 7: Synthesis of Compound 8

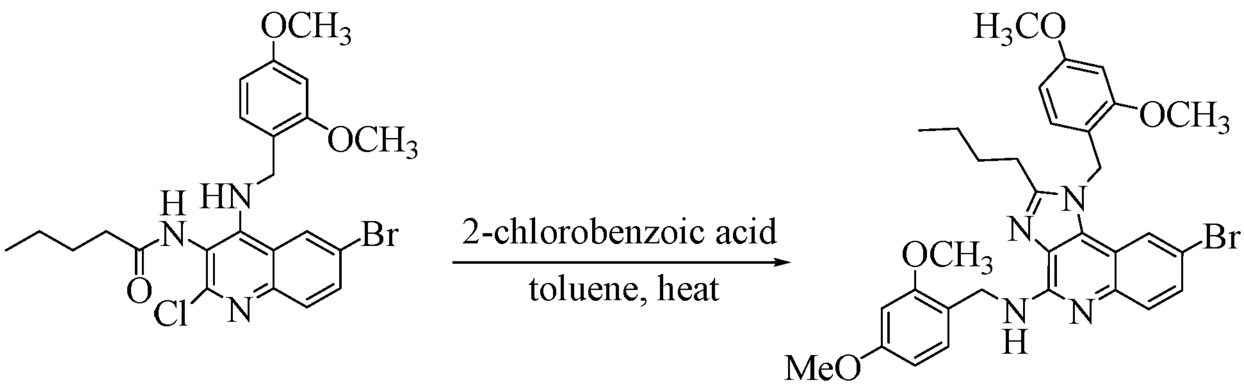


7 8

In a 100 mL beaker a mixture of N-(6-bromo-2-chloro-4-((2,4-dimethoxybenzyl)amino)quinolin-3-yl)pentanamide (5.8 g, 11.4 mmol, 1 eq.) and 2-chlorobenzoic (0.90 g, 5.7 mmol. 0.5 eq.) was boiled in 50 mL toluene for 2 hours. Toluene was added to 50 mL each time the volume reached 25 mL. 2,4-dimethoxybenzylamine (9.5 g, 57 mmol, 5 eq.) was added and the reaction was maintained at 120 °C for 2 hours. The reaction was cooled to room temperature and water (80 mL) then acetic acid (3.5 mL) was added. The supernatant was decanted and the crude product was washed with water (80

mL). The wet solid was triturated with methanol (100 mL) to provide 8-bromo-2-butyl-N,1-bis(2,4-dimethoxybenzyl)-1H-imidazo[4,5-c]quinolin-4-amine (4.80 g, 7.7 mmol, 68%) as an off-white solid. The compound was used without further purification.

Example 8: Synthesis of Compound 9

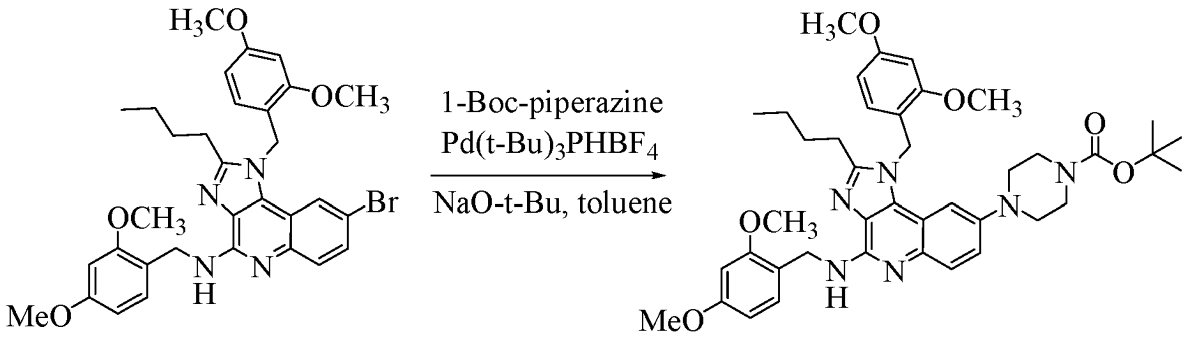


8 9

A mixture of 8-bromo-2-butyl-N,1-bis(2,4-dimethoxybenzyl)-1H-imidazo[4,5-c]quinolin-4-amine (0.31 g, 0.5 mmol, 1 eq.) and tert-butyl piperazine-1-carboxylate (0.19 g, 1 mmol, 2 eq.) were combined in toluene (2 mL) then degassed with argon. $Pd_2dba_3$ (45 mg, 0.05 mmol, 0.1 eq.), tri-tert-butylphosphine tetrafluoroborate (29 mg, 0.10 mmol, 0.2 eq) and sodium tert-butoxide (144 mg, 1.5 mmol, 3 eq) were added. The mixture was heated in a capped vial at 110 °C for 30 minutes. The mixture was cooled then partitioned between ethyl acetate (50 mL) and water (50 mL). The organic layer was washed with brine (50 mL), dried with sodium sulfate, filtered, and concentrated in vacuo. The crude product was purified on silica gel (20 g) eluted with 50% ethyl acetate/hexanes to yield tert-butyl 4-(2-butyl-1-(2,4-dimethoxybenzyl)-4-((2,4-dimethoxybenzyl)amino)-1H-imidazo[4,5-c]quinolin-8-yl)piperazine-1-carboxylate (0.28 g, 0.39 mmol, 78%) as an off-white solid. LC/MS [M+H] 725.40 (calculated); LC/MS [M+H] 725.67 (observed).

Example 9: Synthesis of Compound 10

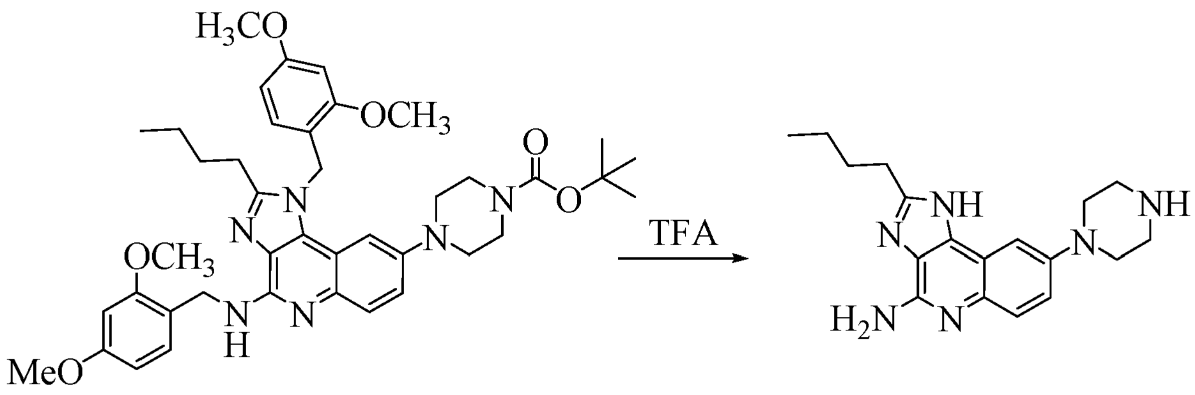


9 10

Tert-butyl 4-(2-butyl-1-(2,4-dimethoxybenzyl)-4-((2,4-dimethoxybenzyl)amino)-1H-imidazo[4,5-c]quinolin-8-yl) piperazine-1-carboxylate (0.28 g, 0.39 mmol, 1 eq.) was dissolved in TFA (3 mL) and heated to reflux for 5 min. The TFA was removed in vacuo and the crude product was dissolved in acetonitrile, filtered then concentrated to obtain the TFA salt of 2-butyl-8-(piperazin-1-yl)-1H-imidazo[4,5-c]quinolin-4-amine (0.16 g, 0.37 mmol, 95%) as an off-white solid. LC/MS [M+H] 325.21 (calculated); LC/MS [M+H] 325.51 (observed).

Example 10: Synthesis of Compound 11

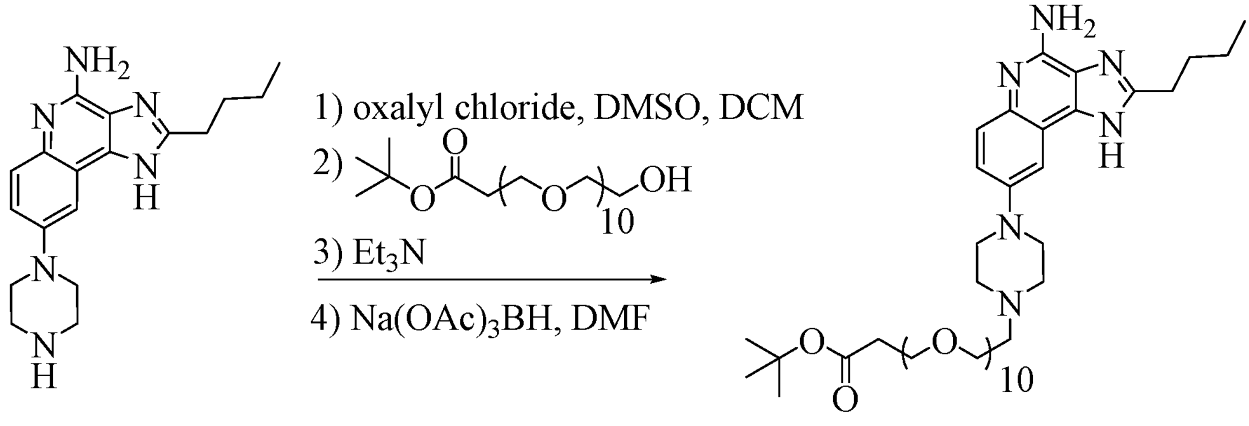


10 11

To a solution of oxalyl chloride (127 mg, 86 µL, 1 mmol, 2 eq) in DCM (1 mL) at 80 °C was added dropwise a solution of DMSO (156 mg, 142 µL, 2 mmol, 4 eq) in DCM (1 mL). The mixture was stirred for 15 min at 80 °C. To this mixture was added a solution of hydroxyl-$PEG_{10}$-t-butyl ester (602 mg, 0.5 mmol, 1 eq) in DCM (1 mL). After stirring for 15 min, $Et_3N$ (303 mg, 418 µL) was added and the mixture was stirred at 80 °C for 15 min then removed from the cold bath and allowed to warm to 20 °C over 30 min. To a suspension of the TFA salt of 2-butyl-8-(piperazin-1-yl)-1H-imidazo[4,5-c]quinolin-4-amine and sodium triacetoxyborohydride (212 mg, 1 mmol, 2 eq) in DMF (3 mL) was added the previous mixture slowly at 20 °C. The combined mixture was stirred at 20 °C for 45 min. Solvent was removed under reduced pressure and to the remaining was added 3 mL of 10% $Na_2CO_3$ and stirred vigorously for 15 min. Water (20 mL) was added and the crude product was extracted into DCM (25 mL). The organic layer was washed with brine, dried ($Na_2SO_4$), filtered and concentrated. The crude material was purified by flash chromatography using a gradient elution of 2-15% MeOH/DCM + 1% $Et_3N$ to yield *tert*-butyl 1-(4-(4-amino-2-butyl-1*H*-imidazo[4,5-c]quinolin-8-yl)piperazin-1-yl)-3,6,9,12,15,18,21,24,27,30-decaoxatritriacontan-33-oate in a 56% yield. LC/MS [M+H] 893.55 (calculated); LC/MS [M+H] 893.79 (observed).

Example 11: Synthesis of Compound 12

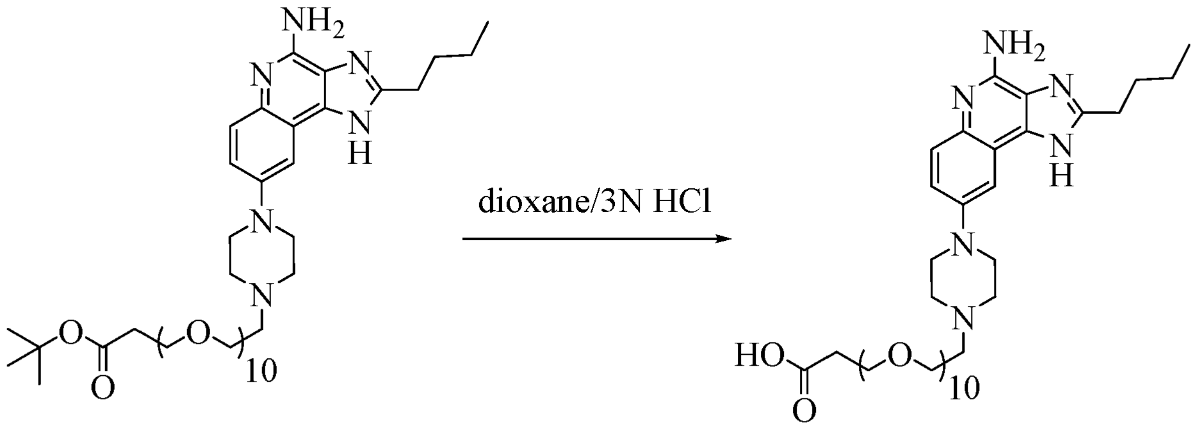


11 12

Tert-butyl 1-(4-(4-amino-2-butyl-1*H*-imidazo[4,5-c]quinolin-8-yl)piperazin-1-yl)-3,6,9,12,15,18,21,24,27,30-decaoxatritriacontan-33-oate was dissolved in a 1:1 mixture of dioxane and 3 N HCl (5 mL) then heated to 60 °C for 90 min. The solvent was removed and the residue was azeotroped four times with acetonitrile (5 mL). The resulting 1-(4-(4-amino-2-butyl-1*H*-imidazo[4,5-c]quinolin-8-yl)piperazin-1-yl)-3,6,9,12,15,18,21,24,27,30-decaoxatritriacontan-33-oic

acid HCL salt was used without further purification.

Example 12: Synthesis of Compound 13

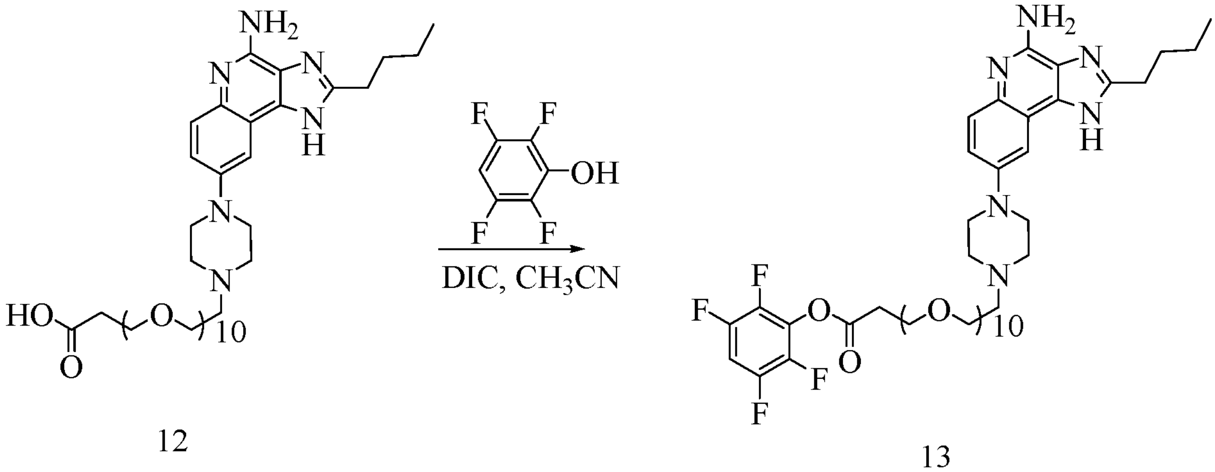


12

13

To 1-(4-(4-amino-2-butyl-1*H*-imidazo[4,5-c]quinolin-8-yl)piperazin-1-yl)-3,6,9,12,15,18,21,24,27,30-decaoxatritriacontan-33-oic acid HCL salt (0.13 mmol, 1 eq) was added a mixture of 2,3,5,6-tetrafluorophenol (66 mg, 0.4 mmol, 3 eq) and diisopropylcarbodiimide (51 mg, 62 μL, 0.4 mmol, 3 eq) dissolved in acetonitrile (3 mL) and the mixture was stirred at 20 °C for 16 h. The mixture was diluted with water (12 mL) and purified by reverse phase chromatography using a gradient eluent of 30-80% acetonitrile/water + 0.1% TFA over 10 min. The pooled fractions were concentrated under reduced pressure and the glassy film was azeotroped with acetonitrile four times (20 mL) to yield 2,3,5,6-tetrafluorophenyl 1-(4-(4-amino-2-butyl-1*H*-imidazo[4,5-c]quinolin-8-yl)piperazin-1-yl)-3,6,9,12,15,18,21,24,27,30-decaoxatritriacontan-33-oate in a 54% yield. LC/MS [M+H] 985.49 (calculated); LC/MS [M+H] 985.71 (observed).

Example 13: Synthesis of Compound 15

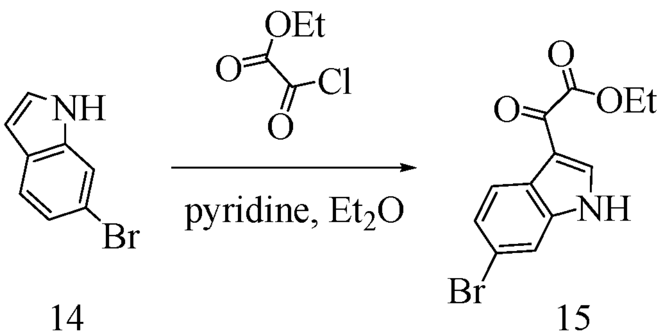


14

15

To a mixture of 6-bromo-1H-indole (5.00 g, 25.50 mmol, 1 eq) and pyridine (2.62 g, 33.16 mmol, 2.68 mL, 1.3 eq) in $Et_2O$ (80 mL) was added ethyl 2-chloro-2-oxo-acetate (4.18 g, 30.61 mmol, 3.43 mL, 1.2 eq) slowly at 0 °C under $N_2$. The mixture was stirred at 0 °C for 2 hours. A yellow solid precipitated. The mixture was filtered and the cake was washed by $H_2O$. The crude product was triturated with $H_2O$ at 20 °C for 20 min to provide ethyl 2-(6-bromo-1H-indol-3-yl)-2-oxo-acetate (5.4 g, 18.24 mmol, 71.50% yield) as a yellow solid. $^1H$ NMR (DMSO-$d_6$, 400 MHz) δ 12.46 (s, 1H), 8.46 (d, *J* = 3.6 Hz, 1H), 8.10 (d, *J* = 8.8 Hz, 1H), 7.75 (s, 1H), 7.43 (d, *J* = 8.8 Hz, 1H), 4.36 (q, *J* = 7.2 Hz, 2H), 1.33 (t, *J* = 7.2 Hz, 3H).

Example 14: Synthesis of Compound 16

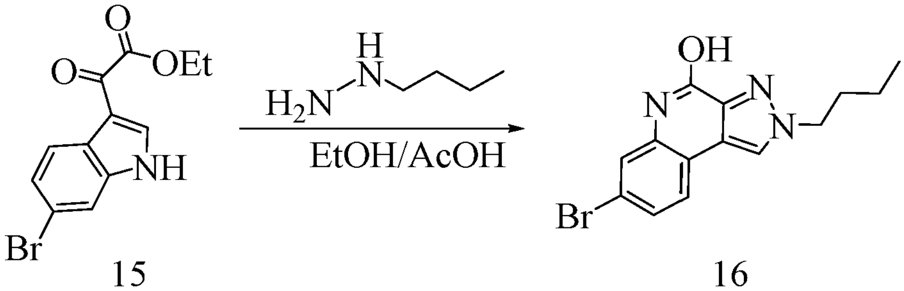


15 16

To a mixture of ethyl 2-(6-bromo-1H-indol-3-yl)-2-oxo-acetate (5.4 g, 18.24 mmol, 1 eq) and butylhydrazine (3.41 g, 27.35 mmol, 1.5 eq, HCl) in EtOH (60 mL) was added AcOH (10.95 g, 182.36 mmol, 10.43 mL, 10 eq) at 25 °C under $N_2$. The mixture was stirred at 90 °C for 16 hours. LCMS showed the reaction was completed. The mixture was concentrated in vacuum. The residue was purified by silica gel chromatography (column height: 250 mm, diameter: 100 mm, 100-200 mesh silica gel, petroleum ether/ethyl acetate = 5/1, 1/2) to provide 7-bromo-2-butyl-pyrazolo[3,4-c] quinolin-4-ol (3 g, 9.37 mmol, 51.38% yield) as a brown solid. $^1$H NMR ($CDCl_3$, 400 MHz) δ 11.40 (s, 1H), 8.72 (s, 1H), 7.81 (*d, J* = 8.4 Hz, 1H), 7.50 (s, 1H), 7.34 (dd, J=8.4, 2.0 Hz, 1H), 4.37 (t, *J*= 6.8 Hz, 2H), 1.91-1.84 (m, 2H), 1.32-1.25 (m, 2H), 0.91 (t, *J* = 7.2 Hz, 3H).

Example 15: Synthesis of Compound 17

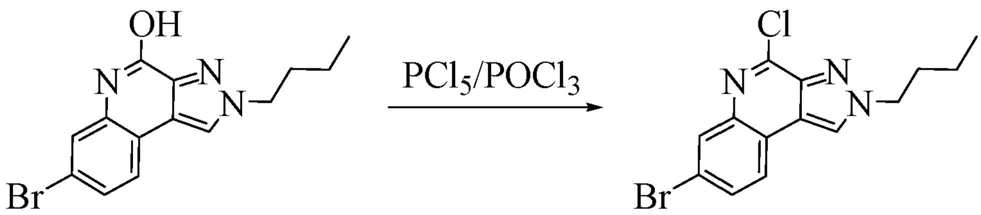


16 17

To a mixture of 7-bromo-2-butyl-pyrazolo[3,4-c]quinolin-4-ol (2.8 g, 8.74 mmol, 1 eq) in $POCl_3$ (13.41 g, 87.45 mmol, 8.13 mL, 10 eq) was added $PCl_5$ (910.52 mg, 4.37 mmol, 0.5 eq) in one portion at 25 °C. The mixture was stirred at 100 °C for 1 hour. LCMS showed the reaction was completed. The mixture was concentrated. The residue was poured into ice water (100 mL) and diluted with $CH_2Cl_2$ (30 mL) and washed with saturated $NaHCO_3$, dried over $Na_2SO_4$, filtered, and concentrated. The residue was purified by silica gel chromatography (column height: 250 mm, diameter: 100 mm, 100-200 mesh silica gel, petroleum ether/ethyl acetate = 10/1, 3/1) to provide 7-bromo-2-butyl-4-chloro-pyrazolo[3,4-c]quinoline (2.6 g, 7.68 mmol, 87.80% yield) as a yellow oil. $^1$H NMR (DMSO-*d*$_6$, 400 MHz) δ 8.30 (s, 1H), 8.22 (d, *J*= 2.0 Hz, 1H), 7.85 (d, *J*= 8.4 Hz, 1H), 7.68 (dd, J=8.4, 2.0 Hz, 1H), 4.53 (t, *J* = 7.2 Hz, 2H), 2.08-2.04 (m, 2H), 1.46-1.37 (m, 2H), 0.10 (t, *J*= 7.2 Hz, 3H).

Example 16: Synthesis of Compound 18

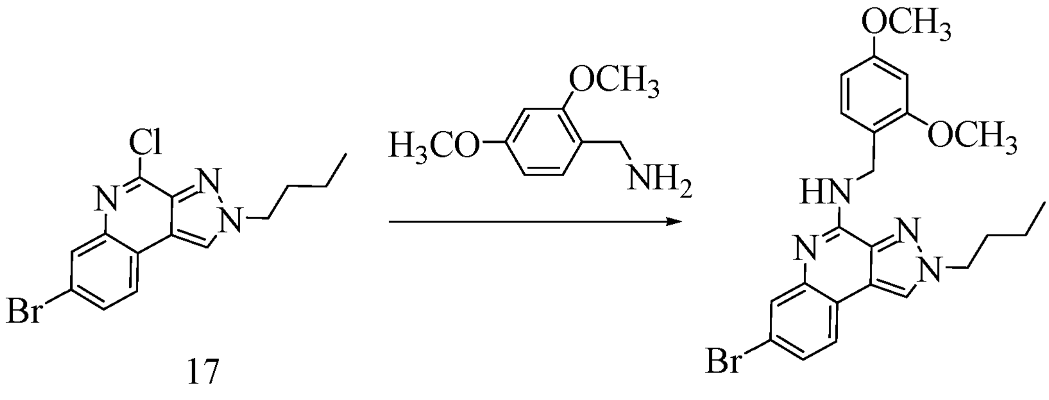

17

18

A mixture of 7-bromo-2-butyl-4-chloro-pyrazolo[3,4-c]quinoline (2.6 g, 7.68 mmol, 1 eq) and 2,4-dimethoxyphenyl)methanamine (6.42 g, 38.39 mmol, 5.78 mL, 5 eq) was stirred at 120 °C for 4 hours. LCMS showed the reaction was completed. The mixture was dissolved in EtOAc/$H_2O$ (10 mL/10 mL) and adjusted pH = 3 with aq. HCl (4 M). The aqueous phase was filtered to give 7-bromo-2-butyl-N-[(2,4-dimethoxyphenyl)methyl]pyrazolo[3,4-c]quinolin-4-amine (2.9 g, 6.18 mmol, 80.47% yield) as a yellow solid which was used into the next step without further purification. $^1H$ NMR ($CDCl_3$, 400 MHz) δ 9.03 (s, 1H), 8.34 (s, 1H), 8.04 (d, *J* = 8.4 Hz, 1H), 7.64 (s, 1H), 7.20 (d, *J* = 8.4 Hz, 1H), 6.61 (d, *J* = 2.4 Hz, 1H), 6.51 (d, *J* = 8.4 Hz, 1H), 4.89 (d, *J* = 4.2 Hz, 2H), 4.49 (t, *J* = 6.8 Hz, 2H), 3.75 (m, 6H), 1.96-1.89 (m, 2H), 1.35-1.27 (m, 2H), 0.91 (t, *J*= 7.2 Hz, 3H).

Example 17: Synthesis of Compound 19

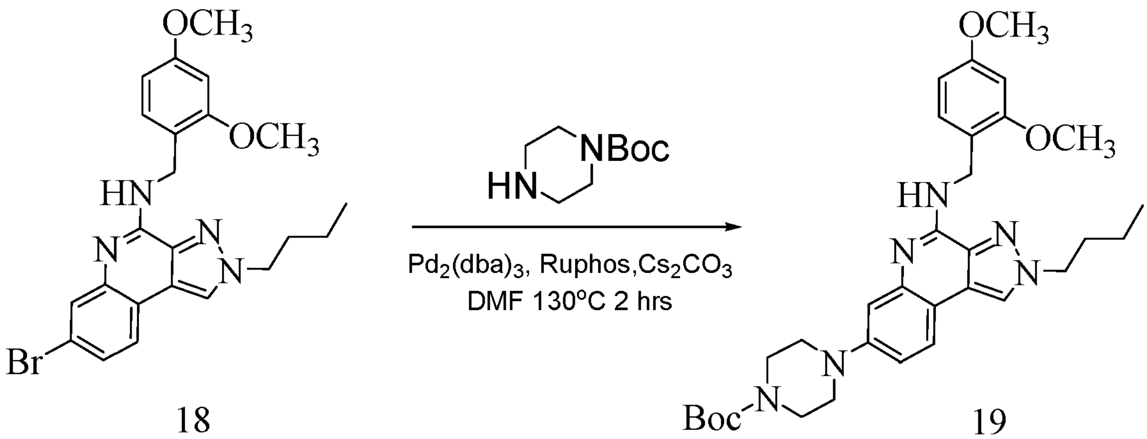


18

19

To a mixture of 7-bromo-2-butyl-N-[(2,4-dimethoxyphenyl)methyl]pyrazolo[3,4-c] quinolin-4-amine (0.45 g, 958.73 μmol, 1 eq) and tert-butyl piperazine-1-carboxylate (535.69 mg, 2.88 mmol, 3 eq) in DMF (10 mL) was added $Pd_2(dba)_3$ (43.90 mg, 47.94 μmol, 0.05 eq), $Cs_2CO_3$ (624.74 mg, 1.92 mmol, 2 eq) and RuPhos (44.74 mg, 95.87 μmol, 0.1 eq) in one portion at 25 °C under $N_2$. The mixture was stirred at 140 °C for 2 hours. LCMS showed the reaction was completed. The mixture was cooled to 25 °C and poured into ice water (30 mL) and stirred for 1 min. The aqueous phase was extracted with ethyl acetate (10 mL × 3). The combined organic phase was washed with brine (10 mL), dried with anhydrous $Na_2SO_4$, filtered, and concentrated in vacuum. The residue was purified by silica gel chromatography (petroleum ether/ethyl acetate = 10/1, 1/1) to provide tert-butyl 4-[2-butyl-4-[(2,4-dimethoxyphenyl) methylamino]pyrazolo[3,4-c]quinolin-7-yl]piperazine-1-carboxylate (0.45 g, 783.00 μmol, 81.67% yield) as a yellow oil. $^1H$ NMR ($CDCl_3$, 400 MHz) δ 7.95 (s, 1H), 7.67 (d, *J* = 8.8 Hz, 1H), 7.40 (d, J=8.0 Hz, 1H), 7.28 (d, *J* = 2.4 Hz, 1H), 6.91 (dd, *J* = 8.8, 2.4 Hz, 1H), 6.49 (d, *J* =2.4 Hz, 1H), 6.45 (dd, *J* = 8.4, 2.4 Hz, 1H), 5.98 (s, 1H), 4.87 (d, *J* = 4.4 Hz, 2H), 4.33 (t, *J* = 7.6 Hz, 2H), 3.86 (s, 3H), 3.80 (s, 3H), 3.64-3.61 (m, 4H), 3.26-3.23 (m, 4H), 1.99-1.92 (m, 2H), 1.51 (s, 9H), 1.40-1.34 (m, 2H), 0.96 (t, *J*= 7.2 Hz, 3H).

Example 18: Synthesis of Compound 20

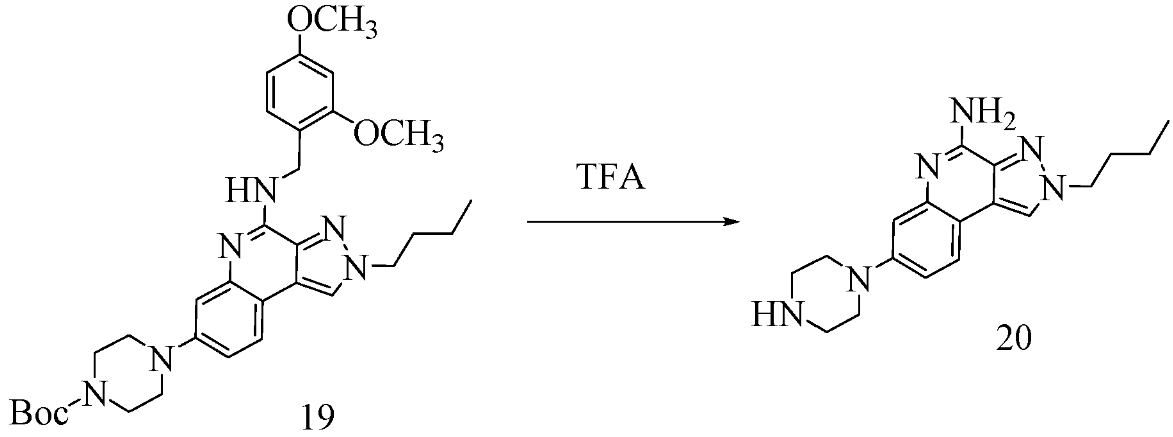


19

20

To a mixture of tert-butyl 4-[2-butyl-4-[(2,4-dimethoxyphenyl)methylamino] pyrazolo[3,4-c]quinolin-7-yl]piperazine-1-carboxylate (0.2 g, 348.00 μmol, 1 eq) in DCM (20 mL) was added TFA (1.98 g, 17.40 mmol, 1.29 mL, 50 eq) in one portion at 25 °C. The mixture was stirred at 50 °C for 36 hours. LCMS and HPLC showed the reaction was completed. The mixture was concentrated and purified by prep-HPLC (column: Nano-micro KROMASIL™ (Sigma-Aldrich) C18 100*30mm 5um; mobile phase: [water (0.1%TFA)-ACN]; B%: 20%-55%, 10min) to provide 2-butyl-7-piperazin-1-yl-pyrazolo[3,4-c]quinolin-4-amine (0.088 g, 200.71 μmol, 57.67% yield, TFA) as an off-white solid. $^1$H NMR (DMSO-*d*$_6$, 400 MHz) δ 9.01 (s, 2H), 8.88 (s, 1H), 7.96 (d, *J* = 8.8 Hz, 1H), 7.23 (dd, *J* = 8.8, 2.4 Hz, 1H), 7.11 (d, *J* = 2.4 Hz, 1H), 4.49 (t, *J* = 7.2 Hz, 2H), 3.45-3.44 (m, 4H), 3.35-3.29 (m, 4H), 1.98-1.90 (m, 2H), 1.36-1.27 (m, 2H), 0.93 (t, *J* = 7.2 Hz, 3H). LCMS (ESI): mass calcd. for $C_{18}H_{24}N_6$ 324.21, m/z found 325.3 $[M+H]^+$.

Example 19: Synthesis of Compound 21

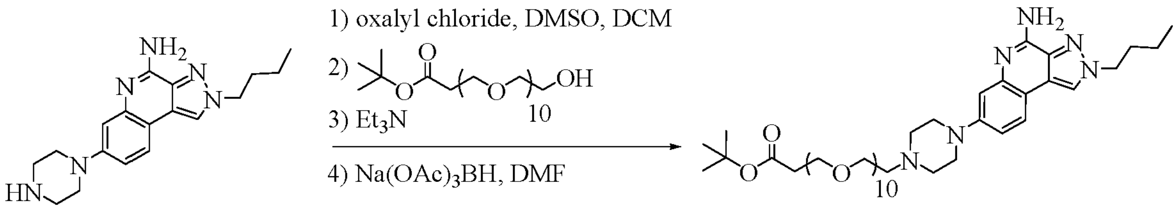


20

21

2-butyl-7-(piperazin-1-yl)-2*H*-pyrazolo[3,4-c]quinolin-4-amine was converted into tert-butyl 1-(4-(4-amino-2-butyl-2*H*-pyrazolo[3,4-c]quinolin-7-yl)piperazin-1-yl)-3,6,9,12,15,18,21,24,27,30-decaoxatritriacontan-33-oate in a 65% yield using the procedure described in Example 10. LC/MS [M+H] 893.56 (calculated); LC/MS [M+H] 893.82 (observed).

Example 20: Synthesis of Compound 22

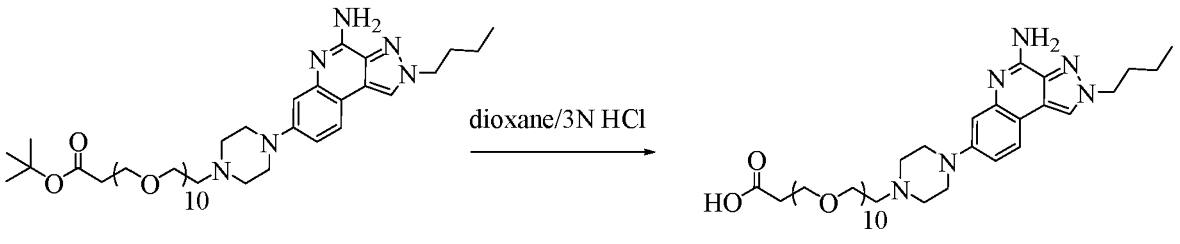


21 22

Tert-butyl 1-(4-(4-amino-2-butyl-2*H*-pyrazolo[3,4-c]quinolin-7-yl)piperazin-1-yl)-3,6,9,12,15,18,21,24,27,30-decaoxatritriacontan-33-oate was converted into 1-(4-(4-amino-2-butyl-2*H*-pyrazolo[3,4-*c*]quinolin-7-yl)piperazin-1-yl)-3,6,9,12,15,18,21,24,27,30-decaoxatritriacontan-33-oic acid in a 92% yield using the procedure described in Example 11. The compound was used without further purification.

Example 21: Synthesis of Compound 23

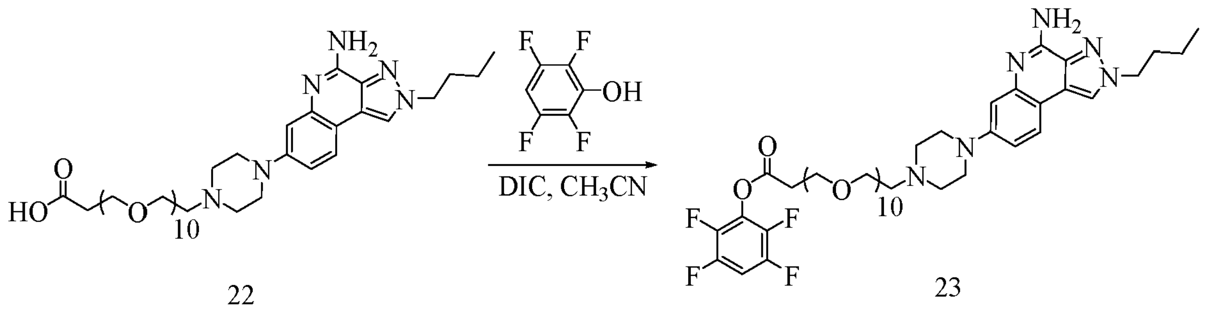


22 23

1-(4-(4-amino-2-butyl-2*H*-pyrazolo[3,4-c]quinolin-7-yl)piperazin-1-yl)-3,6,9,12,15,18,21,24,27,30-decaoxatritriacontan-33-oic acid was converted into 2,3,5,6-tetrafluorophenyl 1-(4-(4-amino-2-butyl-2*H*-pyrazolo[3,4-c]quinolin-7-yl)piperazin-1-yl)-3,6,9,12, 15, 18,21,24,27,30-decaoxatritriacontan-33-oate in a 46% yield using the procedure described in Example 12. LC/MS [M+H] 985.49 (calculated); LC/MS [M+H] 985.73 (observed).

Example 22: Synthesis of Compound 25

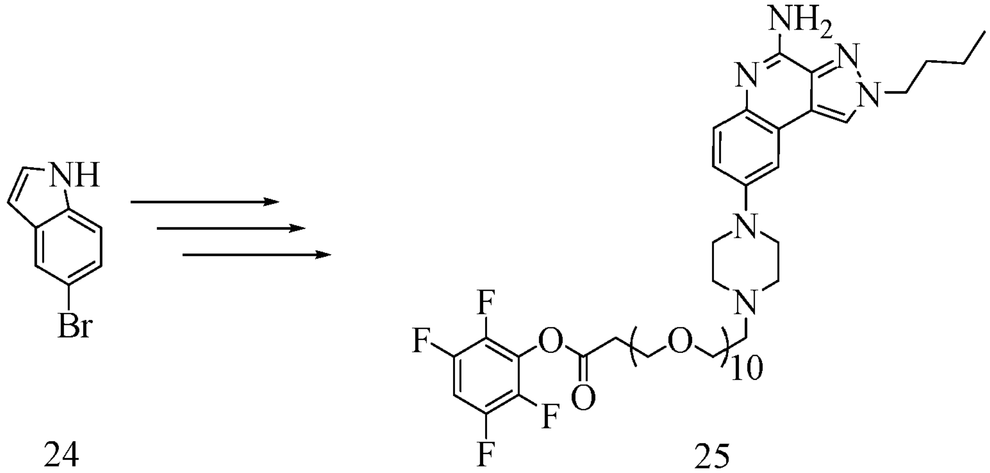

24 25

5-bromo-1*H*-indole was converted into 2,3,5,6-tetrafluorophenyl 1-(4-(4-amino-2-butyl-2*H*-pyrazolo[3,4-*c*]quinolin-8-yl)piperazin-1-yl)-3,6,9,12,15,18,21,24,27,30-decaoxatritriacontan-33-oate using the route described in Examples 13-21. LC/MS [M+H] 985.49 (calculated); LC/MS [M+H] 985.73 (observed).

Example 23: Synthesis of Compound 27

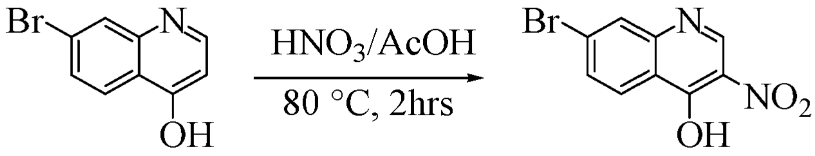


26 27

7-bromoquinolin-4-ol (9.66 g, 43.11 mmol, 1 equiv.) was converted into 7-bromo-3-nitroquinolin-4-ol (7.46 g, 27.7 mmol, 64%) according to the procedure described in Example 2. LC/MS [M+H] 268.96/270.95 (calculated); LC/MS [M+H] 268.99/271.02 (observed).

Example 24: Synthesis of Compound 28

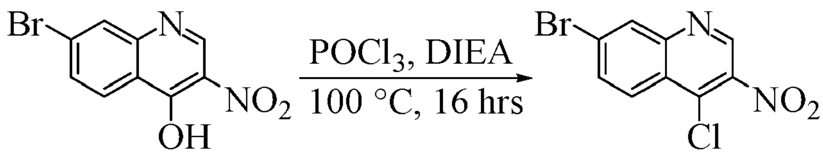


27 28

7-bromo-3-nitroquinolin-4-ol (7.46 g, 27.7 mmol, 1 equiv.) was converted into 7-bromo-4-chloro-3-nitroquinoline (6.88 g, 23.9 mmol, 86%) according to the procedure described in Example 3. LC/MS [M+H] 286.92/288.92 (calculated); LC/MS [M+H] 286.98/288.97 (observed).

Example 25: Synthesis of Compound 29

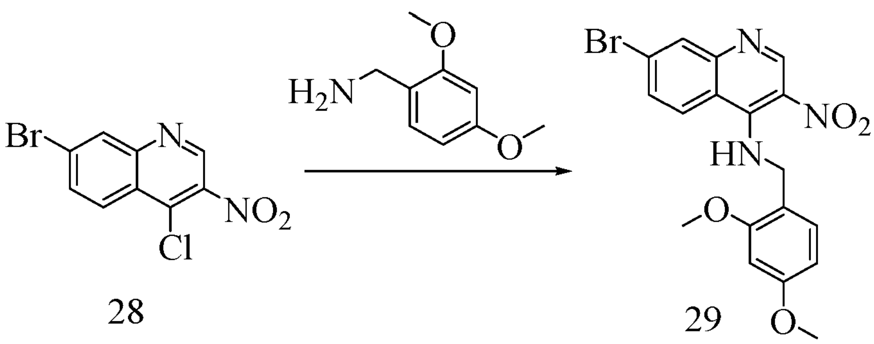

28 29

7-bromo-4-chloro-3-nitroquinoline (2.86 g, 10 mmol, 1 equiv.) was added to (2,4-dimethoxyphenyl) methanamine (100 mmol, 10 *eq)* at 20 °C. The mixture was stirred at 120 °C for 3 hrs. The mixture was diluted with water (200 mL) and extracted with EtOAc (100 ml x 3). The organic layer was washed with brine (100 mL), dried over $Na_2SO_4$, filtered and concentrated. The residue was purified by flash silica gel chromatography (Teledyne Isco, 10 g, SEPAFLASH™ silica flash column, eluent of 0 to about 50% ethyl acetate/petroleum ether gradient at 100 mL/min) to provide 7-bromo-N-(2,4-dimethoxybenzyl)-3-nitroquinolin-4-amine (4.2 g, 10.0 mmol, 100%). LC/MS [M+H] 418.04/420.04 (calculated); LC/MS [M+H] 418.19/420.16 (observed).

Example 26: Synthesis of Compound 30

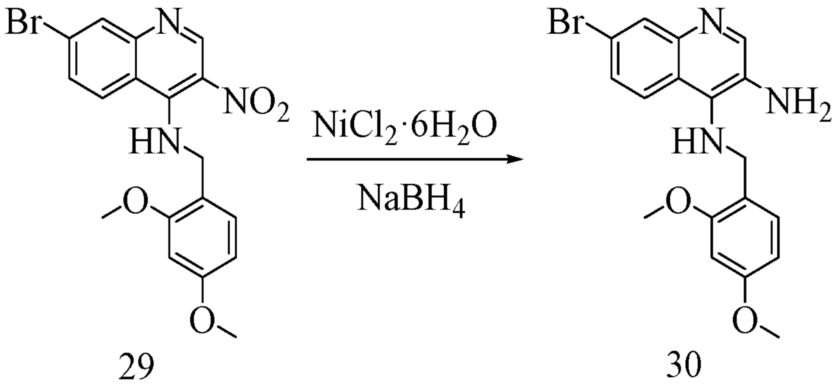


29 30

7-bromo-N-(2,4-dimethoxybenzyl)-3-nitroquinolin-4-amine (4.2 g, 10.0 mmol, 1 equiv.) was suspended in acetonitrile (24 ml). Water (4 ml) was added, followed by nickel(II) chloride hexahydrate (0.48 g, 2 mmol, 0.2 equiv.). Sodium borohydride (1.52 g, 40.2 mmol, 4 equiv.) was added to the green suspension and the exothermic reaction was stirred for 30 minutes. The reaction mixture was filtered, concentrated, and purified by flash chromatography to give 7-bromo-N4-(2,4-dimethoxybenzyl)quinoline-3,4-diamine (2.15 g, 5.5 mmol, 55%). LC/MS [M+H] 388.07/390.06 (calculated); LC/MS [M+H] 388.22/390.21 (observed).

Example 27: Synthesis of Compound 31

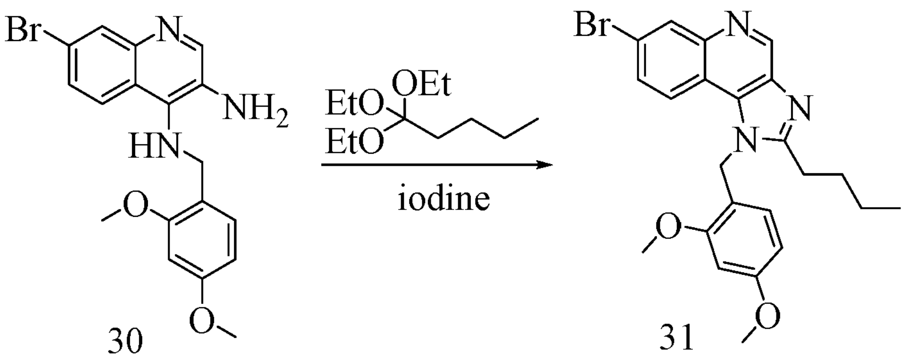


30 31

7-bromo-N-4-(2,4-dimethoxybenzyl)quinoline-3,4-diamine (2.15 g, 5.53 mmol, 1 equiv.) was dissolved in acetonitrile (25 ml). To the stirring solution was added triethyl orthovalerate (2.57 ml, 11.1 mmol, 2 equiv.) followed by iodine (0.140 g, 0.55 mmol, 0.1 equiv.). The reaction was stirred at room temperature until no starting material was observed by LCMS. The reaction mixture was concentrated, diluted in dichloromethane, and purified by flash chromatography to give 7-bromo-2-butyl-1-(2,4-dimethoxybenzyl)-1H-imidazo[4,5-c]quinoline (2.43 g, 5.3 mmol, 97%). LC/MS [M+H] 454.11/456.11 (calculated); LC/MS [M+H] 454.28/456.23 (observed).

Example 28: Synthesis of Compound 32

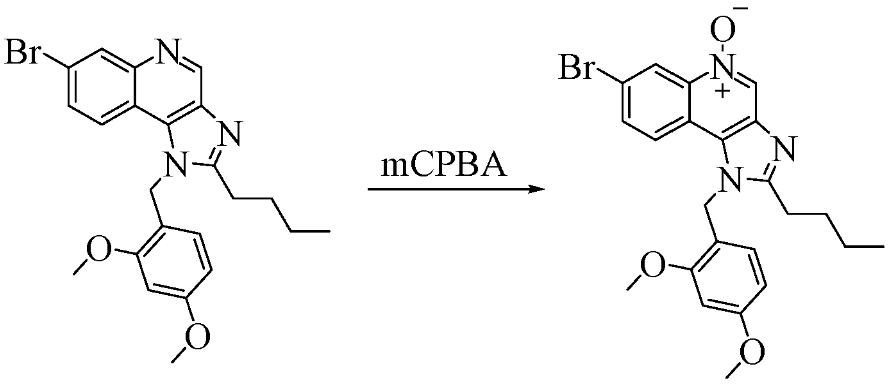


31 32

7-bromo-2-butyl-1-(2,4-dimethoxybenzyl)-1H-imidazo[4,5-c]quinoline (2.7 g, 5.94 mmol, 1 equiv.) was dissolved in 15 ml DCM. To the stirring reaction was added 4-chloroperoxybenzoic acid (4.39 g, 17.83 mmol, 3 equiv.). The reaction was stirred at room temperature and monitored by LCMS. Upon consumption of starting material, the reaction was quenched with 10% aqueous sodium carbonate, extracted with ethyl acetate, concentrated, and purified by flash chromatography to give 7-bromo-2-butyl-1-(2,4-dimethoxybenzyl)-1H-imidazo[4,5-c]quinoline 5-oxide (0.88 g, 1.87 mmol, 31%). LC/MS [M+H] 470.11/472.11 (calculated); LC/MS [M+H] 470.27/472.25 (observed).

Example 29: Synthesis of Compound 33

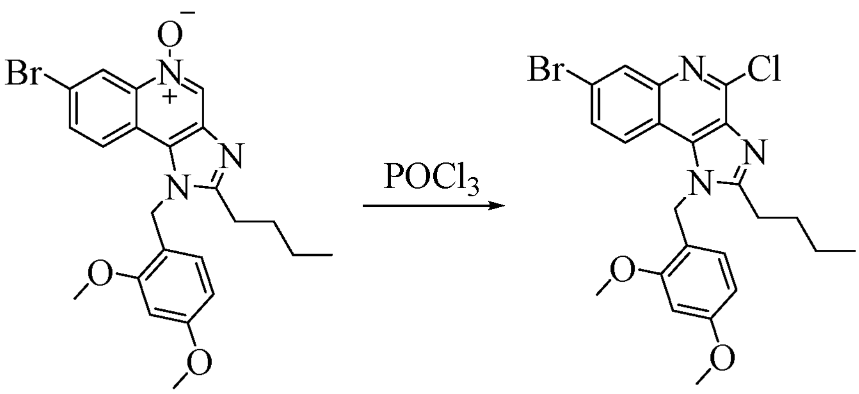


32 33

7-bromo-2-butyl-1-(2,4-dimethoxybenzyl)-1H-imidazo[4,5-c]quinoline 5-oxide (0.88 g, 1.87 mmol, 1 equiv.) was dissolved in dichloromethane (20 ml) and cooled on ice. Phosphoryl chloride (0.21 ml, 2.2 mmol, 1.2 equiv.) was added dropwise to the rapidly stirring solution, followed by N,N-dimethylformamide (0.072 ml, 0.94 mmol, 0.5 equiv.). After five minutes, the reaction was warmed to ambient temperature and monitored by LCMS. Upon consumption of starting material, the solution was washed with a mixture of ice and 10% aqueous sodium carbonate. The organic and aqueous layers were separated, and the aqueous layer extracted with dichloromethane (15 ml). The combined organic fractions were dried over sodium sulfate, filtered, and concentrated to provide 7-bromo-2-butyl-4-chloro-1-(2,4-dimethoxybenzyl)-1H-imidazo[4,5-c]quinoline as a brown foam (1.02 g, 2.09 mmol, 100%). LC/MS [M+H] 488.07/490.07 (calculated); LC/MS [M+H] 488.22/490.21 (observed).

Example 30: Synthesis of Compound 34

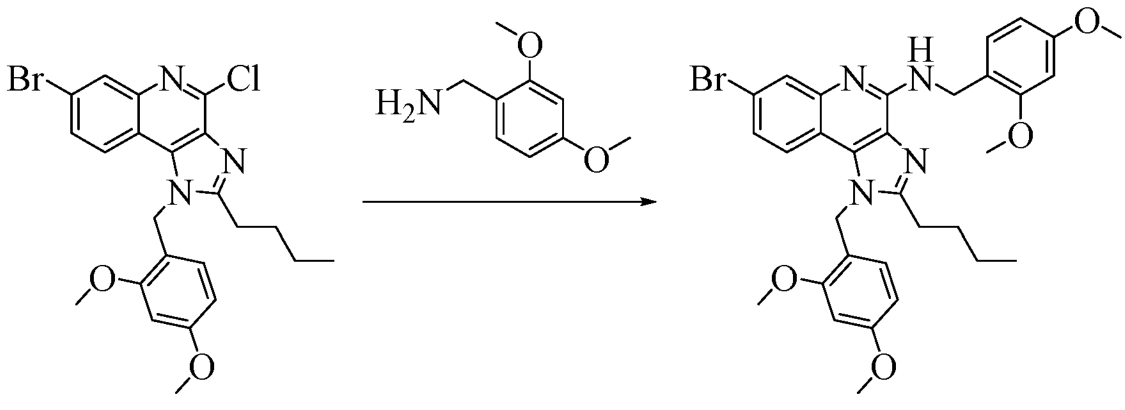

33 34

A mixture of 7-bromo-2-butyl-4-chloro-1-(2,4-dimethoxybenzyl)-1H-imidazo[4,5-c]quinoline (1 g, 2 mmol, 1 equiv.) and (2,4-dimethoxyphenyl) methanamine (20 mmol, 10 eq) was stirred at 120 °C for 2 hours. To the mixture was added 2M HCl to adjust to pH~4 and extracted with ethyl acetate (50 mL x 3). The combined organic phase was washed with brine (50 mL), dried with anhydrous $Na_2SO_4$, filtered and concentrated in vacuum. The residue was purified by column chromatography ($SiO_2$, petroleum ether/ethyl acetate = 20/1 to 0:1) to obtain 7-bromo-2-butyl-1-(2,4-dimethoxybenzyl)-N-(2,4-dimethoxyphenyl)-1H-imidazo[4,5-c]quinolin-4-amine (0.694 g, 1.12 mmol, 57%). LC/MS [M+H] 619.19/621.32 (calculated); LC/MS [M+H] 619.37/621.32 (observed).

Example 31: Synthesis of Compound 35

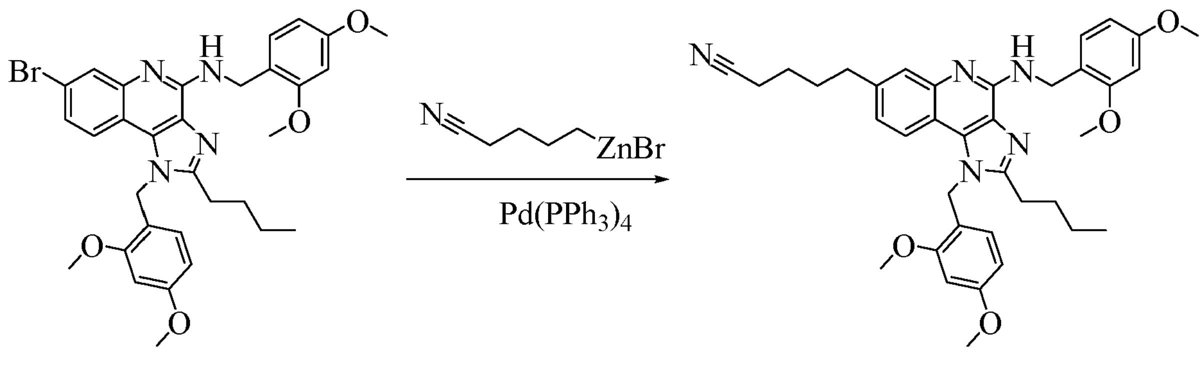


34 35

7-bromo-2-butyl-1-(2,4-dimethoxybenzyl)-N-(2,4-dimethoxyphenyl)-1H-imidazo[4,5-c]quinolin-4-amine (0.154 g, 0.25 mmol, 1 equiv.) and $Pd(PPH_3)_4$ (28.7 mg, 0.0025 mmol, 0.1 equiv.) were combined under dry dinitrogen. Cyanobutylzinc bromide (2.5 ml, 0.5 M in THF, 1.24 mmol, 5 equiv.) was added under dry dinitrogen and the reaction was heated to 75 °C. After 30 minutes, another portion of cyanobutylzinc bromide was added (2.5 ml, 0.5 M in THF, 1.24 mmol, 5 equiv.) and the reaction allowed to stir for an additional 90 minutes. The solution was concentrated to a syrup and the crude material purified by flash chromatography to provide a mixture of the desired 5-(2-butyl-1-(2,4-dimethoxybenzyl)-4-((2,4-dimethoxyphenyl)amino)-1H-imidazo[4,5-c]quinolin-7-yl)pentanenitrile along with 2-butyl-1-(2,4-dimethoxybenzyl)-N-(2,4-dimethoxyphenyl)-1H-imidazo[4,5-c]quinolin-4-amine and residual solvent that was carried on as a crude mixture (0.288 g). LC/MS [M+H] 622.34 (calculated); LC/MS [M+H] 622.96 (observed).

Example 32: Synthesis of Compound 36

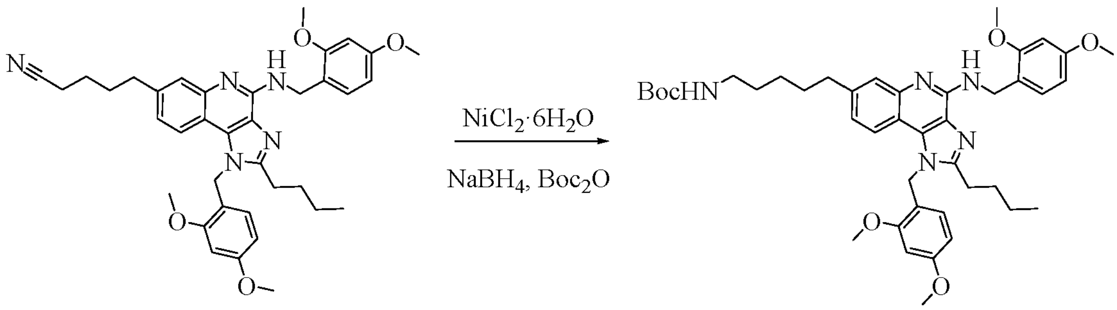


35 36

5-(2-butyl-1-(2,4-dimethoxybenzyl)-4-((2,4-dimethoxyphenyl)amino)-1H-imidazo[4,5-c]quinolin-7-yl)pentanenitrile (0.69 g, 1.1 mmol, 1 equiv.) was dissolved in methanol (20 ml) and cooled on ice. Nickel(II) chloride hexahydrate (0.053 g, 0.22 mmol, 0.2 equiv.) and Boc anhydride (0.51 ml, 2.22 mmol, 2 equiv.) were added to the stirring mixture. Sodium borohydride (1 g, 26.4 mmol, 23.8 equiv.) was added slowly in portions over 1 hour. The reaction was warmed and allowed to stand at ambient temperature for 1 hour, then concentrated. The crude material was taken up in ethyl acetate and washed with saturated sodium bicarbonate. The organic fraction was dried over sodium sulfate, filtered, concentrated, and then purified by flash chromatography to provide tert-butyl (5-(2-butyl-1-(2,4-dimethoxybenzyl)-4-((2,4-dimethoxyphenyl)amino)-1H-imidazo[4,5-c]quinolin-7-yl)pentyl)carbamate (0.265 g, 0.37 mmol, 33%). LC/MS [M+H] 726.42 (calculated); LC/MS [M+H] 726.64 (observed).

Example 33: Synthesis of Compound 37

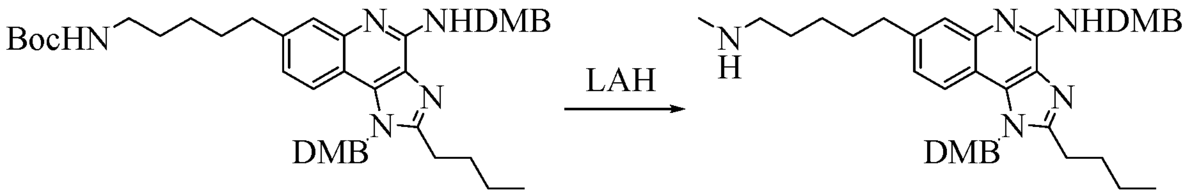


36 37

To a solution of tert-Butyl (5-(2-butyl-1-(2,4-dimethoxybenzyl)-4-((2,4-dimethoxyphenyl)amino)-1H-imidazo[4,5-c]quinolin-7-yl)pentyl)carbamate (94.3 mg, 0.13 mmol, 1 equiv.) in THF (20 mL) was added $LiAlH_4$ (0.65 mmol, 5 eq) in portions at 25 °C under $N_2$. The mixture was stirred at 60 °C for 3 hours. The mixture was added saturated aqueous $Na_2SO_4$ (2 mL) at 0 °C and dried with anhydrous $Na_2SO_4$, filtered and concentrated in vacuum to afford 2-butyl-N,1-bis(3,4-dimethylbenzyl)-7-(5-(methylamino)pentyl)-1H-imidazo[4,5-c]quinolin-4-amine. LC/MS [M+H] 640.39 (calculated); LC/MS [M+H] 640.55 (observed).

Example 34: Synthesis of Compound 38

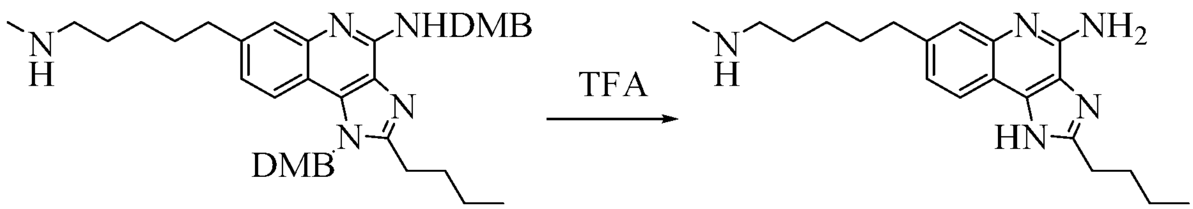


37 38

To a solution of 2-butyl-N,1-bis(3,4-dimethylbenzyl)-7-(5-(methylamino)pentyl)-1H-imidazo[4,5-c]quinolin-4-amine (348.57 µmol, 1 eq) in DCM (20 mL) was added TFA (24.56 mmol, 70.45 eq) in one portion at 25 °C. The mixture

was stirred at 40 °C for 12 hours. The mixture was concentrated in reduced pressure at 45 °C. The residue was purified by prep-HPLC (column: LUNA™ C18 100 × 30 5u (Phenomenex, Inc.); mobile phase: [water (0.1%TFA)-ACN]; B%: 5%-25%, 10 min) to afford 2-butyl-7-(5-(methylamino)pentyl)-1H-imidazo[4,5-c]quinolin-4-amine. LC/MS [M+H] 340.25 (calculated); LC/MS [M+H] 340.36 (observed).

Example 35: Synthesis of Compound 39

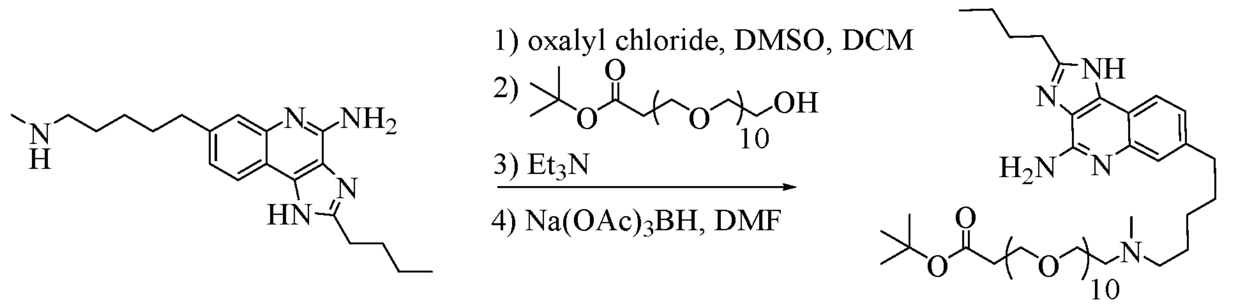


38 39

2-butyl-7-(5-(methylamino)pentyl)-1H-imidazo[4,5-c]quinolin-4-amine (50 mg, 0.15 mmol, 1 equiv.) was converted into tert-butyl 39-(4-amino-2-butyl-1H-imidazo[4,5-c]quinolin-7-yl)-34-methyl-4,7,10,13,16,19,22,25,28,31-decaoxa-34-azanonatriacontanoate using the procedure described in Example 10. LC/MS [M+H] 908.60 (calculated); LC/MS [M+H] 908.75 (observed).

Example 36: Synthesis of Compound 40

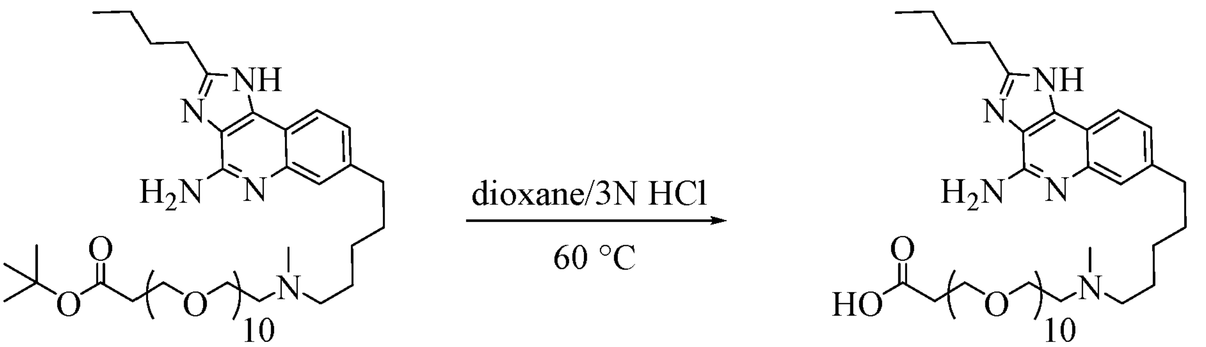


39 40

*Tert*-butyl 39-(4-amino-2-butyl-1H-imidazo[4,5-c]quinolin-7-yl)-34-methyl-4,7,10,13,16,19,22,25,28,31-decaoxa-34-azanonatriacontanoate was converted into 39-(4-amino-2-butyl-1H-imidazo[4,5-c]quinolin-7-yl)-34-methyl-4,7,10,13,16,19,22,25,28,31-decaoxa-34-azanonatriacontanoic acid (45 mg, 0.15 mmol, 33% from compound 38) using the procedure described in Example 11. LC/MS [M+H] 852.53 (calculated); LC/MS [M+H] 852.75 (observed).

Example 37: Synthesis of Compound 41

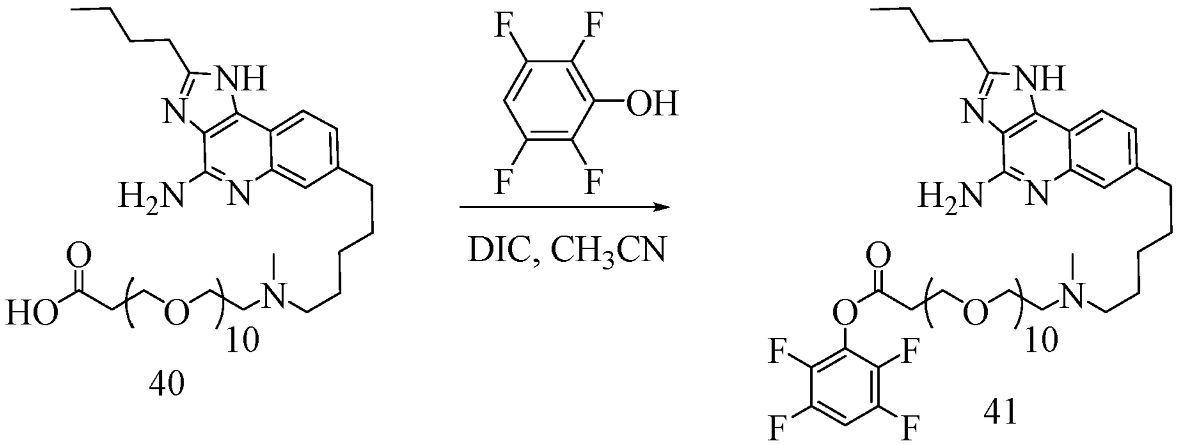


40 41

39-(4-amino-2-butyl-1H-imidazo[4,5-c]quinolin-7-yl)-34-methyl-4,7,10,13,16,19,22,25,28,31-decaoxa-34-azanonatriacontanoic acid (45 mg, 0.053 mmol, 1 equiv.) was converted into 2,3,5,6-tetrafluorophenyl 39-(4-amino-2-butyl-1H-imidazo[4,5-c]quinolin-7-yl)-34-methyl-4,7,10,13,16,19,22,25,28,31-decaoxa-34-azanonatriacontanoate (28.5 mg, 0.053 mmol, 54%) according to the procedure described in Example 12. LC/MS [M+H] 1000.53 (calculated); LC/MS [M+H] 1000.72 (observed).

Example 38: Synthesis of Compound 43

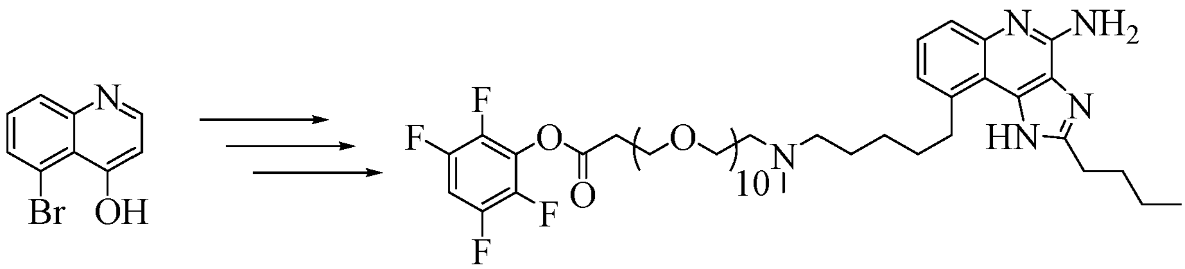

42 43

5-bromoquinolin-4-ol was converted into 2,3,5,6-tetrafluorophenyl 39-(4-amino-2-butyl-1H-imidazo[4,5-c]quinolin-9-yl)-34-methyl-4,7,10,13,16,19,22,25,28,31-decaoxa-34-azanonatriacontanoate using the route described in Examples 23-37. LC/MS [M+H] 1000.53 (calculated); LC/MS [M+H] 1000.94 (observed).

Example 39: Synthesis of Compound 44

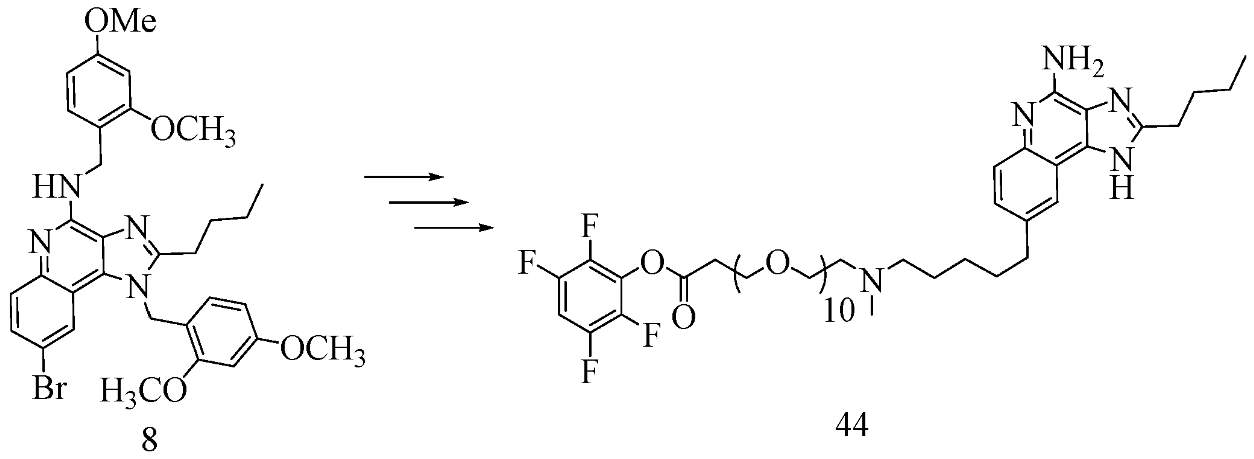

8

44

Compound 8 was converted into 2,3,5,6-tetrafluorophenyl 39-(4-amino-2-butyl-1H-imidazo[4,5-c]quinolin-8-yl)-34-methyl-4,7,10,13,16,19,22,25,28,31-decaoxa-34-azanonatriacontanoate using the route described in Examples 31-37. LC/MS [M+H] 1000.53 (calculated); LC/MS [M+H] 1000.92 (observed).

Example 40: Synthesis of Compound 45

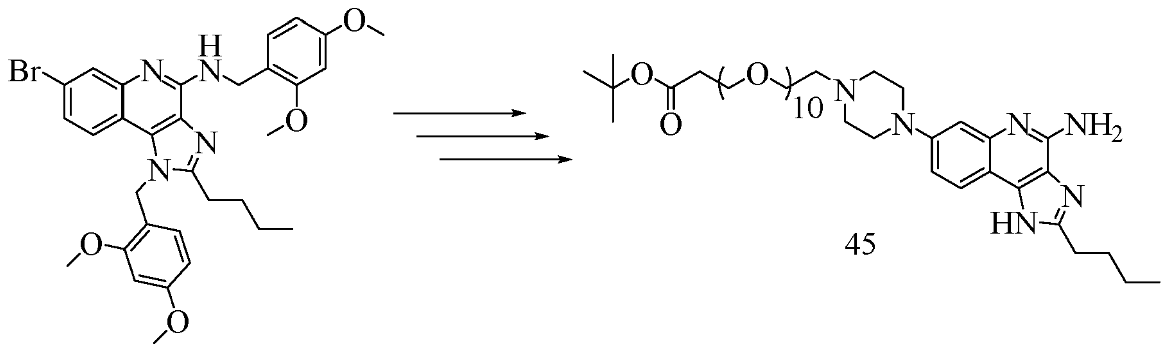

45

34

7-bromo-2-butyl-N,1-bis(2,4-dimethoxybenzyl)-1H-imidazo[4,5-c]quinolin-4-amine was converted into tert-butyl 1-(4-(4-amino-2-butyl-1H-imidazo[4,5-c]quinolin-7-yl)piperazin-1-yl)-3,6,9,12,15,18,21,24,27,30-decaoxatritriacontan-33-oate according to the procedures described in Examples 8-10. LC/MS [M+H] 893.56 (calculated); LC/MS [M+H] 893.79.

Example 41: Synthesis of Compound 46

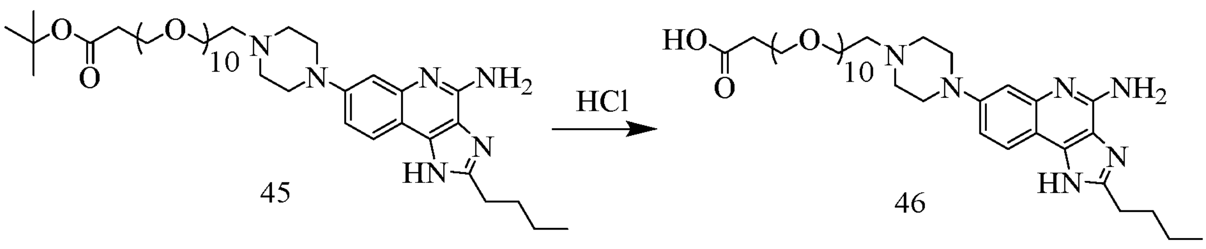


45

46

tert-butyl 1-(4-(4-amino-2-butyl-1H-imidazo[4,5-c]quinolin-7-yl)piperazin-1-yl)-3,6,9,12,15,18,21,24,27,30-decaoxatritriacontan-33-oate was converted to 1-(4-(4-amino-2-butyl-1H-imidazo[4,5-c]quinolin-7-yl)piperazin-1-yl)-3,6,9,12,15,18,21,24,27,30-decaoxatritriacontan-33-oic acid according to the procedure set forth in Example 11.

LC/MS [M+H] 837.49 (calculated); LC/MS [M+H] 837.84 (observed).

Example 42: Synthesis of Compound 47

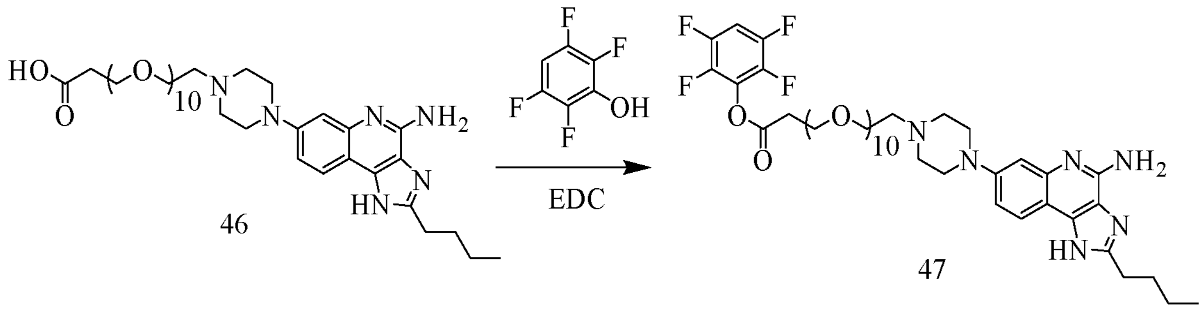


46

47

1-(4-(4-amino-2-butyl-1H-imidazo[4,5-c]quinolin-7-yl)piperazin-1-yl)-3,6,9,12,15,18,21,24,27,30-decaoxatritriacontan-33-oic acid was converted to 2,3,5,6-tetrafluorophenyl 1-(4-(4-amino-2-butyl-1H-imidazo[4,5-c]quinolin-7-yl)piperazin-1-yl)-3,6,9,12,15,18,21,24,27,30-decaoxatritriacontan-33-oate according to the procedure set forth in Example 12. LC/MS [M+H] 985.49 (calculated); [M+H] 985.71 (observed).

Example 43: Synthesis of Compound 48

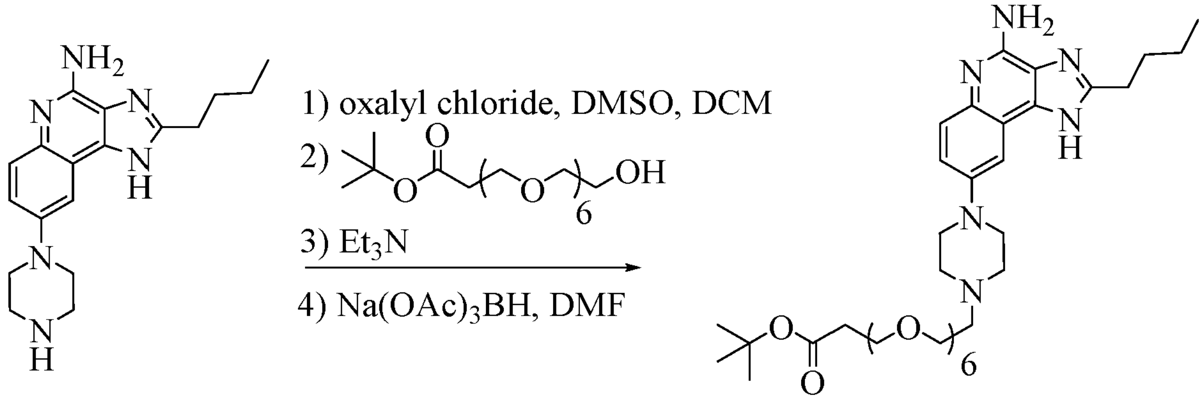


10

48

2-butyl-8-(piperazin-1-yl)-1*H*-imidazo[4,5-c]quinolin-4-amine was converted into *tert*-butyl 1-(4-(4-amino-2-butyl-1H-imidazo[4,5-c]quinolin-8-yl)piperazin-1-yl)-3,6,9,12,15,18-hexaoxahenicosan-21-oate according to the procedure described in Example 10. LC/MS [M+H] 717.45 (calculated); LC/MS [M+H] 717.75 (observed).

Example 44: Synthesis of Compound 49

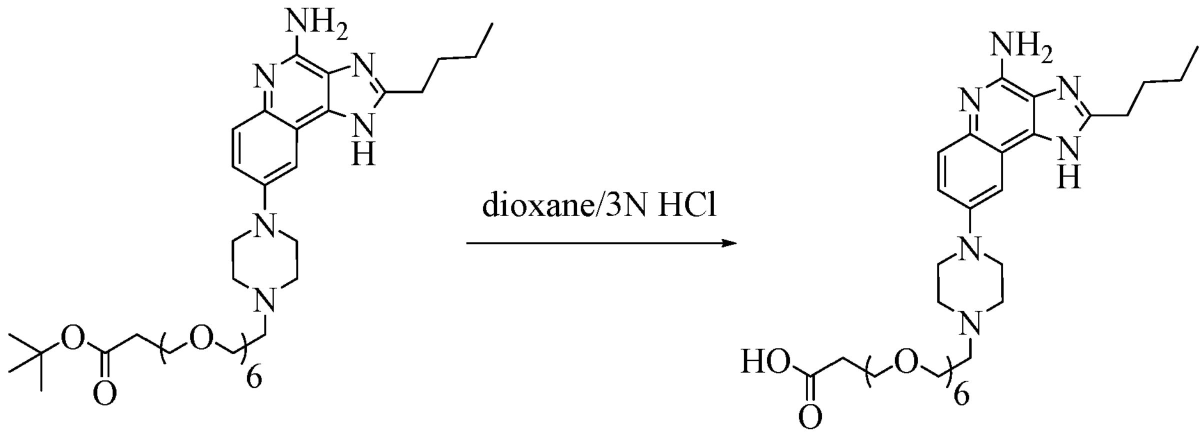


48 49

*Tert*-butyl 1-(4-(4-amino-2-butyl-1H-imidazo[4,5-c]quinolin-8-yl)piperazin-1-yl)-3,6,9,12,15,18-hexaoxahenicosan-21-oate was converted into 1-(4-(4-amino-2-butyl-1H-imidazo[4,5-c]quinolin-8-yl)piperazin-1-yl)-3,6,9,12,15,18-hexaoxahenicosan-21-oic acid according to the procedure described in Example 11. LC/MS [M+H] 661.39 (calculated); LC/MS [M+H] 661.60 (observed).

Example 45: Synthesis of Compound 50

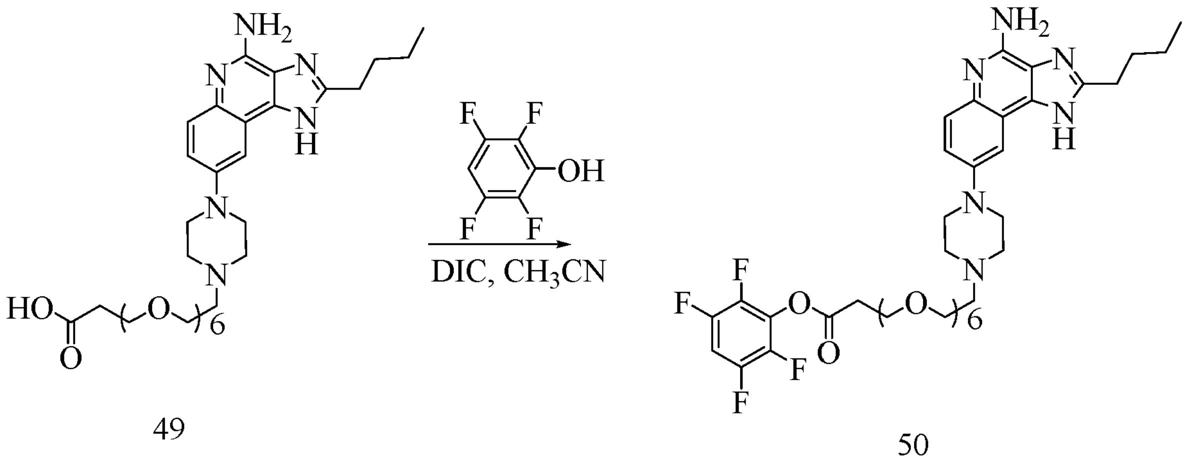


49 50

1-(4-(4-amino-2-butyl-1H-imidazo[4,5-c]quinolin-8-yl)piperazin-1-yl)-3,6,9,12,15,18-hexaoxahenicosan-21-oic acid was converted into 2,3,5,6-tetrafluorophenyl 1-(4-(4-amino-2-butyl-1H-imidazo[4,5-c]quinolin-8-yl)piperazin-1-yl)-3,6,9,12,15,18-hexaoxahenicosan-21-oate according to the procedure described in Example 12. LC/MS [M+H] 809.39 (calculated); LC/MS [M+H] 809.62 (observed).

Example 46: Synthesis of Compound 51

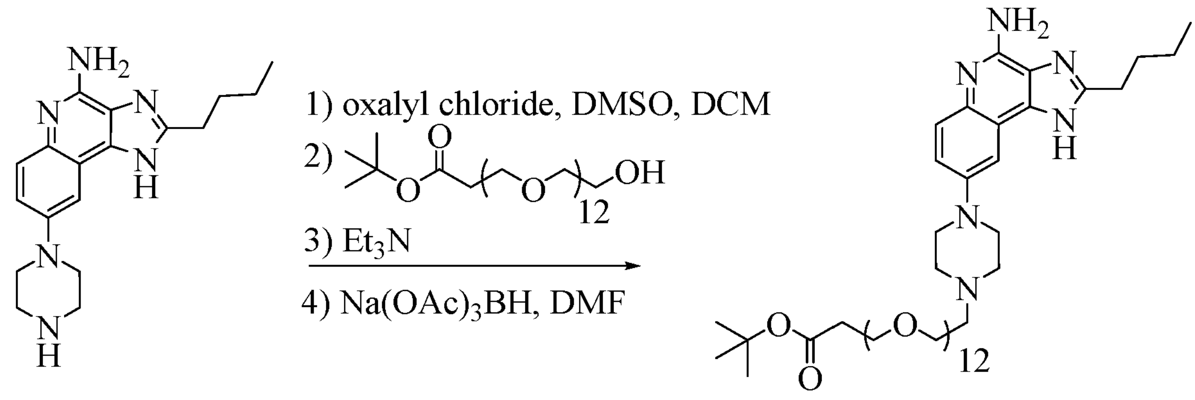


10 51

2-butyl-8-(piperazin-1-yl)-1*H*-imidazo[4,5-c]quinolin-4-amine was converted into tert-butyl 1-(4-(4-amino-2-butyl-1H-imidazo[4,5-c]quinolin-8-yl)piperazin-1-yl)-3,6,9,12,15,18,21,24,27,30,33,36-dodecaoxanonatriacontan-39-oate according to the procedure described in Example 10. LC/MS [M+H] 981.61 (calculated); LC/MS [M+H] 981.86 (observed).

Example 47: Synthesis of Compound 52

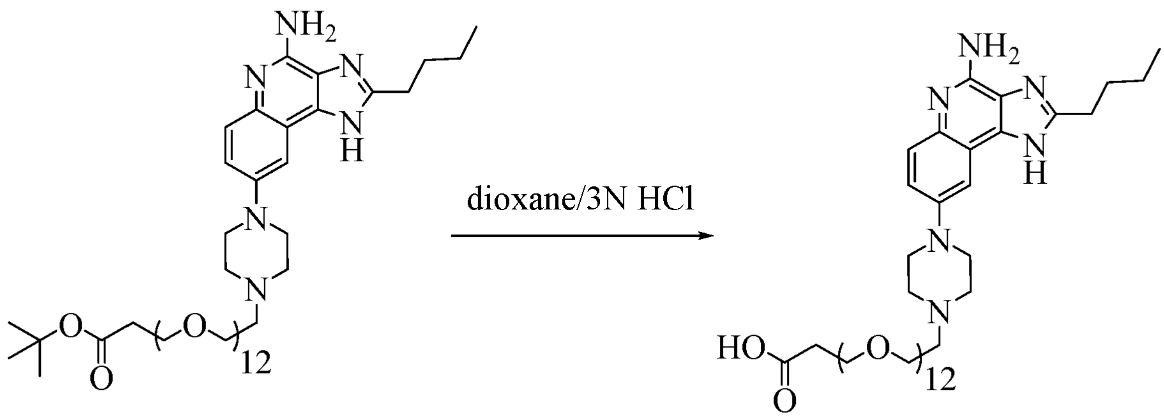


51 52

*Tert*-butyl 1-(4-(4-amino-2-butyl-1H-imidazo[4,5-c]quinolin-8-yl)piperazin-1-yl)-3,6,9,12,15,18,21,24,27,30,33,36-dodecaoxanonatriacontan-39-oate was converted into 1-(4-(4-amino-2-butyl-1H-imidazo[4, 5-c]quinolin-8-yl)piperazin-1-yl)-3,6,9,12,15,18,21,24,27,30,33,36-dodecaoxanonatriacontan-39-oic acid according to the procedure described in Example 11. The compound was used without further purification.

Example 48: Synthesis of Compound 53

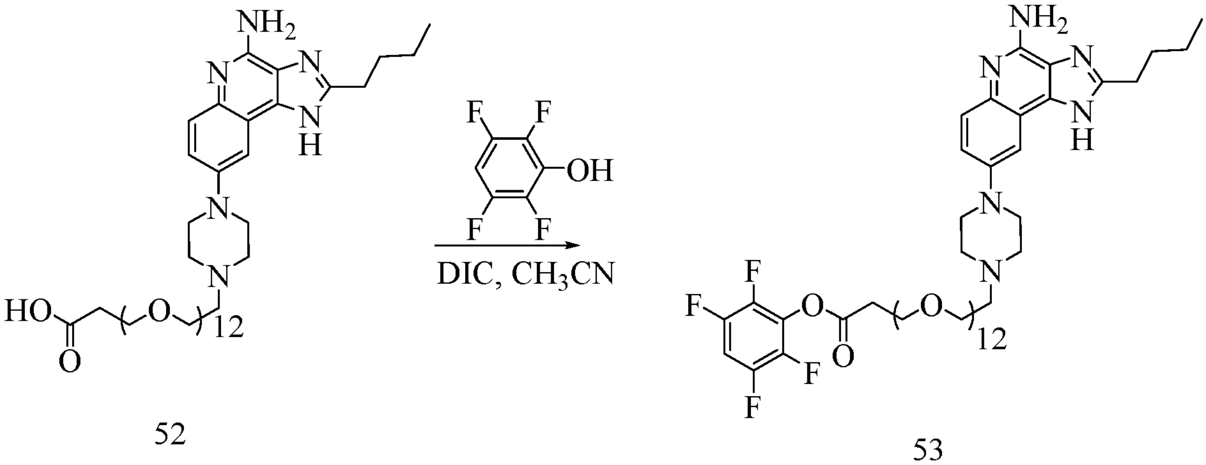


52 53

1-(4-(4-amino-2-butyl-1H-imidazo[4,5-c]quinolin-8-yl)piperazin-1-yl)-3,6,9,12,15,18,21,24,27,30,33,36-dodecaoxanonatriacontan-39-oic acid was converted into 2,3,5,6-tetrafluorophenyl 1-(4-(4-amino-2-butyl-1H-imidazo[4,5-c]quinolin-8-yl)piperazin-1-yl)-3,6,9,12,15,18,21,24,27,30,33,36-dodecaoxanonatriacontan-39-oate according to the procedure described in Example 12. LC/MS [M+H] 1073.54 (calculated); LC/MS [M+H] 1073.81 (observed).

Example 49: Synthesis of Compound 55

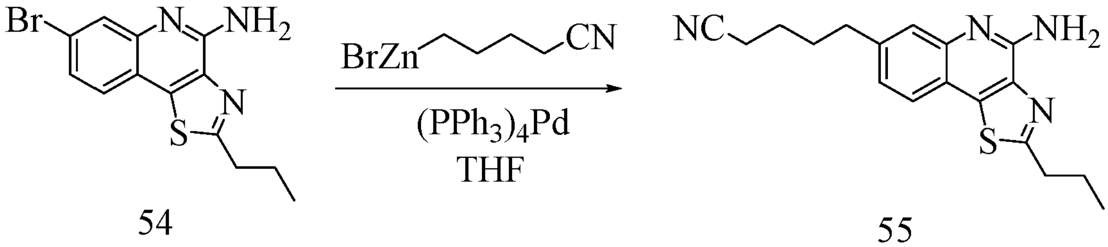


54 55

To 7-bromo-2-propylthiazolo[4,5-c]quinolin-4-amine (prepared according to WO 2006/93514) (320 mg, 1 mmol, 1 eq) was added a solution of 4-cyanobutylzinc bromide (0.5 M in THF, 20 mL, 5 eq.) under $N_2$. Tetrakis(triphenylphosphine)palladium (115 mg, 0.1 mmol, 0.1 eq) was added and mixture was stirred at 60 °C or 90 minutes. The mixture was cooled room temperature and solid sodium bicarbonate (1.0 g, 11.9 mmol, 11.9 eq) was added. To this stirred suspension was added water (0.8 mL) and the suspension was stirred vigorously for 20 minutes. The suspension was filtered through a plug of Celite and the solid cake cake was washed with dichloromethane (25 mL). The filtrate was diluted with dichloromethane (60 mL) and the organics were washed with saturated sodium bicarbonate solution (60 mL) and then brine (60 mL). The crude product was purified by flash chromatography (ethyl acetate/hexanes) to obtain 5-(4-amino-2-propylthiazolo[4,5-c]quinolin-7-yl)pentanenitrile (268 mg, 0.82 mmol, 82%) as a yellow solid after concentration. LC/MS: [M+H] calculated 325.14; [M+H] observed 325.26.

Example 50: Synthesis of Compound 56

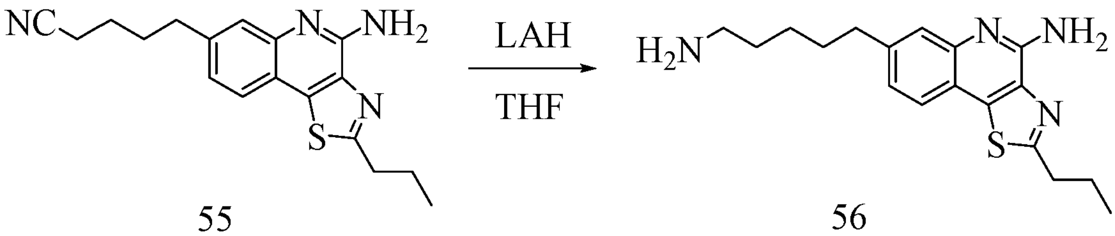


55 56

To 5-(4-amino-2-propylthiazolo[4,5-c]quinolin-7-yl)pentanenitrile) (260 mg, 0.79 mmol, 1 eq) in anhydrous THF

(9 mL) was added in portions solid lithiumaluminum hydride (117 mg, 3.2 mmol, 4 eq) under $N_2$. After gas evolution ceased, the mixture was stirred at 60 °C for 30 minutes. The mixture was cooled room temperature and solid sodium bicarbonate (1.0 g, 11.9 mmol, 14.8 eq) was added. To this stirred suspension was added water (0.3 mL) and the suspension was stirred vigorously for 20 minutes. The suspension was filtered through a plug of Celite and the solid cake cake was washed with dichloromethane (25 mL). The filtrate was diluted with dichloromethane (30 mL) solvent was evaporated. The crude product was purified by flash chromatography (methanol/dichloromethane) to obtain 7-(5-aminopentyl)-2-propylthiazolo [4,5-*c*]quinolin-4-amine (220 mg, 0.67 mmol, 82%) as a yellow solid after concentration. LC/MS: [M+H] calculated 329.17; [M+H] observed 329.31.

Example 51: Synthesis of Compound 58

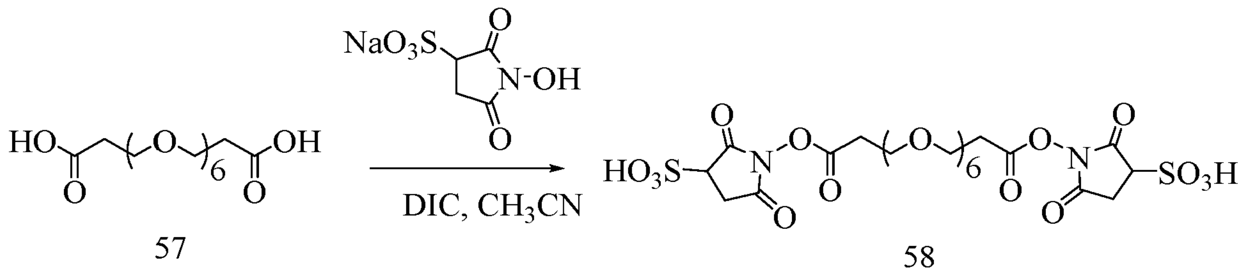


57

58

To a solution of 4,7,10,13,16,19-hexaoxadocosanedioic acid (286 mg, 0.75 mmol, 1.1 equiv.) in acetonitrile (3 mL) was added a mixture of disopropylcarbodiimide (283 mg, 2.25 mmol, 3.3 equiv.) and sodium N-hydroxysuccinimide-3-sulfonate in acetonitrile (3 mL). The mixture was sonicated for 1 minute and heated for 10 minutes at 50 °C. The crude 1,1'-(4,7,10,13,16,19-hexaoxadocosanedioylbis(oxy))bis(2,5-dioxopyrrolidine-3-sulfonic acid) obtained was used without further purification.

Example 52: Synthesis of Compound 59

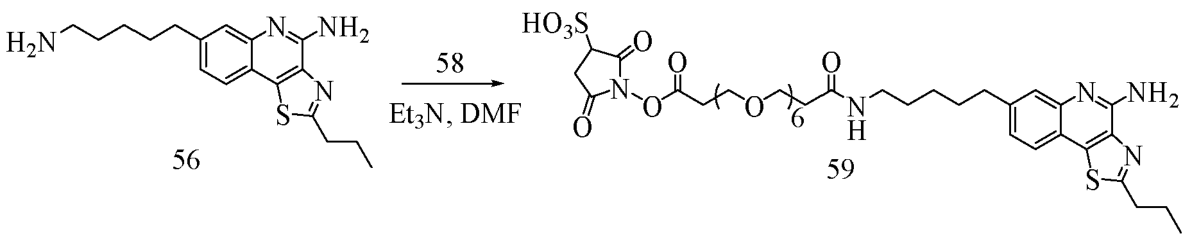


56

59

The crude 1,1'-(4,7,10,13,16,19-hexaoxadocosanedioylbis(oxy))bis(2,5-dioxopyrrolidine-3-sulfonic acid) in acetonitrile (6 mL) obtained in the preceding reaction was added to 7-(5-aminopentyl)-2-propylthiazolo[4,5-c]quinolin-4-amine (220 mg, 0.67 mmol, 1 equiv.) and the reaction was stirred at room temperature for 1h. The solvent was evaporated and the crude product was purified by reverse phase C18 chromatography (acetonitrile/water +0.1% TFA) to obtain 28-(4-amino-2-propylthiazolo[4,5-c]quinolin-7-yl)-22-oxo-4,7,10,13,16,19-hexaoxa-23-azaoctacosanoic acid sulfo-NHS ester (146 mg, 0.17 mmol, 25%) as a pale yellow film after evaporation of solvent. LC/MS: [M+H] calculated 870.32; [M+H] observed 870.53.

Example 53: Synthesis of Immunoconjugate A

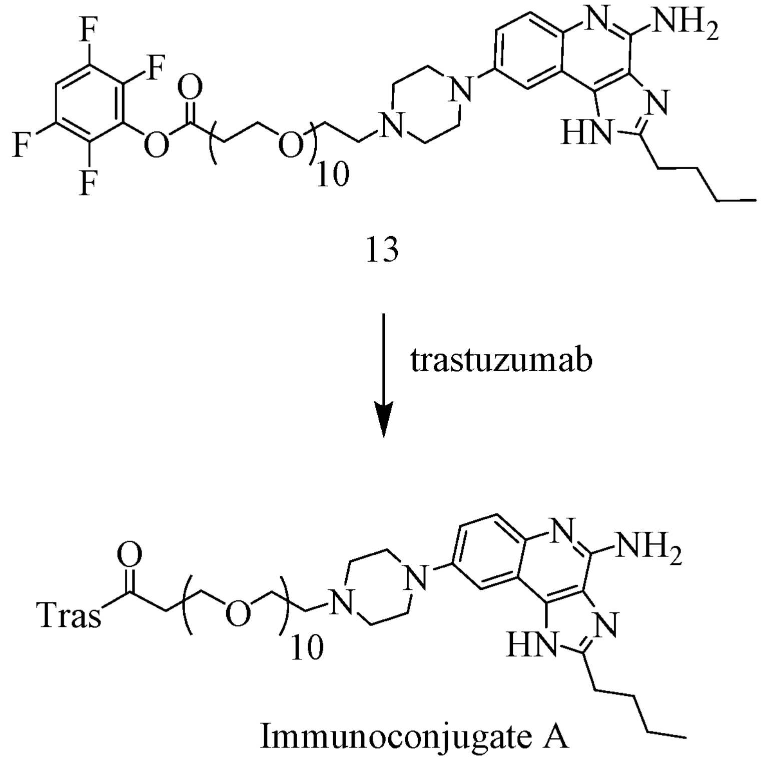


13

Immunoconjugate A

This example demonstrates the synthesis of Immunoconjugate A with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich, St. Louis, MO). The eluates were then each adjusted to 6 mg/ml using the buffer and sterile filtered. Trastuzumab at 6 mg/ml was pre-warmed to 30 °C and rapidly mixed with 7 molar equivalents of Compound 13. The reaction was allowed to proceed for 16 hours at 30 °C and Immunoconjugate A was separated from reactants by running over two successive G-25 desalting columns equilibrated in phosphate buffered saline (PBS) at pH 7.2. Adjuvant-antibody ratio (DAR) was determined by liquid chromatography mass spectrometry analysis using a C4 reverse phase column on an ACQUITY™ UPLC H-class (Waters Corporation, Milford, Massachusetts) connected to a XEVO™ G2-XS TOF mass spectrometer (Waters Corporation). Immunoconjugate A had a DAR of 2.5.

Example 54: Synthesis of Immunoconjugate B

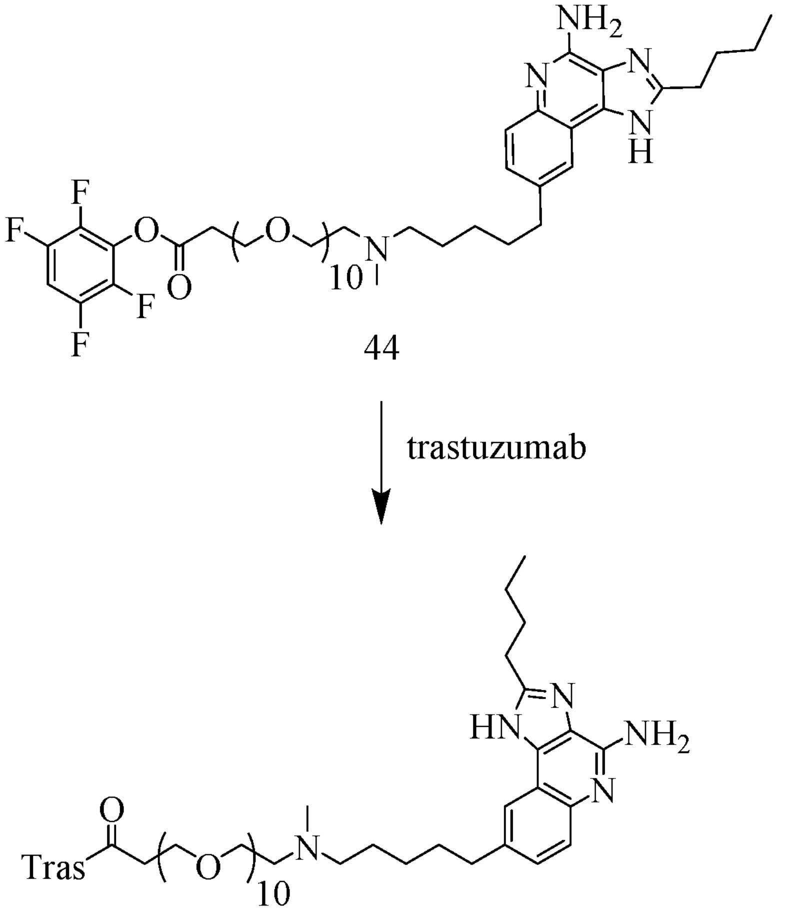


44

Immunoconjugate B

This example demonstrates the synthesis of Immunoconjugate B with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich). The eluates were then each adjusted to 6 mg/ml using the buffer and sterile filtered. Trastuzumab at 6 mg/ml was pre-warmed to 30 °C and rapidly mixed with 7.4 molar equivalents of Compound 44. The reaction was allowed to proceed for 16 hours at 30 °C and Immunoconjugate B was separated from reactants by running over two successive G-25 desalting columns equilibrated in PBS at pH 7.2. DAR was determined by liquid chromatography mass spectrometry analysis using a C4 reverse phase column on an ACQUITY™ UPLC H-class (Waters Corporation) connected to a XEVO™ G2-XS TOF mass spectrometer (Waters Corporation). Immunoconjugate B had a DAR of 2.56.

Example 55: Synthesis of Immunoconjugate C

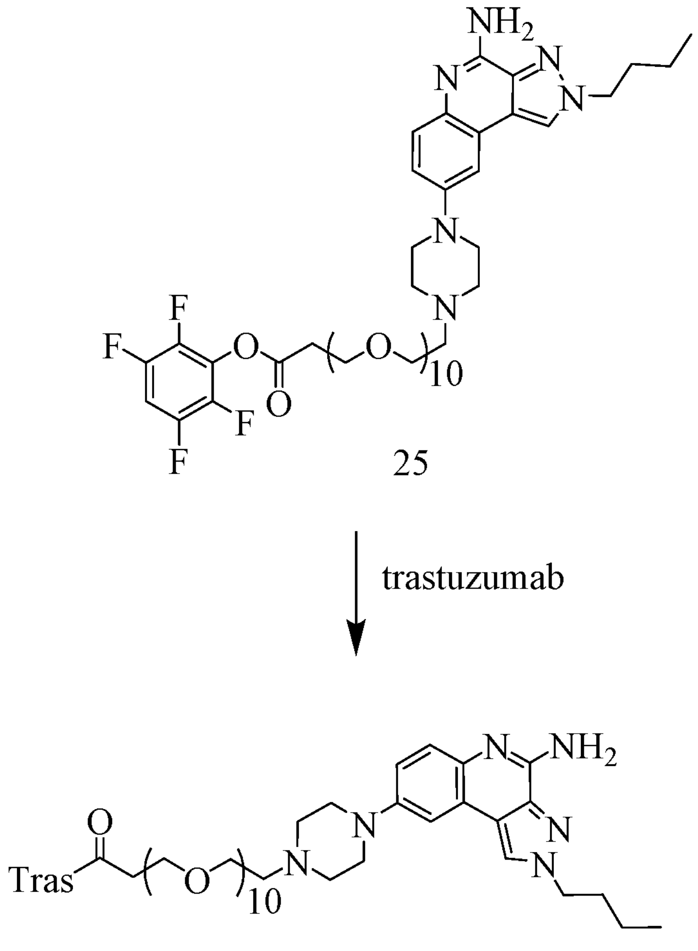


25

Immunoconjugate C

This example demonstrates the synthesis of Immunoconjugate C with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich). The eluates were then each adjusted to 6 mg/ml using the buffer and sterile filtered. Trastuzumab at 6 mg/ml was pre-warmed to 30 °C and rapidly mixed with 7.5 molar equivalents of Compound 25. The reaction was allowed to proceed for 16 hours at 30 °C and Immunoconjugate C was separated from reactants by running over two successive G-25 desalting columns equilibrated in PBS at pH 7.2. DAR was determined by liquid chromatography mass spectrometry analysis using a C4 reverse phase column on an ACQUITY™ UPLC H-class (Waters Corporation) connected to a XEVO™ G2-XS TOF mass spectrometer (Waters Corporation). Immunoconjugate C had a DAR of 2.65.

Example 56: Synthesis of Immunoconjugate D

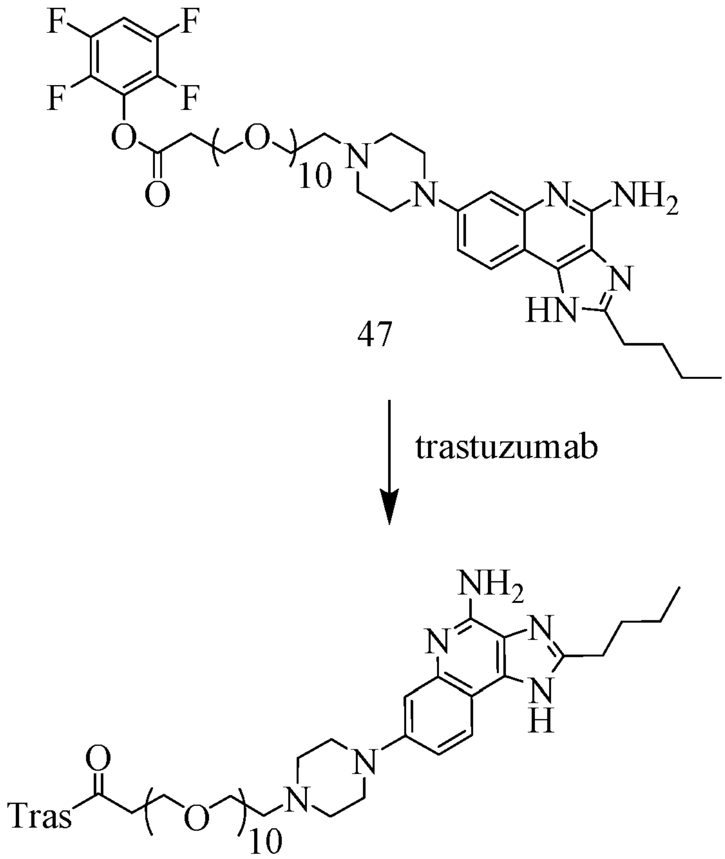


47

Immunoconjugate D

This example demonstrates the synthesis of Immunoconjugate D with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich). The eluates were then each adjusted to 6 mg/ml using the buffer and sterile filtered. Trastuzumab at 6 mg/ml was pre-warmed to 30 °C and rapidly mixed with 8.5 molar equivalents of Compound 47. The reaction was allowed to proceed for 16 hours at 30 °C and Immunoconjugate D was separated from reactants by running over two successive G-25 desalting columns equilibrated in PBS at pH 7.2. DAR was determined by liquid chromatography mass spectrometry analysis using a C4 reverse phase column on an ACQUITY™ UPLC H-class (Waters Corporation) connected to a XEVO™ G2-XS TOF mass spectrometer (Waters Corporation). Immunoconjugate D had a DAR of 2.26.

Example 57: Synthesis of Immunoconjugate E

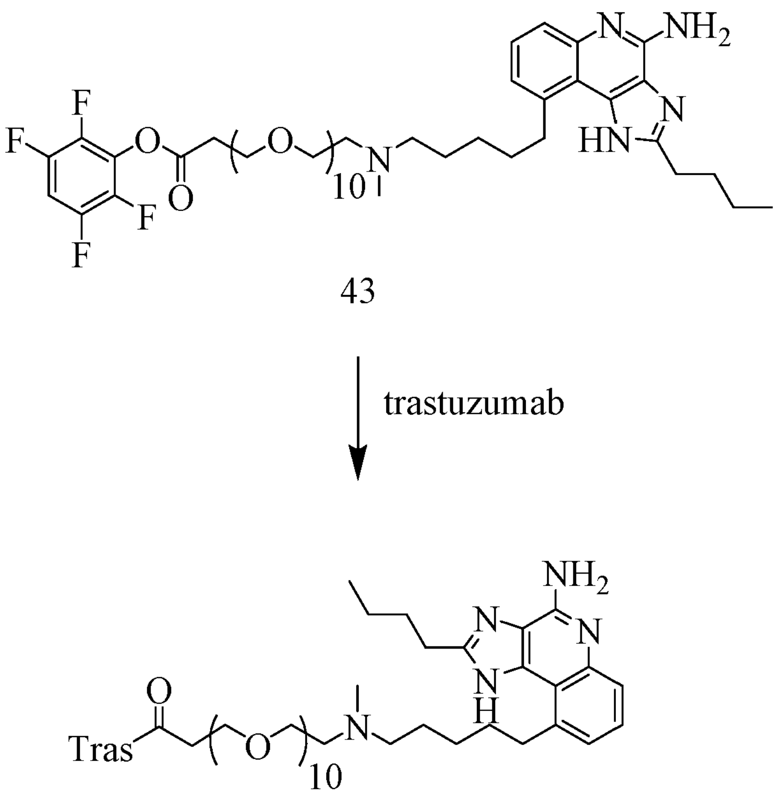


43

Immunoconjugate E

This example demonstrates the synthesis of Immunoconjugate E with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich). The eluates were then each adjusted to 6 mg/ml using the buffer and sterile filtered. Trastuzumab at 6 mg/ml was pre-warmed to 30 °C and rapidly mixed with 8 molar equivalents of Compound 43. The reaction was allowed to proceed for 16 hours at 30 °C and Immunoconjugate E was separated from reactants by running over two successive G-25 desalting columns equilibrated in PBS at pH 7.2. DAR was determined by liquid chromatography mass spectrometry analysis using a C4 reverse phase column on an ACQUITY™ UPLC H-class (Waters Corporation) connected to a XEVO™ G2-XS TOF mass spectrometer (Waters Corporation). Immunoconjugate E had a DAR of 2.8.

Example 58: Synthesis of Immunoconjugate F

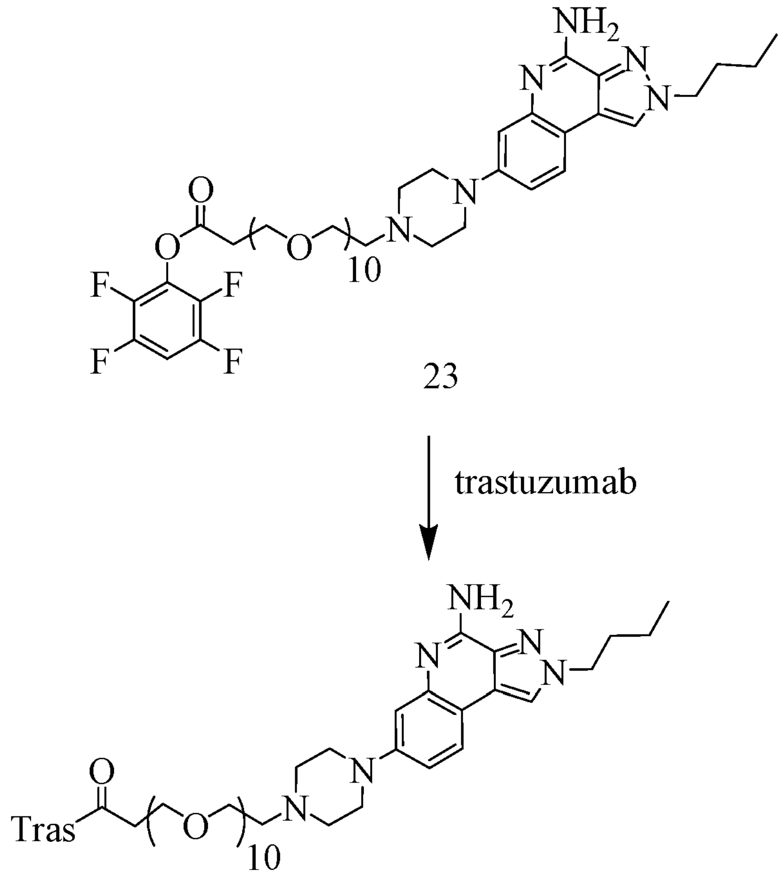


23

Immunoconjugate F

This example demonstrates the synthesis of Immunoconjugate F with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich). The eluates were then each adjusted to 6 mg/ml using the buffer and sterile filtered. Trastuzumab at 6 mg/ml was pre-warmed to 30 °C and rapidly mixed with 7.8 molar equivalents of Compound 23. The reaction was allowed to proceed for 16 hours at 30 °C and Immunoconjugate F was separated from reactants by running over two successive G-25 desalting columns equilibrated in PBS at pH 7.2. DAR was determined by liquid chromatography mass spectrometry analysis using a C4 reverse phase column on an ACQUITY™ UPLC H-class (Waters Corporation) connected to a XEVO™ G2-XS TOF mass spectrometer (Waters Corporation). Immunoconjugate F had a DAR of 2.39.

Example 59: Synthesis of Immunoconjugate G

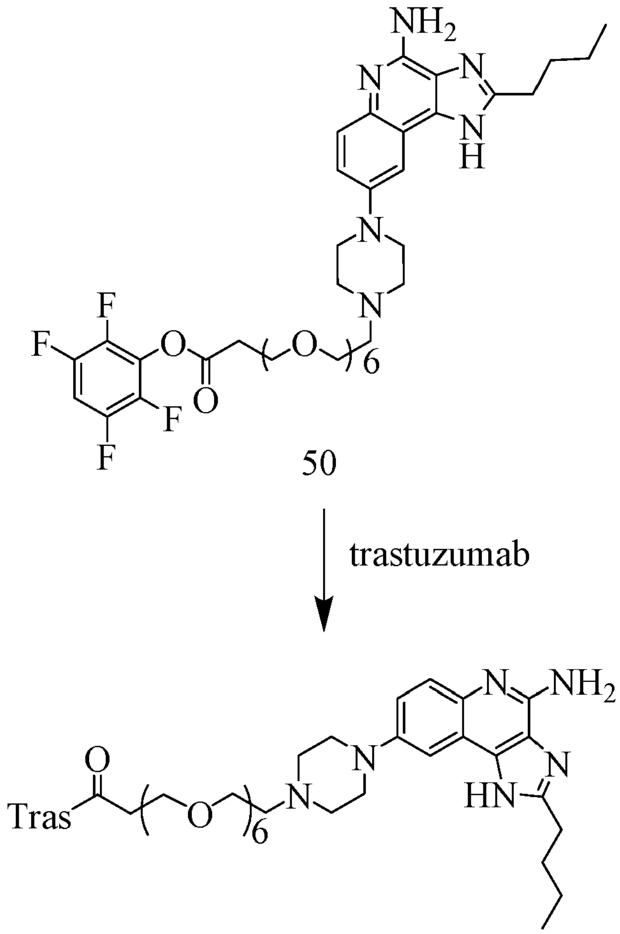


50

Immunoconjugate G

This example demonstrates the synthesis of Immunoconjugate G with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich). The eluates were then each adjusted to 6 mg/ml using the buffer and sterile filtered. Trastuzumab at 6 mg/ml was pre-warmed to 30 °C and rapidly mixed with 8.5 molar equivalents of Compound 50. The reaction was allowed to proceed for 16 hours at 30 °C and Immunoconjugate G was separated from reactants by running over two successive G-25 desalting columns equilibrated in PBS at pH 7.2. DAR was determined by liquid chromatography mass spectrometry analysis using a C4 reverse phase column on an ACQUITY™ UPLC H-class (Waters Corporation) connected to a XEVO™ G2-XS TOF mass spectrometer (Waters Corporation). Immunoconjugate G had a DAR of 2.37.

Example 60: Synthesis of Immunoconjugate H

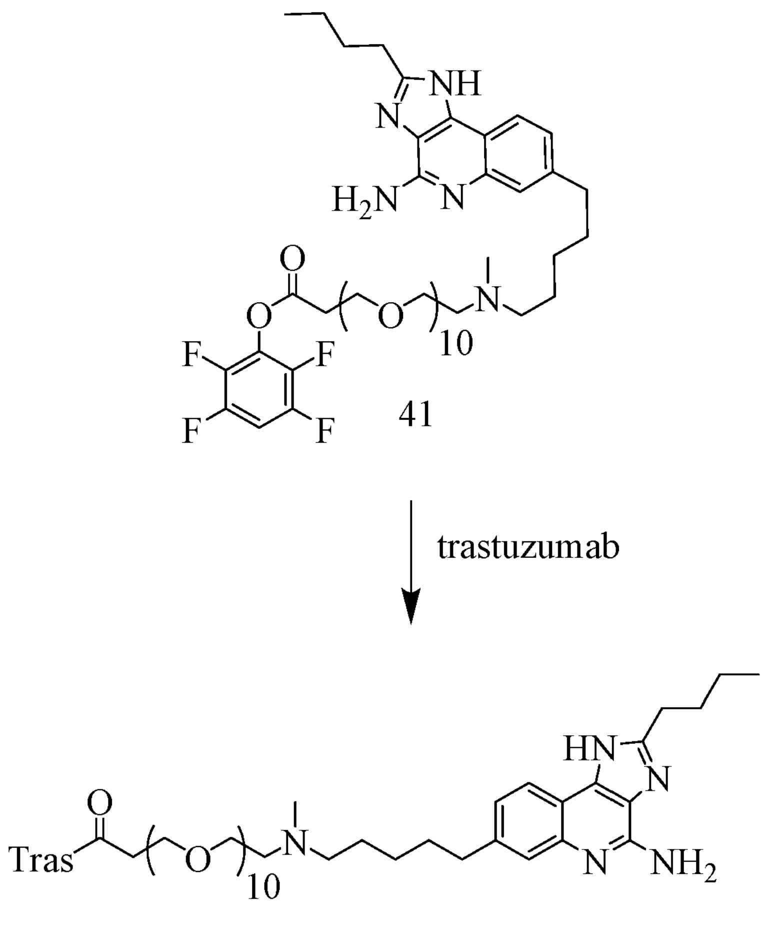


Immunoconjugate H

This example demonstrates the synthesis of Immunoconjugate H with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich). The eluates were then each adjusted to 6 mg/ml using the buffer and sterile filtered. Trastuzumab at 6 mg/ml was pre-warmed to 30 °C and rapidly mixed with 6 molar equivalents of Compound 41. The reaction was allowed to proceed for 16 hours at 30 °C and Immunoconjugate H was separated from reactants by running over two successive G-25 desalting columns equilibrated in PBS at pH 7.2. DAR was determined by liquid chromatography mass spectrometry analysis using a C4 reverse phase column on an ACQUITY™ UPLC H-class (Waters Corporation) connected to a XEVO™ G2-XS TOF mass spectrometer (Waters Corporation). Immunoconjugate H had a DAR of 1.98.

Example 61: Synthesis of Immunoconjugate I

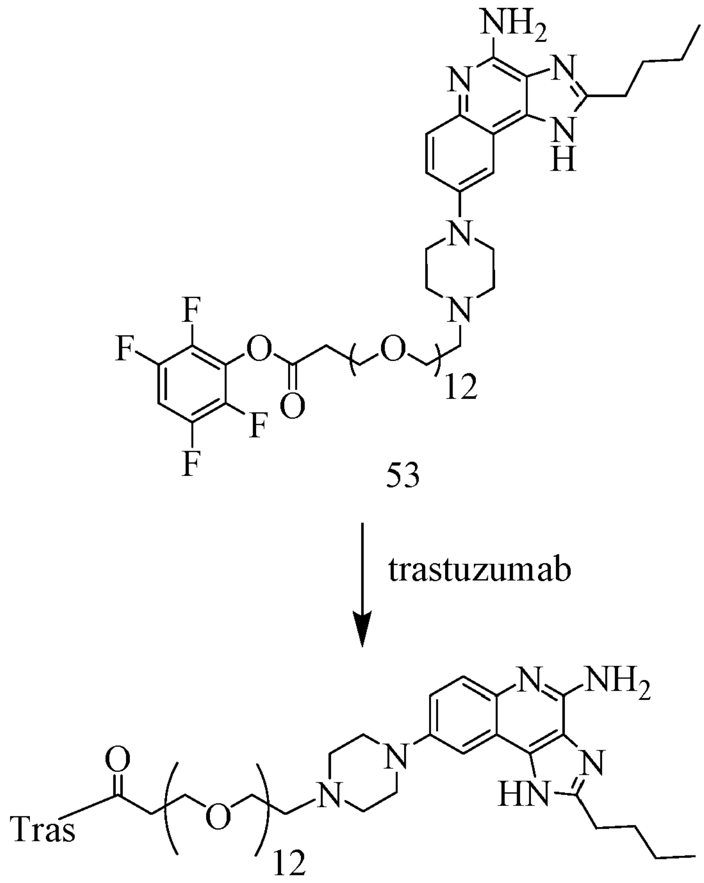


53

Immunoconjugate I

This example demonstrates the synthesis of Immunoconjugate I with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich). The eluates were then each adjusted to 6 mg/ml using the buffer and sterile filtered. Trastuzumab at 6 mg/ml was pre-warmed to 30 °C and rapidly mixed with 6 molar equivalents of Compound 53. The reaction was allowed to proceed for 16 hours at 30 °C and Immunoconjugate I was separated from reactants by running over two successive G-25 desalting columns equilibrated in PBS at pH 7.2. DAR was determined by liquid chromatography mass spectrometry analysis using a C4 reverse phase column on an ACQUITY™ UPLC H-class (Waters Corporation) connected to a XEVO™ G2-XS TOF mass spectrometer (Waters Corporation). Immunoconjugate I had a DAR of 2.15.

Example 62: Synthesis of Immunoconjugate J

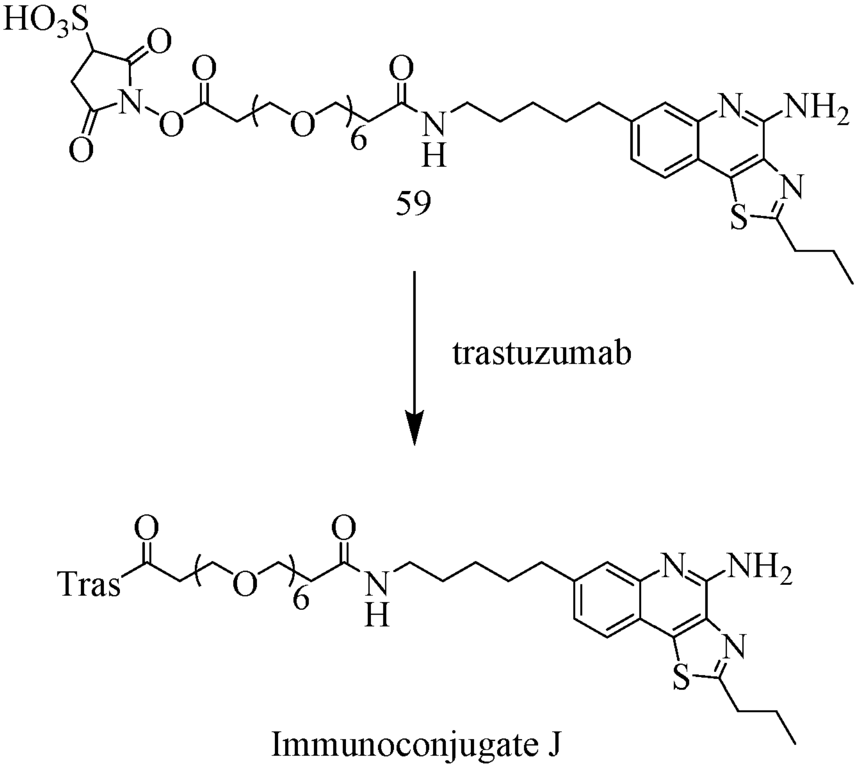


59

Immunoconjugate J

This example demonstrates the synthesis of Immunoconjugate J with trastuzumab as the antibody construct (Tras).

Trastuzumab was buffer exchanged into the conjugation buffer containing 100 mM boric acid, 50 mM sodium chloride, 1 mM ethylenediaminetetraacetic acid at pH 8.3, using G-25 SEPHADEX™ desalting columns (Sigma-Aldrich). The eluates were then each adjusted to 6 mg/ml using the buffer and sterile filtered. Trastuzumab at 6 mg/ml was pre-warmed to 30 °C and rapidly mixed with 6 molar equivalents of Compound 59. The reaction was allowed to proceed for 16 hours at 30 °C and Immunoconjugate J was separated from reactants by running over two successive G-25 desalting columns equilibrated in PBS at pH 7.2. DAR was determined by liquid chromatography mass spectrometry analysis using a C4 reverse phase column on an ACQUITY™ UPLC H-class (Waters Corporation) connected to a XEVO™ G2-XS TOF mass spectrometer (Waters Corporation). Immunoconjugate J had a DAR of 2.07.

Example 63. Assessment of Immunoconjugate Activity *In Vitro*

This example shows that Immunoconjugates A-J are effective at eliciting myeloid activation, and therefore are useful for the treatment of cancer.

*Isolation of Human Antigen Presenting Cells.* Human myeloid antigen presenting cells (APCs) were negatively selected from human peripheral blood obtained from healthy blood donors (Stanford Blood Center, Palo Alto, California) by density gradient centrifugation using a ROSETTESEP™ Human Monocyte Enrichment Cocktail (Stem Cell Technologies, Vancouver, Canada) containing monoclonal antibodies against CD14, CD16, CD40, CD86, CD123, and HLA-DR. Immature APCs were subsequently purified to >97% purity via negative selection using an EASYSEP™ Human Monocyte Enrichment Kit (Stem Cell Technologies) without CD16 depletion containing monoclonal antibodies against CD14, CD16, CD40, CD86, CD123, and HLA-DR.

*Preparation of Tumor Cells.* Three tumor cell lines were used: HCC1954, JIMT-1, and COLO 205. HCC1954 (American Type Culture Collection (ATCC), Manassas, Virginia) was derived from a primary stage IIA, grade 3 invasive ductal carcinoma with no lymph node metastases. HCC1954 is positive for the epithelial cell specific marker Epithelial Glycoprotein 2 and for cytokeratin 19, and is negative for expression of estrogen receptor (ER) and progesterone receptor (PR). HCC1954 overexpresses HER2 (as determined by enzyme-linked immunosorbent assay (ELISA)) with a relatively "high" level of overexpression. JIMT-1 (DSMZ, Braunschweig, Germany) was derived from the pleural effusion of a woman with ductal breast cancer (grade 3 invasive, stage IIB) following postoperative radiation. JIMT-1 overexpresses HER2 at what is considered to be a "medium" level of overexpression, but is insensitive to HER2-inhibiting drugs (e.g., trastuzumab). COLO 205 (ATCC) was derived from the ascites fluid of man with carcinoma of the colon. COLO 205 expresses carcinoembryonic antigen (CEA), keratin, interleukin 10 (IL-10), and is considered to overexpress HER2 at a relatively

"low" level of overexpression.

Tumor cells from each cell line were separately re-suspended in PBS with 0.1% fetal bovine serum (FBS) at 1 to 10 × $10^6$ cells/mL. Cells were subsequently incubated with 2 μM carboxyfluorescein succinimidyl ester (CFSE) to yield a final concentration of 1 μM. The reaction was quenched after 2 minutes via the addition of 10 mL complete medium with 10% FBS and washed twice with complete medium. Cells were either fixed in 2% paraformaldehyde and washed three times with PBS or left viable prior to use.

*APC-Tumor Co-cultures.* 2 × $10^5$ APCs were incubated with (e.g., FIG. 2A-2I) or without (e.g., FIG. 3A-11C) CFSE-labeled tumor cells between a 5:1 and 10:1 effector to target (tumor) cell ratio in 96-well plates (Corning, Corning, NY) containing iscove's modified dulbecco's medium (IMDM) (Thermo Fisher Scientific) supplemented with 10% FBS, 100 U/mL penicillin, 100 μg/mL streptomycin, 2 mM L-glutamine, sodium pyruvate, non-essential amino acids, and where indicated, various concentrations of unconjugated HER2 antibody and Immunoconjugate A (as prepared according to the examples above). Cells and cell-free supernatants were analyzed after 18 hours via flow cytometry or ELISA.

The results of this assay are shown in the figures, for example, FIG. 2A (CD40) and FIG. 2B (CD86) for Immunoconjugate A on the HCC1954 cell line, FIG. 2D (CD40) and FIG. 2E (CD86) for Immunoconjugate A on the JIMT-1 cell line, and FIG. 2G (CD40) and FIG. 2H (CD86) for Immunoconjugate A on the COLO 205 cell line.

While the expression of T cell stimulatory molecules such as CD40 and CD86 are necessary for effective T cell activation, APCs also influence the nature of the ensuing immune response through the secretion of proinflammatory cytokines. Therefore, the capacity of immunoconjugates to elicit cytokine secretion in human APCs following stimulation was investigated. The data indicate that the immunoconjugate-stimulated cells secreted high levels of TNFα. See FIG. 2C for Immunoconjugate A co-cultured with the HCC1954 cell line, FIG. 2F for Immunoconjugate A co-cultured with the JIMT-1 cell line, and FIG. 2I for Immunoconjugate A co-cultured with the COLO 205 cell line.

Similar data is provided for Immunoconjugates B-J at Figures 3A-11C (without co-culture).

Example 64. Comparison of Immunoconjugate K to Immunoconjugate L

This example shows the increase in immunoconjugate activity demonstrated by an immunoconjugate comprising an adjuvant moiety that further comprises a hydrophobic substituent with at least 1 carbon atom.

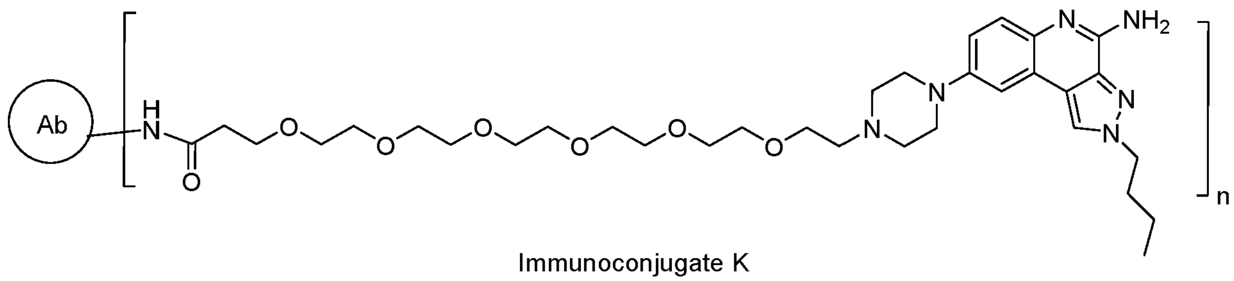

Immunoconjugate K

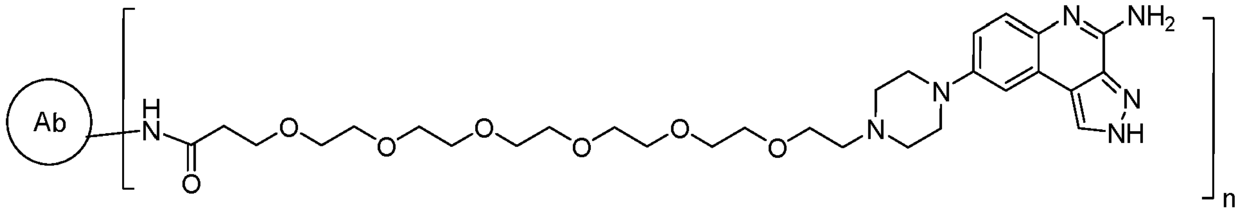

Immunoconjugate L

To determine the DAR, Immunoconjugates K and L are acidified (diluted 5 fold or more in water, 0.2 % formic acid) and injected onto a Waters BEH-C4 reverse phase column (product number 186004495) hooked up to a Waters Aquity H-class UPLC and separated using a linear gradient of 1-90 % acetonitrile, 0.1 % formic acid. C4 column eluates are continuously analyzed via electrospray ionization onto a Waters Xevo G2-XS time of flight (TOF) mass spectrometer. To determine the DAR for a conjugate, it is first necessary to identify the time window in the total ion current chromatogram (TIC) that corresponds to the elution window for the antibody conjugate from the C4 column. Once selected, the observed ions, representing several co-eluting families of mass/charge (m/z) species (one family for each protein species) within the given time window are deconvoluted using Water's MassLynx v4.1 software into accurate masses for each DAR species present. The intensity of the peaks for each DAR species is then combined using equation 1:

$$\text{Average DAR} = \frac{(1 \times \text{iDAR1}) + (2 \times \text{iDAR2}) + (3 \times \text{iDAR3}) + (4 \times \text{iDAR4})}{\text{iDAR0} + \text{iDAR1} + \text{iDAR2} + \text{iDAR3} + \text{iDAR4}} \quad \text{Eq. 1}$$

wherein iDAR is equal to the observed peak intensity (observed ions) for a given DAR species and the total number of observed species is five (four DAR species + unlabeled antibody). The equation may be adjusted as required for the number of species present. This equation is for an antibody conjugate that has been deglycosylated prior to LC-MS analysis. For analysis of a glycosylated antibody each DAR species may be represented by multiple peaks within the deconvoluted time window. In this case $iDARn = [n \times (iDARn_{gly1} + iDARn_{gly2} + iDARn_{gly3})]$ where n is the DAR species and the number of observed glycosylation variants is three for example.

Immunoconjugate K with Trastuzumab as the antibody and a DAR of 2 and Immunoconjugate L with Trastuzumab as the antibody and a DAR of 2 are analyzed using the adjuvant activity and immunoconjugate activity procedures described herein.

Immunoconjugate K increased activity *in vitro* as compared to Immunoconjugate L, as evidenced by the myeloid activation. Thus, an adjuvant moiety that further comprises a hydrophobic substituent with at least 1 carbon atom enhances the activity of the immunoconjugate.

Example 65. Comparison of Immunoconjugate K to Immunoconjugate M

This example shows the importance of the location of the point of attachment of a linker exhibited by two immunoconjugates linked at different locations relative to the 2-amino moiety.

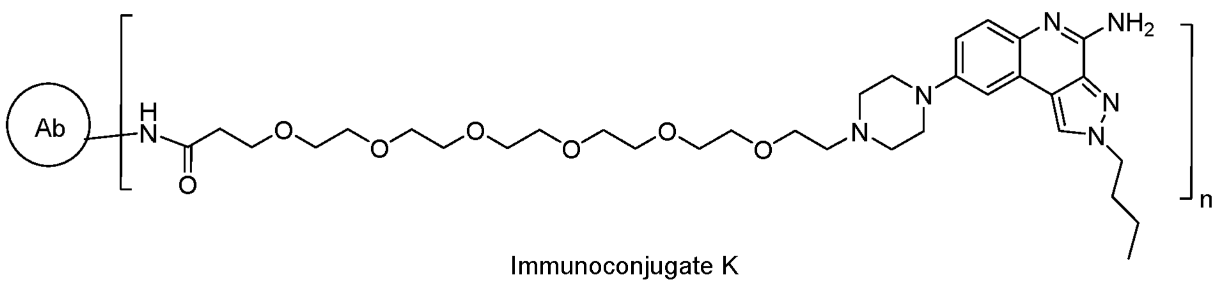

Immunoconjugate K

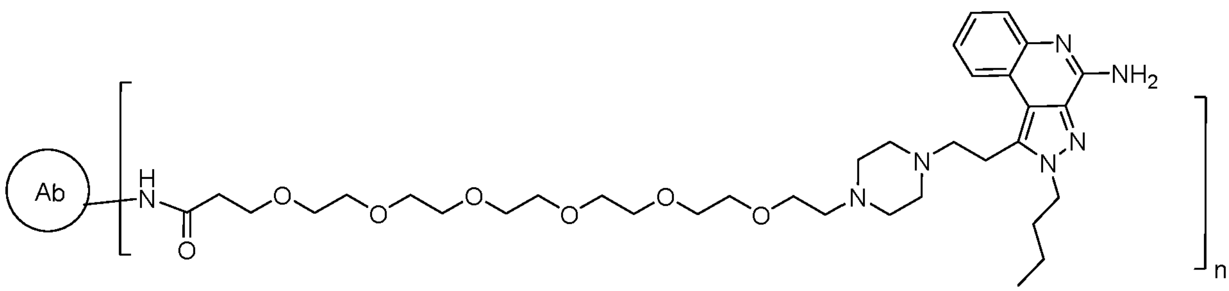

Immunoconjugate M

The DARs of Immunoconjugates K and M were determined according to the procedure provided in Example 64.

Immunoconjugate K with Trastuzumab as the antibody and a DAR of 2 and Immunoconjugate M with Trastuzumab as the antibody and a DAR of 2 were analyzed using the adjuvant activity and immunoconjugate activity procedures described herein.

Immunoconjugate K increased activity *in vitro* as compared to Immunoconjugate M, as evidenced by the myeloid activation. Thus, an adjuvant moiety that has a point of attachment of a linker further from the 2-amino moiety can enhance the activity of the immunoconjugate, thereby demonstrating the importance of the point of attachment of the linker.

Example 66. Comparison of Immunoconjugate K to Immunoconjugate N

This example shows the synergistic effect of an immunoconjugate comprising an adjuvant moiety that further comprises a hydrophobic substituent with at least 1 carbon atom and a preferred point of attachment of the linker, as evidenced by myeloid activation.

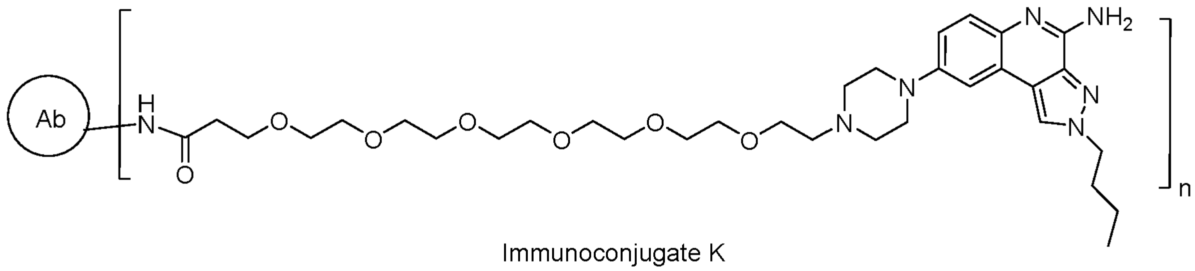

Immunoconjugate K

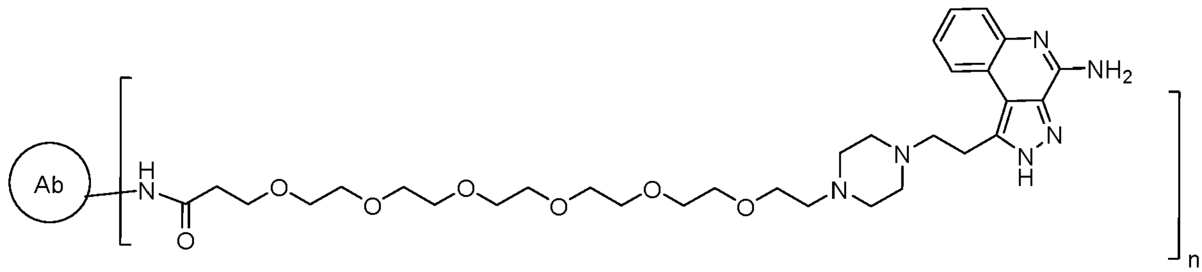

Immunoconjugate N

The DARs of Immunoconjugates K and N were determined according to the procedure provided in Example 64.

Immunoconjugate K with Trastuzumab as the antibody and a DAR of 2 and Immunoconjugate N with Trastuzumab as the antibody and a DAR of 2 were analyzed using the adjuvant activity and immunoconjugate activity procedures described herein.

Immunoconjugate K increased activity *in vitro* as compared to Immunoconjugate N, as evidenced by the myeloid activation. The increase in activity of Immunoconjugate K, relative to Immuonjugate N, is greater than the sum of the benefit achieved relative to Immunoconjugates L and M (see Examples 64 and 65). Thus, this Example demonstrates the synergistic effect of an immunoconjugate comprising an adjuvant moiety that further comprises a hydrophobic substituent with at least 1 carbon atom and a preferred point of attachment of the linker, as evidenced by myeloid activation.

Example 67. Comparison of Adjuvant 1 to Adjuvant 2

This example shows the importance of the pendant nitrogen of the 2-amino nitrogen moiety for maintaining activity of an adjuvant, as evidenced by HEK293 reporter cells expressing human TLR7 or human TLR8.

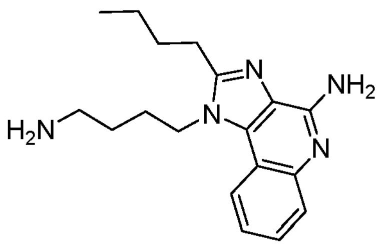

Adjuvant 1

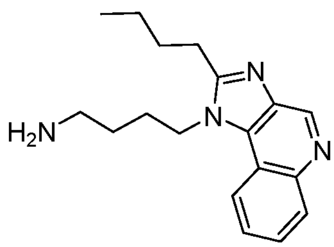

Adjuvant 2

The activities of Adjuvants 1 and 2 were measured using a HEK293 reporter assay in which NF-KB activity is measured. Details of the assay are as follows.

HEK293 reporter cells expressing human TLR7 or human TLR8 were purchased from Invivogen (San Diego, CA) and vendor protocols were followed for cellular propagation and experimentation. Cells were grown to 80-85% confluence at 5% $CO_2$ in Dulbecco's Modified Eagle's Medium (DMEM) supplemented with 10% fetal bovine serum (FBS), Zeocin, and Blasticidin. Cells were then seeded in 96-well flat plates at $4\times10^4$ cells/well with substrate containing HEK detection medium and Adjuvant 1 or Adjuvant 2 in amounts specified in FIG. 12. Activity was measured using a plate reader at 620-655 nm. The results are set forth in FIG. 12.

FIG. 12 shows that Adjuvant 2 was completely inactive in both the TLR7 and TLR8 reporter cells as compared to Adjuvant 1. This result demonstrates that the pendant nitrogen of the 2-amino nitrogen moiety is necessary to maintain activity of an adjuvant for TLR7 and/or TLR8.

Example 68. Comparison of Immunoconjugate O to Immunoconjugate P

This example shows the importance of the pendant nitrogen of the 2-amino nitrogen moiety for maintaining activity of an immunoconjugate, as evidenced by immunoconjugate activity and dendritic cell differentiation.

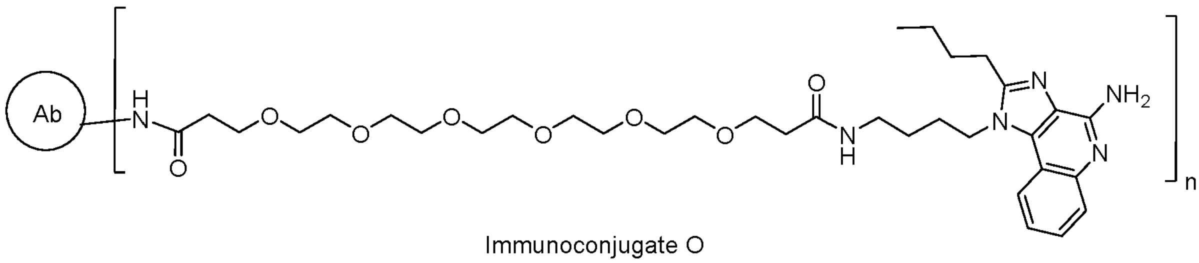

Immunoconjugate O

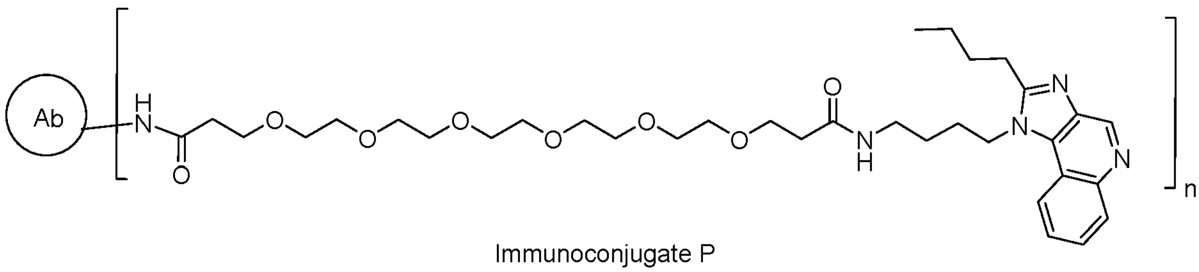

Immunoconjugate P

The DARs of Immunoconjugates O and P were determined according to the procedure provided in Example 64.

Immunoconjugate O with Rituximab as the antibody and a DAR of 1.9 and Immunoconjugate P with Rituximab as the antibody and a DAR of 2.2 were analyzed using the adjuvant activity and immunoconjugate activity procedures described herein, and the results are set forth in FIGs. 13 and 14.

FIG. 13 shows that ablation of TLR activity (see Example 67) in Immunoconjugate P resulted in a complete halt in activation, as measured by upregulation of costimulatory molecules CD40 and CD86. FIG. 13 further shows that upregulation of costimulatory molecules CD40 and CD86 by Immunoconjugate P was comparable to the antibody control and significantly reduced as compared to Immunoconjugate O. These results demonstrate that the pendant nitrogen of the 2-amino nitrogen moiety is necessary to maintain immunoconjugate activity as measured by upregulation of costimulatory molecules CD40 and CD86.

FIG. 14 shows that ablation of TLR activity (see Example 67) in Immunoconjugate P resulted in a complete halt in dendritic cell differentiation, as measured by CD14, CD16, and CD123 expression. When treated with Immunoconjugate O, CD14 and CD16, both markers found on monocytes, were downregulated. In contrast, CD123, a marker expressed on inflammatory myeloid derived dendritic cells was upregulated following treatment with Immunoconjugate O. However, when treated with Immunoconjugate P, the results were comparable to the antibody control, producing no dendritic cell differentiation, as measured by CD14, CD16, and CD123 expression. These results demonstrate that the pendant nitrogen of the 2-amino nitrogen moiety is necessary to induce dendritic cell differentiation as measured by CD14, CD16, and CD 123 expression.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items

(A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

**1.** An immunoconjugate comprising:

(a) an antibody construct comprising (i) an antigen binding domain and (ii) an Fc domain and
(b) 1-8 adjuvant cores, wherein each adjuvant core is covalently bonded to the antibody construct via a linker, wherein each adjuvant core comprises a 2-amino nitrogen moiety with a pendant nitrogen atom and a point of attachment of the linker to the adjuvant core, and wherein the distance between the pendant nitrogen atom and the point of attachment of the linker is greater than 5 Å,
wherein

the immunoconjugate is of formula:

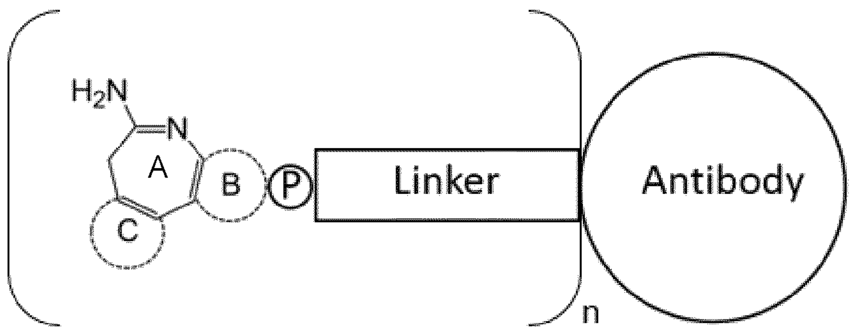

Immunoconjugate D2,

wherein the adjuvant core is represented by fused rings A, B and C, wherein A and B are present and C is optionally present, and B and C denote 5-, 6-, or 7-membered rings, optionally comprising double bonds, and optionally comprising heteroatoms in addition to the 2-amino nitrogen moiety, and the adjuvant core is optionally substituted such that the substitution pattern around the adjuvant core is not particularly limited, as long as the 2-amino nitrogen moiety remains unsubstituted, P represents the point of attachment of the linker to the B ring, and n is an integer from 1 to 8, or
the immunoconjugate is of formula:

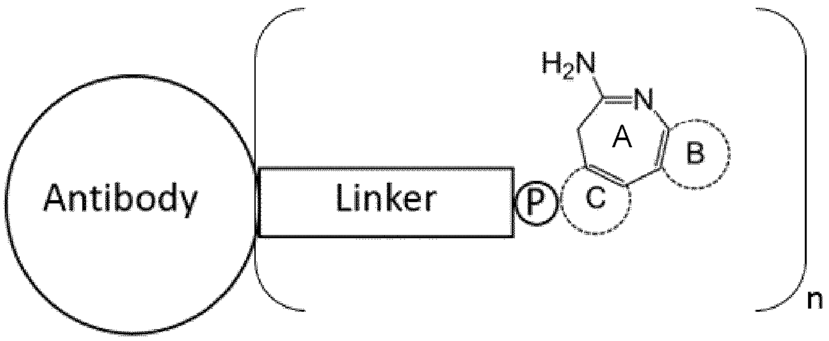

Immunoconjugate D4,

wherein the adjuvant core is represented by fused rings A, B and C, wherein A and C are present and B is optionally present, and B and C denote 5-, 6-, or 7-membered rings, optionally comprising double bonds, and optionally comprising heteroatoms in addition to the 2-amino nitrogen moiety, and the adjuvant core is optionally substituted such that the substitution pattern around the adjuvant core is not particularly limited, as long as the 2-amino nitrogen moiety remains unsubstituted, P represents the point of attachment of the linker to the C ring, and n is an integer from 1 to 8.

**2.** The immunoconjugate of claim 1, wherein the distance between the pendant nitrogen atom and the point of attachment of the linker is greater than 5.5 Å, and preferably the distance between the pendant nitrogen atom and the point of attachment of the linker is greater than 6 Å.

**3.** The immunoconjugate of claim 1 or 2, wherein when bound to a binding domain of a TLR8 comprising an aspartic acid residue, the pendant nitrogen atom of the 2-amino nitrogen moiety is less than 5 Å from a carbonyl oxygen of an acidic side chain of the aspartic acid residue.

**4.** The immunoconjugate of claim 3, wherein the aspartic acid residue is Asp543.

**5.** The immunoconjugate of claim 3 or 4, wherein the pendant nitrogen atom of the 2-amino nitrogen moiety is less than 3 Å from the carbonyl oxygen of the acidic side chain of the aspartic acid residue.

**6.** The immunoconjugate of any one of claims 1-5, wherein when bound to a binding domain of a TLR7 comprising an aspartic acid residue, the pendant nitrogen atom of the 2-amino nitrogen moiety is less than 5 Å from a carbonyl oxygen of an acidic side chain of the aspartic acid residue.

**7.** The immunoconjugate of claim 6, wherein the aspartic acid residue is Asp555.

**8.** The immunoconjugate of claim 6 or 7, wherein the pendant nitrogen atom of the 2-amino nitrogen moiety is less than 3 Å from the carbonyl oxygen of the acidic side chain of the aspartic acid residue.

**9.** The immunoconjugate of any one of claims 1-8, wherein when bound to the binding domain of a TLR8 comprising an arginine and serine residue, the point of attachment of the linker to the adjuvant core is from 3 Å to 10 Å from an oxygen atom of a side chain of the serine residue and/or the point of attachment of the linker to the adjuvant core is from 3 Å to 10 Å from a nitrogen atom of a side chain of the arginine residue, and preferably the point of attachment of the linker to the adjuvant core is from 3 Å to 7 Å from the oxygen atom of the side chain of the serine residue and the point of attachment of the linker to the adjuvant core is from 3 Å to 7 Å from the nitrogen atom of the side chain of the arginine residue.

**10.** The immunoconjugate of claim 9, wherein the serine residue is Ser352 and the arginine residue is Arg429.

**11.** The immunoconjugate of any one of claims 1-10, wherein when bound to the binding domain of a TLR7 comprising a lysine and valine residue, the point of attachment of the linker to the adjuvant core is from 3 Å to 10 Å from a methine carbon atom of a side chain of the valine residue and/or the point of attachment of the linker to the adjuvant core is from 3 Å to 10 Å from a nitrogen atom of a side chain of the lysine residue, and preferably the point of attachment of the linker to the adjuvant core is from 3 Å to 7 Å from the methine carbon atom of the side chain of the valine residue and the point

of attachment of the linker to the adjuvant core is from 3 Å to 7 Å from the nitrogen atom of the side chain of the lysine residue.

**12.** The immunoconjugate of claim 11, wherein the valine residue is Val355 and the lysine residue is Lys432.

**13.** The immunoconjugate of any one of claims 1-12, wherein the antigen binding domain binds to an antigen selected from the group consisting ofHER2, EGFR, PD-L1, CEA CDH1, CD 19, CD20, CD29, CD30, CD40, CD47, EpCAM, SLAMF7, PDGFRa, gp75, MSLN, CA6, CA9, CDH6, CTAG1B/NY-ESO-1, LAMP1, LeY, MAGEA3/A6, P-cadherin, BCMA, CD38, HLA-DR, ROR1, WT1, GFRA1, FR-alpha, LI-CAM, LRRC15, MUC1, MUC16, PSMA, SLC34A2, TROP2, GPC3, CCR8, and VEGF, and preferably HER2, EGFR, PD-L1, and CEA.

**14.** The immunoconjugate of any one of claims 1-12, wherein the antibody construct is an antibody, preferably selected from the group consisting of olaratumab, obinutuzumab, trastuzumab, cetuximab, rituximab, pertuzumab, bevacizumab, daratumumab, etanercept, pembrolizumab, nivolumab, atezolizumab, ipilimumab, panitumumab, zalutumumab, nimotuzumab, matuzumab, and elotuzumab.

**15.** A composition comprising a plurality of immunoconjugates according to any one of claims 1-14, optionally further comprising one or more pharmaceutically acceptable excipients.

**16.** An immunoconjugate according to any one of claims 1-14 or a composition according to claim 15 for use in treating cancer.

**Patentansprüche**

**1.** Ein Immunkonjugat, umfassend:

(a) ein Antikörperkonstrukt, das (i) eine Antigenbindungsdomäne und (ii) eine Fc-Domäne umfasst und
(b) 1-8 Adjuvanskerne, wobei jeder Adjuvanskern über einen Linker kovalent an das Antikörperkonstrukt gebunden ist, wobei jeder Adjuvanskern eine 2-Amino-Stickstoffeinheit mit einem anhängenden Stickstoffatom und einen Anbindungspunkt des Linkers an den Adjuvanskern umfasst, und wobei der Abstand zwischen dem anhängenden Stickstoffatom und dem Anbindungspunkt des Linkers größer als 5 Å ist,
wobei

das Immunkonjugat die Formel aufweist:

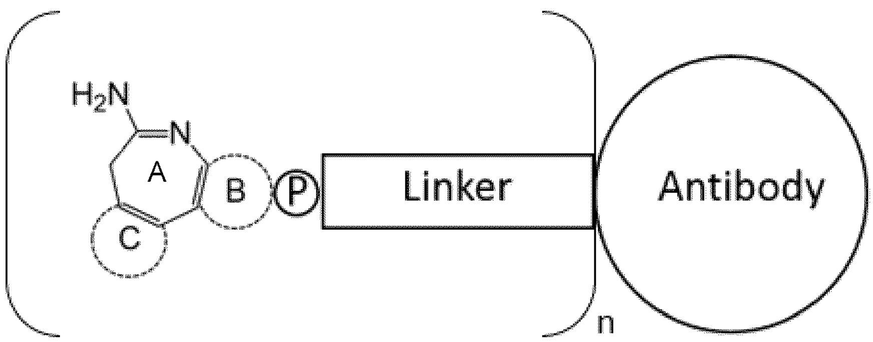

Immunkonjugat D2,

wobei der Adjuvanskern durch kondensierte Ringe A, B und C dargestellt wird, wobei A und B vorhanden sind und C gegebenenfalls vorhanden ist, und B und C 5-, 6- oder 7-gliedrige Ringe bezeichnen, die gegebenenfalls Doppelbindungen umfassen und gegebenenfalls Heteroatome zusätzlich zu der 2-Amino-Stickstoffeinheit umfassen, und der Adjuvanskern gegebenenfalls substituiert ist, so dass das Substitutionsmuster um den Adjuvanskern herum nicht besonders begrenzt ist, solange die 2-Amino-Stickstoffeinheit unsubstituiert bleibt, P den Anbindungspunkt des Linkers an den B-Ring darstellt und n eine ganze Zahl von 1 bis 8 ist, oder

das Immunkonjugat die Formel aufweist:

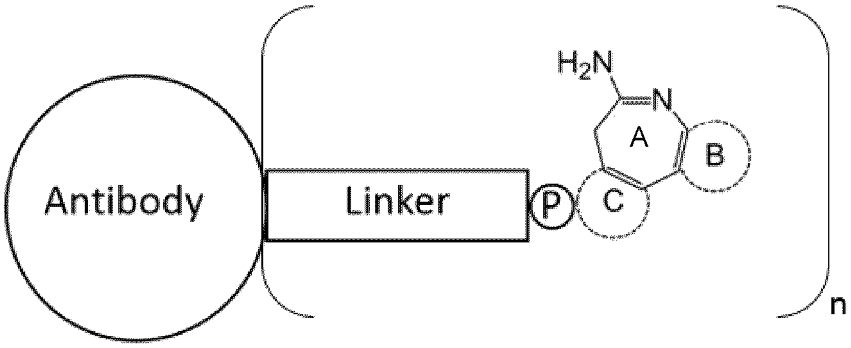

Immunkonjugat D4,

wobei der Adjuvanskern durch kondensierte Ringe A, B und C dargestellt wird, wobei A und C vorhanden sind und B gegebenenfalls vorhanden ist und B und C 5-, 6- oder 7-gliedrige Ringe bezeichnen, die gegebenenfalls Doppelbindungen umfassen und gegebenenfalls Heteroatome zusätzlich zu der 2-Amino-Stickstoffeinheit umfassen, und der Adjuvanskern gegebenenfalls substituiert ist, so dass das Substitutionsmuster um den Adjuvanskern herum nicht besonders begrenzt ist, solange die 2-Amino-Stickstoffeinheit unsubstituiert bleibt, P den Anbindungspunk des Linkers an den C-Ring darstellt und n eine ganze Zahl von 1 bis 8 ist.

2. Das Immunkonjugat nach Anspruch 1, wobei der Abstand zwischen dem anhängenden Stickstoffatom und dem Anbindungspunkt des Linkers größer als 5,5 Å, und vorzugsweise der Abstand zwischen dem anhängenden Stickstoffatom und dem Anbindungspunkt des Linkers größer als 6 Å ist.

3. Das Immunkonjugat nach Anspruch 1 oder 2, wobei, wenn es an eine Bindungsdomäne eines TLR8 gebunden ist, die einen Asparaginsäurerest umfasst, das anhängende Stickstoffatom der 2-Amino-Stickstoffeinheit weniger als 5 Å von einem Carbonylsauerstoff einer sauren Seitenkette des Asparaginsäurerestes entfernt ist.

4. Das Immunkonjugat nach Anspruch 3, wobei der Asparaginsäurerest Asp543 ist.

5. Das Immunkonjugat nach Anspruch 3 oder 4, wobei das anhängende Stickstoffatom der 2-Amino-Stickstoffeinheit weniger als 3 Å vom Carbonylsauerstoff der sauren Seitenkette des Asparaginsäurerests entfernt ist.

6. Das Immunkonjugat nach einem der Ansprüche 1-5, wobei, wenn es an eine Bindungsdomäne eines TLR7 gebunden ist, die einen Asparaginsäurerest umfasst, das anhängende Stickstoffatom der 2-Amino-Stickstoffeinheit weniger als 5 Å von einem Carbonylsauerstoff einer sauren Seitenkette des Asparaginsäurerestes entfernt ist.

7. Das Immunkonjugat nach Anspruch 6, wobei der Asparaginsäurerest Asp555 ist.

8. Das Immunkonjugat nach Anspruch 6 oder 7, wobei das anhängende Stickstoffatom der 2-Amino-Stickstoffeinheit weniger als 3 Å vom Carbonylsauerstoff der sauren Seitenkette des Asparaginsäurerests entfernt ist.

9. Das Immunkonjugat nach einem der Ansprüche 1-8, wobei, wenn es an die Bindungsdomäne eines TLR8 gebunden ist, die einen Arginin- und einen Serinrest umfasst, der Anbindungspunkt des Linkers an den Adjuvanskern von 3 Å bis 10 Å von einem Sauerstoffatom einer Seitenkette des Serinrestes und/oder der Anbindungspunkt des Linkers an den Adjuvanskern von 3 Å bis 10 Å von einem Stickstoffatom einer Seitenkette des Argininrestes entfernt ist, und vorzugsweise der Anbindungspunkt des Linkers an den Adjuvanskern 3 Å bis 7 Å von dem Sauerstoffatom der Seitenkette des Serinrestes entfernt ist und der Anbindungspunkt des Linkers an den Adjuvanskern ist 3 Å bis 7 Å von dem Stickstoffatom der Seitenkette des Argininrestes entfernt ist.

10. Das Immunkonjugat nach Anspruch 9, wobei der Serinrest Ser352 und der Argininrest Arg429 ist.

11. Das Immunkonjugat nach einem der Ansprüche 1-10, wobei, wenn es an die Bindungsdomäne eines TLR7 gebunden ist, die einen Lysin- und Valinrest umfasst, der Anbindungspunkt des Linkers an den Adjuvanskern von 3 Å bis 10 Å

von einem Methinkohlenstoffatom einer Seitenkette des Valinrestes und/oder der Anbindungspunkt des Linkers an den Adjuvanskern von 3 Å bis 10 Å von einem Stickstoffatom einer Seitenkette des Lysinrestes entfernt ist, und vorzugsweise der Anbindungspunkt des Linkers an den Adjuvanskern 3 Å bis 7 Å von dem Methinkohlenstoffatom der Seitenkette des Valinrestes entfernt ist und der Anbindungspunkt des Linkers an den Adjuvanskern 3 Å bis 7 Å von dem Stickstoffatom der Seitenkette des Lysinrestes entfernt ist.

**12.** Das Immunkonjugat nach Anspruch 11, wobei der Valinrest Val355 und der Lysinrest Lys432 ist.

**13.** Das Immunkonjugat nach einem der Ansprüche 1-12, wobei die Antigenbindungsdomäne an ein Antigen bindet, das aus der Gruppe ausgewählt ist, die aus HER2, EGFR, PD-L1, CEA CDH1, CD 19, CD20, CD29, CD30, CD40, CD47, EpCAM, SLAMF7, PDGFRa, gp75, MSLN, CA6, CA9, CDH6, CTAG1B/NY-ESO-1, LAMP1, LeY, MAGEA3/A6, P-Cadherin, BCMA, CD38, HLA-DR, ROR1, WT1, GFRA1, FR-alpha, LI-CAM, LRRC15, MUC1, MUC16, PSMA, SLC34A2, TROP2, GPC3, CCR8 und VEGF, und vorzugsweise aus HER2, EGFR, PD-L1 und CEA, besteht.

**14.** Das Immunkonjugat nach einem der Ansprüche 1-12, wobei das Antikörperkonstrukt ein Antikörper ist, vorzugsweise ausgewählt aus der Gruppe bestehend aus Olaratumab, Obinutuzumab, Trastuzumab, Cetuximab, Rituximab, Pertuzumab, Bevacizumab, Daratumumab, Etanercept, Pembrolizumab, Nivolumab, Atezolizumab, Ipilimumab, Panitumumab, Zalutumumab, Nimotuzumab, Matuzumab und Elotuzumab.

**15.** Eine Zusammensetzung, die eine Vielzahl von Immunkonjugaten nach einem der Ansprüche 1 bis 14 umfasst, die gegebenenfalls weiterhin einen oder mehrere pharmazeutisch akzeptable Hilfsstoffe umfasst.

**16.** Ein Immunkonjugat nach einem der Ansprüche 1 bis 14 oder eine Zusammensetzung nach Anspruch 15 zur Verwendung bei der Behandlung von Krebs.

**Revendications**

**1.** Immunoconjugué comprenant :

(a) une construction d'anticorps comprenant (i) un domaine de liaison d'antigène et (ii) un domaine Fc et
(b) 1 à 8 noyaux d'adjuvant, sachant que chaque noyau d'adjuvant est lié de manière covalente à la construction d'anticorps via un lieur, sachant que chaque noyau d'adjuvant comprend un groupe fonctionnel amino-2 azote avec un atome d'azote pendant et un point d'attache du lieur au noyau d'adjuvant, et sachant que la distance entre l'atome d'azote pendant et le point d'attache du lieur est supérieure à 5 Å,
sachant que

l'immunoconjugué est de formule :

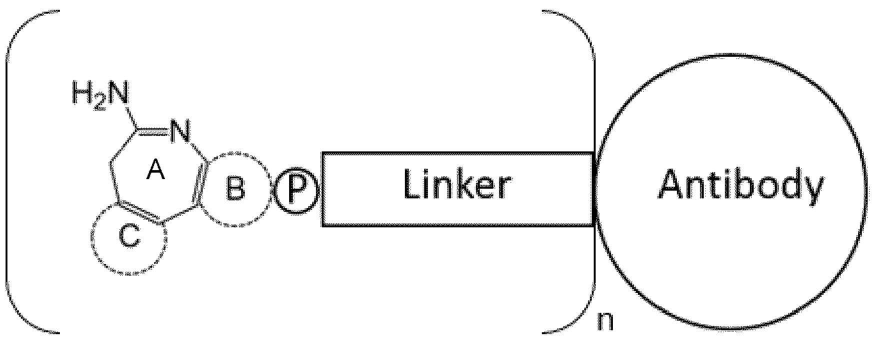

Immunoconjugué D2,

sachant que le noyau d'adjuvant est représenté par des cycles condensés A, B et C, sachant que A et B sont présents et C est présent facultativement, et B et C désignent des cycles à 5, 6, ou 7 chaînons, comprenant facultativement des liaisons doubles, et comprenant facultativement des hétéroatomes en plus du groupe fonctionnel amino-2 azote, et le noyau d'adjuvant est facultativement substitué de telle sorte que le motif de

substitution autour du noyau d'adjuvant ne soit pas particulièrement limité, tant que le groupe fonctionnel amino-2 azote reste non substitué, P représente le point d'attache du lieur au cycle B, et n est un entier de 1 à 8, ou

l'immunoconjugué est de formule :

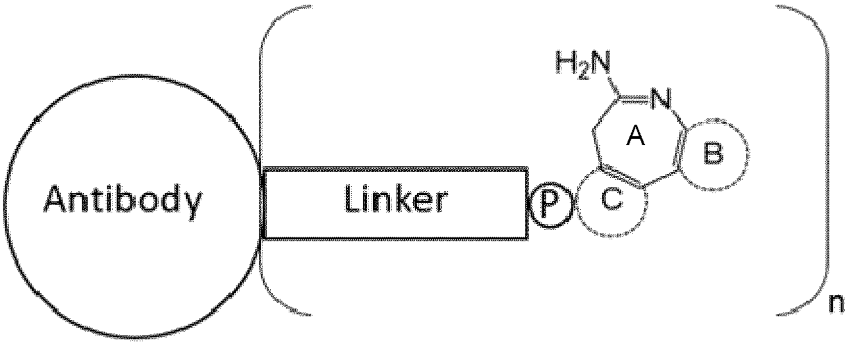

Immunoconjugué D4,

sachant que le noyau d'adjuvant est représenté par des cycles condensés A, B et C, sachant que A et C sont présents et B est présent facultativement, et B et C désignent des cycles à 5, 6, ou 7 chaînons, comprenant facultativement des liaisons doubles, et comprenant facultativement des hétéroatomes en plus du groupe fonctionnel amino-2 azote, et le noyau d'adjuvant est facultativement substitué de telle sorte que le motif de substitution autour du noyau d'adjuvant ne soit pas particulièrement limité, tant que le groupe fonctionnel amino-2 azote reste non substitué, P représente le point d'attache du lieur au cycle C, et n est un entier de 1 à 8.

**2.** L'immunoconjugué de la revendication 1, sachant que la distance entre l'atome d'azote pendant et le point d'attache du lieur est supérieure à 5,5 Å, et de préférence la distance entre l'atome d'azote pendant et le point d'attache du lieur est supérieure à 6 Å.

**3.** L'immunoconjugué de la revendication 1 ou 2, sachant que lorsqu'il est lié à un domaine de liaison d'un TLR8 comprenant un résidu d'acide aspartique, l'atome d'azote pendant du groupe fonctionnel amino-2 azote est à moins de 5 Å d'un oxygène carbonylique d'une chaîne latérale acide du résidu d'acide aspartique.

**4.** L'immunoconjugué de la revendication 3, sachant que le résidu d'acide aspartique est Asp543.

**5.** L'immunoconjugué de la revendication 3 ou 4, sachant que l'atome d'azote pendant du groupe fonctionnel amino-2 azote est à moins de 3 Å de l'oxygène carbonylique de la chaîne latérale acide du résidu d'acide aspartique.

**6.** L'immunoconjugué de l'une quelconque des revendications 1 à 5, sachant que lorsqu'il est lié à un domaine de liaison d'un TLR7 comprenant un résidu d'acide aspartique, l'atome d'azote pendant du groupe fonctionnel amino-2 azote est à moins de 5 Å d'un oxygène carbonylique d'une chaîne latérale acide du résidu d'acide aspartique.

**7.** L'immunoconjugué de la revendication 6, sachant que le résidu d'acide aspartique est Asp555.

**8.** L'immunoconjugué de la revendication 6 ou 7, sachant que l'atome d'azote pendant du groupe fonctionnel amino-2 azote est à moins de 3 Å de l'oxygène carbonylique de la chaîne latérale acide du résidu d'acide aspartique.

**9.** L'immunoconjugué de l'une quelconque des revendications 1 à 8, sachant que lorsqu'il est lié au domaine de liaison d'un TLR8 comprenant un résidu d'arginine et de serine, le point d'attache du lieur au noyau d'adjuvant est à de 3 Å à 10 Å d'un atome d'oxygène d'une chaîne latérale du résidu de serine et/ou le point d'attache du lieur au noyau d'adjuvant est à de 3 Å à 10 Å d'un atome d'azote d'une chaîne latérale du résidu d'arginine, et de préférence le point d'attache du lieur au noyau d'adjuvant est à de 3 Å à 7 Å de l'atome d'oxygène de la chaîne latérale du résidu de serine et le point d'attache du lieur au noyau d'adjuvant est à de 3 Å à 7 Å de l'atome d'azote de la chaîne latérale du résidu d'arginine.

**10.** L'immunoconjugué de la revendication 9, sachant que le résidu de serine est Ser352 et le résidu d'arginine est Arg429.

**11.** L'immunoconjugué de l'une quelconque des revendications 1 à 10, sachant que lorsqu'il est lié au domaine de liaison d'un TLR7 comprenant un résidu de lysine et de valine, le point d'attache du lieur au noyau d'adjuvant est à de 3 Å à 10 Å d'un atome de carbone méthinique d'une chaîne latérale du résidu de valine et/ou le point d'attache du lieur au noyau d'adjuvant est à de 3 Å à 10 Å d'un atome d'azote d'une chaîne latérale du résidu de lysine, et de préférence le point d'attache du lieur au noyau d'adjuvant est à de 3 Å à 7 Å de l'atome de carbone méthinique de la chaîne latérale du résidu de valine et le point d'attache du lieur au noyau d'adjuvant est à de 3 Å à 7 Å de l'atome d'azote de la chaîne latérale du résidu de lysine.

**12.** L'immunoconjugué de la revendication 11, sachant que le résidu de valine est Val355 et le résidu de lysine est Lys432.

**13.** L'immunoconjugué de l'une quelconque des revendications 1 à 12, sachant que le domaine de liaison d'antigène se lie à un antigène sélectionné dans le groupe constitué par HER2, EGFR, PD-L1, CEA CDH1, CD 19, CD20, CD29, CD30, CD40, CD47, EpCAM, SLAMF7, PDGFRa, gp75, MSLN, CA6, CA9, CDH6, CTAG1B/NY-ESO-1, LAMP1, LeY, MAGEA3/A6, P-cadherin, BCMA, CD38, HLA-DR, ROR1, WT1, GFRA1, FR-alpha, LI-CAM, LRRC15, MUC1, MUC16, PSMA, SLC34A2, TROP2, GPC3, CCR8, et VEGF, et de préférence HER2, EGFR, PD-L1, et CEA.

**14.** L'immunoconjugué de l'une quelconque des revendications 1 à 12, sachant que la construction d'anticorps est un anticorps, de préférence sélectionné dans le groupe constitué par l'olaratumab, l'obinutuzumab, le trastuzumab, le cetuximab, le rituximab, le pertuzumab, le bevacizumab, le daratumumab, l'etanercept, le pembrolizumab, le nivolumab, l'atezolizumab, l'ipilimumab, le panitumumab, le zalutumumab, le nimotuzumab, le matuzumab, et l'elotuzumab.

**15.** Composition comprenant une pluralité d'immunoconjugués selon l'une quelconque des revendications 1 à 14, comprenant en outre facultativement un ou plusieurs excipients pharmaceutiquement acceptables.

**16.** Immunoconjugué selon l'une quelconque des revendications 1 à 14 ou composition selon la revendication 15 pour utilisation dans le traitement du cancer.

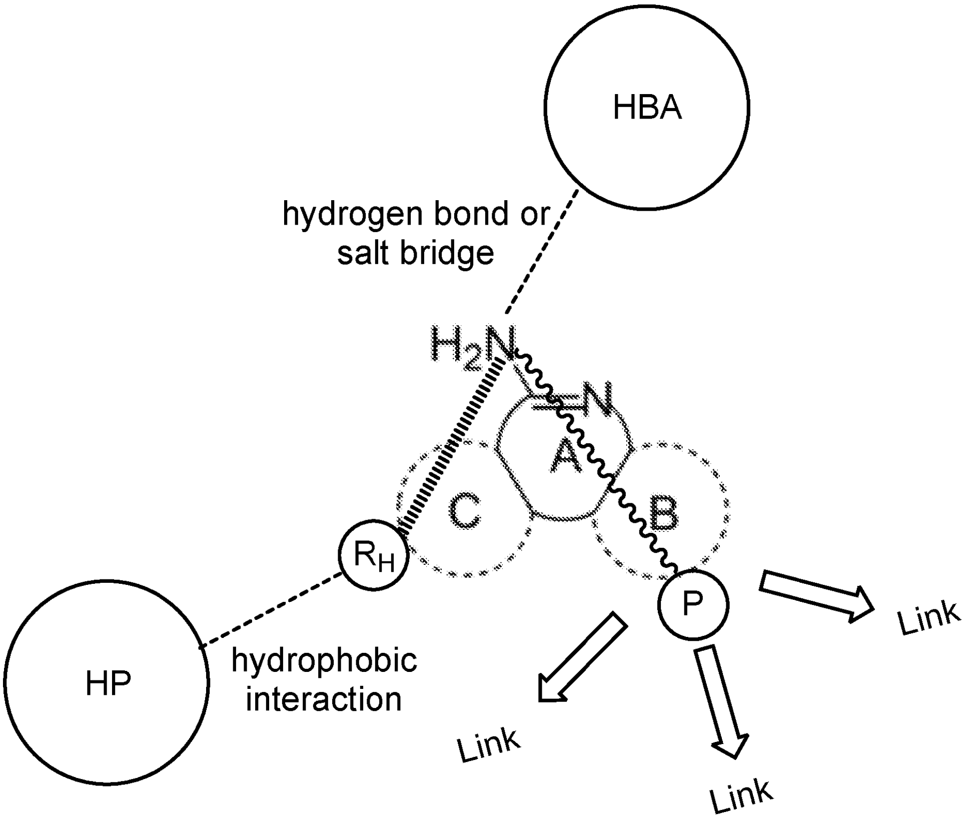
HBA
hydrogen bond or salt bridge
H2N
N
A
C
B
RH
P
HP
hydrophobic interaction
Link
Link
Link

FIG. 1

HCC1954

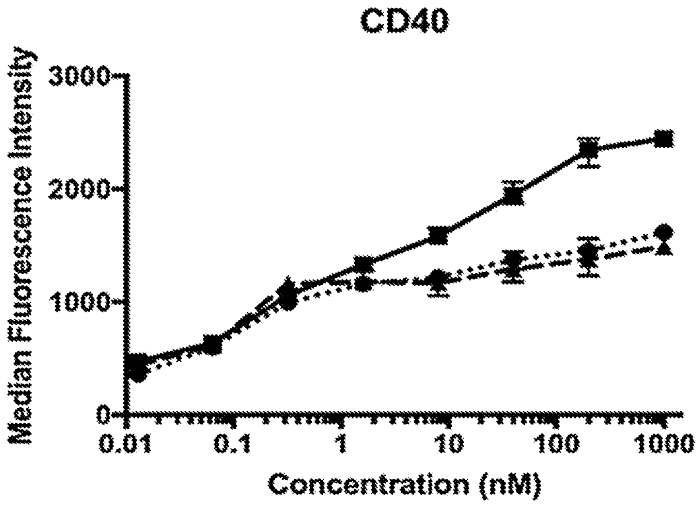


FIG. 2A

HCC1954

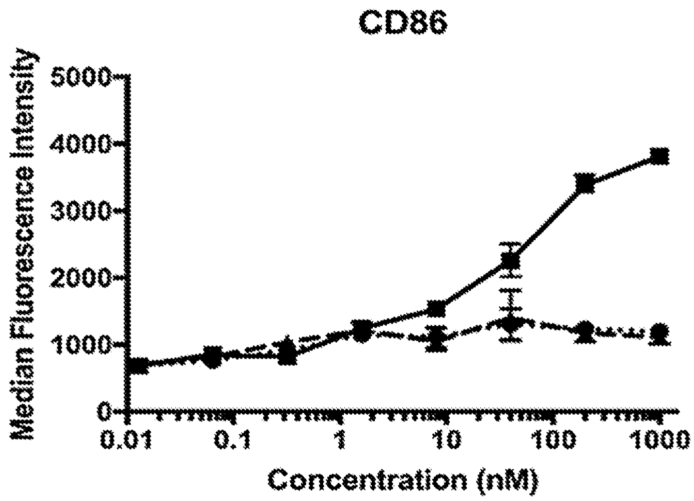


FIG. 2B

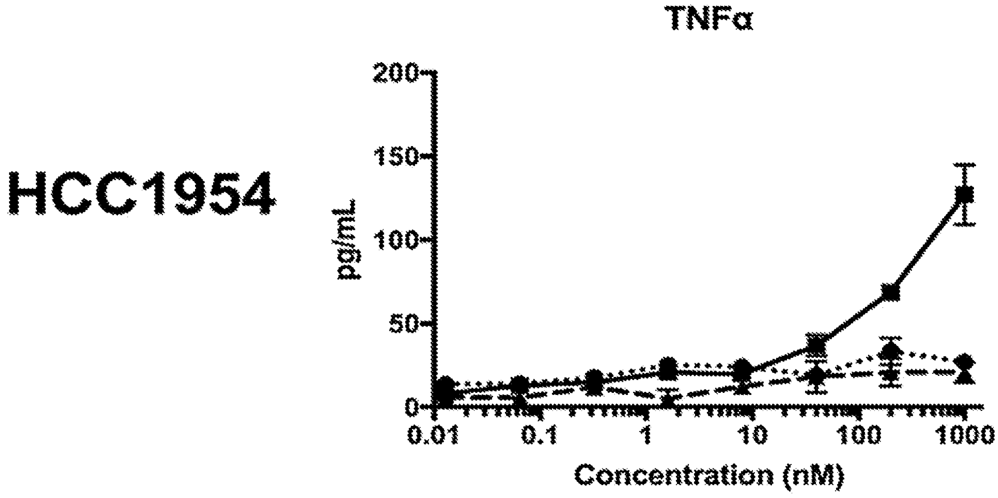
HCC1954
TNFα
pg/mL
200
150
100
50
0
0.01
0.1
1
10
100
1000
Concentration (nM)

FIG. 2C

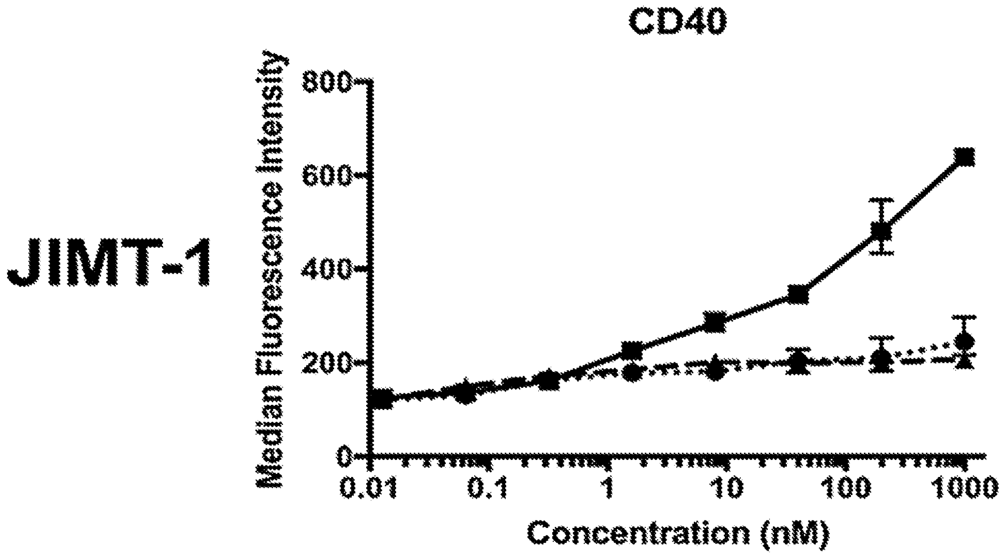
JIMT-1
CD40
Median Fluorescence Intensity
800
600
400
200
0
0.01
0.1
1
10
100
1000
Concentration (nM)

FIG. 2D

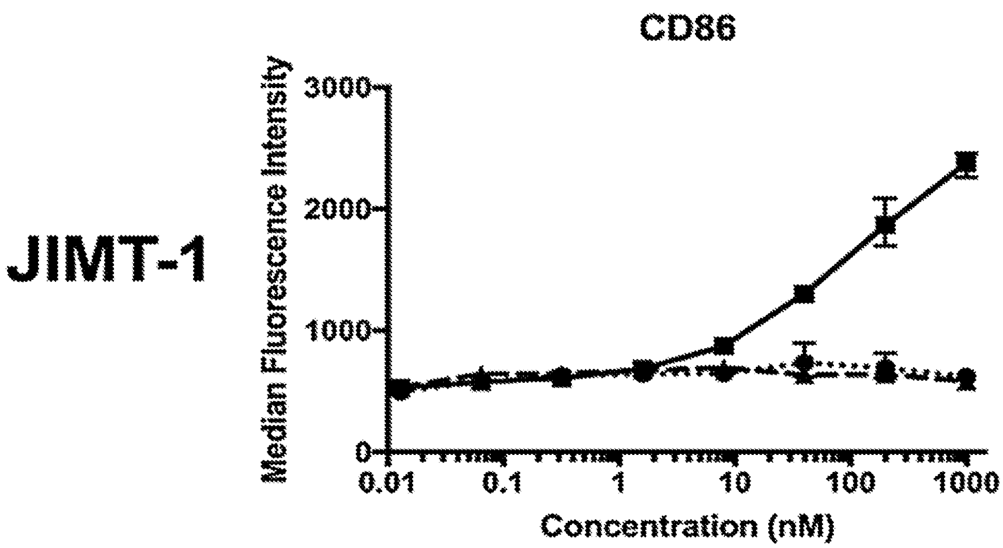
JIMT-1
CD86
Median Fluorescence Intensity
3000
2000
1000
0
0.01
0.1
1
10
100
1000
Concentration (nM)

FIG. 2E

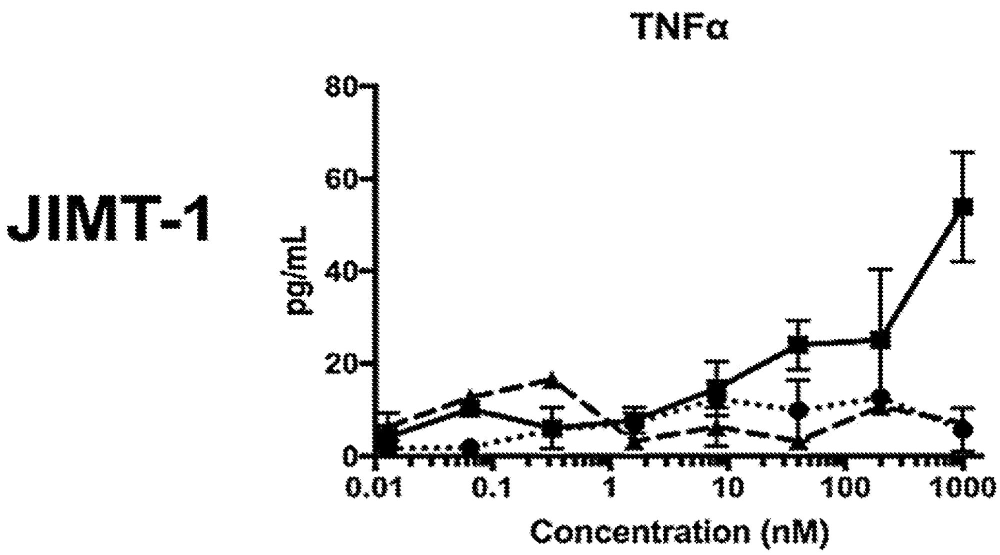
JIMT-1
TNFα
pg/mL
80
60
40
20
0
0.01
0.1
1
10
100
1000
Concentration (nM)

FIG. 2F

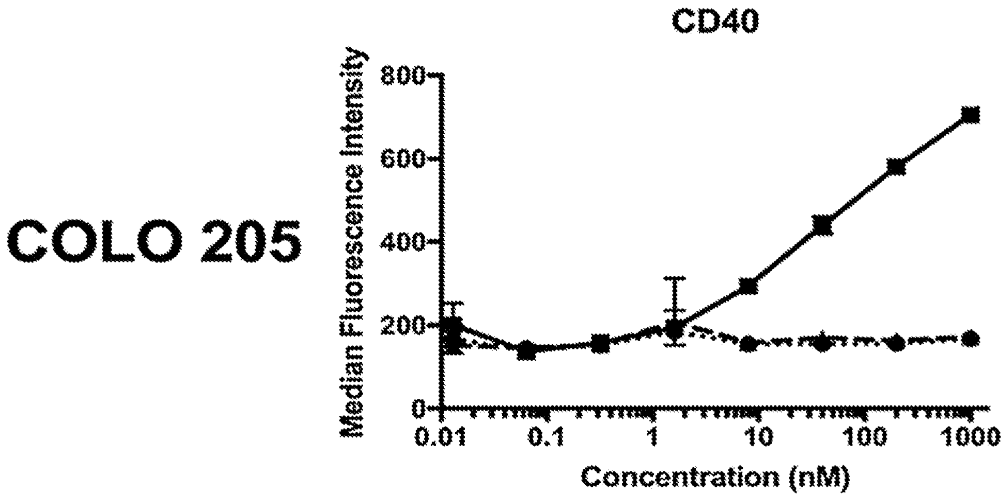
COLO 205
CD40
Median Fluorescence Intensity
800
600
400
200
0
0.01
0.1
1
10
100
1000
Concentration (nM)

FIG. 2G

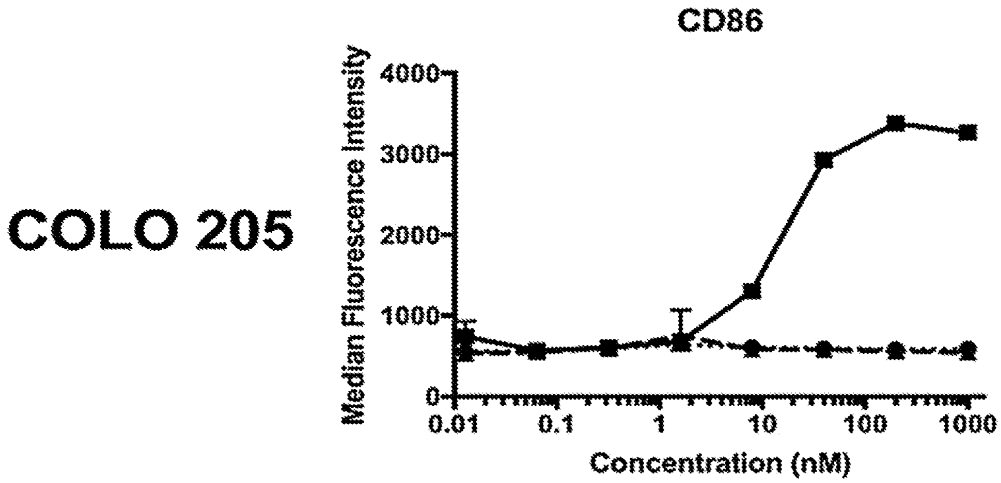
COLO 205
CD86
Median Fluorescence Intensity
4000
3000
2000
1000
0
0.01
0.1
1
10
100
1000
Concentration (nM)

FIG. 2H

COLO 205

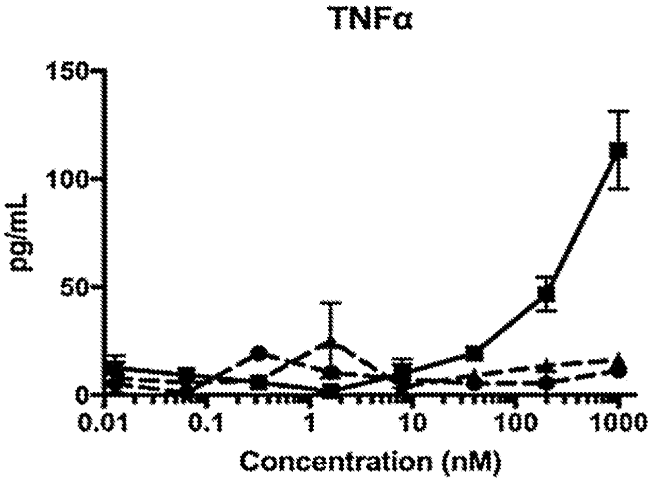


FIG. 2I

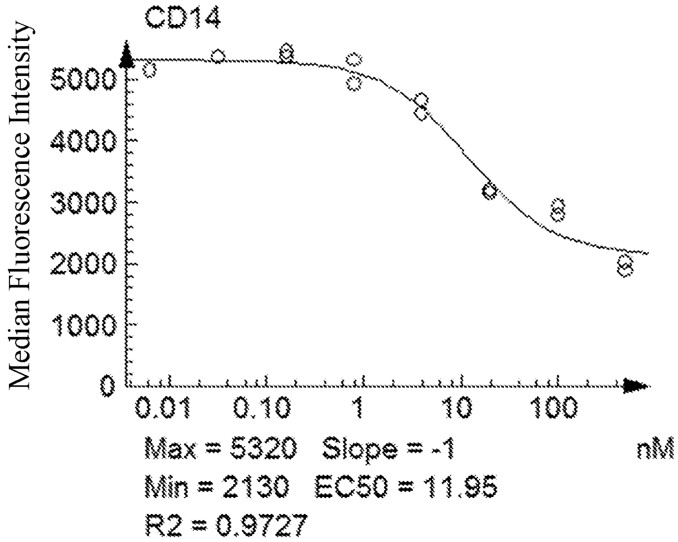
CD14
Median Fluorescence Intensity
5000
4000
3000
2000
1000
0
0.01
0.10
1
10
100
nM
Max = 5320 Slope = -1
Min = 2130 EC50 = 11.95
R2 = 0.9727

**FIG. 3A**

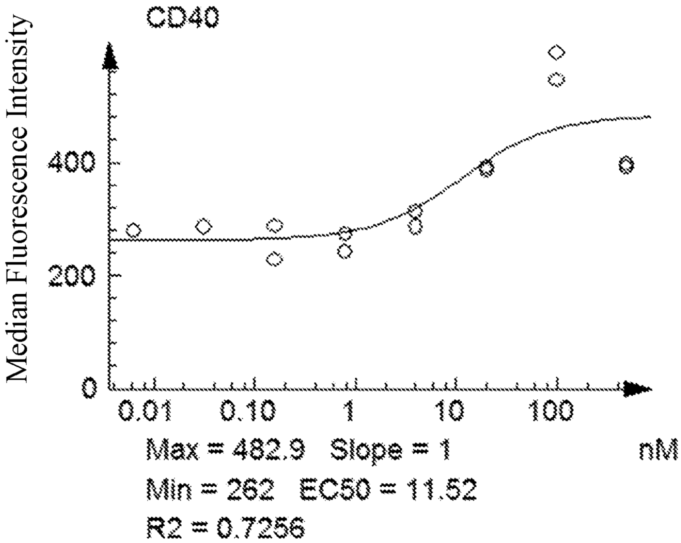
CD40
Median Fluorescence Intensity
400
200
0
0.01
0.10
1
10
100
nM
Max = 482.9 Slope = 1
Min = 262 EC50 = 11.52
R2 = 0.7256

**FIG. 3B**

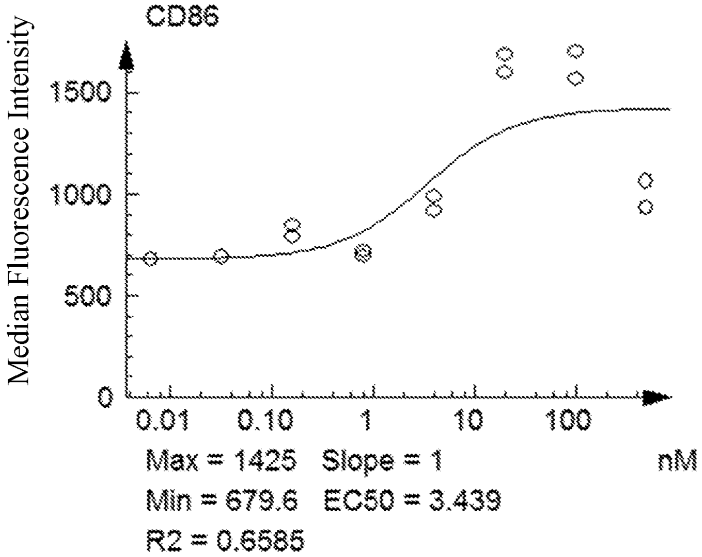
CD86
Median Fluorescence Intensity
1500
1000
500
0
0.01
0.10
1
10
100
nM
Max = 1425 Slope = 1
Min = 679.6 EC50 = 3.439
R2 = 0.6585

**FIG. 3C**

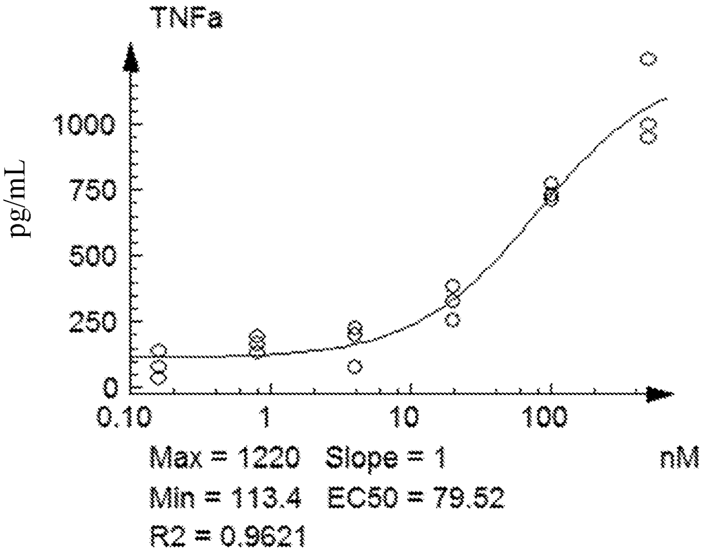
TNFa
pg/mL
1000
750
500
250
0
0.10
1
10
100
nM
Max = 1220 Slope = 1
Min = 113.4 EC50 = 79.52
R2 = 0.9621

**FIG. 3D**

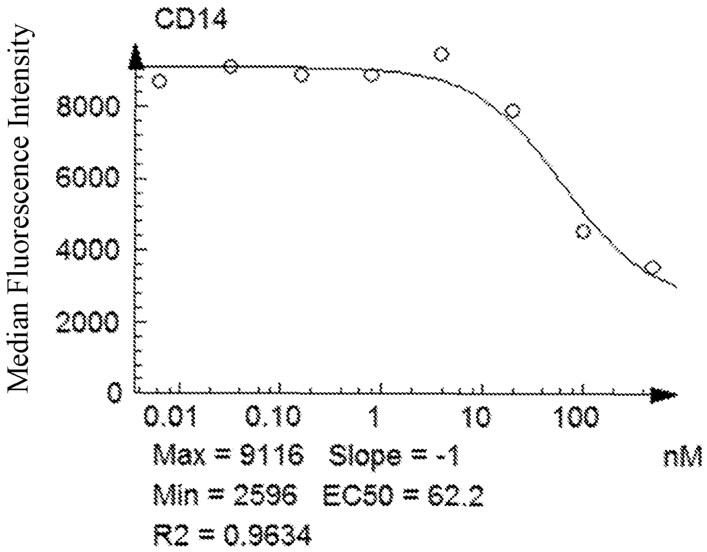
CD14
Median Fluorescence Intensity
8000
6000
4000
2000
0
0.01
0.10
1
10
100
nM
Max = 9116 Slope = -1
Min = 2596 EC50 = 62.2
R2 = 0.9634

**FIG. 4A**

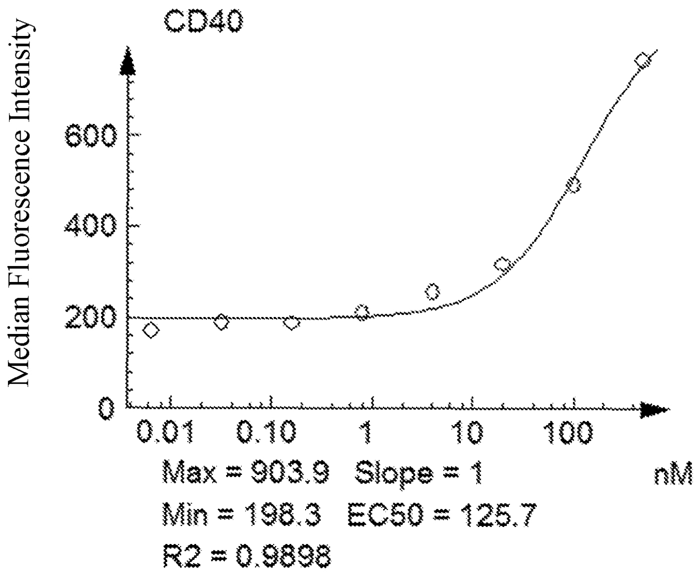
CD40
Median Fluorescence Intensity
600
400
200
0
0.01
0.10
1
10
100
nM
Max = 903.9 Slope = 1
Min = 198.3 EC50 = 125.7
R2 = 0.9898

**FIG. 4B**

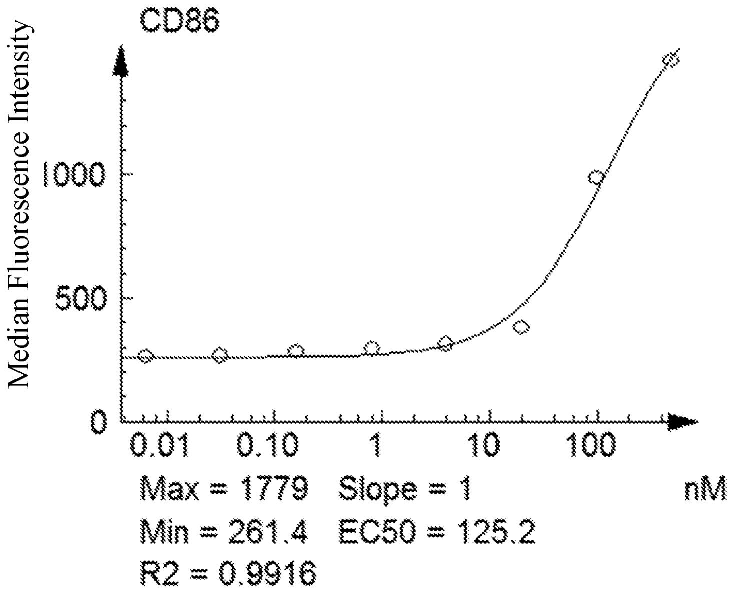
CD86
Median Fluorescence Intensity
1000
500
0
0.01
0.10
1
10
100
nM
Max = 1779 Slope = 1
Min = 261.4 EC50 = 125.2
R2 = 0.9916

**FIG. 4C**

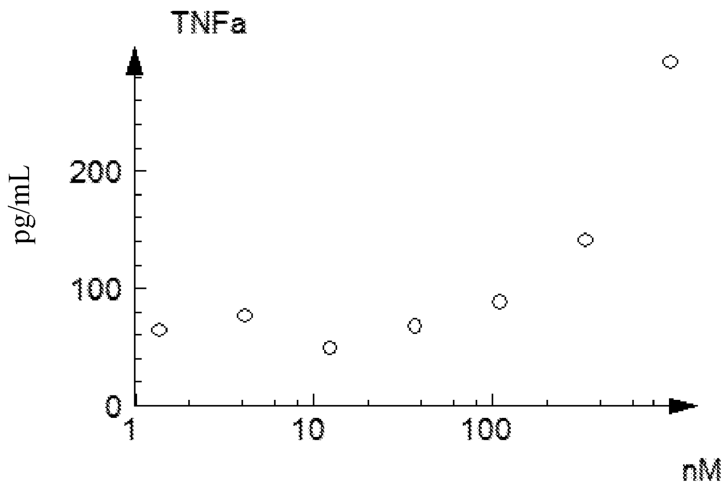
TNFa
pg/mL
200
100
0
1
10
100
nM

**FIG. 4D**

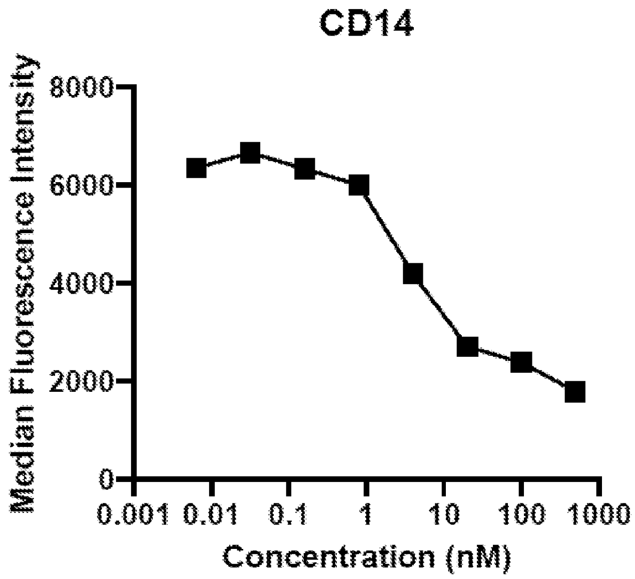
CD14
Median Fluorescence Intensity
8000
6000
4000
2000
0
0.001 0.01 0.1 1 10 100 1000
Concentration (nM)

Fig. 5A

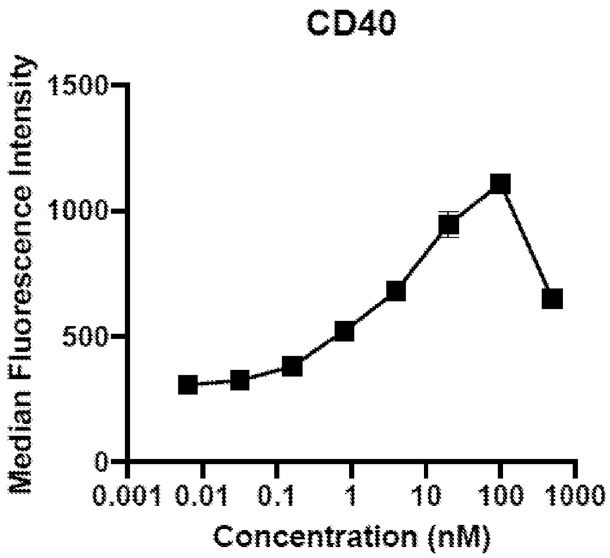
CD40
Median Fluorescence Intensity
1500
1000
500
0
0.001 0.01 0.1 1 10 100 1000
Concentration (nM)

Fig. 5B

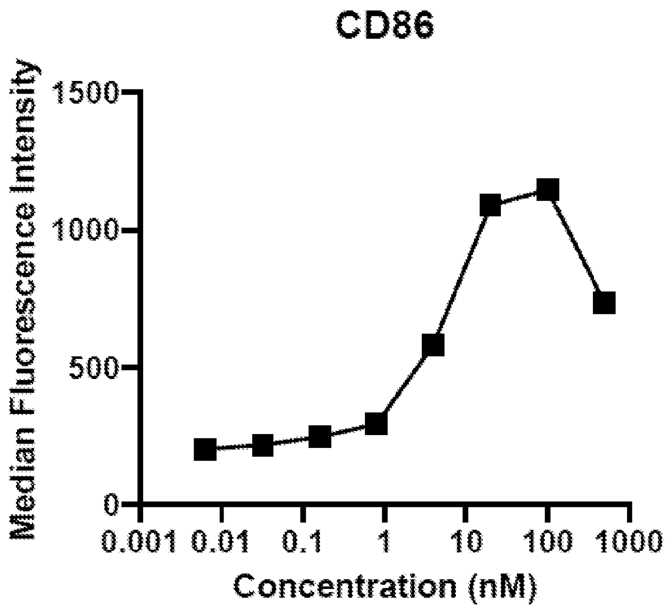
CD86
Median Fluorescence Intensity
1500
1000
500
0
0.001 0.01 0.1 1 10 100 1000
Concentration (nM)

Fig. 5C

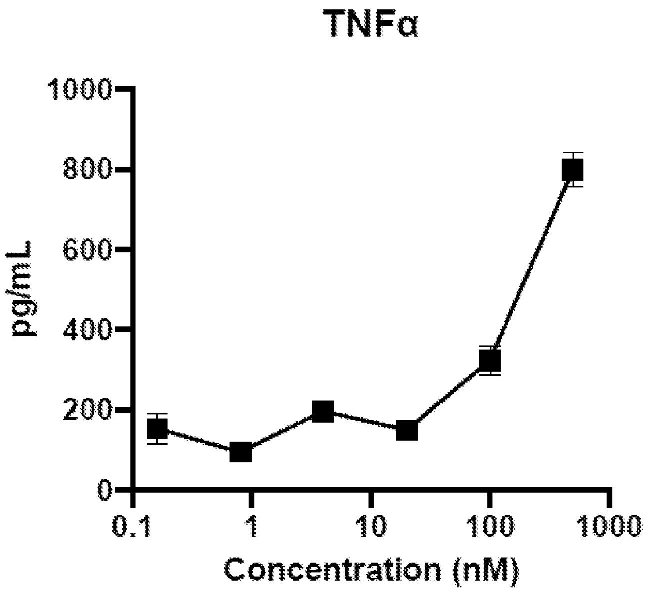
TNFα
pg/mL
1000
800
600
400
200
0
0.1 1 10 100 1000
Concentration (nM)

Fig. 5D

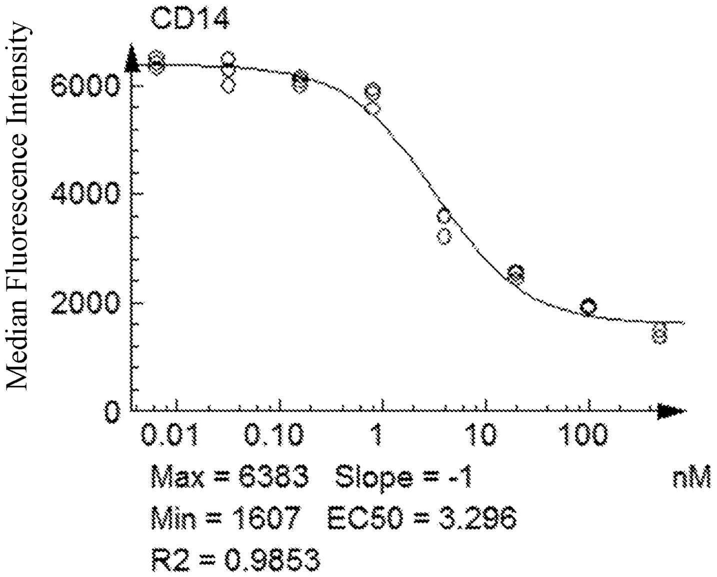
CD14
Median Fluorescence Intensity
6000
4000
2000
0
0.01
0.10
1
10
100
nM
Max = 6383 Slope = -1
Min = 1607 EC50 = 3.296
R2 = 0.9853

FIG. 6A

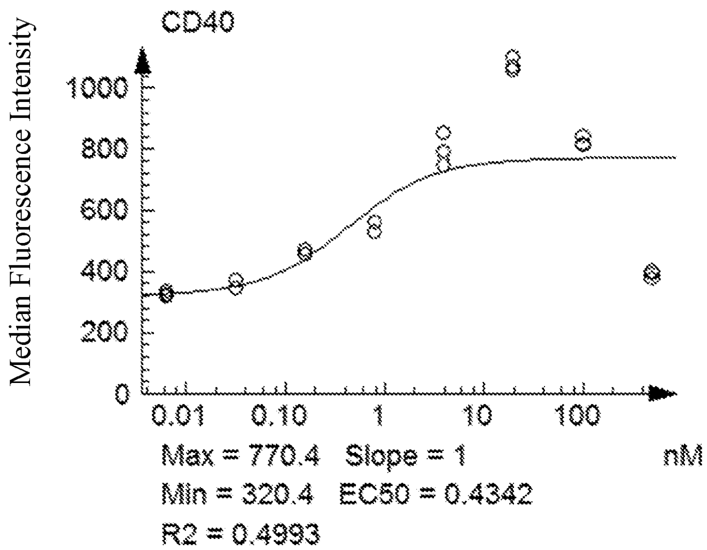
CD40
Median Fluorescence Intensity
1000
800
600
400
200
0
0.01
0.10
1
10
100
nM
Max = 770.4 Slope = 1
Min = 320.4 EC50 = 0.4342
R2 = 0.4993

FIG. 6B

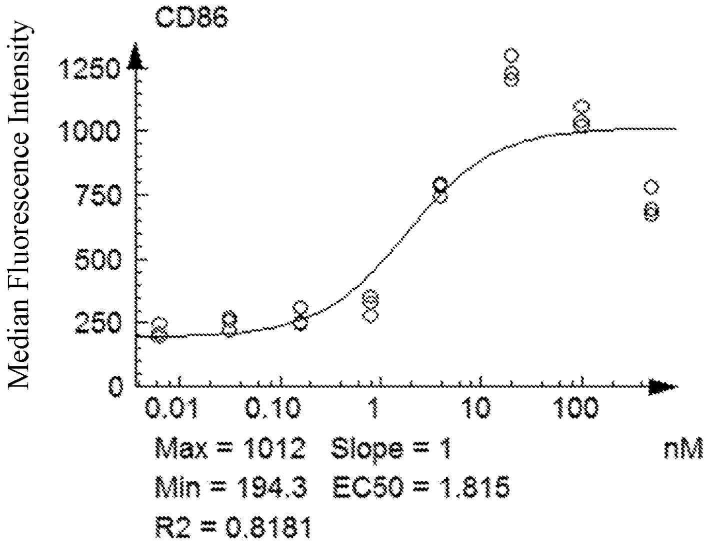
CD86
Median Fluorescence Intensity
1250
1000
750
500
250
0
0.01
0.10
1
10
100
nM
Max = 1012 Slope = 1
Min = 194.3 EC50 = 1.815
R2 = 0.8181

FIG. 6C

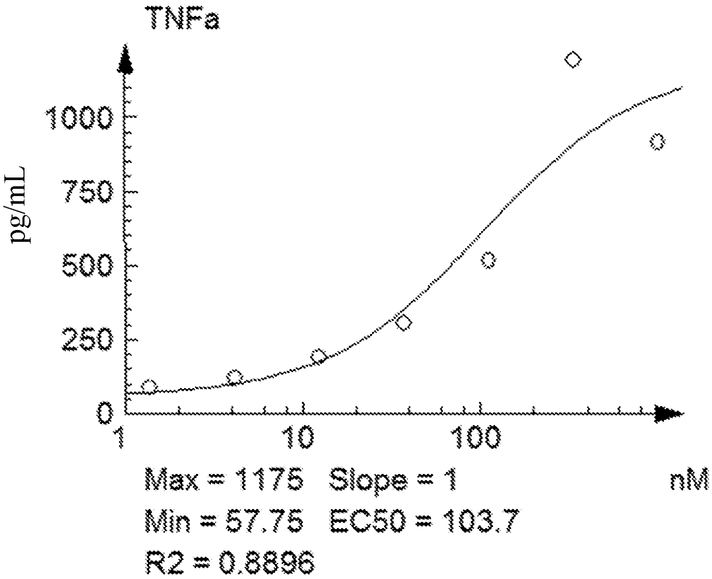
TNFa
pg/mL
1000
750
500
250
0
1
10
100
nM
Max = 1175 Slope = 1
Min = 57.75 EC50 = 103.7
R2 = 0.8896

FIG. 6D

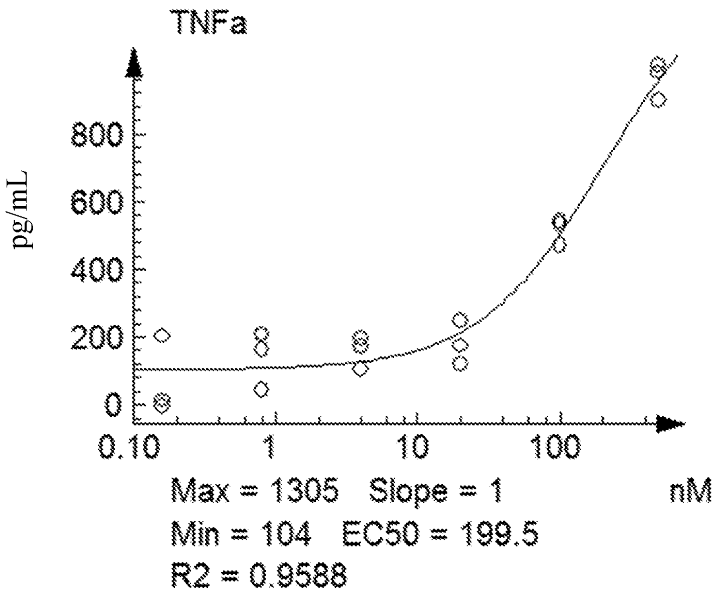
TNFa
pg/mL
800
600
400
200
0
0.10
1
10
100
nM
Max = 1305 Slope = 1
Min = 104 EC50 = 199.5
R2 = 0.9588

**FIG. 6E**

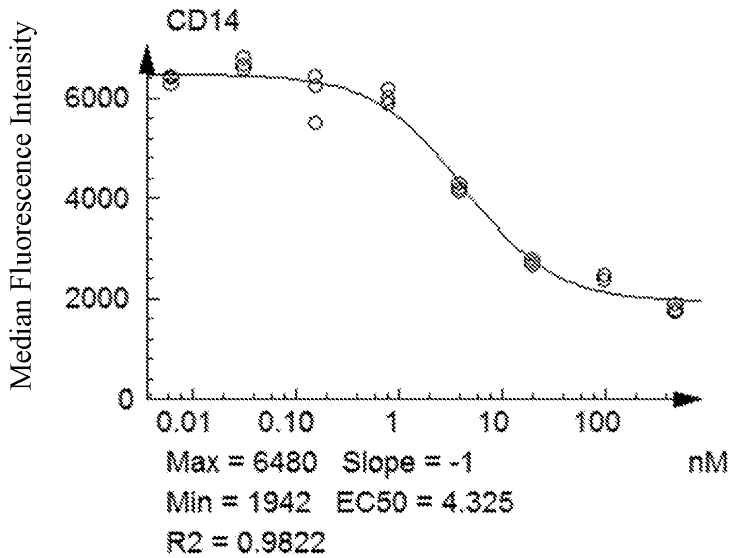
CD14
Median Fluorescence Intensity
6000
4000
2000
0
0.01
0.10
1
10
100
nM
Max = 6480 Slope = -1
Min = 1942 EC50 = 4.325
R2 = 0.9822

**FIG. 7A**

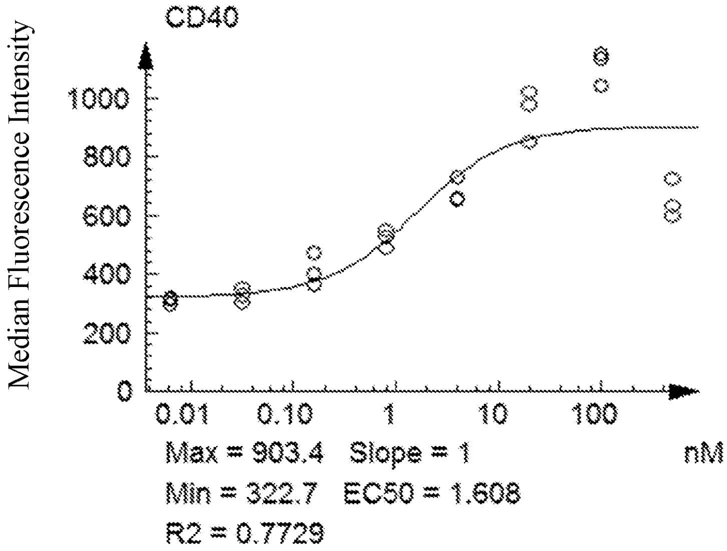
CD40
Median Fluorescence Intensity
0
200
400
600
800
1000
0.01
0.10
1
10
100
nM
Max = 903.4 Slope = 1
Min = 322.7 EC50 = 1.608
R2 = 0.7729

FIG. 7B

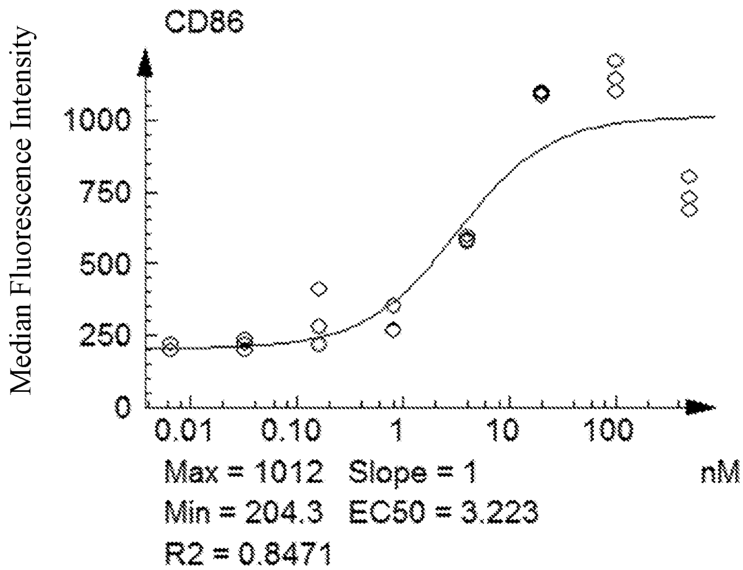
CD86
Median Fluorescence Intensity
0
250
500
750
1000
0.01
0.10
1
10
100
nM
Max = 1012 Slope = 1
Min = 204.3 EC50 = 3.223
R2 = 0.8471

FIG. 7C

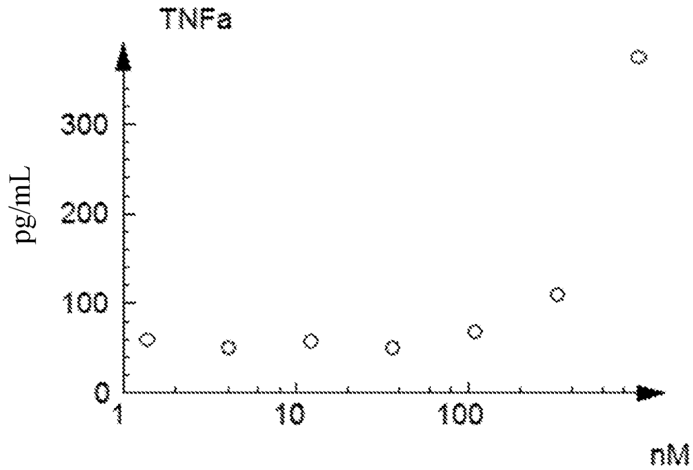
TNFa
pg/mL
300
200
100
0
1
10
100
nM

FIG. 7D

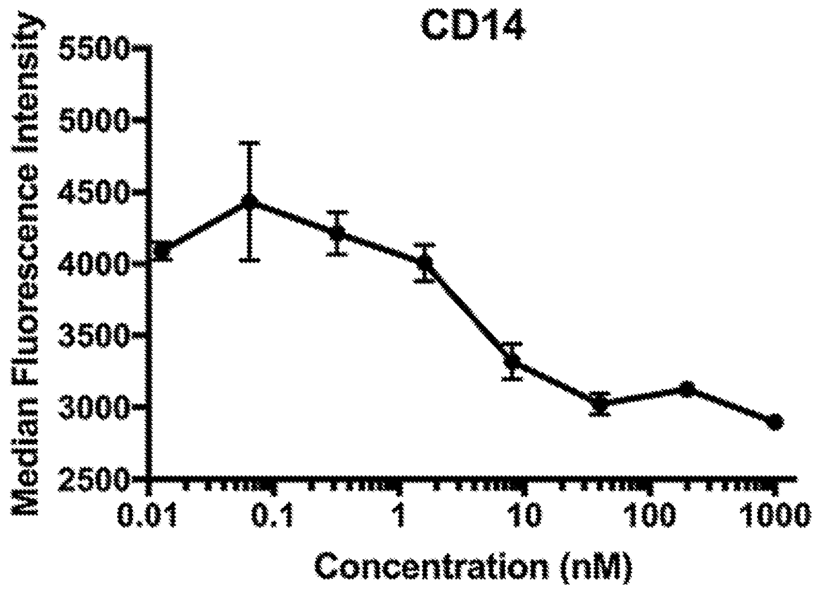
CD14
Median Fluorescence Intensity
5500
5000
4500
4000
3500
3000
2500
0.01
0.1
1
10
100
1000
Concentration (nM)

FIG. 8A

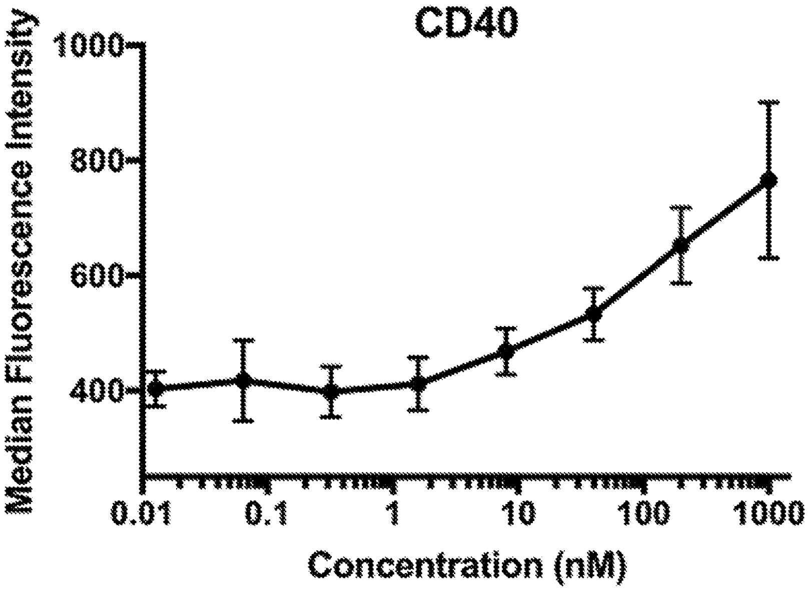
CD40
Median Fluorescence Intensity
1000
800
600
400
0.01
0.1
1
10
100
1000
Concentration (nM)

FIG. 8B

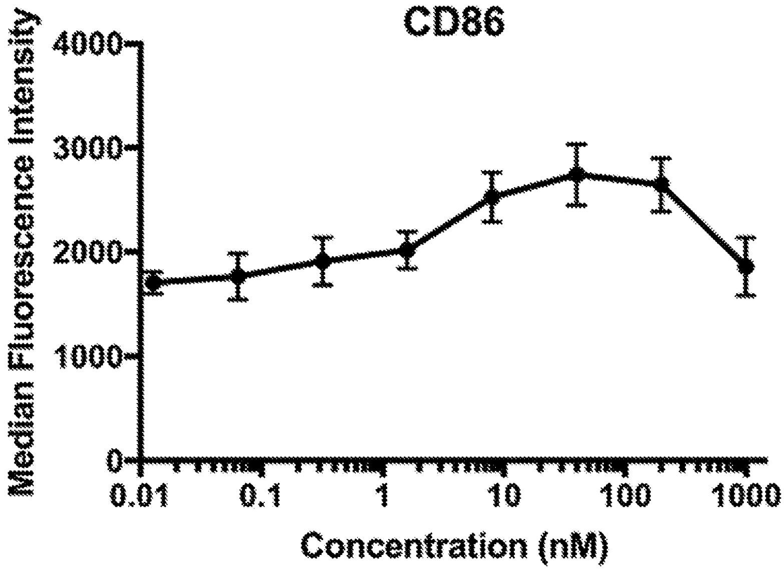
CD86
Median Fluorescence Intensity
4000
3000
2000
1000
0
0.01
0.1
1
10
100
1000
Concentration (nM)

FIG. 8C

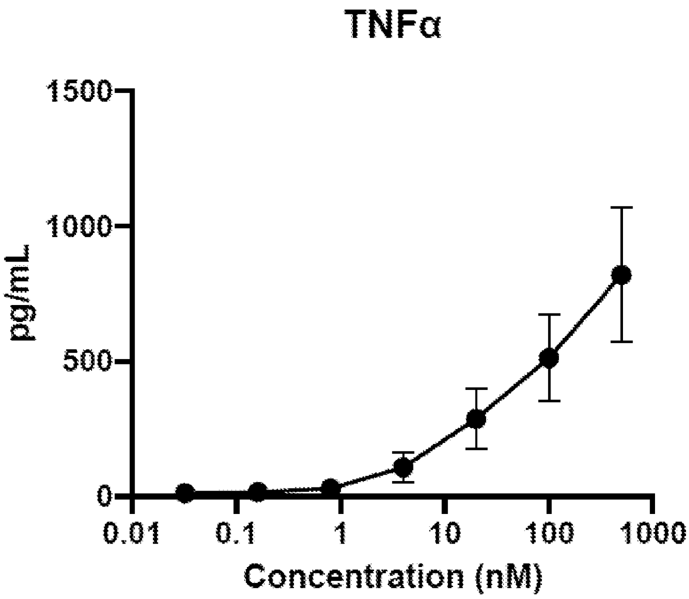
TNFα
1500
1000
500
0
pg/mL
0.01
0.1
1
10
100
1000
Concentration (nM)

FIG. 8D

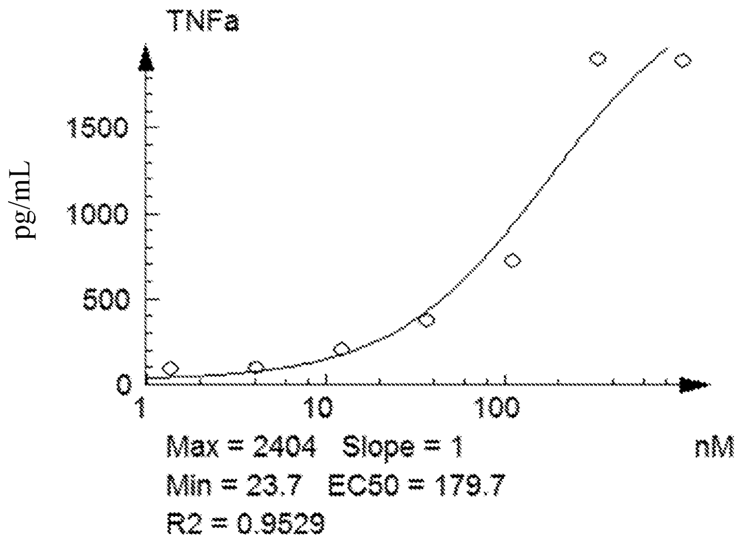
TNFa
1500
1000
500
0
pg/mL
1
10
100
nM
Max = 2404 Slope = 1
Min = 23.7 EC50 = 179.7
R2 = 0.9529

FIG. 9

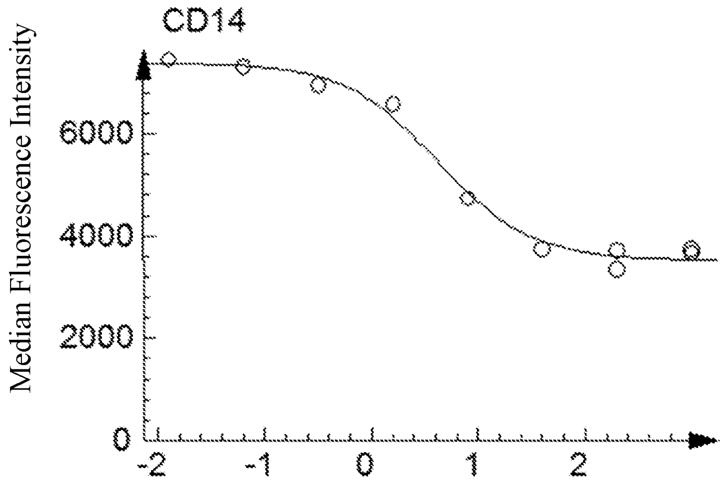


Max = 7386 Slope = -1
Min = 3521 EC50 = 4.149
R2 = 0.9897

**FIG. 10A**

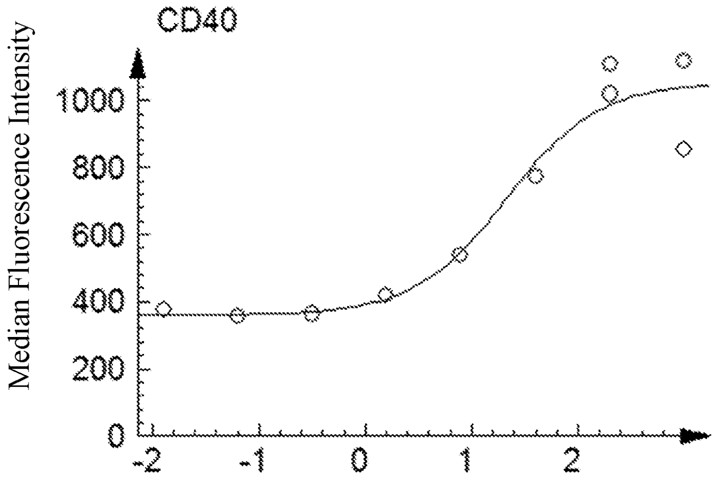


Max = 1053 Slope = 1
Min = 360 EC50 = 21.29
R2 = 0.9354

**FIG. 10B**

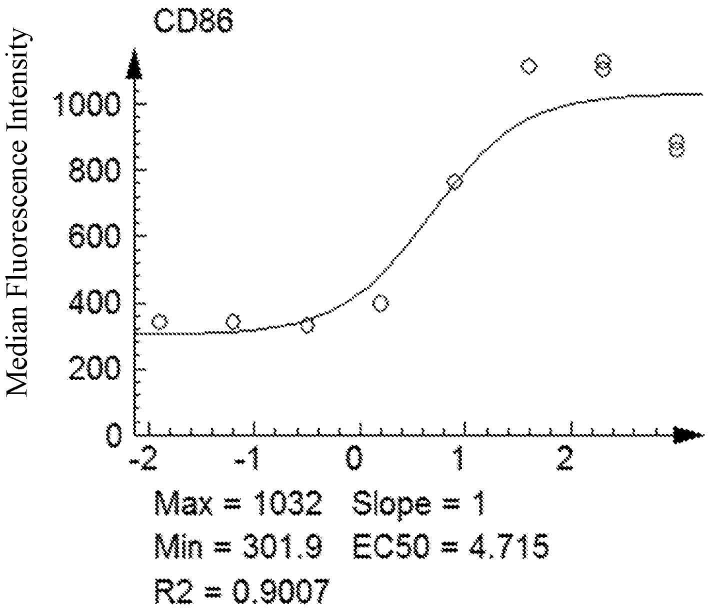


**FIG. 10C**

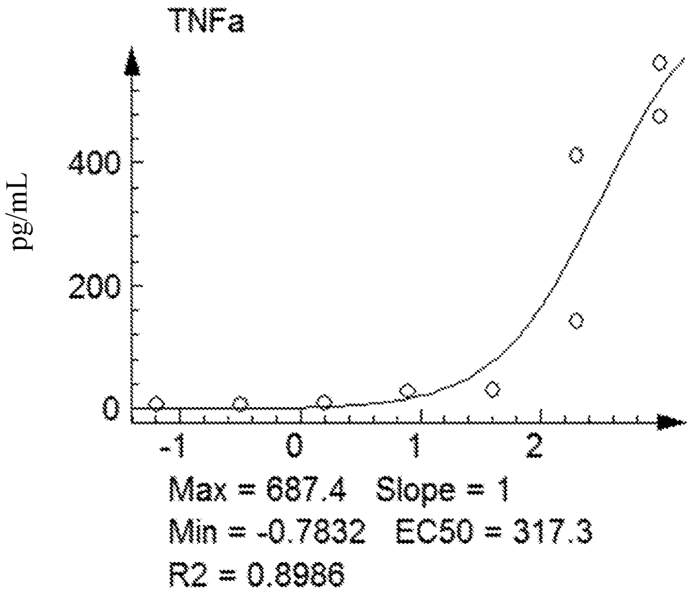


**FIG. 10D**

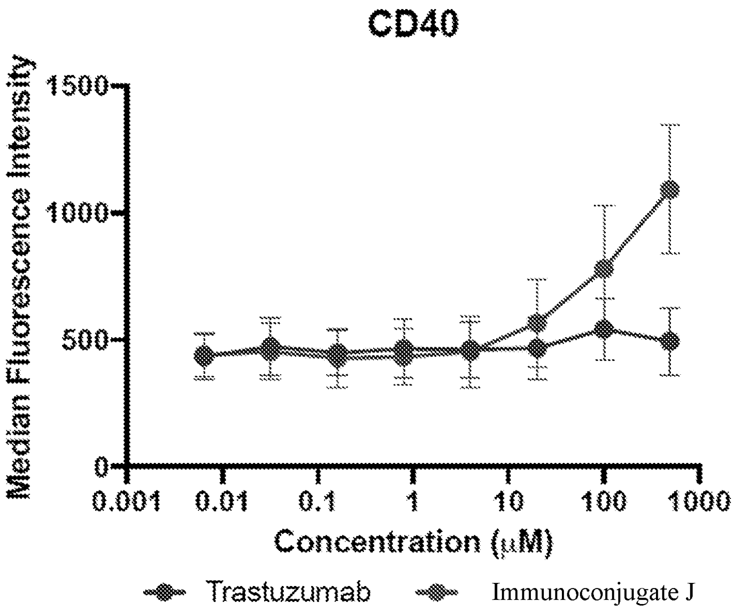


FIG. 11A

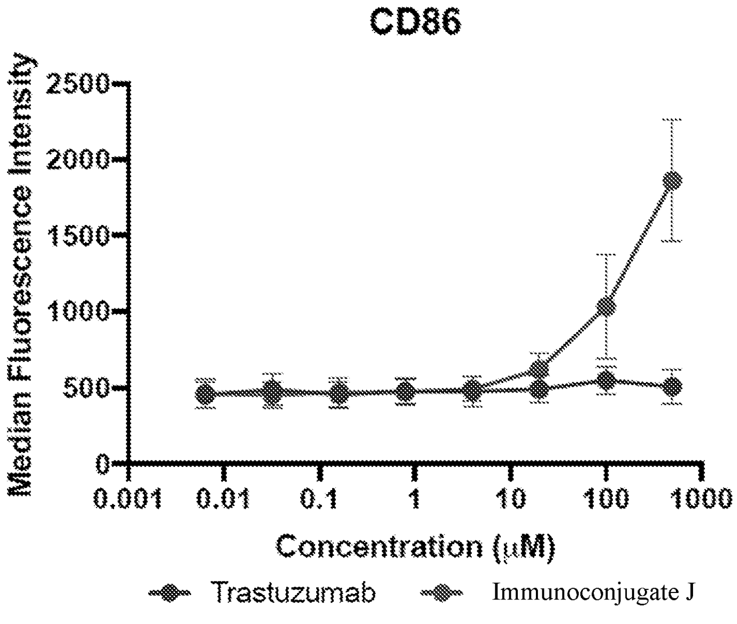


FIG. 11B

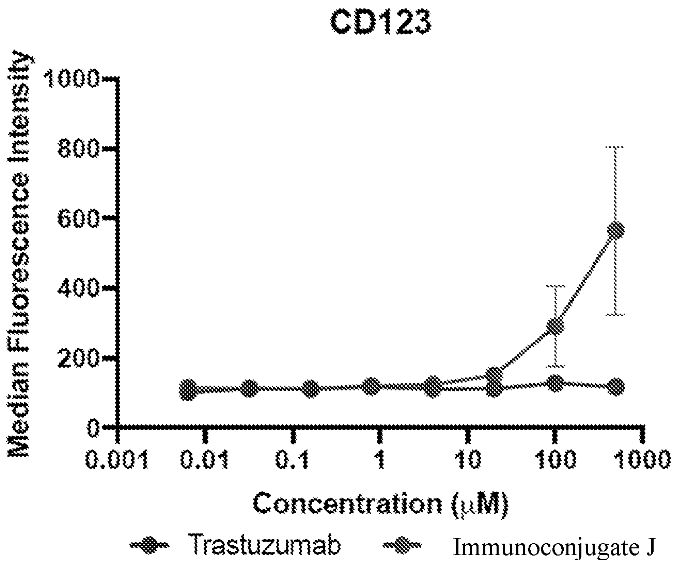
CD123
Median Fluorescence Intensity
1000
800
600
400
200
0
0.001
0.01
0.1
1
10
100
1000
Concentration (μM)
Trastuzumab
Immunoconjugate J

**FIG. 11C**

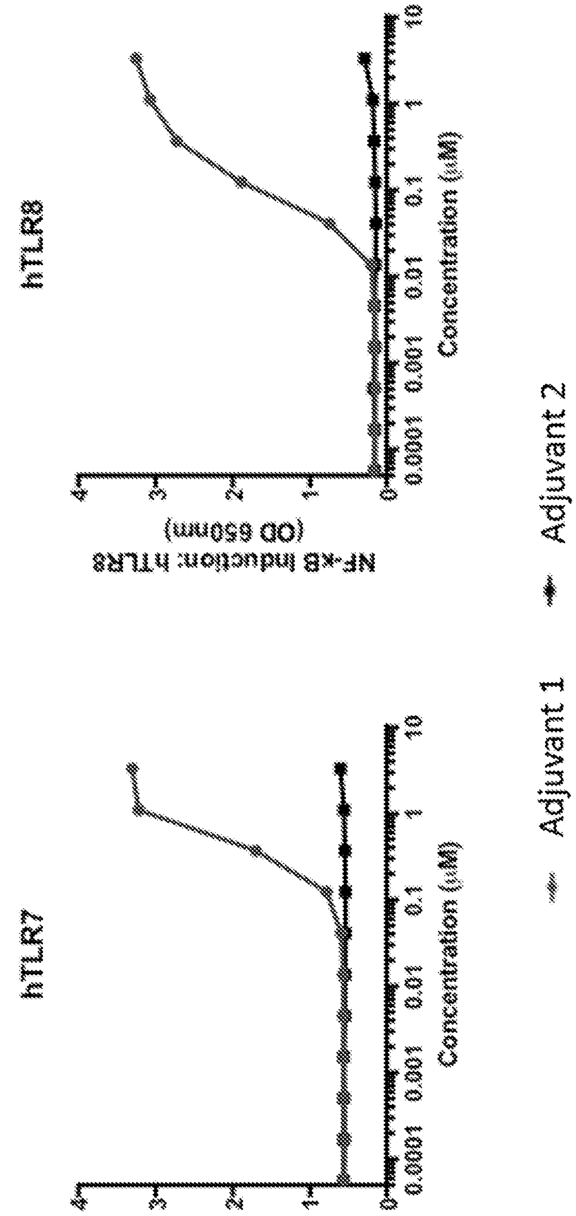
hTLR7
NF-κB Induction: hTLR7 (OD 650nm)
Concentration (μM)
0.0001
0.001
0.01
0.1
1
10
0
1
2
3
4
hTLR8
NF-κB Induction: hTLR8 (OD 650nm)
Concentration (μM)
Adjuvant 1
Adjuvant 2

FIG. 12

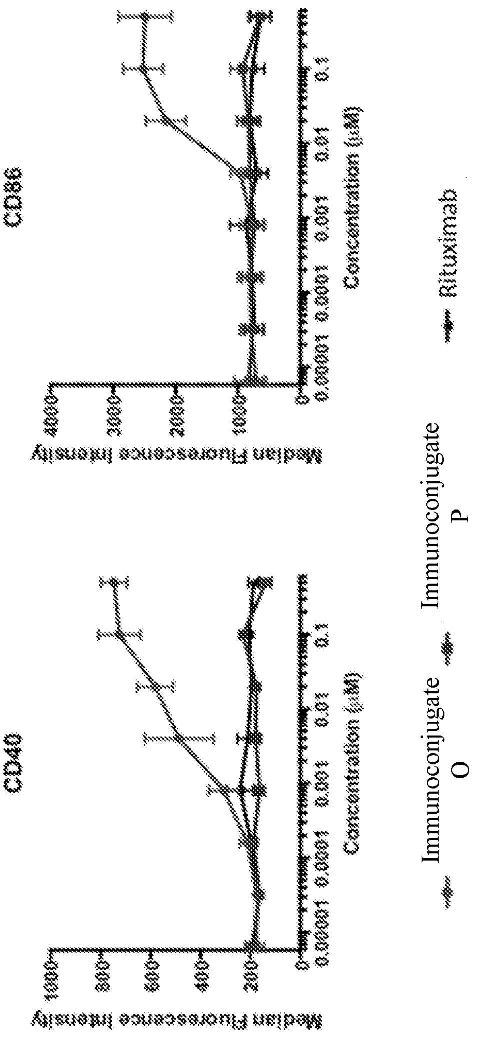
CD40
Median Fluorescence Intensity
Concentration (µM)
CD86
Median Fluorescence Intensity
Concentration (µM)
Immunoconjugate O
Immunoconjugate P
Rituximab

FIG. 13

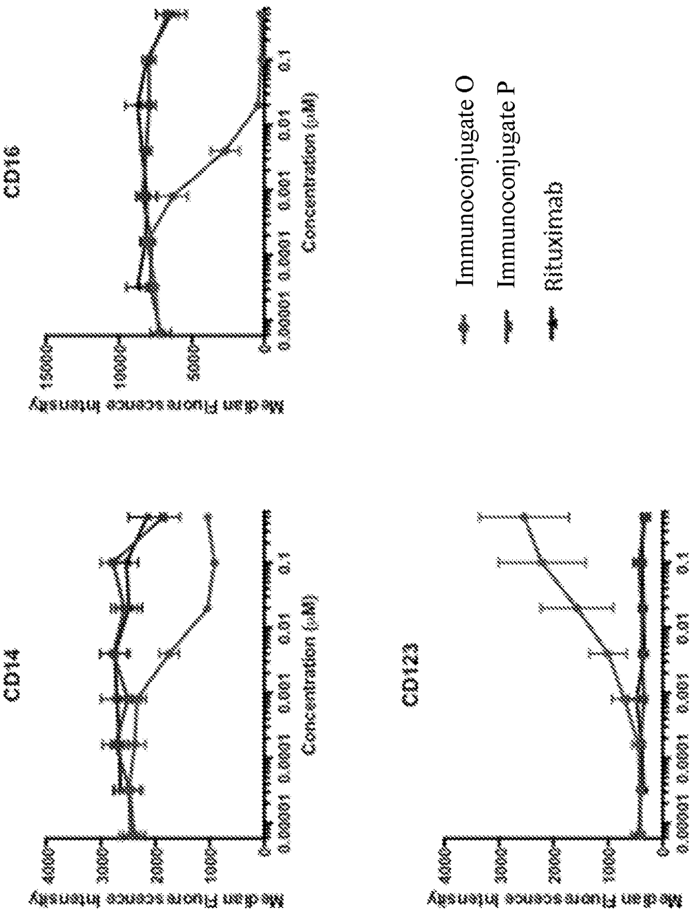
CD14
CD16
CD123
Median Fluorescence Intensity
Concentration (μM)
Immunoconjugate O
Immunoconjugate P
Rituximab

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018009916 A1
- WO 2007100634 A2
- US 20130165455, Carson
- US 20100150942
- US 20040101909
- US 20130177574
- US 20130108619
- US 20130011388
- US 20160145350
- US 7416726 B
- US 5624821 A
- US 20070014795
- US 20080286819
- US 9676863 B
- US 20170158772 A


### Non-patent literature cited in the description


- **CARMI et al.** *Nature*, 2015, vol. 521, 99-104
- Fundamental Immunology. 2012
- **MCCAFFERTY et al.** *Nature*, 1990, vol. 348, 552-554
- **LIEBERMAN**. *Pharmaceutical Dosage Forms*, 1992, vol. 1-3
- **LLOYD**. *The Art, Science and Technology of Pharmaceutical Compounding*, 1999
- **PICKAR**. Dosage Calculations. 1999
- Goodman & Gilman's The Pharmacological Basis of Therapeutics. McGraw-Hill, 2006
- Remington: The Science and Practice of Pharmacy. Lippincott, Williams & Wilkins, 2005
- **VACCHELLI et al.** *OncoImmunology*, 2013, vol. 2 (8), e25238
- **JEFFERIS et al.** *mAbs*, 2009, vol. 1 (4), 332-338
- **HERMANSON**. Bioconjugate Techniques. Academic Press, 2008